# EUROPEAN PATENT APPLICATION

(11) **EP 1 403 367 A1**
(43) Date of publication of application: **31.03.2004**
(21) Application number: 02733390.5
(22) Date of filing: 07.06.2002
(51) Int. Cl.: C12N 15/09, C12N 15/12, C07K 14/47, C07K 16/08, C12P 21/08, C12Q 1/02, C12Q 1/68, C12N 1/15, C12N 1/19, C12N 1/21, C12N 5/10, C12P 21/02, G01N 33/15, G01N 33/53

(54) **METHOD OF TESTING DRUG FOR TREATING OR PREVENTING DISEASES SUCH AS HYPERLIPEMIA**

(30) Priority: 08.06.2001 JP 2001173758; 13.06.2001 JP 2001178548; 13.07.2001 JP 2001213334; 28.09.2001 JP 2001300715; 28.09.2001 JP 2001300716; 22.11.2001 JP 2001357037; 18.12.2001 JP 2001384103; 05.04.2002 JP 2002103583
(71) Applicant: Sankyo Company Limited, Chuo-ku, Tokyo 103-8426 (JP)
(72) Inventor: KOISHI, Ryuta, c/o Sankyo Company, Limeted, Shinagawa-ku, Tokyo 140-8710 (JP); ANDO, Yosuke, c/o Sankyo Company, Limited, Fukuroi-shi, Shizuoka 437-0065 (JP); ONO, Mitsuru, c/o Sankyo Company, Limited, Shinagawa-ku, Tokyo 140-8710 (JP); YASUMO, Hiroaki, c/o Sankyo Company, Limited, Shinagawa-ku, Tokyo 140-8710 (JP); SHIMIZUGAWA, Tetsuya, c/o Sankyo Company, Limited, Shinagawa-ku, Tokyo 140-8710 (JP); YOSHIDA, Kenichi, c/o Sankyo Company, Limited, Shinagawa-ku, Tokyo 140-8710 (JP); SHIMAMURA, Mitsuru, c/o Sankyo Company, Limited, Shinagawa-ku, Tokyo 140-8710 (JP); FURUKAWA, Hidehiko, c/o Sankyo Company, Limited, Shinagawa-ku, Tokyo 140-8710 (JP)
(74) Representative: Gibson, Christian John Robert
(86) International application number: PCT/JP2002/005657
(87) International publication number: WO 2002/101039

(57) **Abstract**

It is intended to provide a novel method of testing a therapeutic or preventive agent for at least one disease selected from among hyperlipemia, arteriosclerosis and hyperglycemia. More specifically, a method is provided which comprises culturing cells in the presence or absence of a test substance, detecting mRNA expression level, promoter activity or protein production level of an "angiopoetin-related protein 3 or 4" gene, and thus selecting a test substance that decreases mRNA expression levels, promoter activity or protein production levels thus detected in cells cultured in the presence thereof compared with cells cultured in the absence thereof.

SEQUENCE LISTING

<110> Sankyo Company, Limited

<120> Methods of testing therapeutic or preventive agents for hyperlipidemia.

<130> FP0225

<140>
<141>

<150>JP2001/173758
<151>2001-6-8

<150>JP2001/178548
<151>2001-6-13

<150>JP2001/213334
<151>2001-7-13

<150>JP2001/300715
<151>2001-9-28

<150>JP2001/300716
<151>2001-9-28

<150>JP2001/357037
<151>2001-11-22

<150>JP2001/384103
<151>2001-12-18

<150>JP2002/103583
<151>2001-4-5

<160> 66

<170> PatentIn Ver. 2.0

<210> 1
<211> 1604
<212> DNA
<213> Mus musculus

<220>
<221> CDS
<222> (47)..(1411)

<400> 1
ggcacgaggt tccaaattgc ttaaaattga ataattgaga caaaaa atg cac aca 55
Met His Thr
1

att aaa tta ttc ctt ttt gtt gtt cct tta gta att gca tcc aga gtg 103
Ile Lys Leu Phe Leu Phe Val Val Pro Leu Val Ile Ala Ser Arg Val
    5                   10                  15

gat cca gac ctt tca tca ttt gat tct gca cct tca gag cca aaa tca 151
Asp Pro Asp Leu Ser Ser Phe Asp Ser Ala Pro Ser Glu Pro Lys Ser
20                  25                  30                  35

aga ttt gct atg ttg gat gat gtc aaa att tta gcg aat ggc ctc ctg 199
Arg Phe Ala Met Leu Asp Asp Val Lys Ile Leu Ala Asn Gly Leu Leu
                40                  45                  50

cag ctg ggt cat gga ctt aaa gat ttt gtc cat aag act aag gga caa 247
Gln Leu Gly His Gly Leu Lys Asp Phe Val His Lys Thr Lys Gly Gln
            55                  60                  65

att aac gac ata ttt cag aag ctc aac ata ttt gat cag tct ttt tat 295
Ile Asn Asp Ile Phe Gln Lys Leu Asn Ile Phe Asp Gln Ser Phe Tyr
        70                  75                  80

gac cta tca ctt cga acc aat gaa atc aaa gaa gag gaa aag gag cta    343
Asp Leu Ser Leu Arg Thr Asn Glu Ile Lys Glu Glu Glu Lys Glu Leu
    85                  90                  95

aga aga act aca tct aca cta caa gtt aaa aac gag gag gtg aag aac    391
Arg Arg Thr Thr Ser Thr Leu Gln Val Lys Asn Glu Glu Val Lys Asn
100                 105                 110                 115

atg tca gta gaa ctg aac tca aag ctt gag agt ctg ctg gaa gag aag    439
Met Ser Val Glu Leu Asn Ser Lys Leu Glu Ser Leu Leu Glu Glu Lys
                120                 125                 130

aca gcc ctt caa cac aag gtc agg gct ttg gag gag cag cta acc aac    487
Thr Ala Leu Gln His Lys Val Arg Ala Leu Glu Glu Gln Leu Thr Asn
            135                 140                 145

tta att cta agc cca gct ggg gct cag gag cac cca gaa gta aca tca    535
Leu Ile Leu Ser Pro Ala Gly Ala Gln Glu His Pro Glu Val Thr Ser
        150                 155                 160

ctc aaa agt ttt gta gaa cag caa gac aac agc ata aga gaa ctc ctc    583
Leu Lys Ser Phe Val Glu Gln Gln Asp Asn Ser Ile Arg Glu Leu Leu
    165                 170                 175

cag agt gtg gaa gaa cag tat aaa caa tta agt caa cag cac atg cag    631
Gln Ser Val Glu Glu Gln Tyr Lys Gln Leu Ser Gln Gln His Met Gln
180                 185                 190                 195

ata aaa gaa ata gaa aag cag ctc aga aag act ggt att caa gaa ccc    679
Ile Lys Glu Ile Glu Lys Gln Leu Arg Lys Thr Gly Ile Gln Glu Pro
                200                 205                 210

tca gaa aat tct ctt tct tct aaa tca aga gca cca aga act act ccc    727
Ser Glu Asn Ser Leu Ser Ser Lys Ser Arg Ala Pro Arg Thr Thr Pro
            215                 220                 225

cct ctt caa ctg aac gaa aca gaa aat aca gaa caa gat gac ctt cct 775
Pro Leu Gln Leu Asn Glu Thr Glu Asn Thr Glu Gln Asp Asp Leu Pro
        230                 235                 240

gcc gac tgc tct gcc gtt tat aac aga ggc gaa cat aca agt ggc gtg 823
Ala Asp Cys Ser Ala Val Tyr Asn Arg Gly Glu His Thr Ser Gly Val
    245                 250                 255

tac act att aaa cca aga aac tcc caa ggg ttt aat gtc tac tgt gat 871
Tyr Thr Ile Lys Pro Arg Asn Ser Gln Gly Phe Asn Val Tyr Cys Asp
260                 265                 270                 275

acc caa tca ggc agt cca tgg aca tta att caa cac cgg aaa gat ggc 919
Thr Gln Ser Gly Ser Pro Trp Thr Leu Ile Gln His Arg Lys Asp Gly
                280                 285                 290

tca cag gac ttc aac gaa aca tgg gaa aac tac gaa aag ggc ttt ggg 967
Ser Gln Asp Phe Asn Glu Thr Trp Glu Asn Tyr Glu Lys Gly Phe Gly
            295                 300                 305

agg ctc gat gga gaa ttt tgg ttg ggc cta gag aag atc tat gct ata 1015
Arg Leu Asp Gly Glu Phe Trp Leu Gly Leu Glu Lys Ile Tyr Ala Ile
        310                 315                 320

gtc caa cag tct aac tac att tta cga ctc gag cta caa gac tgg aaa 1063
Val Gln Gln Ser Asn Tyr Ile Leu Arg Leu Glu Leu Gln Asp Trp Lys
    325                 330                 335

gac agc aag cac tac gtt gaa tac tcc ttt cac ctg ggc agt cac gaa 1111
Asp Ser Lys His Tyr Val Glu Tyr Ser Phe His Leu Gly Ser His Glu
340                 345                 350                 355

acc aac tac acg cta cat gtg gct gag att gct ggc aat atc cct ggg 1159
Thr Asn Tyr Thr Leu His Val Ala Glu Ile Ala Gly Asn Ile Pro Gly
                360                 365                 370

gcc ctc cca gag cac aca gac ctg atg ttt tct aca tgg aat cac aga 1207
Ala Leu Pro Glu His Thr Asp Leu Met Phe Ser Thr Trp Asn His Arg
            375                 380                 385

gca aag gga cag ctc tac tgt cca gaa agt tac tca ggt ggc tgg tgg 1255
Ala Lys Gly Gln Leu Tyr Cys Pro Glu Ser Tyr Ser Gly Gly Trp Trp
        390                 395                 400

tgg aat gac ata tgt gga gaa aac aac cta aat gga aaa tac aac aaa 1303
Trp Asn Asp Ile Cys Gly Glu Asn Asn Leu Asn Gly Lys Tyr Asn Lys
    405                 410                 415

ccc aga acc aaa tcc aga cca gag aga aga aga ggg atc tac tgg aga 1351
Pro Arg Thr Lys Ser Arg Pro Glu Arg Arg Arg Gly Ile Tyr Trp Arg
420                 425                 430                 435

cct cag agc aga aag ctc tat gct atc aaa tca tcc aaa atg atg ctc 1399
Pro Gln Ser Arg Lys Leu Tyr Ala Ile Lys Ser Ser Lys Met Met Leu
                440                 445                 450

cag ccc acc acc taagaagctt caactgaact gagacaaaat aaaagatcaa 1451
Gln Pro Thr Thr
            455

taaattaaat attaaagtcc tcccgatcac tgtagtaatc tggtattaaa attttaatgg 1511

aaagcttgag aattgaattt caattaggtt taaactcatt gttaagatca gatatcaccg 1571

aatcaacgta aacaaaattt atctttttca atc 1604

<210> 2
<211> 455
<212> PRT
<213> Mus musculus

<400> 2
Met His Thr Ile Lys Leu Phe Leu Phe Val Val Pro Leu Val Ile Ala
1               5                   10                  15

Ser Arg Val Asp Pro Asp Leu Ser Ser Phe Asp Ser Ala Pro Ser Glu
            20                  25                  30

Pro Lys Ser Arg Phe Ala Met Leu Asp Asp Val Lys Ile Leu Ala Asn
        35                  40                  45

Gly Leu Leu Gln Leu Gly His Gly Leu Lys Asp Phe Val His Lys Thr
    50                  55                  60

Lys Gly Gln Ile Asn Asp Ile Phe Gln Lys Leu Asn Ile Phe Asp Gln
65                  70                  75                  80

Ser Phe Tyr Asp Leu Ser Leu Arg Thr Asn Glu Ile Lys Glu Glu Glu
                85                  90                  95

Lys Glu Leu Arg Arg Thr Thr Ser Thr Leu Gln Val Lys Asn Glu Glu
            100                 105                 110

Val Lys Asn Met Ser Val Glu Leu Asn Ser Lys Leu Glu Ser Leu Leu
        115                 120                 125

Glu Glu Lys Thr Ala Leu Gln His Lys Val Arg Ala Leu Glu Glu Gln
    130                 135                 140

Leu Thr Asn Leu Ile Leu Ser Pro Ala Gly Ala Gln Glu His Pro Glu
145                 150                 155                 160

Val Thr Ser Leu Lys Ser Phe Val Glu Gln Gln Asp Asn Ser Ile Arg
                165                 170                 175

Glu Leu Leu Gln Ser Val Glu Glu Gln Tyr Lys Gln Leu Ser Gln Gln

            180                 185                 190

His Met Gln Ile Lys Glu Ile Glu Lys Gln Leu Arg Lys Thr Gly Ile
        195                 200                 205

Gln Glu Pro Ser Glu Asn Ser Leu Ser Ser Lys Ser Arg Ala Pro Arg
    210                 215                 220

Thr Thr Pro Pro Leu Gln Leu Asn Glu Thr Glu Asn Thr Glu Gln Asp
225                 230                 235                 240

Asp Leu Pro Ala Asp Cys Ser Ala Val Tyr Asn Arg Gly Glu His Thr
                245                 250                 255

Ser Gly Val Tyr Thr Ile Lys Pro Arg Asn Ser Gln Gly Phe Asn Val
            260                 265                 270

Tyr Cys Asp Thr Gln Ser Gly Ser Pro Trp Thr Leu Ile Gln His Arg
        275                 280                 285

Lys Asp Gly Ser Gln Asp Phe Asn Glu Thr Trp Glu Asn Tyr Glu Lys
    290                 295                 300

Gly Phe Gly Arg Leu Asp Gly Glu Phe Trp Leu Gly Leu Glu Lys Ile
305                 310                 315                 320

Tyr Ala Ile Val Gln Gln Ser Asn Tyr Ile Leu Arg Leu Glu Leu Gln
                325                 330                 335

Asp Trp Lys Asp Ser Lys His Tyr Val Glu Tyr Ser Phe His Leu Gly
            340                 345                 350

Ser His Glu Thr Asn Tyr Thr Leu His Val Ala Glu Ile Ala Gly Asn
        355                 360                 365

Ile Pro Gly Ala Leu Pro Glu His Thr Asp Leu Met Phe Ser Thr Trp

370                 375                 380

Asn His Arg Ala Lys Gly Gln Leu Tyr Cys Pro Glu Ser Tyr Ser Gly
385                 390                 395                 400

Gly Trp Trp Trp Asn Asp Ile Cys Gly Glu Asn Asn Leu Asn Gly Lys
                405                 410                 415

Tyr Asn Lys Pro Arg Thr Lys Ser Arg Pro Glu Arg Arg Arg Gly Ile
            420                 425                 430

Tyr Trp Arg Pro Gln Ser Arg Lys Leu Tyr Ala Ile Lys Ser Ser Lys
        435                 440                 445

Met Met Leu Gln Pro Thr Thr
    450                 455

<210> 3
<211> 1716
<212> DNA
<213> Homo sapiens

<220>
<221> CDS
<222> (78)..(1457)

<400> 3
gcggccgcgt cgacgtctag gtctgcttcc agaagaaaac agttccacgt tgcttgaaat 60

tgaaaatcaa gataaaa atg ttc aca att aag ctc ctt ctt ttt att gtt     110
                   Met Phe Thr Ile Lys Leu Leu Leu Phe Ile Val
                   1               5                   10

cct cta gtt att tcc tcc aga att gat caa gac aat tca tca ttt gat     158
Pro Leu Val Ile Ser Ser Arg Ile Asp Gln Asp Asn Ser Ser Phe Asp

            15                  20                  25

tct cta tct cca gag cca aaa tca aga ttt gct atg tta gac gat gta 206
Ser Leu Ser Pro Glu Pro Lys Ser Arg Phe Ala Met Leu Asp Asp Val
        30                  35                  40

aaa att tta gcc aat ggc ctc ctt cag ttg gga cat ggt ctt aaa gac 254
Lys Ile Leu Ala Asn Gly Leu Leu Gln Leu Gly His Gly Leu Lys Asp
    45                  50                  55

ttt gtc cat aag acg aag ggc caa att aat gac ata ttt caa aaa ctc 302
Phe Val His Lys Thr Lys Gly Gln Ile Asn Asp Ile Phe Gln Lys Leu
60                  65                  70                  75

aac ata ttt gat cag tct ttt tat gat cta tcg ctg caa acc agt gaa 350
Asn Ile Phe Asp Gln Ser Phe Tyr Asp Leu Ser Leu Gln Thr Ser Glu
                80                  85                  90

atc aaa gaa gaa gaa aag gaa ctg aga aga act aca tat aaa cta caa 398
Ile Lys Glu Glu Glu Lys Glu Leu Arg Arg Thr Thr Tyr Lys Leu Gln
            95                  100                 105

gtc aaa aat gaa gag gta aag aat atg tca ctt gaa ctc aac tca aaa 446
Val Lys Asn Glu Glu Val Lys Asn Met Ser Leu Glu Leu Asn Ser Lys
        110                 115                 120

ctt gaa agc ctc cta gaa gaa aaa att cta ctt caa caa aaa gtg aaa 494
Leu Glu Ser Leu Leu Glu Glu Lys Ile Leu Leu Gln Gln Lys Val Lys
    125                 130                 135

tat tta gaa gag caa cta act aac tta att caa aat caa cct gaa act 542
Tyr Leu Glu Glu Gln Leu Thr Asn Leu Ile Gln Asn Gln Pro Glu Thr
140                 145                 150                 155

cca gaa cac cca gaa gta act tca ctt aaa act ttt gta gaa aaa caa 590
Pro Glu His Pro Glu Val Thr Ser Leu Lys Thr Phe Val Glu Lys Gln

                160                 165                 170

gat aat agc atc aaa gac ctt ctc cag acc gtg gaa gac caa tat aaa 638
Asp Asn Ser Ile Lys Asp Leu Leu Gln Thr Val Glu Asp Gln Tyr Lys
            175                 180                 185

caa tta aac caa cag cat agt caa ata aaa gaa ata gaa aat cag ctc 686
Gln Leu Asn Gln Gln His Ser Gln Ile Lys Glu Ile Glu Asn Gln Leu
        190                 195                 200

aga agg act agt att caa gaa ccc aca gaa att tct cta tct tcc aag 734
Arg Arg Thr Ser Ile Gln Glu Pro Thr Glu Ile Ser Leu Ser Ser Lys
    205                 210                 215

cca aga gca cca aga act act ccc ttt ctt cag ttg aat gaa ata aga 782
Pro Arg Ala Pro Arg Thr Thr Pro Phe Leu Gln Leu Asn Glu Ile Arg
220                 225                 230                 235

aat gta aaa cat gat ggc att cct gct gaa tgt acc acc att tat aac 830
Asn Val Lys His Asp Gly Ile Pro Ala Glu Cys Thr Thr Ile Tyr Asn
                240                 245                 250

aga ggt gaa cat aca agt ggc atg tat gcc atc aga ccc agc aac tct 878
Arg Gly Glu His Thr Ser Gly Met Tyr Ala Ile Arg Pro Ser Asn Ser
            255                 260                 265

caa gtt ttt cat gtc tac tgt gat gtt ata tca ggt agt cca tgg aca 926
Gln Val Phe His Val Tyr Cys Asp Val Ile Ser Gly Ser Pro Trp Thr
        270                 275                 280

tta att caa cat cga ata gat gga tca caa aac ttc aat gaa acg tgg 974
Leu Ile Gln His Arg Ile Asp Gly Ser Gln Asn Phe Asn Glu Thr Trp
    285                 290                 295

gag aac tac aaa tat ggt ttt ggg agg ctt gat gga gaa ttt tgg ttg 1022
Glu Asn Tyr Lys Tyr Gly Phe Gly Arg Leu Asp Gly Glu Phe Trp Leu

300                 305                 310                 315

ggc cta gag aag ata tac tcc ata gtg aag caa tct aat tat gtt tta    1070
Gly Leu Glu Lys Ile Tyr Ser Ile Val Lys Gln Ser Asn Tyr Val Leu
                320                 325                 330

cga att gag ttg gaa gac tgg aaa gac aac aaa cat tat att gaa tat    1118
Arg Ile Glu Leu Glu Asp Trp Lys Asp Asn Lys His Tyr Ile Glu Tyr
            335                 340                 345

tct ttt tac ttg gga aat cac gaa acc aac tat acg cta cat cta gtt    1166
Ser Phe Tyr Leu Gly Asn His Glu Thr Asn Tyr Thr Leu His Leu Val
        350                 355                 360

gcg att act ggc aat gtc ccc aat gca atc ccg gaa aac aaa gat ttg    1214
Ala Ile Thr Gly Asn Val Pro Asn Ala Ile Pro Glu Asn Lys Asp Leu
    365                 370                 375

gtg ttt tct act tgg gat cac aaa gca aaa gga cac ttc aac tgt cca    1262
Val Phe Ser Thr Trp Asp His Lys Ala Lys Gly His Phe Asn Cys Pro
380                 385                 390                 395

gag ggt tat tca gga ggc tgg tgg tgg cat gat gag tgt gga gaa aac    1310
Glu Gly Tyr Ser Gly Gly Trp Trp Trp His Asp Glu Cys Gly Glu Asn
                400                 405                 410

aac cta aat ggt aaa tat aac aaa cca aga gca aaa tct aag cca gag    1358
Asn Leu Asn Gly Lys Tyr Asn Lys Pro Arg Ala Lys Ser Lys Pro Glu
            415                 420                 425

agg aga aga gga tta tct tgg aag tct caa aat gga agg tta tac tct    1406
Arg Arg Arg Gly Leu Ser Trp Lys Ser Gln Asn Gly Arg Leu Tyr Ser
        430                 435                 440

ata aaa tca acc aaa atg ttg atc cat cca aca gat tca gaa agc ttt    1454
Ile Lys Ser Thr Lys Met Leu Ile His Pro Thr Asp Ser Glu Ser Phe

445                    450                    455

gaa tgaactgagg caaatttaaa aggcaataat ttaaacatta acctcattcc        1507
Glu
460

aagttaatgt ggtctaataa tctggtatta aatccttaag agaaagcttg agaaatagat 1567

tttttttatc ttaaagtcac tgtctattta agattaaaca tacaatcaca taaccttaaa 1627

gaataccgtt tacatttctc aatcaaaatt cttataatac tatttgtttt aaattttgtg 1687

atgtgggaat caattttaga tggtcacaa                                   1716

<210> 4
<211> 460
<212> PRT
<213> Homo sapiens

<400> 4
Met Phe Thr Ile Lys Leu Leu Leu Phe Ile Val Pro Leu Val Ile Ser
1               5                   10                  15

Ser Arg Ile Asp Gln Asp Asn Ser Ser Phe Asp Ser Leu Ser Pro Glu
            20                  25                  30

Pro Lys Ser Arg Phe Ala Met Leu Asp Asp Val Lys Ile Leu Ala Asn
        35                  40                  45

Gly Leu Leu Gln Leu Gly His Gly Leu Lys Asp Phe Val His Lys Thr
    50                  55                  60

Lys Gly Gln Ile Asn Asp Ile Phe Gln Lys Leu Asn Ile Phe Asp Gln
65                  70                  75                  80

Ser Phe Tyr Asp Leu Ser Leu Gln Thr Ser Glu Ile Lys Glu Glu Glu
                85                  90                  95

Lys Glu Leu Arg Arg Thr Thr Tyr Lys Leu Gln Val Lys Asn Glu Glu
            100                 105                 110

Val Lys Asn Met Ser Leu Glu Leu Asn Ser Lys Leu Glu Ser Leu Leu
        115                 120                 125

Glu Glu Lys Ile Leu Leu Gln Gln Lys Val Lys Tyr Leu Glu Glu Gln
    130                 135                 140

Leu Thr Asn Leu Ile Gln Asn Gln Pro Glu Thr Pro Glu His Pro Glu
145                 150                 155                 160

Val Thr Ser Leu Lys Thr Phe Val Glu Lys Gln Asp Asn Ser Ile Lys
                165                 170                 175

Asp Leu Leu Gln Thr Val Glu Asp Gln Tyr Lys Gln Leu Asn Gln Gln
            180                 185                 190

His Ser Gln Ile Lys Glu Ile Glu Asn Gln Leu Arg Arg Thr Ser Ile
        195                 200                 205

Gln Glu Pro Thr Glu Ile Ser Leu Ser Ser Lys Pro Arg Ala Pro Arg
    210                 215                 220

Thr Thr Pro Phe Leu Gln Leu Asn Glu Ile Arg Asn Val Lys His Asp
225                 230                 235                 240

Gly Ile Pro Ala Glu Cys Thr Thr Ile Tyr Asn Arg Gly Glu His Thr
                245                 250                 255

Ser Gly Met Tyr Ala Ile Arg Pro Ser Asn Ser Gln Val Phe His Val
            260                 265                 270

Tyr Cys Asp Val Ile Ser Gly Ser Pro Trp Thr Leu Ile Gln His Arg
        275                 280                 285

Ile Asp Gly Ser Gln Asn Phe Asn Glu Thr Trp Glu Asn Tyr Lys Tyr
    290                 295                 300

Gly Phe Gly Arg Leu Asp Gly Glu Phe Trp Leu Gly Leu Glu Lys Ile
305                 310                 315                 320

Tyr Ser Ile Val Lys Gln Ser Asn Tyr Val Leu Arg Ile Glu Leu Glu
                325                 330                 335

Asp Trp Lys Asp Asn Lys His Tyr Ile Glu Tyr Ser Phe Tyr Leu Gly
            340                 345                 350

Asn His Glu Thr Asn Tyr Thr Leu His Leu Val Ala Ile Thr Gly Asn
        355                 360                 365

Val Pro Asn Ala Ile Pro Glu Asn Lys Asp Leu Val Phe Ser Thr Trp
    370                 375                 380

Asp His Lys Ala Lys Gly His Phe Asn Cys Pro Glu Gly Tyr Ser Gly
385                 390                 395                 400

Gly Trp Trp Trp His Asp Glu Cys Gly Glu Asn Asn Leu Asn Gly Lys
                405                 410                 415

Tyr Asn Lys Pro Arg Ala Lys Ser Lys Pro Glu Arg Arg Arg Gly Leu
            420                 425                 430

Ser Trp Lys Ser Gln Asn Gly Arg Leu Tyr Ser Ile Lys Ser Thr Lys
        435                 440                 445

Met Leu Ile His Pro Thr Asp Ser Glu Ser Phe Glu
    450                 455                 460

<210> 5
<211> 23
<212> DNA
<213> Mus musculus

<400> 5
gactgatcaa atatgttgag ctt                                                23

<210> 6
<211> 21
<212> DNA
<213> Mus musculus

<400> 6
tgcatccaga gtggatccag a                                                  21

<210> 7
<211> 21
<212> DNA
<213> Homo sapiens

<400> 7
tcctctagtt atttcctcca g                                                  21

<210> 8
<211> 20
<212> DNA
<213> Homo sapiens

<400> 8
tggtttgcca gcgatagatc                                                    20

<210> 9
<211> 14
<212> PRT
<213> Artificial Sequence

<220>
<223> Description of Artificial Sequence: Synthetic
      oligopeptide which is used as an antigen to obtain
      polyclonal antibody

<400> 9
Glu Pro Lys Ser Arg Phe Ala Met Leu Asp Asp Val Lys Cys
  1               5                   10

<210> 10
<211> 14
<212> PRT
<213> Mus musculus

<400> 10
Cys Gly Glu Asn Asn Leu Asn Gly Lys Tyr Asn Lys Pro Arg
  1               5                   10

<210> 11
<211> 1417
<212> DNA
<213> Artificial Sequence

<220>
<223> Description of Artificial Sequence: DNA encoding
      N-terminal domain of human angiopoietin related
      protein 3

<220>
<221> CDS
<222> (18)..(638)

<400> 11
gaattcggct tgccacc atg gtc aca att aag ctc ctt ctt ttt att gtt     50
                   Met Val Thr Ile Lys Leu Leu Leu Phe Ile Val
                   1               5                   10

cct cta gtt att tcc tcc aga att gat caa gac aat tca tca ttt gat     98
Pro Leu Val Ile Ser Ser Arg Ile Asp Gln Asp Asn Ser Ser Phe Asp
            15                  20                  25

tct cta tct cca gag cca aaa tca aga ttt gct atg tta gac gat gta     146
Ser Leu Ser Pro Glu Pro Lys Ser Arg Phe Ala Met Leu Asp Asp Val
        30                  35                  40

aaa att tta gcc aat ggc ctc ctt cag ttg gga cat ggt ctt aaa gac     194
Lys Ile Leu Ala Asn Gly Leu Leu Gln Leu Gly His Gly Leu Lys Asp
    45                  50                  55

ttt gtc cat aag acg aag ggc caa att aat gac ata ttt caa aaa ctc     242
Phe Val His Lys Thr Lys Gly Gln Ile Asn Asp Ile Phe Gln Lys Leu
60                  65                  70                  75

aac ata ttt gat cag tct ttt tat gat cta tcg ctg caa acc agt gaa     290
Asn Ile Phe Asp Gln Ser Phe Tyr Asp Leu Ser Leu Gln Thr Ser Glu
                80                  85                  90

atc aaa gaa gaa gaa aag gaa ctg aga aga act aca tat aaa cta caa     338
Ile Lys Glu Glu Glu Lys Glu Leu Arg Arg Thr Thr Tyr Lys Leu Gln
            95                  100                 105

gtc aaa aat gaa gag gta aag aat atg tca ctt gaa ctc aac tca aaa     386
Val Lys Asn Glu Glu Val Lys Asn Met Ser Leu Glu Leu Asn Ser Lys
        110                 115                 120

ctt gaa agc ctc cta gaa gaa aaa att cta ctt caa caa aaa gtg aaa 434
Leu Glu Ser Leu Leu Glu Glu Lys Ile Leu Leu Gln Gln Lys Val Lys
    125                 130                 135

tat tta gaa gag caa cta act aac tta att caa aat caa cct gaa act 482
Tyr Leu Glu Glu Gln Leu Thr Asn Leu Ile Gln Asn Gln Pro Glu Thr
140                 145                 150                 155

cca gaa cac cca gaa gta act tca ctt aaa act ttt gta gaa aaa caa 530
Pro Glu His Pro Glu Val Thr Ser Leu Lys Thr Phe Val Glu Lys Gln
                160                 165                 170

gat aat agc atc aaa gac ctt ctc cag acc gtg gaa gac caa tat aaa 578
Asp Asn Ser Ile Lys Asp Leu Leu Gln Thr Val Glu Asp Gln Tyr Lys
            175                 180                 185

caa tta aac caa cag cat agt caa ata aaa gaa ata gaa aat cag ctc 626
Gln Leu Asn Gln Gln His Ser Gln Ile Lys Glu Ile Glu Asn Gln Leu
        190                 195                 200

aga agg act agc tagtattcaa gaacccacag aaatttctct atcttccaag 678
Arg Arg Thr Ser
    205

ccaagagcac caagaactac tccctttctt cagttgaatg aaataagaaa tgtaaaacat 738

gatggcattc ctgctgaatg taccaccatt tataacagag gtgaacatac aagtggcatg 798

tatgccatca gacccagcaa ctctcaagtt tttcatgtct actgtgatgt tatatcaggt 858

agtccatgga cattaattca acatcgaata gatggatcac aaaacttcaa tgaaacgtgg 918

gagaactaca aatatggttt tgggaggctt gatggagaat tttggttggg cctagagaag 978

atatactcca tagtgaagca atctaattat gttttacgaa ttgagttgga agactggaaa 1038

gacaacaaac attatattga atattctttt tacttgggaa atcacgaaac caactatacg 1098

ctacatctag ttgcgattac tggcaatgtc cccaatgcaa tcccggaaaa caaagatttg 1158

gtgttttcta cttgggatca caaagcaaaa ggacacttca actgtccaga gggttattca 1218

ggaggctggt ggtggcatga tgagtgtgga gaaaacaacc taaatggtaa atataacaaa 1278

ccaagagcaa aatctaagcc agagaggaga agaggattat cttggaagtc tcaaaatgga 1338

aggttatact ctataaaatc aaccaaaatg ttgatccatc caacagattc agaaagcttt 1398

gaatgaacga ggctctaga 1417

<210> 12
<211> 207
<212> PRT
<213> Artificial Sequence
<223> Description of Artificial Sequence: Polypeptide encoding
      N-terminal domain of human angiopoietin related
      protein 3

<400> 12
Met Val Thr Ile Lys Leu Leu Leu Phe Ile Val Pro Leu Val Ile Ser
 1               5                  10                  15

Ser Arg Ile Asp Gln Asp Asn Ser Ser Phe Asp Ser Leu Ser Pro Glu
            20                  25                  30

Pro Lys Ser Arg Phe Ala Met Leu Asp Asp Val Lys Ile Leu Ala Asn
        35                  40                  45

Gly Leu Leu Gln Leu Gly His Gly Leu Lys Asp Phe Val His Lys Thr
    50                  55                  60

Lys Gly Gln Ile Asn Asp Ile Phe Gln Lys Leu Asn Ile Phe Asp Gln
65                  70                  75                  80

Ser Phe Tyr Asp Leu Ser Leu Gln Thr Ser Glu Ile Lys Glu Glu Glu
                85                  90                  95

Lys Glu Leu Arg Arg Thr Thr Tyr Lys Leu Gln Val Lys Asn Glu Glu
            100                 105                 110

Val Lys Asn Met Ser Leu Glu Leu Asn Ser Lys Leu Glu Ser Leu Leu
        115                 120                 125

Glu Glu Lys Ile Leu Leu Gln Gln Lys Val Lys Tyr Leu Glu Glu Gln
    130                 135                 140

Leu Thr Asn Leu Ile Gln Asn Gln Pro Glu Thr Pro Glu His Pro Glu
145                 150                 155                 160

Val Thr Ser Leu Lys Thr Phe Val Glu Lys Gln Asp Asn Ser Ile Lys
                165                 170                 175

Asp Leu Leu Gln Thr Val Glu Asp Gln Tyr Lys Gln Leu Asn Gln Gln
            180                 185                 190

His Ser Gln Ile Lys Glu Ile Glu Asn Gln Leu Arg Arg Thr Ser
        195                 200                 205

<210> 13
<211> 858
<212> DNA
<213> Artificial Sequence

<220>
<223> Description of Artificial Sequence: DNA encoding

C-terminal domain of human angiopoietin related
protein 3

<220>
<221> CDS
<222> (18)..(842)

<400> 13
gaattcggct tgccacc atg gtc aca att aag ctc ctt ctt ttt att gtt 50
Met Val Thr Ile Lys Leu Leu Leu Phe Ile Val
1               5                   10

cct cta gtt att tcc tcc aga att gat cct agt att caa gaa ccc aca 98
Pro Leu Val Ile Ser Ser Arg Ile Asp Pro Ser Ile Gln Glu Pro Thr
            15                  20                  25

gaa att tct cta tct tcc aag cca aga gca cca aga act act ccc ttt 146
Glu Ile Ser Leu Ser Ser Lys Pro Arg Ala Pro Arg Thr Thr Pro Phe
        30                  35                  40

ctt cag ttg aat gaa ata aga aat gta aaa cat gat ggc att cct gct 194
Leu Gln Leu Asn Glu Ile Arg Asn Val Lys His Asp Gly Ile Pro Ala
    45                  50                  55

gaa tgt acc acc att tat aac aga ggt gaa cat aca agt ggc atg tat 242
Glu Cys Thr Thr Ile Tyr Asn Arg Gly Glu His Thr Ser Gly Met Tyr
60                  65                  70                  75

gcc atc aga ccc agc aac tct caa gtt ttt cat gtc tac tgt gat gtt 290
Ala Ile Arg Pro Ser Asn Ser Gln Val Phe His Val Tyr Cys Asp Val
                80                  85                  90

ata tca ggt agt cca tgg aca tta att caa cat cga ata gat gga tca 338
Ile Ser Gly Ser Pro Trp Thr Leu Ile Gln His Arg Ile Asp Gly Ser
            95                  100                 105

caa aac ttc aat gaa acg tgg gag aac tac aaa tat ggt ttt ggg agg 386
Gln Asn Phe Asn Glu Thr Trp Glu Asn Tyr Lys Tyr Gly Phe Gly Arg
        110                 115                 120

ctt gat gga gaa ttt tgg ttg ggc cta gag aag ata tac tcc ata gtg 434
Leu Asp Gly Glu Phe Trp Leu Gly Leu Glu Lys Ile Tyr Ser Ile Val
    125                 130                 135

aag caa tct aat tat gtt tta cga att gag ttg gaa gac tgg aaa gac 482
Lys Gln Ser Asn Tyr Val Leu Arg Ile Glu Leu Glu Asp Trp Lys Asp
140                 145                 150                 155

aac aaa cat tat att gaa tat tct ttt tac ttg gga aat cac gaa acc 530
Asn Lys His Tyr Ile Glu Tyr Ser Phe Tyr Leu Gly Asn His Glu Thr
                160                 165                 170

aac tat acg cta cat cta gtt gcg att act ggc aat gtc ccc aat gca 578
Asn Tyr Thr Leu His Leu Val Ala Ile Thr Gly Asn Val Pro Asn Ala
            175                 180                 185

atc ccg gaa aac aaa gat ttg gtg ttt tct act tgg gat cac aaa gca 626
Ile Pro Glu Asn Lys Asp Leu Val Phe Ser Thr Trp Asp His Lys Ala
        190                 195                 200

aaa gga cac ttc aac tgt cca gag ggt tat tca gga ggc tgg tgg tgg 674
Lys Gly His Phe Asn Cys Pro Glu Gly Tyr Ser Gly Gly Trp Trp Trp
    205                 210                 215

cat gat gag tgt gga gaa aac aac cta aat ggt aaa tat aac aaa cca 722
His Asp Glu Cys Gly Glu Asn Asn Leu Asn Gly Lys Tyr Asn Lys Pro
220                 225                 230                 235

aga gca aaa tct aag cca gag agg aga aga gga tta tct tgg aag tct 770
Arg Ala Lys Ser Lys Pro Glu Arg Arg Arg Gly Leu Ser Trp Lys Ser
                240                 245                 250

caa aat gga agg tta tac tct ata aaa tca acc aaa atg ttg atc cat    818
Gln Asn Gly Arg Leu Tyr Ser Ile Lys Ser Thr Lys Met Leu Ile His
        255                 260                 265

cca aca gat tca gaa agc ttt gaa tgaacgaggc tctaga                  858
Pro Thr Asp Ser Glu Ser Phe Glu
    270                 275

<210> 14
<211> 275
<212> PRT
<213> Artificial Sequence
<223> Description of Artificial Sequence: Polypeptide encoding
      C-terminal domain of human angiopoietin related
      protein 3

<400> 14
Met Val Thr Ile Lys Leu Leu Leu Phe Ile Val Pro Leu Val Ile Ser
  1               5                  10                  15

Ser Arg Ile Asp Pro Ser Ile Gln Glu Pro Thr Glu Ile Ser Leu Ser
             20                  25                  30

Ser Lys Pro Arg Ala Pro Arg Thr Thr Pro Phe Leu Gln Leu Asn Glu
         35                  40                  45

Ile Arg Asn Val Lys His Asp Gly Ile Pro Ala Glu Cys Thr Thr Ile
     50                  55                  60

Tyr Asn Arg Gly Glu His Thr Ser Gly Met Tyr Ala Ile Arg Pro Ser
 65                  70                  75                  80

Asn Ser Gln Val Phe His Val Tyr Cys Asp Val Ile Ser Gly Ser Pro
                 85                  90                  95

Trp Thr Leu Ile Gln His Arg Ile Asp Gly Ser Gln Asn Phe Asn Glu
            100                 105                 110

Thr Trp Glu Asn Tyr Lys Tyr Gly Phe Gly Arg Leu Asp Gly Glu Phe
        115                 120                 125

Trp Leu Gly Leu Glu Lys Ile Tyr Ser Ile Val Lys Gln Ser Asn Tyr
    130                 135                 140

Val Leu Arg Ile Glu Leu Glu Asp Trp Lys Asp Asn Lys His Tyr Ile
145                 150                 155                 160

Glu Tyr Ser Phe Tyr Leu Gly Asn His Glu Thr Asn Tyr Thr Leu His
                165                 170                 175

Leu Val Ala Ile Thr Gly Asn Val Pro Asn Ala Ile Pro Glu Asn Lys
            180                 185                 190

Asp Leu Val Phe Ser Thr Trp Asp His Lys Ala Lys Gly His Phe Asn
        195                 200                 205

Cys Pro Glu Gly Tyr Ser Gly Gly Trp Trp Trp His Asp Glu Cys Gly
    210                 215                 220

Glu Asn Asn Leu Asn Gly Lys Tyr Asn Lys Pro Arg Ala Lys Ser Lys
225                 230                 235                 240

Pro Glu Arg Arg Arg Gly Leu Ser Trp Lys Ser Gln Asn Gly Arg Leu
                245                 250                 255

Tyr Ser Ile Lys Ser Thr Lys Met Leu Ile His Pro Thr Asp Ser Glu
            260                 265                 270

Ser Phe Glu
        275

<210> 15
<211> 1943
<212> DNA
<213> Homo sapiens

<220>
<221> CDS
<222> (173)..(1393)

<400> 15
ggagaagaag ccgagctgag cggatcctca cacgactgtg atccgattct ttccagcggc 60

ttctgcaacc aagcgggtct tacccccggt cctccgcgtc tccagtcctc gcacctggaa 120

ccccaacgtc cccgagagtc cccgaatccc cgctcccagg ctacctaaga gg atg agc 178
Met Ser
1

ggt gct ccg acg gcc ggg gca gcc ctg atg ctc tgc gcc gcc acc gcc 226
Gly Ala Pro Thr Ala Gly Ala Ala Leu Met Leu Cys Ala Ala Thr Ala
        5                   10                  15

gtg cta ctg agc gct cag ggc gga ccc gtg cag tcc aag tcg ccg cgc 274
Val Leu Leu Ser Ala Gln Gly Gly Pro Val Gln Ser Lys Ser Pro Arg
    20                  25                  30

ttt gcg tcc tgg gac gag atg aat gtc ctg gcg cac gga ctc ctg cag 322
Phe Ala Ser Trp Asp Glu Met Asn Val Leu Ala His Gly Leu Leu Gln
35                  40                  45                  50

ctc ggc cag ggg ctg cgc gaa cac gcg gag cgc acc cgc agt cag ctg 370
Leu Gly Gln Gly Leu Arg Glu His Ala Glu Arg Thr Arg Ser Gln Leu
                55                  60                  65

agc gcg ctg gag cgg cgc ctg agc gcg tgc ggg tcc gcc tgt cag gga 418

Ser Ala Leu Glu Arg Arg Leu Ser Ala Cys Gly Ser Ala Cys Gln Gly
            70                  75                  80

acc gag ggg tcc acc gac ctc ccg tta gcc cct gag agc cgg gtg gac 466
Thr Glu Gly Ser Thr Asp Leu Pro Leu Ala Pro Glu Ser Arg Val Asp
        85                  90                  95

cct gag gtc ctt cac agc ctg cag aca caa ctc aag gct cag aac agc 514
Pro Glu Val Leu His Ser Leu Gln Thr Gln Leu Lys Ala Gln Asn Ser
    100                 105                 110

agg atc cag caa ctc ttc cac aag gtg gcc cag cag cag cgg cac ctg 562
Arg Ile Gln Gln Leu Phe His Lys Val Ala Gln Gln Gln Arg His Leu
115                 120                 125                 130

gag aag cag cac ctg cga att cag cat ctg caa agc cag ttt ggc ctc 610
Glu Lys Gln His Leu Arg Ile Gln His Leu Gln Ser Gln Phe Gly Leu
                135                 140                 145

ctg gac cac aag cac cta gac cat gag gtg gcc aag cct gcc cga aga 658
Leu Asp His Lys His Leu Asp His Glu Val Ala Lys Pro Ala Arg Arg
            150                 155                 160

aag agg ctg ccc gag atg gcc cag cca gtt gac ccg gct cac aat gtc 706
Lys Arg Leu Pro Glu Met Ala Gln Pro Val Asp Pro Ala His Asn Val
        165                 170                 175

agc cgc ctg cac cgg ctg ccc agg gat tgc cag gag ctg ttc cag gtt 754
Ser Arg Leu His Arg Leu Pro Arg Asp Cys Gln Glu Leu Phe Gln Val
    180                 185                 190

ggg gag agg cag agt gga cta ttt gaa atc cag cct cag ggg tct ccg 802
Gly Glu Arg Gln Ser Gly Leu Phe Glu Ile Gln Pro Gln Gly Ser Pro
195                 200                 205                 210

cca ttt ttg gtg aac tgc aag atg acc tca gat gga ggc tgg aca gta 850

Pro Phe Leu Val Asn Cys Lys Met Thr Ser Asp Gly Gly Trp Thr Val
                215                 220                 225

att cag agg cgc cac gat ggc tca gtg gac ttc aac cgg ccc tgg gaa 898
Ile Gln Arg Arg His Asp Gly Ser Val Asp Phe Asn Arg Pro Trp Glu
            230                 235                 240

gcc tac aag gcg ggg ttt ggg gat ccc cac ggc gag ttc tgg ctg ggt 946
Ala Tyr Lys Ala Gly Phe Gly Asp Pro His Gly Glu Phe Trp Leu Gly
        245                 250                 255

ctg gag aag gtg cat agc atc acg ggg gac cgc aac agc cgc ctg gcc 994
Leu Glu Lys Val His Ser Ile Thr Gly Asp Arg Asn Ser Arg Leu Ala
    260                 265                 270

gtg cag ctg cgg gac tgg gat ggc aac gcc gag ttg ctg cag ttc tcc 1042
Val Gln Leu Arg Asp Trp Asp Gly Asn Ala Glu Leu Leu Gln Phe Ser
275                 280                 285                 290

gtg cac ctg ggt ggc gag gac acg gcc tat agc ctg cag ctc act gca 1090
Val His Leu Gly Gly Glu Asp Thr Ala Tyr Ser Leu Gln Leu Thr Ala
                295                 300                 305

ccc gtg gcc ggc cag ctg ggc gcc acc acc gtc cca ccc agc ggc ctc 1138
Pro Val Ala Gly Gln Leu Gly Ala Thr Thr Val Pro Pro Ser Gly Leu
            310                 315                 320

tcc gta ccc ttc tcc act tgg gac cag gat cac gac ctc cgc agg gac 1186
Ser Val Pro Phe Ser Thr Trp Asp Gln Asp His Asp Leu Arg Arg Asp
        325                 330                 335

aag aac tgc gcc aag agc ctc tct gga ggc tgg tgg ttt ggc acc tgc 1234
Lys Asn Cys Ala Lys Ser Leu Ser Gly Gly Trp Trp Phe Gly Thr Cys
    340                 345                 350

agc cat tcc aac ctc aac ggc cag tac ttc cgc tcc atc cca cag cag 1282

Ser His Ser Asn Leu Asn Gly Gln Tyr Phe Arg Ser Ile Pro Gln Gln
355                 360                 365                 370

cgg cag aag ctt aag aag gga atc ttc tgg aag acc tgg cgg ggc cgc    1330
Arg Gln Lys Leu Lys Lys Gly Ile Phe Trp Lys Thr Trp Arg Gly Arg
                375                 380                 385

tac tac ccg ctg cag gcc acc acc atg ttg atc cag ccc atg gca gca    1378
Tyr Tyr Pro Leu Gln Ala Thr Thr Met Leu Ile Gln Pro Met Ala Ala
            390                 395                 400

gag gca gcc tcc tag cgtcctggct gggcctggtc ccaggcccac gaaagacggt    1433
Glu Ala Ala Ser
        405

gactcttggc tctgcccgag gatgtggccg ttccctgcct gggcaggggc tccaaggagg 1493

ggccatctgg aaacttgtgg acagagaaga agaccacgac tggagaagcc ccctttctga 1553

gtgcaggggg gctgcatgcg ttgcctcctg agatcgaggc tgcaggatat gctcagactc 1613

tagaggcgtg gaccaagggg catggagctt cactccttgc tggccaggga gttggggact 1673

cagagggacc acttggggcc agccagactg gcctcaatgg cggactcagt cacattgact 1733

gacggggacc agggcttgtg tgggtcgaga gcgccctcat ggtgctggtg ctgttgtgtg 1793

taggtcccct ggggacacaa gcaggcgcca atggtatctg ggcggcgtca cagagttctt 1853

ggaataaaag caacctcaga acacttaaaa aaaaaaaaaa aaaaaaaaaa aaaaaaaaaa 1913

aaaaaaaaaa aaaaaaaaaa aaaaaaaaaa                                  1943

<210> 16
<211> 406

<212> PRT
<213> Homo sapiens

<400> 16
Met Ser Gly Ala Pro Thr Ala Gly Ala Ala Leu Met Leu Cys Ala Ala
1               5                   10                  15
Thr Ala Val Leu Leu Ser Ala Gln Gly Gly Pro Val Gln Ser Lys Ser
            20                  25                  30
Pro Arg Phe Ala Ser Trp Asp Glu Met Asn Val Leu Ala His Gly Leu
        35                  40                  45
Leu Gln Leu Gly Gln Gly Leu Arg Glu His Ala Glu Arg Thr Arg Ser
    50                  55                  60
Gln Leu Ser Ala Leu Glu Arg Arg Leu Ser Ala Cys Gly Ser Ala Cys
65                  70                  75                  80
Gln Gly Thr Glu Gly Ser Thr Asp Leu Pro Leu Ala Pro Glu Ser Arg
                85                  90                  95
Val Asp Pro Glu Val Leu His Ser Leu Gln Thr Gln Leu Lys Ala Gln
            100                 105                 110
Asn Ser Arg Ile Gln Gln Leu Phe His Lys Val Ala Gln Gln Gln Arg
        115                 120                 125
His Leu Glu Lys Gln His Leu Arg Ile Gln His Leu Gln Ser Gln Phe
    130                 135                 140
Gly Leu Leu Asp His Lys His Leu Asp His Glu Val Ala Lys Pro Ala
145                 150                 155                 160
Arg Arg Lys Arg Leu Pro Glu Met Ala Gln Pro Val Asp Pro Ala His
                165                 170                 175
Asn Val Ser Arg Leu His Arg Leu Pro Arg Asp Cys Gln Glu Leu Phe
            180                 185                 190
Gln Val Gly Glu Arg Gln Ser Gly Leu Phe Glu Ile Gln Pro Gln Gly
        195                 200                 205
Ser Pro Pro Phe Leu Val Asn Cys Lys Met Thr Ser Asp Gly Gly Trp
    210                 215                 220
Thr Val Ile Gln Arg Arg His Asp Gly Ser Val Asp Phe Asn Arg Pro
225                 230                 235                 240
Trp Glu Ala Tyr Lys Ala Gly Phe Gly Asp Pro His Gly Glu Phe Trp
                245                 250                 255

Leu Gly Leu Glu Lys Val His Ser Ile Thr Gly Asp Arg Asn Ser Arg
            260                 265                 270
Leu Ala Val Gln Leu Arg Asp Trp Asp Gly Asn Ala Glu Leu Leu Gln
        275                 280                 285
Phe Ser Val His Leu Gly Gly Glu Asp Thr Ala Tyr Ser Leu Gln Leu
    290                 295                 300
Thr Ala Pro Val Ala Gly Gln Leu Gly Ala Thr Thr Val Pro Pro Ser
305                 310                 315                 320
Gly Leu Ser Val Pro Phe Ser Thr Trp Asp Gln Asp His Asp Leu Arg
                325                 330                 335
Arg Asp Lys Asn Cys Ala Lys Ser Leu Ser Gly Gly Trp Trp Phe Gly
            340                 345                 350
Thr Cys Ser His Ser Asn Leu Asn Gly Gln Tyr Phe Arg Ser Ile Pro
        355                 360                 365
Gln Gln Arg Gln Lys Leu Lys Lys Gly Ile Phe Trp Lys Thr Trp Arg
    370                 375                 380
Gly Arg Tyr Tyr Pro Leu Gln Ala Thr Thr Met Leu Ile Gln Pro Met
385                 390                 395                 400
Ala Ala Glu Ala Ala Ser
                405

<210> 17
<211> 2066
<212> DNA
<213> Homo sapiens

<220>
<221> CDS
<222> (434)..(1909)

<400> 17
atttctccat gtggcagaca gagcaaagcc acaacgcttt ctctgctgga ttaaagacgg 60

cccacagacc agaacttcca ctatactact taaaattaca taggtggctt gtcaaattca 120

attgattagt attgtaaaag gaaaaagaag ttccttctta cagcttggat tcaacggtcc 180

aaaacaaaaa tgcagctgcc attaaagtca cagatgaaca aacttctaca ctgattttta 240

aaatcaagaa taagggcagc aagtttctgg attcactgaa tcaacagaca caaaaagctg 300

gcaatatagc aactatgaag agaaaagcta ctaataaaat taacccaacg catagaagac 360

ttttttttct cttctaaaaa caactaagta aagacttaaa tttaaacaca tcattttaca 420

acctcatttc aaa atg aag act ttt acc tgg acc cta ggt gtg cta ttc 469
Met Lys Thr Phe Thr Trp Thr Leu Gly Val Leu Phe
1               5                   10

ttc cta cta gtg gac act gga cat tgc aga ggt gga caa ttc aaa att 517
Phe Leu Leu Val Asp Thr Gly His Cys Arg Gly Gly Gln Phe Lys Ile
        15                  20                  25

aaa aaa ata aac cag aga aga tac cct cgt gcc aca gat ggt aaa gag 565
Lys Lys Ile Asn Gln Arg Arg Tyr Pro Arg Ala Thr Asp Gly Lys Glu
    30                  35                  40

gaa gca aag aaa tgt gca tac aca ttc ctg gta cct gaa caa aga ata 613
Glu Ala Lys Lys Cys Ala Tyr Thr Phe Leu Val Pro Glu Gln Arg Ile
45                  50                  55                  60

aca ggg cca atc tgt gtc aac acc aag ggg caa gat gca agt acc att 661
Thr Gly Pro Ile Cys Val Asn Thr Lys Gly Gln Asp Ala Ser Thr Ile
                65                  70                  75

aaa gac atg atc acc agg atg gac ctt gaa aac ctg aag gat gtg ctc 709
Lys Asp Met Ile Thr Arg Met Asp Leu Glu Asn Leu Lys Asp Val Leu
            80                  85                  90

tcc agg cag aag cgg gag ata gat gtt ctg caa ctg gtg gtg gat gta 757
Ser Arg Gln Lys Arg Glu Ile Asp Val Leu Gln Leu Val Val Asp Val

95                  100                  105

gat gga aac att gtg aat gag gta aag ctg ctg aga aag gaa agc cgt     805
Asp Gly Asn Ile Val Asn Glu Val Lys Leu Leu Arg Lys Glu Ser Arg
    110                 115                 120

aac atg aac tct cgt gtt act caa ctc tat atg caa tta tta cat gag     853
Asn Met Asn Ser Arg Val Thr Gln Leu Tyr Met Gln Leu Leu His Glu
125                 130                 135                 140

att atc cgt aag agg gat aat tca ctt gaa ctt tcc caa ctg gaa aac     901
Ile Ile Arg Lys Arg Asp Asn Ser Leu Glu Leu Ser Gln Leu Glu Asn
                145                 150                 155

aaa atc ctc aat gtc acc aca gaa atg ttg aag atg gca aca aga tac     949
Lys Ile Leu Asn Val Thr Thr Glu Met Leu Lys Met Ala Thr Arg Tyr
            160                 165                 170

agg gaa cta gag gtg aaa tac gct tcc ttg act gat ctt gtc aat aac     997
Arg Glu Leu Glu Val Lys Tyr Ala Ser Leu Thr Asp Leu Val Asn Asn
        175                 180                 185

caa tct gtg atg atc act ttg ttg gaa gaa cag tgc ttg agg ata ttt     1045
Gln Ser Val Met Ile Thr Leu Leu Glu Glu Gln Cys Leu Arg Ile Phe
    190                 195                 200

tcc cga caa gac acc cat gtg tct ccc cca ctt gtc cag gtg gtg cca     1093
Ser Arg Gln Asp Thr His Val Ser Pro Pro Leu Val Gln Val Val Pro
205                 210                 215                 220

caa cat att cct aac agc caa cag tat act cct ggt ctg ctg gga ggt     1141
Gln His Ile Pro Asn Ser Gln Gln Tyr Thr Pro Gly Leu Leu Gly Gly
                225                 230                 235

aac gag att cag agg gat cca ggt tat ccc aga gat tta atg cca cca     1189
Asn Glu Ile Gln Arg Asp Pro Gly Tyr Pro Arg Asp Leu Met Pro Pro

            240                 245                 250

cct gat ctg gca act tct ccc acc aaa agc cct ttc aag ata cca ccg 1237
Pro Asp Leu Ala Thr Ser Pro Thr Lys Ser Pro Phe Lys Ile Pro Pro
        255                 260                 265

gta act ttc atc aat gaa gga cca ttc aaa gac tgt cag caa gca aaa 1285
Val Thr Phe Ile Asn Glu Gly Pro Phe Lys Asp Cys Gln Gln Ala Lys
    270                 275                 280

gaa gct ggg cat tcg gtc agt ggg att tat atg att aaa cct gaa aac 1333
Glu Ala Gly His Ser Val Ser Gly Ile Tyr Met Ile Lys Pro Glu Asn
285                 290                 295                 300

agc aat gga cca atg cag tta tgg tgt gaa aac agt ttg gac cct ggg 1381
Ser Asn Gly Pro Met Gln Leu Trp Cys Glu Asn Ser Leu Asp Pro Gly
                305                 310                 315

ggt tgg act gtt att cag aaa aga aca gac ggc tct gtc aac ttc ttc 1429
Gly Trp Thr Val Ile Gln Lys Arg Thr Asp Gly Ser Val Asn Phe Phe
            320                 325                 330

aga aat tgg gaa aat tat aag aaa ggg ttt gga aac att gac gga gaa 1477
Arg Asn Trp Glu Asn Tyr Lys Lys Gly Phe Gly Asn Ile Asp Gly Glu
        335                 340                 345

tac tgg ctt gga ctg gaa aat atc tat atg ctt agc aat caa gat aat 1525
Tyr Trp Leu Gly Leu Glu Asn Ile Tyr Met Leu Ser Asn Gln Asp Asn
    350                 355                 360

tac aag tta ttg att gaa tta gaa gac tgg agt gat aaa aaa gtc tat 1573
Tyr Lys Leu Leu Ile Glu Leu Glu Asp Trp Ser Asp Lys Lys Val Tyr
365                 370                 375                 380

gca gaa tac agc agc ttt cgt ctg gaa cct gaa agt gaa ttc tat aga 1621
Ala Glu Tyr Ser Ser Phe Arg Leu Glu Pro Glu Ser Glu Phe Tyr Arg

                385                 390                 395

ctg cgc ctg gga act tac cag gga aat gca ggg gat tct atg atg tgg 1669
Leu Arg Leu Gly Thr Tyr Gln Gly Asn Ala Gly Asp Ser Met Met Trp
            400                 405                 410

cat aat ggt aaa caa ttc acc aca ctg gac aga gat aaa gat atg tat 1717
His Asn Gly Lys Gln Phe Thr Thr Leu Asp Arg Asp Lys Asp Met Tyr
        415                 420                 425

gca gga aac tgc gcc cac ttt cat aaa gga ggc tgg tgg tac aat gcc 1765
Ala Gly Asn Cys Ala His Phe His Lys Gly Gly Trp Trp Tyr Asn Ala
    430                 435                 440

tgt gca cat tct aac cta aat gga gta tgg tac aga gga ggc cat tac 1813
Cys Ala His Ser Asn Leu Asn Gly Val Trp Tyr Arg Gly Gly His Tyr
445                 450                 455                 460

aga agc aag cac caa gat gga att ttc tgg gcc gaa tac aga ggc ggg 1861
Arg Ser Lys His Gln Asp Gly Ile Phe Trp Ala Glu Tyr Arg Gly Gly
                465                 470                 475

tca tac tcc tta aga gca gtt cag atg atg atc aag cct att gac tga 1909
Ser Tyr Ser Leu Arg Ala Val Gln Met Met Ile Lys Pro Ile Asp
            480                 485                 490

agagagacac tcgccaattt aaatgacaca gaactttgta cttttcagct cttaaaaatg 1969

taaatgttac atgtatatta cttggcacaa tttatttcta cacagaaagt ttttaaaatg 2029

aattttaccg taactataaa agggaaccta taaatgt 2066

<210> 18
<211> 491
<212> PRT

<213> Homo sapiens

<400> 18
Met Lys Thr Phe Thr Trp Thr Leu Gly Val Leu Phe Phe Leu Leu Val
1               5                   10                  15
Asp Thr Gly His Cys Arg Gly Gly Gln Phe Lys Ile Lys Lys Ile Asn
            20                  25                  30
Gln Arg Arg Tyr Pro Arg Ala Thr Asp Gly Lys Glu Glu Ala Lys Lys
        35                  40                  45
Cys Ala Tyr Thr Phe Leu Val Pro Glu Gln Arg Ile Thr Gly Pro Ile
    50                  55                  60
Cys Val Asn Thr Lys Gly Gln Asp Ala Ser Thr Ile Lys Asp Met Ile
65                  70                  75                  80
Thr Arg Met Asp Leu Glu Asn Leu Lys Asp Val Leu Ser Arg Gln Lys
                85                  90                  95
Arg Glu Ile Asp Val Leu Gln Leu Val Val Asp Val Asp Gly Asn Ile
            100                 105                 110
Val Asn Glu Val Lys Leu Leu Arg Lys Glu Ser Arg Asn Met Asn Ser
        115                 120                 125
Arg Val Thr Gln Leu Tyr Met Gln Leu Leu His Glu Ile Ile Arg Lys
    130                 135                 140
Arg Asp Asn Ser Leu Glu Leu Ser Gln Leu Glu Asn Lys Ile Leu Asn
145                 150                 155                 160
Val Thr Thr Glu Met Leu Lys Met Ala Thr Arg Tyr Arg Glu Leu Glu
                165                 170                 175
Val Lys Tyr Ala Ser Leu Thr Asp Leu Val Asn Asn Gln Ser Val Met
            180                 185                 190
Ile Thr Leu Leu Glu Glu Gln Cys Leu Arg Ile Phe Ser Arg Gln Asp
        195                 200                 205
Thr His Val Ser Pro Pro Leu Val Gln Val Val Pro Gln His Ile Pro
    210                 215                 220
Asn Ser Gln Gln Tyr Thr Pro Gly Leu Leu Gly Gly Asn Glu Ile Gln
225                 230                 235                 240
Arg Asp Pro Gly Tyr Pro Arg Asp Leu Met Pro Pro Pro Asp Leu Ala
                245                 250                 255
Thr Ser Pro Thr Lys Ser Pro Phe Lys Ile Pro Pro Val Thr Phe Ile

            260                 265                 270
Asn Glu Gly Pro Phe Lys Asp Cys Gln Gln Ala Lys Glu Ala Gly His
        275                 280                 285
Ser Val Ser Gly Ile Tyr Met Ile Lys Pro Glu Asn Ser Asn Gly Pro
    290                 295                 300
Met Gln Leu Trp Cys Glu Asn Ser Leu Asp Pro Gly Gly Trp Thr Val
305                 310                 315                 320
Ile Gln Lys Arg Thr Asp Gly Ser Val Asn Phe Phe Arg Asn Trp Glu
                325                 330                 335
Asn Tyr Lys Lys Gly Phe Gly Asn Ile Asp Gly Glu Tyr Trp Leu Gly
            340                 345                 350
Leu Glu Asn Ile Tyr Met Leu Ser Asn Gln Asp Asn Tyr Lys Leu Leu
        355                 360                 365
Ile Glu Leu Glu Asp Trp Ser Asp Lys Lys Val Tyr Ala Glu Tyr Ser
    370                 375                 380
Ser Phe Arg Leu Glu Pro Glu Ser Glu Phe Tyr Arg Leu Arg Leu Gly
385                 390                 395                 400
Thr Tyr Gln Gly Asn Ala Gly Asp Ser Met Met Trp His Asn Gly Lys
                405                 410                 415
Gln Phe Thr Thr Leu Asp Arg Asp Lys Asp Met Tyr Ala Gly Asn Cys
            420                 425                 430
Ala His Phe His Lys Gly Gly Trp Trp Tyr Asn Ala Cys Ala His Ser
        435                 440                 445
Asn Leu Asn Gly Val Trp Tyr Arg Gly Gly His Tyr Arg Ser Lys His
    450                 455                 460
Gln Asp Gly Ile Phe Trp Ala Glu Tyr Arg Gly Gly Ser Tyr Ser Leu
465                 470                 475                 480
Arg Ala Val Gln Met Met Ile Lys Pro Ile Asp
                485                 490

<210> 19
<211> 1518
<212> DNA
<213> Homo sapiens

<220>
<221> CDS
<222> (22)..(1503)

<400> 19
aaccaccatt ttgcaaggac c atg agg cca ctg tgc gtg aca tgc tgg tgg 51
Met Arg Pro Leu Cys Val Thr Cys Trp Trp
1               5                   10

ctc gga ctg ctg gct gcc atg gga gct gtt gca ggc cag gag gac ggt 99
Leu Gly Leu Leu Ala Ala Met Gly Ala Val Ala Gly Gln Glu Asp Gly
                15                  20                  25

ttt gag ggc act gag gag ggc tcg cca aga gag ttc att tac cta aac 147
Phe Glu Gly Thr Glu Glu Gly Ser Pro Arg Glu Phe Ile Tyr Leu Asn
            30                  35                  40

agg tac aag cgg gcg ggc gag tcc cag gac aag tgc acc tac acc ttc 195
Arg Tyr Lys Arg Ala Gly Glu Ser Gln Asp Lys Cys Thr Tyr Thr Phe
        45                  50                  55

att gtg ccc cag cag cgg gtc acg ggt gcc atc tgc gtc aac tcc aag 243
Ile Val Pro Gln Gln Arg Val Thr Gly Ala Ile Cys Val Asn Ser Lys
    60                  65                  70

gag cct gag gtg ctt ctg gag aac cga gtg cat aag cag gag cta gag 291
Glu Pro Glu Val Leu Leu Glu Asn Arg Val His Lys Gln Glu Leu Glu
75                  80                  85                  90

ctg ctc aac aat gag ctg ctc aag cag aag cgg cag atc gag aca ctg 339
Leu Leu Asn Asn Glu Leu Leu Lys Gln Lys Arg Gln Ile Glu Thr Leu
                95                  100                 105

cag cag ctg gtg gag gtg gac ggc ggc att gtg agc gag gtg aag ctg 387
Gln Gln Leu Val Glu Val Asp Gly Gly Ile Val Ser Glu Val Lys Leu
            110                 115                 120

ctg cgc aag gag agc cgc aac atg aac tcg cgg gtc acg cag ctc tac 435
Leu Arg Lys Glu Ser Arg Asn Met Asn Ser Arg Val Thr Gln Leu Tyr
        125                 130                 135

atg cag ctc ctg cac gag atc atc cgc aag cgg gac aac gcg ttg gag 483
Met Gln Leu Leu His Glu Ile Ile Arg Lys Arg Asp Asn Ala Leu Glu
    140                 145                 150

ctc tcc cag ctg gag aac agg atc ctg aac cag aca gcc gac atg ctg 531
Leu Ser Gln Leu Glu Asn Arg Ile Leu Asn Gln Thr Ala Asp Met Leu
155                 160                 165                 170

cag ctg gcc agc aag tac aag gac ctg gag cac aag tac cag cac ctg 579
Gln Leu Ala Ser Lys Tyr Lys Asp Leu Glu His Lys Tyr Gln His Leu
                175                 180                 185

gcc aca ctg gcc cac aac caa tca gag atc atc gcg cag ctt gag gag 627
Ala Thr Leu Ala His Asn Gln Ser Glu Ile Ile Ala Gln Leu Glu Glu
            190                 195                 200

cac tgc cag agg gtg ccc tcg gcc agg ccc gtc ccc cag cca ccc ccc 675
His Cys Gln Arg Val Pro Ser Ala Arg Pro Val Pro Gln Pro Pro Pro
        205                 210                 215

gct gcc ccg ccc cgg gtc tac caa cca ccc acc tac aac cgc atc atc 723
Ala Ala Pro Pro Arg Val Tyr Gln Pro Pro Thr Tyr Asn Arg Ile Ile
    220                 225                 230

aac cag atc tct acc aac gag atc cag agt gac cag aac ctg aag gtg 771
Asn Gln Ile Ser Thr Asn Glu Ile Gln Ser Asp Gln Asn Leu Lys Val
235                 240                 245                 250

ctg cca ccc cct ctg ccc act atg ccc act ctc acc agc ctc cca tct 819
Leu Pro Pro Pro Leu Pro Thr Met Pro Thr Leu Thr Ser Leu Pro Ser
                255                 260                 265

tcc acc gac aag ccg tcg ggc cca tgg aga gac tgc ctg cag gcc ctg 867
Ser Thr Asp Lys Pro Ser Gly Pro Trp Arg Asp Cys Leu Gln Ala Leu
            270                 275                 280

gag gat ggc cac gac acc agc tcc atc tac ctg gtg aag ccg gag aac 915
Glu Asp Gly His Asp Thr Ser Ser Ile Tyr Leu Val Lys Pro Glu Asn
        285                 290                 295

acc aac cgc ctc atg cag gtg tgg tgc gac cag aga cac gac ccc ggg 963
Thr Asn Arg Leu Met Gln Val Trp Cys Asp Gln Arg His Asp Pro Gly
    300                 305                 310

ggc tgg acc gtc atc cag aga cgc ctg gat ggc tct gtt aac ttc ttc 1011
Gly Trp Thr Val Ile Gln Arg Arg Leu Asp Gly Ser Val Asn Phe Phe
315                 320                 325                 330

agg aac tgg gag acg tac aag caa ggg ttt ggg aac att gat ggc gaa 1059
Arg Asn Trp Glu Thr Tyr Lys Gln Gly Phe Gly Asn Ile Asp Gly Glu
                335                 340                 345

tac tgg ctg ggc ctg gag aac att tac tgg ctg acg aac caa ggc aac 1107
Tyr Trp Leu Gly Leu Glu Asn Ile Tyr Trp Leu Thr Asn Gln Gly Asn
            350                 355                 360

tac aaa ctc ctg gtg acc atg gag gac tgg tcc ggc cgc aaa gtc ttt 1155
Tyr Lys Leu Leu Val Thr Met Glu Asp Trp Ser Gly Arg Lys Val Phe
        365                 370                 375

gca gaa tac gcc agt ttc cgc ctg gaa cct gag agc gag tat tat aag 1203
Ala Glu Tyr Ala Ser Phe Arg Leu Glu Pro Glu Ser Glu Tyr Tyr Lys
    380                 385                 390

ctg cgg ctg ggg cgc tac cat ggc aat gcg ggt gac tcc ttt aca tgg 1251
Leu Arg Leu Gly Arg Tyr His Gly Asn Ala Gly Asp Ser Phe Thr Trp
395                 400                 405                 410

cac aac ggc aag cag ttc acc acc ctg gac aga gat cat gat gtc tac    1299
His Asn Gly Lys Gln Phe Thr Thr Leu Asp Arg Asp His Asp Val Tyr
                415                 420                 425

aca gga aac tgt gcc cac tac cag aag gga ggc tgg tgg tat aac gcc    1347
Thr Gly Asn Cys Ala His Tyr Gln Lys Gly Gly Trp Trp Tyr Asn Ala
            430                 435                 440

tgt gcc cac tcc aac ctc aac ggg gtc tgg tac cgc ggg ggc cat tac    1395
Cys Ala His Ser Asn Leu Asn Gly Val Trp Tyr Arg Gly Gly His Tyr
        445                 450                 455

cgg agc cgc tac cag gac gga gtc tac tgg gct gag ttc cga gga ggc    1443
Arg Ser Arg Tyr Gln Asp Gly Val Tyr Trp Ala Glu Phe Arg Gly Gly
    460                 465                 470

tct tac tca ctc aag aaa gtg gtg atg atg atc cga ccg aac ccc aac    1491
Ser Tyr Ser Leu Lys Lys Val Val Met Met Ile Arg Pro Asn Pro Asn
475                 480                 485                 490

acc ttc cac taa gccagctccc cctcc                                   1518
Thr Phe His

<210> 20
<211> 493
<212> PRT
<213> Homo sapiens

<400> 20
Met Arg Pro Leu Cys Val Thr Cys Trp Trp Leu Gly Leu Leu Ala Ala
 1               5                  10                  15
Met Gly Ala Val Ala Gly Gln Glu Asp Gly Phe Glu Gly Thr Glu Glu
            20                  25                  30
Gly Ser Pro Arg Glu Phe Ile Tyr Leu Asn Arg Tyr Lys Arg Ala Gly

35                  40                  45
Glu Ser Gln Asp Lys Cys Thr Tyr Thr Phe Ile Val Pro Gln Gln Arg
    50                  55                  60
Val Thr Gly Ala Ile Cys Val Asn Ser Lys Glu Pro Glu Val Leu Leu
65                  70                  75                  80
Glu Asn Arg Val His Lys Gln Glu Leu Glu Leu Leu Asn Asn Glu Leu
                85                  90                  95
Leu Lys Gln Lys Arg Gln Ile Glu Thr Leu Gln Gln Leu Val Glu Val
            100                 105                 110
Asp Gly Gly Ile Val Ser Glu Val Lys Leu Leu Arg Lys Glu Ser Arg
        115                 120                 125
Asn Met Asn Ser Arg Val Thr Gln Leu Tyr Met Gln Leu Leu His Glu
    130                 135                 140
Ile Ile Arg Lys Arg Asp Asn Ala Leu Glu Leu Ser Gln Leu Glu Asn
145                 150                 155                 160
Arg Ile Leu Asn Gln Thr Ala Asp Met Leu Gln Leu Ala Ser Lys Tyr
                165                 170                 175
Lys Asp Leu Glu His Lys Tyr Gln His Leu Ala Thr Leu Ala His Asn
            180                 185                 190
Gln Ser Glu Ile Ile Ala Gln Leu Glu Glu His Cys Gln Arg Val Pro
        195                 200                 205
Ser Ala Arg Pro Val Pro Gln Pro Pro Pro Ala Ala Pro Pro Arg Val
    210                 215                 220
Tyr Gln Pro Pro Thr Tyr Asn Arg Ile Ile Asn Gln Ile Ser Thr Asn
225                 230                 235                 240
Glu Ile Gln Ser Asp Gln Asn Leu Lys Val Leu Pro Pro Pro Leu Pro
                245                 250                 255
Thr Met Pro Thr Leu Thr Ser Leu Pro Ser Ser Thr Asp Lys Pro Ser
            260                 265                 270
Gly Pro Trp Arg Asp Cys Leu Gln Ala Leu Glu Asp Gly His Asp Thr
        275                 280                 285
Ser Ser Ile Tyr Leu Val Lys Pro Glu Asn Thr Asn Arg Leu Met Gln
    290                 295                 300
Val Trp Cys Asp Gln Arg His Asp Pro Gly Gly Trp Thr Val Ile Gln
305                 310                 315                 320
Arg Arg Leu Asp Gly Ser Val Asn Phe Phe Arg Asn Trp Glu Thr Tyr

                325                 330                 335
Lys Gln Gly Phe Gly Asn Ile Asp Gly Glu Tyr Trp Leu Gly Leu Glu
            340                 345                 350
Asn Ile Tyr Trp Leu Thr Asn Gln Gly Asn Tyr Lys Leu Leu Val Thr
        355                 360                 365
Met Glu Asp Trp Ser Gly Arg Lys Val Phe Ala Glu Tyr Ala Ser Phe
    370                 375                 380
Arg Leu Glu Pro Glu Ser Glu Tyr Tyr Lys Leu Arg Leu Gly Arg Tyr
385                 390                 395                 400
His Gly Asn Ala Gly Asp Ser Phe Thr Trp His Asn Gly Lys Gln Phe
                405                 410                 415
Thr Thr Leu Asp Arg Asp His Asp Val Tyr Thr Gly Asn Cys Ala His
            420                 425                 430
Tyr Gln Lys Gly Gly Trp Trp Tyr Asn Ala Cys Ala His Ser Asn Leu
        435                 440                 445
Asn Gly Val Trp Tyr Arg Gly Gly His Tyr Arg Ser Arg Tyr Gln Asp
    450                 455                 460
Gly Val Tyr Trp Ala Glu Phe Arg Gly Gly Ser Tyr Ser Leu Lys Lys
465                 470                 475                 480
Val Val Met Met Ile Arg Pro Asn Pro Asn Thr Phe His
                485                 490

<210> 21
<211> 1931
<212> DNA
<213> Homo sapiens

<220>
<221> CDS
<222> (242)..(1654)

<400> 21
cctgattccg gaaatgctaa atatttatag ttaggggctc ctgggcccag catcccatct 60

ccactcccag gctctggggg ctggggagtg nttaccaagc ctcctctctc cttctgtccc 120

actgccctct ccccgtctct agctcagagg ccccactgga ccctcggctc ttccttggac 180

ttcttgtgtg ttctgtgagc ttcgctggat tcagggtctt gggcatcaga ggtccgccgc 240

g atg ggg aag ccc tgg ctg cgt gcg cta cag ctg ctg ctc ctg ctg ggc 289
  Met Gly Lys Pro Trp Leu Arg Ala Leu Gln Leu Leu Leu Leu Leu Gly
  1               5                   10                  15

gcg tcg tgg gcg cgg gcg ggc gcc ccg cgc tgc acc tac acc ttc gtg 337
Ala Ser Trp Ala Arg Ala Gly Ala Pro Arg Cys Thr Tyr Thr Phe Val
            20                  25                  30

ctg ccc ccg cag aag ttc acg ggc gct gtg tgc tgg agc ggc ccc gca 385
Leu Pro Pro Gln Lys Phe Thr Gly Ala Val Cys Trp Ser Gly Pro Ala
        35                  40                  45

tcc acg cgg gcg acg ccc gag gcc gcc aac gcc agc gag ctg gcg gcg 433
Ser Thr Arg Ala Thr Pro Glu Ala Ala Asn Ala Ser Glu Leu Ala Ala
    50                  55                  60

ctg cgc atg cgc gtc ggc cgc cac gag gag ctg tta cgc gag ctg cag 481
Leu Arg Met Arg Val Gly Arg His Glu Glu Leu Leu Arg Glu Leu Gln
65                  70                  75                  80

agg ctg gcg gcg gcc gac ggc gcc gtg gcc ggc aag gtg cgc gcg ctg 529
Arg Leu Ala Ala Ala Asp Gly Ala Val Ala Gly Lys Val Arg Ala Leu
                85                  90                  95

cgc aag gag agc cgc ggc ctg agc gcg cgc ctg ggc cag ttg cgc gcg 577
Arg Lys Glu Ser Arg Gly Leu Ser Ala Arg Leu Gly Gln Leu Arg Ala
            100                 105                 110

cag ctg cag cac gag gcg ggg ccc ggg gcg ggc ccg ggg gcg gat ctg 625
Gln Leu Gln His Glu Ala Gly Pro Gly Ala Gly Pro Gly Ala Asp Leu
        115                 120                 125

ggg gcg gag cct gcc gcg gcg ctg gcg ctg ctc ggg gag cgc gtg ctc 673
Gly Ala Glu Pro Ala Ala Ala Leu Ala Leu Leu Gly Glu Arg Val Leu
    130                 135                 140

aac gcg tcc gcc gag gct cag cgc gca gcc gcc cgg ttc cac cag ctg 721
Asn Ala Ser Ala Glu Ala Gln Arg Ala Ala Ala Arg Phe His Gln Leu
145                 150                 155                 160

gac gtc aag ttc cgc gag ctg gcg cag ctc gtc acc cag cag agc agt 769
Asp Val Lys Phe Arg Glu Leu Ala Gln Leu Val Thr Gln Gln Ser Ser
                165                 170                 175

ctc atc gcc cgc ctg gag cgc ctg tgc ccg gga ggc gcg ggc ggg cag 817
Leu Ile Ala Arg Leu Glu Arg Leu Cys Pro Gly Gly Ala Gly Gly Gln
            180                 185                 190

cag cag gtc ctg ccg cca ccc cca ctg gtg cct gtg gtt ccg gtc cgt 865
Gln Gln Val Leu Pro Pro Pro Pro Leu Val Pro Val Val Pro Val Arg
        195                 200                 205

ctt gtg ggt agc acc agt gac acc agt agg atg ctg gac cca gcc cca 913
Leu Val Gly Ser Thr Ser Asp Thr Ser Arg Met Leu Asp Pro Ala Pro
    210                 215                 220

gag ccc cag aga gac cag acc cag aga cag cag gag ccc atg gct tct 961
Glu Pro Gln Arg Asp Gln Thr Gln Arg Gln Gln Glu Pro Met Ala Ser
225                 230                 235                 240

ccc atg cct gca ggt cac cct gcg gtc ccc acc aag cct gtg ggc ccg 1009
Pro Met Pro Ala Gly His Pro Ala Val Pro Thr Lys Pro Val Gly Pro
                245                 250                 255

tgg cag gat tgt gca gag gcc cgc cag gca ggc cat gaa cag agt gga 1057
Trp Gln Asp Cys Ala Glu Ala Arg Gln Ala Gly His Glu Gln Ser Gly
            260                 265                 270

gtg tat gaa ctg cga gtg ggc cgt cac gta gtg tca gta tgg tgt gag     1105
Val Tyr Glu Leu Arg Val Gly Arg His Val Val Ser Val Trp Cys Glu
        275                 280                 285

cag caa ctg gag ggt gga ggc tgg act gtg atc cag cgg agg caa gat     1153
Gln Gln Leu Glu Gly Gly Gly Trp Thr Val Ile Gln Arg Arg Gln Asp
    290                 295                 300

ggt tca gtc aac ttc ttc act acc tgg cag cac tat aag gcg ggc ttt     1201
Gly Ser Val Asn Phe Phe Thr Thr Trp Gln His Tyr Lys Ala Gly Phe
305                 310                 315                 320

ggg cgg cca gac gga gaa tac tgg ctg ggc ctt gaa ccc gtg tat cag     1249
Gly Arg Pro Asp Gly Glu Tyr Trp Leu Gly Leu Glu Pro Val Tyr Gln
                325                 330                 335

ctg acc agc cgt ggg gac cat gag ctg ctg gtt ctc ctg gag gac tgg     1297
Leu Thr Ser Arg Gly Asp His Glu Leu Leu Val Leu Leu Glu Asp Trp
            340                 345                 350

ggg ggc cgt gga gca cgt gcc cac tat gat ggc ttc tcc ctg gaa ccc     1345
Gly Gly Arg Gly Ala Arg Ala His Tyr Asp Gly Phe Ser Leu Glu Pro
        355                 360                 365

gag agc gac cac tac cgc ctg cgg ctt ggc cag tac cat ggt gat gct     1393
Glu Ser Asp His Tyr Arg Leu Arg Leu Gly Gln Tyr His Gly Asp Ala
    370                 375                 380

gga gac tct ctt tcc tgg cac aat gac aag ccc ttc agc acc gtg gat     1441
Gly Asp Ser Leu Ser Trp His Asn Asp Lys Pro Phe Ser Thr Val Asp
385                 390                 395                 400

agg gac cga gac tcc tat tct ggt aac tgt gcc ctg tac cag cgg gga     1489
Arg Asp Arg Asp Ser Tyr Ser Gly Asn Cys Ala Leu Tyr Gln Arg Gly
                405                 410                 415

ggc tgg tgg tac cat gcc tgt gcc cac tcc aac ctc aac ggt gtg tgg    1537
Gly Trp Trp Tyr His Ala Cys Ala His Ser Asn Leu Asn Gly Val Trp
            420                 425                 430

cac cac ggc ggc cac tac cga agc cgc tac cag gat ggt gtc tac tgg    1585
His His Gly Gly His Tyr Arg Ser Arg Tyr Gln Asp Gly Val Tyr Trp
        435                 440                 445

gct gag ttt cgt ggt ggg gca tat tct ctc agg aag gcc gcc atg ctc    1633
Ala Glu Phe Arg Gly Gly Ala Tyr Ser Leu Arg Lys Ala Ala Met Leu
    450                 455                 460

att cgg ccc ctg aag ctg tga ctctgtgttc ctctgtcccc taggccctag       1684
Ile Arg Pro Leu Lys Leu
465                 470

aggacattgg tcagcaggag cccaagttgt tctggccaca cnttntttgt ggntcagtgc 1744

caatgtgtcc cacanaantt cccactgtgg atcttgtnac cctgggcgct gaaaatggga 1804

cccaggaatc ccccccgtca atatnttggc ctcanatggc tccccaaggt cattcatatc 1864

tcggtttgag ctcatatntt ataataacac aaagtagccg cgaaaaaaaa aaaaaaaaaa 1924

aaaaaaa                                                            1931

<210> 22
<211> 470
<212> PRT
<213> Homo sapiens

<400> 22
Met Gly Lys Pro Trp Leu Arg Ala Leu Gln Leu Leu Leu Leu Leu Gly
1               5                   10                  15

Ala Ser Trp Ala Arg Ala Gly Ala Pro Arg Cys Thr Tyr Thr Phe Val
            20                  25                  30
Leu Pro Pro Gln Lys Phe Thr Gly Ala Val Cys Trp Ser Gly Pro Ala
        35                  40                  45
Ser Thr Arg Ala Thr Pro Glu Ala Ala Asn Ala Ser Glu Leu Ala Ala
    50                  55                  60
Leu Arg Met Arg Val Gly Arg His Glu Glu Leu Leu Arg Glu Leu Gln
65                  70                  75                  80
Arg Leu Ala Ala Ala Asp Gly Ala Val Ala Gly Lys Val Arg Ala Leu
                85                  90                  95
Arg Lys Glu Ser Arg Gly Leu Ser Ala Arg Leu Gly Gln Leu Arg Ala
            100                 105                 110
Gln Leu Gln His Glu Ala Gly Pro Gly Ala Gly Pro Gly Ala Asp Leu
        115                 120                 125
Gly Ala Glu Pro Ala Ala Ala Leu Ala Leu Leu Gly Glu Arg Val Leu
    130                 135                 140
Asn Ala Ser Ala Glu Ala Gln Arg Ala Ala Ala Arg Phe His Gln Leu
145                 150                 155                 160
Asp Val Lys Phe Arg Glu Leu Ala Gln Leu Val Thr Gln Gln Ser Ser
                165                 170                 175
Leu Ile Ala Arg Leu Glu Arg Leu Cys Pro Gly Gly Ala Gly Gly Gln
            180                 185                 190
Gln Gln Val Leu Pro Pro Pro Pro Leu Val Pro Val Val Pro Val Arg
        195                 200                 205
Leu Val Gly Ser Thr Ser Asp Thr Ser Arg Met Leu Asp Pro Ala Pro
    210                 215                 220
Glu Pro Gln Arg Asp Gln Thr Gln Arg Gln Gln Glu Pro Met Ala Ser
225                 230                 235                 240
Pro Met Pro Ala Gly His Pro Ala Val Pro Thr Lys Pro Val Gly Pro
                245                 250                 255
Trp Gln Asp Cys Ala Glu Ala Arg Gln Ala Gly His Glu Gln Ser Gly
            260                 265                 270
Val Tyr Glu Leu Arg Val Gly Arg His Val Val Ser Val Trp Cys Glu
        275                 280                 285
Gln Gln Leu Glu Gly Gly Gly Trp Thr Val Ile Gln Arg Arg Gln Asp
    290                 295                 300

Gly Ser Val Asn Phe Phe Thr Thr Trp Gln His Tyr Lys Ala Gly Phe
305                 310                 315                 320
Gly Arg Pro Asp Gly Glu Tyr Trp Leu Gly Leu Glu Pro Val Tyr Gln
                325                 330                 335
Leu Thr Ser Arg Gly Asp His Glu Leu Leu Val Leu Leu Glu Asp Trp
            340                 345                 350
Gly Gly Arg Gly Ala Arg Ala His Tyr Asp Gly Phe Ser Leu Glu Pro
        355                 360                 365
Glu Ser Asp His Tyr Arg Leu Arg Leu Gly Gln Tyr His Gly Asp Ala
    370                 375                 380
Gly Asp Ser Leu Ser Trp His Asn Asp Lys Pro Phe Ser Thr Val Asp
385                 390                 395                 400
Arg Asp Arg Asp Ser Tyr Ser Gly Asn Cys Ala Leu Tyr Gln Arg Gly
                405                 410                 415
Gly Trp Trp Tyr His Ala Cys Ala His Ser Asn Leu Asn Gly Val Trp
            420                 425                 430
His His Gly Gly His Tyr Arg Ser Arg Tyr Gln Asp Gly Val Tyr Trp
        435                 440                 445
Ala Glu Phe Arg Gly Gly Ala Tyr Ser Leu Arg Lys Ala Ala Met Leu
    450                 455                 460
Ile Arg Pro Leu Lys Leu
465                 470

<210> 23
<211> 2006
<212> DNA
<213> Homo sapiens

<220>
<221> CDS
<222> (8)..(1048)

<400> 23
acaaaag atg ctg aaa aag cct ctc tca gct gtg acc tgg ctc tgc att    49

Met Leu Lys Lys Pro Leu Ser Ala Val Thr Trp Leu Cys Ile
1               5                   10

ttc atc gtg gcc ttt gtc agc cac cca gcg tgg ctg cag aag ctc tct    97
Phe Ile Val Ala Phe Val Ser His Pro Ala Trp Leu Gln Lys Leu Ser
15                  20                  25                  30

aag cac aag aca cca gca cag cca cag ctc aaa gcg gcc aac tgc tgt    145
Lys His Lys Thr Pro Ala Gln Pro Gln Leu Lys Ala Ala Asn Cys Cys
                35                  40                  45

gag gag gtg aag gag ctc aag gcc caa gtt gcc aac ctt agc agc ctg    193
Glu Glu Val Lys Glu Leu Lys Ala Gln Val Ala Asn Leu Ser Ser Leu
            50                  55                  60

ctg agt gaa ctg aac aag aag cag gag agg gac tgg gtc agc gtg gtc    241
Leu Ser Glu Leu Asn Lys Lys Gln Glu Arg Asp Trp Val Ser Val Val
        65                  70                  75

atg cag gtg atg gag ctg gag agc aac agc aag cgc atg gag tcg cgg    289
Met Gln Val Met Glu Leu Glu Ser Asn Ser Lys Arg Met Glu Ser Arg
    80                  85                  90

ctc aca gat gct gag agc aag tac tcc gag atg aac aac caa att gac    337
Leu Thr Asp Ala Glu Ser Lys Tyr Ser Glu Met Asn Asn Gln Ile Asp
95                  100                 105                 110

atc atg cag ctg cag gca gca cag acg gtc act cag acc tcc gca gat    385
Ile Met Gln Leu Gln Ala Ala Gln Thr Val Thr Gln Thr Ser Ala Asp
                115                 120                 125

gcc atc tac gac tgc tct tcc ctc tac cag aag aac tac cgc atc tct    433
Ala Ile Tyr Asp Cys Ser Ser Leu Tyr Gln Lys Asn Tyr Arg Ile Ser
            130                 135                 140

gga gtg tat aag ctt cct cct gat gac ttc ctg ggc agc cct gaa ctg    481

Gly Val Tyr Lys Leu Pro Pro Asp Asp Phe Leu Gly Ser Pro Glu Leu
        145                 150                 155

gag gtg ttc tgt gac atg gag act tca ggc gga ggc tgg acc atc atc 529
Glu Val Phe Cys Asp Met Glu Thr Ser Gly Gly Gly Trp Thr Ile Ile
    160                 165                 170

cag aga cga aaa agt ggc ctt gtc tcc ttc tac cgg gac tgg aag cag 577
Gln Arg Arg Lys Ser Gly Leu Val Ser Phe Tyr Arg Asp Trp Lys Gln
175                 180                 185                 190

tac aag cag ggc ttt ggc agc atc cgt ggg gac ttc tgg ctg ggg aac 625
Tyr Lys Gln Gly Phe Gly Ser Ile Arg Gly Asp Phe Trp Leu Gly Asn
                195                 200                 205

gaa cac atc cac cgg ctc tcc aga cag cca acc cgg ctg cgt gta gag 673
Glu His Ile His Arg Leu Ser Arg Gln Pro Thr Arg Leu Arg Val Glu
            210                 215                 220

atg gag gac tgg gag ggc aac ctg cgc tac gct gag tat agc cac ttt 721
Met Glu Asp Trp Glu Gly Asn Leu Arg Tyr Ala Glu Tyr Ser His Phe
        225                 230                 235

gtt ttg ggc aat gaa ctc aac agc tat cgc ctc ttc ctg ggg aac tac 769
Val Leu Gly Asn Glu Leu Asn Ser Tyr Arg Leu Phe Leu Gly Asn Tyr
    240                 245                 250

act ggc aat gtg ggg aac gac gcc ctc cag tat cat aac aac aca gcc 817
Thr Gly Asn Val Gly Asn Asp Ala Leu Gln Tyr His Asn Asn Thr Ala
255                 260                 265                 270

ttc agc acc aag gac aag gac aat gac aac tgc ttg gac aag tgt gca 865
Phe Ser Thr Lys Asp Lys Asp Asn Asp Asn Cys Leu Asp Lys Cys Ala
                275                 280                 285

cag ctc cgc aaa ggt ggc tac tgg tac aac tgc tgc aca gac tcc aac 913

Gln Leu Arg Lys Gly Gly Tyr Trp Tyr Asn Cys Cys Thr Asp Ser Asn
            290                 295                 300

ctc aat gga gtg tac tac cgc ctg ggt gag cac aat aag cac ctg gat    961
Leu Asn Gly Val Tyr Tyr Arg Leu Gly Glu His Asn Lys His Leu Asp
        305                 310                 315

ggc atc acc tgg tat ggc tgg cat gga tct acc tac tcc ctc aaa cgg    1009
Gly Ile Thr Trp Tyr Gly Trp His Gly Ser Thr Tyr Ser Leu Lys Arg
    320                 325                 330

gtg gag atg aaa atc cgc cca gaa gac ttc aag cct taa aaggaggctg    1058
Val Glu Met Lys Ile Arg Pro Glu Asp Phe Lys Pro
335                 340                 345

ccgtggagca cggatacaga aactgagaca cgtggagact ggatgagggc agatgaggac 1118

aggaagagag tgttagaaag ggtaggactg agaaacagcc tataatctcc aaagaaagaa 1178

taagtctcca aggagcacaa aaaaatcata tgtaccaagg atgttacagt aaacaggatg 1238

aactatttaa acccactggg tcctgccaca tccttctcaa ggtggtagac tgagtggggt 1298

ctctctgccc aagatccctg acatagcagt agcttgtctt ttccacatga tttgtctgtg 1358

aaagaaaata attttgagat cgttttatct attttctcta cggcttaggc tatgtgaggg 1418

caaaacacaa atccctttgc taaaaagacc catattattt tgattctcaa aggataggcc 1478

tttgagtgtt agagaaagga gtgaaggagc caggtgggaa atggtatttc tatttttaaa 1538

ctccagtgaa attatcttga gtctacacat tatttttaaa acacaaaaat tgttcggctg 1598

gaactgaccc aggctggact tgcggggagg aaactccagg gcactgcatc tggcgatcag 1658

actctgagca ctgcccctgc tcgccttggt catgtacagc actgaaagga atgaggcacc 1718

agcaggaggt ggacagagtc tctcatggat gccggcacaa aactgcctta aaatattcat 1778

agttaataca ggtatatcta tttttattta ctttgtaaga aacaagctca aggagcttcc 1838

ttttaaattt tgtctgtagg aaatggttga aaactgaagg tagatggtgt tatagttaat 1898

aataaatgct gtaaataagc atctcacttt gtaaaaataa aatattgtgg ttttgtttta 1958

aacattcaac gtttcttttc cttctacaat aaacactttc aaaatgtg 2006

<210> 24
<211> 346
<212> PRT
<213> Homo sapiens

<400> 24
Met Leu Lys Lys Pro Leu Ser Ala Val Thr Trp Leu Cys Ile Phe Ile
1               5                   10                  15
Val Ala Phe Val Ser His Pro Ala Trp Leu Gln Lys Leu Ser Lys His
            20                  25                  30
Lys Thr Pro Ala Gln Pro Gln Leu Lys Ala Ala Asn Cys Cys Glu Glu
        35                  40                  45
Val Lys Glu Leu Lys Ala Gln Val Ala Asn Leu Ser Ser Leu Leu Ser
    50                  55                  60
Glu Leu Asn Lys Lys Gln Glu Arg Asp Trp Val Ser Val Val Met Gln
65                  70                  75                  80
Val Met Glu Leu Glu Ser Asn Ser Lys Arg Met Glu Ser Arg Leu Thr
                85                  90                  95
Asp Ala Glu Ser Lys Tyr Ser Glu Met Asn Asn Gln Ile Asp Ile Met
            100                 105                 110
Gln Leu Gln Ala Ala Gln Thr Val Thr Gln Thr Ser Ala Asp Ala Ile
        115                 120                 125
Tyr Asp Cys Ser Ser Leu Tyr Gln Lys Asn Tyr Arg Ile Ser Gly Val
    130                 135                 140

Tyr Lys Leu Pro Pro Asp Asp Phe Leu Gly Ser Pro Glu Leu Glu Val
145                 150                 155                 160
Phe Cys Asp Met Glu Thr Ser Gly Gly Gly Trp Thr Ile Ile Gln Arg
                165                 170                 175
Arg Lys Ser Gly Leu Val Ser Phe Tyr Arg Asp Trp Lys Gln Tyr Lys
            180                 185                 190
Gln Gly Phe Gly Ser Ile Arg Gly Asp Phe Trp Leu Gly Asn Glu His
        195                 200                 205
Ile His Arg Leu Ser Arg Gln Pro Thr Arg Leu Arg Val Glu Met Glu
    210                 215                 220
Asp Trp Glu Gly Asn Leu Arg Tyr Ala Glu Tyr Ser His Phe Val Leu
225                 230                 235                 240
Gly Asn Glu Leu Asn Ser Tyr Arg Leu Phe Leu Gly Asn Tyr Thr Gly
                245                 250                 255
Asn Val Gly Asn Asp Ala Leu Gln Tyr His Asn Asn Thr Ala Phe Ser
            260                 265                 270
Thr Lys Asp Lys Asp Asn Asp Asn Cys Leu Asp Lys Cys Ala Gln Leu
        275                 280                 285
Arg Lys Gly Gly Tyr Trp Tyr Asn Cys Cys Thr Asp Ser Asn Leu Asn
    290                 295                 300
Gly Val Tyr Tyr Arg Leu Gly Glu His Asn Lys His Leu Asp Gly Ile
305                 310                 315                 320
Thr Trp Tyr Gly Trp His Gly Ser Thr Tyr Ser Leu Lys Arg Val Glu
                325                 330                 335
Met Lys Ile Arg Pro Glu Asp Phe Lys Pro
            340                 345

<210> 25
<211> 1441
<212> DNA
<213> Artificial Sequence

<220>
<223> Description of Artificial Sequence: DNA encoding
      KhAP5 CC/His

<220>
<221> CDS
<222> (18)..(662)

<400> 25
gaattcggct tgccacc atg gtc aca att aag ctc ctt ctt ttt att gtt 50
Met Val Thr Ile Lys Leu Leu Leu Phe Ile Val
1               5                   10

cct cta gtt att tcc tcc aga att gat cag cat cat cac cat cac cac 98
Pro Leu Val Ile Ser Ser Arg Ile Asp Gln His His His His His His
            15                  20                  25

gat caa gac aat tca tca ttt gat tct cta tct cca gag cca aaa tca 146
Asp Gln Asp Asn Ser Ser Phe Asp Ser Leu Ser Pro Glu Pro Lys Ser
        30                  35                  40

aga ttt gct atg tta gac gat gta aaa att tta gcc aat ggc ctc ctt 194
Arg Phe Ala Met Leu Asp Asp Val Lys Ile Leu Ala Asn Gly Leu Leu
    45                  50                  55

cag ttg gga cat ggt ctt aaa gac ttt gtc cat aag acg aag ggc caa 242
Gln Leu Gly His Gly Leu Lys Asp Phe Val His Lys Thr Lys Gly Gln
60                  65                  70                  75

att aat gac ata ttt caa aaa ctc aac ata ttt gat cag tct ttt tat 290
Ile Asn Asp Ile Phe Gln Lys Leu Asn Ile Phe Asp Gln Ser Phe Tyr
                80                  85                  90

gat cta tcg ctg caa acc agt gaa atc aaa gaa gaa gaa aag gaa ctg 338
Asp Leu Ser Leu Gln Thr Ser Glu Ile Lys Glu Glu Glu Lys Glu Leu
            95                  100                 105

aga aga act aca tat aaa cta caa gtc aaa aat gaa gag gta aag aat 386
Arg Arg Thr Thr Tyr Lys Leu Gln Val Lys Asn Glu Glu Val Lys Asn

        110                 115                 120

atg tca ctt gaa ctc aac tca aaa ctt gaa agc ctc cta gaa gaa aaa 434
Met Ser Leu Glu Leu Asn Ser Lys Leu Glu Ser Leu Leu Glu Glu Lys
    125                 130                 135

att cta ctt caa caa aaa gtg aaa tat tta gaa gag caa cta act aac 482
Ile Leu Leu Gln Gln Lys Val Lys Tyr Leu Glu Glu Gln Leu Thr Asn
140                 145                 150                 155

tta att caa aat caa cct gaa act cca gaa cac cca gaa gta act tca 530
Leu Ile Gln Asn Gln Pro Glu Thr Pro Glu His Pro Glu Val Thr Ser
                160                 165                 170

ctt aaa act ttt gta gaa aaa caa gat aat agc atc aaa gac ctt ctc 578
Leu Lys Thr Phe Val Glu Lys Gln Asp Asn Ser Ile Lys Asp Leu Leu
            175                 180                 185

cag acc gtg gaa gac caa tat aaa caa tta aac caa cag cat agt caa 626
Gln Thr Val Glu Asp Gln Tyr Lys Gln Leu Asn Gln Gln His Ser Gln
        190                 195                 200

ata aaa gaa ata gaa aat cag ctc aga agg act agc tagtattcaa 672
Ile Lys Glu Ile Glu Asn Gln Leu Arg Arg Thr Ser
    205                 210                 215

gaacccacag aaatttctct atcttccaag ccaagagcac caagaactac tccctttctt 732

cagttgaatg aaataagaaa tgtaaaacat gatggcattc ctgctgaatg taccaccatt 792

tataacagag gtgaacatac aagtggcatg tatgccatca gacccagcaa ctctcaagtt 852

tttcatgtct actgtgatgt tatatcaggt agtccatgga cattaattca acatcgaata 912

gatggatcac aaaacttcaa tgaaacgtgg gagaactaca aatatggttt tgggaggctt 972

gatggagaat tttggttggg cctagagaag atatactcca tagtgaagca atctaattat 1032

gttttacgaa ttgagttgga agactggaaa gacaacaaac attatattga atattctttt 1092

tacttgggaa atcacgaaac caactatacg ctacatctag ttgcgattac tggcaatgtc 1152

cccaatgcaa tcccggaaaa caaagatttg gtgttttcta cttgggatca caaagcaaaa 1212

ggacacttca actgtccaga gggttattca ggaggctggt ggtggcatga tgagtgtgga 1272

gaaaacaacc taaatggtaa atataacaaa ccaagagcaa aatctaagcc agagaggaga 1332

agaggattat cttggaagtc tcaaaatgga aggttatact ctataaaatc aaccaaaatg 1392

ttgatccatc caacagattc agaaagcttt gaatgaacga ggctctaga 1441

<210> 26
<211> 215
<212> PRT
<213> Artificial Sequence
<223> Description of Artificial Sequence: Polypeptide encoding
      KhAP5 CC/His

<400> 26
Met Val Thr Ile Lys Leu Leu Leu Phe Ile Val Pro Leu Val Ile Ser
 1               5                  10                  15

Ser Arg Ile Asp Gln His His His His His His Asp Gln Asp Asn Ser
            20                  25                  30

Ser Phe Asp Ser Leu Ser Pro Glu Pro Lys Ser Arg Phe Ala Met Leu
        35                  40                  45

Asp Asp Val Lys Ile Leu Ala Asn Gly Leu Leu Gln Leu Gly His Gly
    50                  55                  60

Leu Lys Asp Phe Val His Lys Thr Lys Gly Gln Ile Asn Asp Ile Phe
65                  70                  75                  80

Gln Lys Leu Asn Ile Phe Asp Gln Ser Phe Tyr Asp Leu Ser Leu Gln
                85                  90                  95

Thr Ser Glu Ile Lys Glu Glu Glu Lys Glu Leu Arg Arg Thr Thr Tyr
            100                 105                 110

Lys Leu Gln Val Lys Asn Glu Glu Val Lys Asn Met Ser Leu Glu Leu
        115                 120                 125

Asn Ser Lys Leu Glu Ser Leu Leu Glu Glu Lys Ile Leu Leu Gln Gln
    130                 135                 140

Lys Val Lys Tyr Leu Glu Glu Gln Leu Thr Asn Leu Ile Gln Asn Gln
145                 150                 155                 160

Pro Glu Thr Pro Glu His Pro Glu Val Thr Ser Leu Lys Thr Phe Val
                165                 170                 175

Glu Lys Gln Asp Asn Ser Ile Lys Asp Leu Leu Gln Thr Val Glu Asp
            180                 185                 190

Gln Tyr Lys Gln Leu Asn Gln Gln His Ser Gln Ile Lys Glu Ile Glu
        195                 200                 205

Asn Gln Leu Arg Arg Thr Ser
    210                 215

<210> 27
<211> 526
<212> DNA
<213> Mus musculus

<400> 27
gctgtttcca gataaacaaa ctacaaacag ggttacagat gcttaatttt tcctcaagct 60
aaggaaactc acacttaagt ttaatgacaa acttaatcca tcaaaaacta aggtttattt 120
ataaatctct tatagtaaga aaaaatttaa atgatctttt aaaaacaggt aaataccttt 180
atcctgaaag aataattatc aatacagcaa atttaactat tttaacaatt ttgaaaatta 240
cctcctatta gttgctcatt tccttgattt cagttgccat tgatctaact caacatgggg 300
gagggttaca ttcgtgcaag ttagcacagc ttaatggtta actccattct cctgccaacc 360
ttgtgtgtgc atgcagcaaa tagccgtgta tataaagtca agaggtccag gtctccttac 420
aggagggaga agttccaaat tgcttaaaat tgaataattg agacaaaaaa tgcacacaat 480
taaattattc ctttttgttg ttcctttagt aattgcatcc agagtg 526

<210> 28
<211> 260
<212> DNA
<213> Mus musculus

<400> 28
acaaagcctg tggcattgca ccctaaaact tggccactcg ctccgccccc gggccccgcc 60
tccaatgctc tccctcccac tcccacaccc gctttataaa gtggggcttt aggtgcaacc 120
gtgaaacgct tatgagctac gggctccaga tcttcttctg caccagagca agtctaagtc 180
tgagccggct cccccagaac tccagctgct gggtcttgaa ctcctgcgtt ccggagtcct 240
agcgttgctg cacccaaggc 260

<210> 29
<211> 199
<212> DNA
<213> Mus musculus

<400> 29
ttgcatccag agtggatcca gacctttcat catttgattc tgcaccttca gagccaaaat 60

caagatttgc tatgttggat gatgtcaaaa ttttagcgaa tggcctcctg cagctgggtc 120

atggacttaa agattttgtc cataagacta agggacaaat taacgacata tttcagaagc 180

tcaacatatt tgatcagtc 199

<210> 30
<211> 24
<212> DNA
<213> Rattus norvegicus

<400> 30
caaagcttga aagtctactg gagg 24

<210> 31
<211> 21
<212> DNA
<213> Rattus norvegicus

<400> 31
ggttctgaac caagctggtc a 21

<210> 32
<211> 26
<212> DNA
<213> Rattus norvegicus

<400> 32
aagatggcgc tccaacacag agtcag 26

<210> 33
<211> 23
<212> DNA
<213> Mus musculus

<400> 33
ggctaaatag taaaaccctg gcg                                                23

<210> 34
<211> 23
<212> DNA
<213> Mus musculus

<400> 34
gtgcttgctg tctttccagt ctt                                                23

<210> 35
<211> 25
<212> DNA
<213> Mus musculus

<400> 35
aggatctcgt cgtgacccat ggcga                                              25

<210> 36
<211> 26
<212> DNA
<213> Mus musculus

<400> 36
gagcggcgat accgtaaagc acgagg                                             26

<210> 37
<211> 23
<212> DNA
<213> Mus musculus

<400> 37
tcccaagtca cacaagaact gac                                              23

<210> 38
<211> 23
<212> DNA
<213> Mus musculus

<400> 38
catccagaag gctaaagaag gca                                              23

<210> 39
<211> 17
<212> DNA
<213> Mus musculus

<400> 39
tgacctggag aatctct                                                     17

<210> 40
<211> 17
<212> DNA
<213> Mus musculus

<400> 40
catccaggct ctctggc                                                     17

<210> 41
<211> 17
<212> DNA
<213> Mus musculus

<400> 41
tgacctggag aatctcc                                                    17

<210> 42
<211> 37
<212> DNA
<213> Artificial Sequence

<220>
<223> Description of Artificial Sequence: PCR primer
      with Kozak's consensus sequence

<400> 42
gccaccatgg tcacaattaa gctccttctt tttattg                              37

<210> 43
<211> 36
<212> DNA
<213> Artificial Sequence

<220>
<223> Description of Artificial Sequence: PCR primer
      with the specific sequence for a restriction
      endonuclease XhoI

<400> 43
tctagagcct cgttcattca aagctttctg aatctg                               36

<210> 44
<211> 26
<212> DNA
<213> Artificial Sequence

<220>
<223> Description of Artificial Sequence: PCR sense
      primer to amplify a DNA fragment encoding a part
      of GST-CC, GST-CCdel or GST-C1

<400> 44
cggaattccc tccagaattg atcaag                                          26

<210> 45
<211> 27
<212> DNA
<213> Artificial Sequence

<220>
<223> Description of Artificial Sequence: PCR antisense
      primer to amplify a DNA fragment encoding a part
      of GST-CC

<400> 45
ccgctcgaga ctagtccttc tgagctg                                         27

<210> 46
<211> 27
<212> DNA
<213> Artificial Sequence

<220>
<223> Description of Artificial Sequence: PCR antisense
      primer to amplify a DNA fragment encoding a part
      of GST-CCdel

<400> 46
ccgctcgagt tgactatgct gttggtt                                         27

<210> 47
<211> 27
<212> DNA
<213> Artificial Sequence

<220>
<223> Description of Artificial Sequence: PCR antisense
      primer to amplyfy a DNA fragment encoding a
      part of GST-C1

<400> 47
ccgctcgagg ttagttagtt gctcttc                                          27


<210> 48
<211> 24
<212> DNA
<213> Artificial Sequence

<220>
<223> Description of Artificial Sequence:
      Oligonucleotide for insertion of a sequence
      encoding histidine tag

<400> 48
gatccgcatc atcaccatca ccat                                             24


<210> 49
<211> 24
<212> DNA
<213> Artificial Sequence

<220>

<223> Description of Artificial Sequence:
      Oligonucleotide for insertion of a sequence
      encoding a hisitidine tag

<400> 49
gatcatggtg atggtgatga tgcg                                              24

<210> 50
<211> 33
<212> DNA
<213> Artificial Sequence

<220>
<223> Description of Artificial Sequence:
      Sense/antisense primer to amplify a DNA fragment
      which is a part of a plasmid pME18S/KhAP5/His

<400> 50
gaattgatca gcatcatcac catcaccacg atc                                    33

<210> 51
<211> 27
<212> DNA
<213> Artificial Sequence

<220>
<223> Description of Artificial Sequence: PCR sense
      primer primer to amplify a DNA fragment encoding a
      part of GST-ARP4N

<400> 51
tcccccgggg gacccgtgca gtccaag                                           27

<210> 52
<211> 27
<212> DNA
<213> Artificial Sequence

<220>
<223> Description of Artificial Sequence: PCR antisense
      primer to amplify a DNA fragment encoding a part
      of GST-ARP4N

<400> 52
ccgctcgagc tggctttgca gatgctg                                    27


<210> 53
<211> 1480
<212> DNA
<213> Homo sapiens

<220>
<221> CDS
<222> (25)..(1407)

<400> 53
ggcacgagga aaatcaagat aaaa atg ttc aca att aag ctc ctt ctt ttt     51
                           Met Phe Thr Ile Lys Leu Leu Leu Phe
                             1               5

att gtt cct cta gtt att tcc tcc aga att gat caa gac aat tca tca     99
Ile Val Pro Leu Val Ile Ser Ser Arg Ile Asp Gln Asp Asn Ser Ser
 10                  15                  20                  25

ttt gat tct cta tct cca gag cca aaa tca aga ttt gct atg tta gac    147
Phe Asp Ser Leu Ser Pro Glu Pro Lys Ser Arg Phe Ala Met Leu Asp
                 30                  35                  40

gat gta aaa att tta gcc aat ggc ctc ctt cag ttg gga cat ggt ctt 195
Asp Val Lys Ile Leu Ala Asn Gly Leu Leu Gln Leu Gly His Gly Leu
            45                  50                  55

aaa gac ttt gtc cat aag acg aag ggc caa att aat gac ata ttt caa 243
Lys Asp Phe Val His Lys Thr Lys Gly Gln Ile Asn Asp Ile Phe Gln
        60                  65                  70

aaa ctc aac ata ttt gat cag tct ttt tat gat cta tcg ctg caa acc 291
Lys Leu Asn Ile Phe Asp Gln Ser Phe Tyr Asp Leu Ser Leu Gln Thr
    75                  80                  85

agt gaa atc aaa gaa gaa gaa aag gaa ctg aga aga act aca tat aaa 339
Ser Glu Ile Lys Glu Glu Glu Lys Glu Leu Arg Arg Thr Thr Tyr Lys
90                  95                  100                 105

cta caa gtc aaa aat gaa gag gta aag aat atg tca ctt gaa ctc aac 387
Leu Gln Val Lys Asn Glu Glu Val Lys Asn Met Ser Leu Glu Leu Asn
                110                 115                 120

tca aaa ctt gaa agc ctc cta gaa gaa aaa att cta ctt caa caa aaa 435
Ser Lys Leu Glu Ser Leu Leu Glu Glu Lys Ile Leu Leu Gln Gln Lys
            125                 130                 135

gtg aaa tat tta gaa gag caa cta act aac tta att caa aat caa cct 483
Val Lys Tyr Leu Glu Glu Gln Leu Thr Asn Leu Ile Gln Asn Gln Pro
        140                 145                 150

gaa act cca gaa cac cca gaa gta act tca ctt aaa act ttt gta gaa 531
Glu Thr Pro Glu His Pro Glu Val Thr Ser Leu Lys Thr Phe Val Glu
    155                 160                 165

aaa caa gat aat agc atc aaa gac ctt ctc cag acc gtg gaa gac caa 579
Lys Gln Asp Asn Ser Ile Lys Asp Leu Leu Gln Thr Val Glu Asp Gln
170                 175                 180                 185

tat aaa caa tta aac caa cag cat agt caa ata aaa gaa ata gaa aat 627
Tyr Lys Gln Leu Asn Gln Gln His Ser Gln Ile Lys Glu Ile Glu Asn
                190                 195                 200

cag ctc aga agg act agt att caa gaa ccc aca gaa att tct cta tct 675
Gln Leu Arg Arg Thr Ser Ile Gln Glu Pro Thr Glu Ile Ser Leu Ser
            205                 210                 215

tcc aag cca aga gca cca aga act act ccc ttt ctt cag ttg aat gaa 723
Ser Lys Pro Arg Ala Pro Arg Thr Thr Pro Phe Leu Gln Leu Asn Glu
        220                 225                 230

ata aga aat gta aaa cat gat ggc att cct gct gaa tgt acc acc att 771
Ile Arg Asn Val Lys His Asp Gly Ile Pro Ala Glu Cys Thr Thr Ile
    235                 240                 245

tat aac aga ggt gaa cat aca agt ggc atg tat gcc atc aga ccc agc 819
Tyr Asn Arg Gly Glu His Thr Ser Gly Met Tyr Ala Ile Arg Pro Ser
250                 255                 260                 265

aac tct caa gtt ttt cat gtc tac tgt gat gtt ata tca ggt agt cca 867
Asn Ser Gln Val Phe His Val Tyr Cys Asp Val Ile Ser Gly Ser Pro
                270                 275                 280

tgg aca tta att caa cat cga ata gat gga tca caa aac ttc aat gaa 915
Trp Thr Leu Ile Gln His Arg Ile Asp Gly Ser Gln Asn Phe Asn Glu
            285                 290                 295

acg tgg gag aac tac aaa tat ggt ttt ggg agg ctt gat gga gaa ttt 963
Thr Trp Glu Asn Tyr Lys Tyr Gly Phe Gly Arg Leu Asp Gly Glu Phe
        300                 305                 310

tgg ttg ggc cta gag aag ata tac tcc ata gtg aag caa tct aat tat 1011
Trp Leu Gly Leu Glu Lys Ile Tyr Ser Ile Val Lys Gln Ser Asn Tyr
    315                 320                 325

gtt tta cga att gag ttg gaa gac tgg aaa gac aac aaa cat tat att 1059
Val Leu Arg Ile Glu Leu Glu Asp Trp Lys Asp Asn Lys His Tyr Ile
330                 335                 340                 345

gaa tat tct ttt tac ttg gga aat cac gaa acc aac tat acg cta cat 1107
Glu Tyr Ser Phe Tyr Leu Gly Asn His Glu Thr Asn Tyr Thr Leu His
                350                 355                 360

cta gtt gcg att act ggc aat gtc ccc aat gca atc ccg gaa aac aaa 1155
Leu Val Ala Ile Thr Gly Asn Val Pro Asn Ala Ile Pro Glu Asn Lys
            365                 370                 375

gat ttg gtg ttt tct act tgg gat cac aaa gca aaa gga cac ttc aac 1203
Asp Leu Val Phe Ser Thr Trp Asp His Lys Ala Lys Gly His Phe Asn
        380                 385                 390

tgt cca gag ggt tat tca gga ggc tgg tgg tgg cat gat gag tgt gga 1251
Cys Pro Glu Gly Tyr Ser Gly Gly Trp Trp Trp His Asp Glu Cys Gly
    395                 400                 405

gaa aac aac cta aat ggt aaa tat aac aaa cca aga gca aaa tct aag 1299
Glu Asn Asn Leu Asn Gly Lys Tyr Asn Lys Pro Arg Ala Lys Ser Lys
410                 415                 420                 425

cca gag agg aga aga gga tta tct tgg aag tct caa aat gga agg tta 1347
Pro Glu Arg Arg Arg Gly Leu Ser Trp Lys Ser Gln Asn Gly Arg Leu
                430                 435                 440

tac tct ata aaa tca acc aaa atg ttg atc cat cca aca gat tca gaa 1395
Tyr Ser Ile Lys Ser Thr Lys Met Leu Ile His Pro Thr Asp Ser Glu
            445                 450                 455

agc ttt gaa tga actgaggcaa atttaaaagg caataattta aacattaacc 1447
Ser Phe Glu
        460

tcattccaag ttaatgtggt ctaataatct ggt                                1480

<210> 54
<211> 460
<212> PRT
<213> Homo sapiens

<400> 54
Met Phe Thr Ile Lys Leu Leu Leu Phe Ile Val Pro Leu Val Ile Ser
1               5                   10                  15
Ser Arg Ile Asp Gln Asp Asn Ser Ser Phe Asp Ser Leu Ser Pro Glu
            20                  25                  30
Pro Lys Ser Arg Phe Ala Met Leu Asp Asp Val Lys Ile Leu Ala Asn
        35                  40                  45
Gly Leu Leu Gln Leu Gly His Gly Leu Lys Asp Phe Val His Lys Thr
    50                  55                  60
Lys Gly Gln Ile Asn Asp Ile Phe Gln Lys Leu Asn Ile Phe Asp Gln
65                  70                  75                  80
Ser Phe Tyr Asp Leu Ser Leu Gln Thr Ser Glu Ile Lys Glu Glu Glu
                85                  90                  95
Lys Glu Leu Arg Arg Thr Thr Tyr Lys Leu Gln Val Lys Asn Glu Glu
            100                 105                 110
Val Lys Asn Met Ser Leu Glu Leu Asn Ser Lys Leu Glu Ser Leu Leu
        115                 120                 125
Glu Glu Lys Ile Leu Leu Gln Gln Lys Val Lys Tyr Leu Glu Glu Gln
    130                 135                 140
Leu Thr Asn Leu Ile Gln Asn Gln Pro Glu Thr Pro Glu His Pro Glu
145                 150                 155                 160
Val Thr Ser Leu Lys Thr Phe Val Glu Lys Gln Asp Asn Ser Ile Lys
                165                 170                 175
Asp Leu Leu Gln Thr Val Glu Asp Gln Tyr Lys Gln Leu Asn Gln Gln
            180                 185                 190
His Ser Gln Ile Lys Glu Ile Glu Asn Gln Leu Arg Arg Thr Ser Ile
        195                 200                 205
Gln Glu Pro Thr Glu Ile Ser Leu Ser Ser Lys Pro Arg Ala Pro Arg

210                 215                 220
Thr Thr Pro Phe Leu Gln Leu Asn Glu Ile Arg Asn Val Lys His Asp
225                 230                 235                 240
Gly Ile Pro Ala Glu Cys Thr Thr Ile Tyr Asn Arg Gly Glu His Thr
                245                 250                 255
Ser Gly Met Tyr Ala Ile Arg Pro Ser Asn Ser Gln Val Phe His Val
            260                 265                 270
Tyr Cys Asp Val Ile Ser Gly Ser Pro Trp Thr Leu Ile Gln His Arg
        275                 280                 285
Ile Asp Gly Ser Gln Asn Phe Asn Glu Thr Trp Glu Asn Tyr Lys Tyr
    290                 295                 300
Gly Phe Gly Arg Leu Asp Gly Glu Phe Trp Leu Gly Leu Glu Lys Ile
305                 310                 315                 320
Tyr Ser Ile Val Lys Gln Ser Asn Tyr Val Leu Arg Ile Glu Leu Glu
                325                 330                 335
Asp Trp Lys Asp Asn Lys His Tyr Ile Glu Tyr Ser Phe Tyr Leu Gly
            340                 345                 350
Asn His Glu Thr Asn Tyr Thr Leu His Leu Val Ala Ile Thr Gly Asn
        355                 360                 365
Val Pro Asn Ala Ile Pro Glu Asn Lys Asp Leu Val Phe Ser Thr Trp
    370                 375                 380
Asp His Lys Ala Lys Gly His Phe Asn Cys Pro Glu Gly Tyr Ser Gly
385                 390                 395                 400
Gly Trp Trp Trp His Asp Glu Cys Gly Glu Asn Asn Leu Asn Gly Lys
                405                 410                 415
Tyr Asn Lys Pro Arg Ala Lys Ser Lys Pro Glu Arg Arg Arg Gly Leu
            420                 425                 430
Ser Trp Lys Ser Gln Asn Gly Arg Leu Tyr Ser Ile Lys Ser Thr Lys
        435                 440                 445
Met Leu Ile His Pro Thr Asp Ser Glu Ser Phe Glu
    450                 455                 460

<210> 55
<211> 27
<212> DNA

<213> Artificial Sequence

<220>
<223> Description of Artificial Sequence: PCR sense
      primer to amplify a DNA fragment encoding a
      part of GST-ARP4Full

<400> 55
tcccccgggg gacccgtgca gtccaag                                          27

<210> 56
<211> 27
<212> DNA
<213> Artificial Sequence

<220>
<223> Description of Artificial Sequence: PCR antisense
      primer to amplify a DNA fragment encoding a
      part of GST-ARP4Full

<400> 56
ccgctcgagg gaggctgcct ctgctgc                                          27

<210> 57
<211> 18
<212> DNA
<213> Artificial Sequence

<220>
<223> Description of Artificial Sequence: Sequencing
      primer -21M13forward

<400> 57
tgtaaaacga cggccagt                                                    18

<210> 58
<211> 18
<212> DNA
<213> Artificial Sequence

<220>
<223> Description of Artificial Sequence: Sequencing
      primer M13reverse

<400> 58
caggaaacag ctatgacc                                                    18


<210> 59
<211> 28
<212> DNA
<213> Artificial Sequence

<220>
<223> Description of Artificial Sequence: PCR primer 1

<400> 59
aaggtaccgc tgtttccaga taaacaaa                                         28


<210> 60
<211> 35
<212> DNA
<213> Artificial Sequence

<220>
<223> Description of Artificial Sequence: PCR primer 2

<400> 60
tcaaatgatg aaaggtctgg atccactctg gatgc                                 35

<210> 61
<211> 31
<212> DNA
<213> Artificial Sequence

<220>
<223> Description of Artificial Sequence:
      Oligonucleotide to prepare a fragment inserted in
      the plasmid pGL13-1

<400> 61
aaggtaccaa ttgcatccag agtggatcca a                                    31

<210> 62
<211> 31
<212> DNA
<213> Artificial Sequence

<220>
<223> Description of Artificial Sequence:
      Oligonucleotide to prepare a fragment inserted in
      the plasmid pGL13-1

<400> 62
ttggatccac tctggatgca attggtacct t                                    31

<210> 63
<211> 29
<212> DNA
<213> Artificial Sequence

<220>

<223> Description of Artificial Sequence:PCR sense
      primer with the specific sequence for a
      restriction endonuclease KpnI

<400> 63
cggggtaccg agtgggtgct gggaagcaa                                        29

<210> 64
<211> 29
<212> DNA
<213> Artificial Sequence

<220>
<223> Description of Artificial Sequence:PCR antisense
      primer with the specific sequence for a
      restriction
      endonuclease HindIII

<400> 64
cccaagcttg ccttgggtgc agcaacgct                                        29

<210> 65
<211> 29
<212> DNA
<213> Artificial Sequence

<220>
<223> Description of Artificial Sequence:PCR sense
      primer with the specific sequence for a
      restriction endonuclease KpnI

<400> 65
cggggtacca caaagcctgt ggcattgca                                        29

<210> 66
<211> 29
<212> DNA
<213> Artificial Sequence

<220>
<223> Description of Artificial Sequence:PCR antisense
      primer with the specific sequence for a
      restriction
      endonuclease HindIII

<400> 66
cggggtaccc tcccccagaa ctccagctg                                    29

## Description

### [Field of the Invention]

The present invention relates to a novel method for testing a therapeutic or preventive agent for one or more diseases selected from hyperlipidemia, arteriosclerosis, and hyperglycemia.

Moreover, the present invention relates to a novel polypeptide useful for identifying or testing a therapeutic or preventive agent for one or more diseases selected from hyperlipidemia, arteriosclerosis, and hyperglycemia and a novel testing method using the polypeptide.

Moreover, the present invention relates to a novel method for testing a substance which regulates a lipase activity useful as a lipid reducing agent, especially a therapeutic or preventive agent for one or more diseases selected from hyperlipidemia, arteriosclerosis, and hyperglycemia.

Moreover, the present invention relates to a novel DNA which has a useful promoter activity in a test of a therapeutic or preventive agent for one or more of diseases selected from hyperlipidemia, arteriosclerosis, and hyperglycemia.

### [Background of the Invention]

Heart vessel disease is still a main cause of death in almost all of the advanced nations in the world. Atherosclerosis is a main cause of an acute coronary syndrome (unstable angina and acute myocardial infarction, ischemic sudden death), cerebral thrombosis, and cerebral infarction. In the United States, it is believed that 550,000 people die of myocardial infarction caused by atherosclerosis every year. In the statistics in 1991, the primary cause of death in Japan is heart vessel disease by atherosclerosis. It has become clear from epidemiology research that atherosclerosis is caused by a lot of risk factors such as hypercholesterolemia, hyperlipidemia, hypertension, and smoking.

An unusual increase in concentration of serum lipid is called hyperlipidemia or hyperlipemia. The blood serum lipids include a cholesterol (a cholesterol ester, a free cholesterol), a phospholipid (a lecithin and a sphingomyelin), a triglyceride (a neutral fat), a free fatty acid, and other sterols. Among them, increase of a neutral fat or cholesterol in blood may become a clinical problem (COMMON DISEASE SERIES No.19 hyperlipidemia, edited by Haruo Nakamura, October 10, 1991, published by Nankodo, Ausin et al., Circulation 101, 2777 -2782 (2000)).

The relation between hypercholesterolemia and ischemic heart disease incidence has been made clear. The position of hypercholesterolemia as an independent risk factor in ischemic heart disease is established, and prevention and treatment are performed with positive effects. Moreover, hypertriglyceridemia has been considered to be a risk factor in many diseases relevant to arteriosclerosis in recent years, although epidemiologically it is not as indicative as a blood serum cholesterol value (Rubins et al., N Engl J Med. 341, 410-418 (1999)). Therefore, suitable control of lipid value in blood is very important for prevention or treatment of many. diseases related to arteriosclerosis represented by ischemic heart disease, cerebral infarction, or the like (Brewer et al., Am J Cardiol. 83, 3F-12F (1999)).

Fibrin acid compounds, have been provided as medicines for reducing neutral fat, for example, clofibrate, fenofibrate, bezafibrate, gemfibrozil, or the like. It is known that they reduce neutral fat levels and cholesterol level in plasma, and are useful for prevention of ischemic heart disease in an individual with an increased cholesterol level (Havel, R. J. and Kane, J. P. (1973) 13 Ann. Rev. Pharmac. 287-308, Circulation 102, 21-27 (2000), Frick, M. H. et al. (1987) N. Eng. J. Med. 317. 1237-1245).

Medicines which trap bile acid and inhibit absorption thereof are known for reducing cholesterol values in blood, e.g. such as cholestyramine and colestipol (for example, U.S. Pat. No. 4027009). Also known are medicines which inhibit acyl-coenzyme A cholesterol acyl transferase enzymes (ACAT) and inhibit parenteral absorption of cholesterol, such as Melinamide (French patent No. 1476569); and medicines which suppress biosynthesis of cholesterol. Cholesterol biosynthesis depressor agents, in particular include lovastatin which inhibits 3-hydroxy 3-methyl glutaryl coenzyme A (HMG-CoA) reductase (U.S. Pat. No. 4231938), simvastatin (U.S. Pat. No. 4444784), pravastatin (U.S. Pat. No. 4346227), and the like.

A lipid reducing agent which has more effective, safe activity for reducing neutral fat will provide an improved medicine for prevention or treatment of hyperlipidemia, and for treatment or prevention of arteriosclerosis.

Diabetes is a disease with many causative factors, and is characterized by elevation of glucose level in plasma (hyperglycemia). Hyperglycemia which is not controlled increases the risk of premature mortality due to an increased danger of a disease of the microvascular and large artery systems, including nephropathy, retinopathy, hypertension, apoplexy, and cardiac disease. Therefore, control of glucose homeostasis is quite important as a method of treating hyperglycemia, and thus diabetes.

The sequence of cDNA shown in SEQ ID No.1 of the Sequence Listing which is used in the method of the present invention is disclosed in the Genbank database as a nucleotide sequence which encodes "angiopoietin-related protein 3", and the same nucleotide sequence is disclosed in International patent publication No. WO 99/55869 as a nucleotide sequence which encodes "zalpha5". However, the relationship between the gene with this nucleotide sequence and hyperlipidemia, arteriosclerosis or hyperglycemia is not known. Furthermore, the DNA sequence for control of transcription thereof is not known. Therefore, it was not known that a part of the nucleotide sequence which encodes the "angiopoietin-related protein 3 " or "zalpha5" or a promoter thereof is useful for testing a therapeutic or preventive agent for hyperlipidemia, arteriosclerosis, and hyperglycemia.

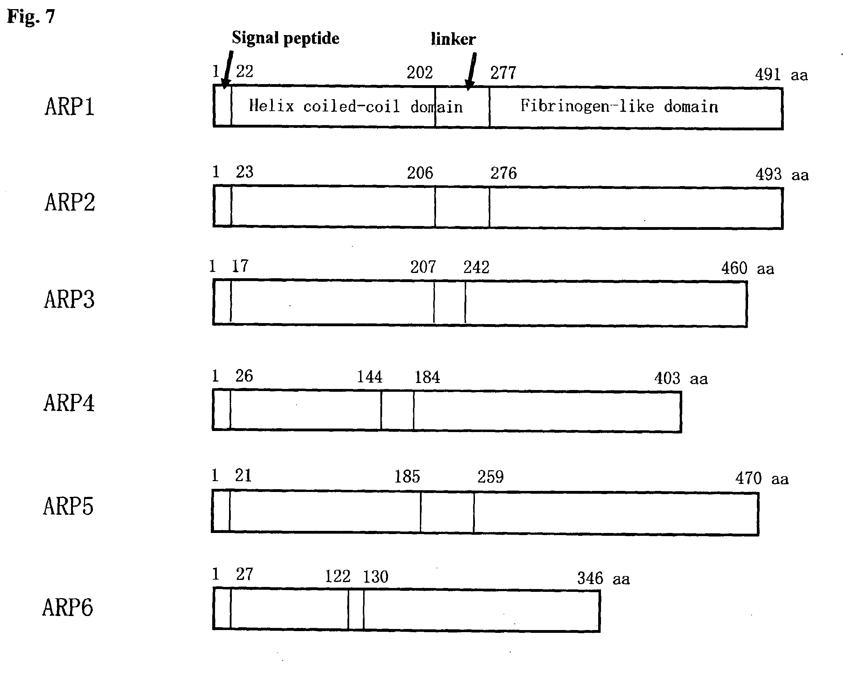

Six proteins are known within the family of "angiopoietin related proteins" ("angiopoietin related proteins 1, 2, 3, 4, 5, and 6"). The angiopoietin related proteins 1, 2, 3, 4, 5, and 6 are secreted proteins, characterized by a coil structure at the amino terminal end, and a fibrinogen like domain at the carboxyl terminal end, similar to angiopoietin (Fig. 7). For example, in "angiopoietin-related protein 3 ", it is believed that within 460 amino acids, the domain from the amino terminal to the 16th amino acid is a signal peptide, the domain from the 17th to the 207th amino acid is a helix domain in which a coil function is included, the domain from the 208th to the 241st amino acid is a linker domain, and the domain from the 242nd to the 460th amino acid is a fibrinogen connecting domain.

The "angiopoietin related protein 1" and the "angiopoietin related protein 2" are identified from human heart by PCR based on homology with angiopoietin (Kim, I., et al. (1999) FEBS Lett. 443: 353-356; Kim I., et al (1999) J. Biol. Chem. 274: 26523-26528). The homology between "angiopoietin related protein 1" and "angiopoietin related protein 2" is 59%. The homology of "angiopoietin related protein 1 and 2" to "angiopoietin 1" is 26-29%. The homology of "angiopoietin related protein 1 and 2" to "angiopoietin 2" is 34%. Although "angiopoietin related protein 1" is expressed in various organs, it does not have an endothelial-cell stimulus activity like an angiopoietin. On the other hand, "angiopoietin related protein 2" is expressed in heart, small intestine, spleen, and stomach and has a stimulus activity on an endothelial cell. However it does not bind to "TIE2" which is a receptor for angiopoietin 1 or angiopoietin 2.

On the other hand, "angiopoietin-related protein 3 " was found by a homology search using EST, and it has been shown that it is expressed only in liver and kidney (Conklin, D. et al. (1999) Genomics 62, 477-482). Although a function of the C-terminal side domain of "angiopoietin protein 3" has been reported (Conklin, D. et al. (1999) Genomics 62, 477-482), the function of the N terminal side domain thereof is not known. Furthermore, it is not known if protein having only the N terminal domain and lacking the C-terminal side domain has a function. Moreover, no partner factor of "angiopoietin-related protein 3 " is known, and a method of testing a medicine which regulates the concentration of neutral fat in blood in vivo using "angiopoietin-related protein 3 " and a partner factor thereof is not known.

The "angiopoietin related protein 4" is identified as a protein which participates in lipid metabolism or glucose homeostasis under control of PPARγ (Yoon, J. C. et al. (2000) Molec. Cell. Biol 20, 5343-5349), a lipid factor induced by fasting with a PPARα target gene (Kersten, S. 28488-28493) and a novel "angiopoietin related protein" originating from a liver which induces apotosis in an endothelial cell (Kim I. et al. (2000) Biochem. J. 346,603-610). Although it has been reported that "angiopoietin related protein 4" is expressed in white fat cells (adipocytes) and placenta, it has not been reported whether it has an angiopoietin-like activity.

Moreover, a specific DNA domain (a promoter / enhancer domain) involved in control of expression of "angiopoietin related protein 4" has not been identified, and it is not known if a promoter of this gene would be useful for testing of a therapeutic or preventive agent for hyperlipidemia, arteriosclerosis or hyperglycemia.

As for the "angiopoietin related protein 5", the gene has been recognised, but no function thereof is known.

The "angiopoietin related protein 6" was identified as a cornea-derived transcript 6 (CDT6), which gene is specifically expressed in a cornea (Peek, R. et al. (1998) Invest. Ophthalmol. Vis. Sci. 39. 1782-1788). The "angiopoietin related protein 6" prevented tumor proliferation in a mouse transplant cancer model (Peek, R. et al. (2001) J. Biol. Chem. epub ahead of print).

As described above, there is a possibility that "angiopoietin related proteins" have a different function from the vessel endothelium growth factor-like (VEGF-like) activity that angiopoietin has.

It has not been reported that a fibrinogen like domain on the carboxyl terminal end, which is common to the "angiopoietin related protein" family, is a domain which participates in binding to a "TIE receptor". No function is known for the coil structure on the amino terminus which is common to the "angiopoietin related proteins".

Furthermore, it was not known at all that a protein having only the amino-terminal domain and lacking a fibrinogen like domain at the side of the carboxyl terminal of a "angiopoietin related protein" family would have a function.

The purpose of the present invention is to provide a novel method for testing a therapeutic or preventive agent for one or more diseases selected from hyperlipidemia, arteriosclerosis, and hyperglycemia

Moreover, the purpose of the present invention is to provide a novel polypeptide useful for identifying or testing a therapeutic or preventive agent for one or more diseases selected from hyperlipidemia, arteriosclerosis, and hyperglycemia, and a novel testing method using the polypeptide.

Moreover, the purpose of the present invention is especially to provide a new method for testing a substance which regulates a lipase activity, especially a substance for reducing lipid concentration in blood, by raising LPL and/or HTGL activity which is useful as a preventive or therapeutic agent for hyperlipidemia and/or arteriosclerosis.

Moreover, the purpose of the present invention is to specify the promoter domain involved in transcriptional control of the "angiopoietin-related protein 3 " gene and the "angiopoietin related protein 4" gene, and provide a method for screening for a compound which regulates an activity of the promoter. More specifically, a new method for testing a therapeutic or preventive agent for hyperlipidemia, arteriosclerosis, or hyperglycemia and DNA used in the method are provided.

### [Disclosure of invention]

The present invention provides a method for testing the effect of a test substance as a therapeutic or preventive agent for one or more diseases selected from hyperlipidemia, arteriosclerosis, and hyperglycemia using as an index expression of a gene which participates in regulation of neutral-fat concentration in the blood of a mammal as shown in SEQ ID Nos. 1, 3, 11, 15, 17, 19, 21, 23, and 25 of the Sequence Listing; a nucleic acid probe, a primer, and an antibody being used in the method.

The inventors of the present invention have narrowed the position on a chromosome of a gene causing hyperlipidemia, in order to search for a target gene for a therapeutic or preventive agent for hyperlipidemia, by comparing the gene of a native hypolipidemia mouse with the gene of a hyperlipidemia mouse. As a result, they succeeded in identifying a gene highly expressed in the hyperlipidemia mouse. Although cDNA originating from this gene has been disclosed as a nucleotide sequence coding for "angiopoietin-related protein 3 " on a GenBank database as a result of homology search, the inventors have found that concentration of the neutral fat in blood is increased as a result of induction of expression of the gene which has this nucleotide sequence in hypolipidemia mouse or administration to the mouse of a human recombinant protein obtained by expression in animal cells and confirmed that the gene has a novel function which had not been reported earlier.

Furthermore, it was confirmed that progress of arteriosclerosis is controlled in a mouse obtained by crossing a congenic mouse in which the "angiopoietin-related protein 3 " gene is not normally expressed in nature, and an apolipoprotein E-deficient mouse which shows the symptoms of arteriosclerosis. Then, they succeeded in constructing a novel method for testing a therapeutic or preventive agent for arteriosclerosis using a system for detection of gene expression using the nucleotide sequence or a part thereof as a probe or a primer. Moreover, they succeeded in preparing a specific antibody to the polypeptide encoded by the nucleotide sequence and constructing a novel method for testing a therapeutic or preventive agent for arteriosclerosis using an experimental system in which the amount of production of the polypeptide is detected using the antibody.

Furthermore, it was confirmed that hyperglycemia status is improved in the mouse obtained by crossing a congenic mouse in which the "angiopoietin-related protein 3 " gene is not normally expressed in nature, and an apolipoprotein E-deficient mouse which shows the symptoms of arteriosclerosis or a mouse having a leptin variant gene. Then, they succeeded in constructing a novel method for testing a therapeutic or preventive agent for hyperglycemia using a system for detection of gene expression using the nucleotide sequence or a part thereof as a probe or a primer. Moreover, they succeeded in preparing a specific antibody to the polypeptide encoded by the nucleotide sequence and constructing a novel method for testing a therapeutic or preventive agent for hyperglycemia using an experimental system in which the amount of production of the polypeptide is detected using the antibody.

Moreover, these inventors noted the coil structure at the amino terminal end which the "angiopoietin related proteins" have in common, and found out that "angiopoietin related protein" and the polypeptide that has only the amino-terminal domain and lacks a fibrinogen-like domain at the carboxyl terminal end of the "angiopoietin related protein" had a function of raising a neutral fat concentration in blood in a mammal. Furthermore, the inventors have found that when "angiopoetin related protein" was administrated to blood, the fibrinogen-like domain at the carboxyl terminal end is cleaved, a polypeptide which has a helix domain at the amino terminal end is generated, which participates in raising fat concentration in blood. Furthermore, the inventors established a method for testing a therapeutic or preventive agent for one or more diseases selected from hyperlipidemia, arteriosclerosis, and hyperglycemia in vivo and in vitro, using the "angiopoietin related protein" or the polypeptide that has only the amino-terminal domain and lacks a fibrinogen-like domain at the carboxyl terminal side of the "angiopoietin related protein".

Although a helix domain, a linker domain, and a fibrinogen-like domain in the "angiopoietin related protein", can be identified, for example, by 3-dimensional crystal structure analysis, it can also be predicted using a computer program. As such, a suitable known computer program is GCG Package Ver. 10.2 (COIL SCAN) (Lupas, et al., Science 2252: 1162-1164. 1991.). A coil structure exists in the helix domain.

According to the above computer program or the results of the experiments, in "angiopoietin related protein 1", amino acids 1 to 21 of SEQ ID No. 18 of the Sequence Listing are a signal peptide, 22 to 202 are the helix domain where the coil function is included, 277 to 491 are the fibrinogen-like domain. In "angiopoietin related protein 2", amino acids 1 to 22 of SEQ ID No. 20 of the Sequence Listing are a signal peptide, 23 to 206 are the helix domain where the coil function is included, and 276 to 493 are fibrinogen-like domain. In "angiopoietin-related protein 3 ", amino acids 1 to 16 of SEQ ID No. 4 of the Sequence Listing are a signal peptide, 17 to 207 are the helix domain where the coil function is included, and 242 to 460 are the fibrinogen-like domain. In "angiopoietin related protein 4", amino acids 1 to 25 of SEQ ID No. 16 of the Sequence Listing are a signal peptide, 26 to 144 are the helix domain where the coil function is included, and 184 to 406 are the fibrinogen-like domain. In "angiopoietin related protein 5", amino acids 1 to 20 of SEQ ID No. 22 of the Sequence Listing are a signal peptide, 21 to 185 are the helix domain where the coil function is included, and 259 to 470 are the fibrinogen-like domain. In "angiopoietin related protein 6", amino acids 1 to 26 of SEQ ID No. 24 of the Sequence Listing are a signal peptide, 27 to 122 are the helix domain where the coil function is included, and 130 to 346 are the fibrinogen-like domain.

Moreover, these inventors found out that the lipase activity of LPL and/or HTGL was inhibited by "angiopoietin-related protein 3 ", as a result of performing a search for the partner factor of the "angiopoietin-related protein 3 " which acts in vivo to increase neutral fat concentration in blood. Furthermore, they have developed a method for testing in vitro a substance which regulates the activity of the "angiopoietin-related protein 3 " which raises the concentration of neutral fat in blood by means of the lipase activity inhibiting action of the LPL and/or HTGL.

Since the "angiopoietin-related protein 3 " inhibits LPL activity, if a substance is found which raises LPL activity substantially by controlling the interaction of this protein and LPL, a useful medicine for treatment or prevention of hyperlipidemia or arteriosclerosis which has a new action mechanism will be provided.

Moreover, the inventors have introduced a reporter gene wherein DNA encoding luciferase is connected to a DNA which consists of the upstream 418 nucleotides on 5'-end side, and the downstream 108 nucleotides on 3'-end side of the site which is predicted to be a transcription initiation site of the "angiopoietin-related protein 3 " gene in a mouse genome (nucleotide number 419 of SEQ ID No. 27 of the Sequence Listing), which is defined to be "1" (hereinafter shown as "-418 to +108", corresponding to nucleotide numbers 1 to 526 of SEQ ID No. 27 of the Sequence Listing) into human HepG2 cell, and measured the amount of production of luciferase, and thereby found that the "-418 to +108" domain of the "angiopoietin-related protein 3 " gene has a promoter activity. Moreover, the inventors of the present invention have also found that when the 5'-end side domain of the mouse "angiopoietin-related protein 3 " gene connected with a luciferase gene was shortened to "+93 to +108" (corresponding to nucleotide numbers 511 to 526 of SEQ ID No. 27 of the Sequence Listing), the promoter activity was lost. Furthermore, the inventors have found that a compound which regulates the promoter activity can be screened by using the above-mentioned DNA having the promoter activity.

Moreover, the inventors of the present invention have introduced a reporter gene wherein DNA encoding luciferase is connected to a DNA which consists of the upstream 90 nucleotides on 5'-end side, and the downstream 169 nucleotides on 3'-end side of the site which is predicted to be a transcription initiation site of the "angiopoietin related protein 4" gene in a mouse genome (nucleotide number 91 of SEQ ID No. 28 of the Sequence Listing), which is defined to be "1" (hereinafter shown as "-90 to +170", corresponding to nucleotide numbers 1 to 260 of SEQ ID No. 28 of the Sequence Listing), and measured the amount of production of luciferase, and thereby found that the "-90 to +170" domain of the "angiopoietin related protein 4" gene has a promoter activity. Moreover, the inventors of the present invention have also found that when the 5'-end side domain of the mouse "angiopoietin related protein 4" gene connected with a luciferase gene was shortened to "+99 to +170" (corresponding to nucleotide numbers 189 to 260 of SEQ ID No. 28 of the Sequence Listing), the promoter activity was lost.

Furthermore, the inventors have found out that a compound which regulates the promoter activity can be screened by using the above-mentioned DNA having the promoter activity.

The DNA of the present invention may include any nucleotide sequence of the 5'-end side upstream domain of the mouse "angiopoietin-related protein 3 " gene, as long as it has a promoter activity in a cell originating from mammalian liver. DNA which consists of a nucleotide sequence shown in nucleotide numbers 1 to 526 of SEQ ID No. 27 of the Sequence Listing is preferable, but the present invention is not limited thereto.

On the other hand, DNA which consists of the nucleotide sequence corresponding to +93 to +108 on the 5'-end side of the mouse "angiopoietin-related protein 3 " gene (nucleotide numbers 511 to 526 of SEQ ID No. 27 of the Sequence Listing) does not have a substantial promoter activity. Therefore, it is believed that a domain essential for promoter activity is included in the nucleotide sequence corresponding to -418 to +92 of the 5'-end side upstream domain of the mouse "angiopoietin-related protein 3 " gene (nucleotide numbers 1 to 510 of SEQ ID No. 27 of the Sequence Listing), and thus the promoter DNA of the present invention preferably contains at least a part of the nucleotide sequence shown by nucleotide numbers 1 to 510 of SEQ ID No. 27 of the Sequence Listing.

Moreover, DNA of the present invention may contain any nucleotide sequence of the 5'-end side upstream domain of the mouse "angiopoietin related protein 4" gene, as long as it has a promoter activity in a cell originating from a mammal. DNA which consists of a nucleotide sequence shown in nucleotide numbers 1 to 260 of SEQ ID No. 28 of the Sequence Listing is preferable, but the present invention is not limited thereto.

On the other hand, the nucleotide sequence corresponding to +99 to + 170 on the 5'-end side of a mouse "angiopoietin related protein 4" gene, (DNA corresponding to nucleotide numbers 189 to 260 of SEQ ID No. 28 of the Sequence Listing) does not have a substantial promoter activity. Therefore, it is thought that a domain essential for promoter activity is included in the nucleotide sequence corresponding to -90 to +98 of the 5'-end side upstream domain of the mouse "angiopoietin related protein 4" gene (corresponding to nucleotide numbers 1 to 188 of SEQ ID No. 28 of the Sequence Listing), and thus the promoter DNA of the present invention contains at least the nucleotide sequence more suitably shown by nucleotide numbers 1 to 188 of SEQ ID No. 28 of the Sequence Listing in part.

First, the present invention relates to a novel method for testing a therapeutic or preventive effect of a test substance for one or more diseases selected from hyperlipidemia, arteriosclerosis, and hyperglycemia comprising the following steps :
1) culturing cells originating from a mammal in the presence or absence of the test substance;
2) detecting the amount of expression of mRNA which has a nucleotide sequence of any one of the following sequences a) to e) (however, t in the sequence is read as u), in the cultured cells obtained in 1 above):
   a) the nucleotide sequence shown in nucleotide numbers 47-1411 of SEQ ID No. 1 of the Sequence Listing;
   b) the nucleotide sequence shown in nucleotide numbers 78-1457 of SEQ ID No. 3 of the Sequence Listing;
   c) the nucleotide sequence of DNA incorporated into a phagemid carried by transformed Escherichia coli E.coli pBK/m55-1 SANK72199 (FERM BP-6940);
   d) the nucleotide sequence of DNA incorporated into a phagemid carried by transformed Escherichia coli E.coli pTrip/h55-1 SANK72299 (FERM BP-6941);
   e) the nucleotide sequence which hybridizes to a polynucleotide consisting of an antisense sequence of a nucleotide sequence described in any of the above a) to d) under stringent conditions, and encodes a polypeptide having an activity of raising the concentration of neutral fat in blood; and
3) comparing the amount of the detected mRNA expression between cells cultured in the absence of the test substance and cells cultured in the presence of the test substance, as a result of the detection in the above step 2).

Secondly, the present invention relates to the above-mentioned method characterized in that the cultured cells originating from a mammal originate from the liver.

Thirdly, the present invention relates to the above-mentioned method of the first or second aspect characterized in that the cultured cells originating from a mammal originate from a primate or a rodent.

The fourth aspect of the present invention relates to the method of any one of the first to the third aspects characterized in that the cultured cells originating from a mammal originate from a human or a mouse.

The fifth aspect of the present invention relates to the method of any one of the first to the fourth aspects characterized in that the method for detecting the amount of expression of mRNA is a Northern blot, a dot blot or a slot blot.

The sixth aspect of the present invention relates to the method of any one of the first to the fourth aspects characterized in that the method for detecting the amount of expression of mRNA is RT-PCR.

The seventh aspect of the present invention relates to the method of any one of the first to the fourth aspects characterized in that the method for detecting the amount of expression of mRNA is a ribonuclease protection assay.

The eighth aspect of the present invention relates to the method of any one of the first to the fourth aspects characterized in that the method for detecting the amount of expression of mRNA is a run-on assay.

The ninth aspect of the present invention relates to the method of any one of the first to the fourth aspects characterized in that the method for detecting the amount of expression of mRNA is conducted using a gene chip produced with a group of complementary DNAs originating from a tissue or a cell of animals or partial sequences of the DNA.

The tenth aspect of the present invention relates to a method for testing a therapeutic or preventive effect of a test substance against one or more diseases selected from hyperlipidemia, arteriosclerosis, and hyperglycemia comprising the following steps:
1) culturing cells in the presence or absence of the test substance;
2) detecting the amount of production of a polypeptide, having an amino acid sequence encoded by the nucleotide sequence of any one of the following a) to e) or a part thereof, in the supernatant of the cultured cells obtained in 1) above using an antibody specifically binding to the polypeptide:
   a) the nucleotide sequence shown in nucleotide numbers 47-1411 of SEQ ID No. 1 of the Sequence Listing;
   b) the nucleotide sequence shown in nucleotide numbers 78-1457 of SEQ ID No. 3 of the Sequence Listing;
   c) the nucleotide sequence of DNA incorporated into a phagemid carried by transformed Escherichia coli E.coli pBK/m55-1 SANK72199 (FERM BP-6940);
   d) the nucleotide sequence of DNA incorporated into a phagemid carried by transformed Escherichia coli E.coli pTrip/h55-1 SANK72299 (FERM BP-6941);
   e) the nucleotide sequence which hybridizes to a polynucleotide consisting of an antisense sequence of the nucleotide sequence described in any of the above a) to d) under stringent conditions, and encodes a polypeptide having the activity of raising neutral fat concentration in blood; and
3) comparing the amount of the detected polypeptide between the cells cultured in the absence of the test substance and the cells cultured in the presence of the test substance, as a result of the above mentioned step 2).

The eleventh aspect of the present invention relates to the method of the tenth aspect characterized in that the antibody which specifically binds to a polypeptide consisting of an amino acid sequence encoded by any one of the above-mentioned nucleotide sequences a) to e) or a part thereof is an antibody which specifically binds to a polypeptide consisting of the amino acid sequence shown in amino acid numbers 17-455 of SEQ ID No. 2 of the Sequence Listing or a part thereof, a polypeptide which consists of the amino acid sequence shown in amino acid numbers 19-455 of the same sequence as the above or a part thereof, or a polypeptide which consists of amino acid sequence shown in amino acid numbers 17-460 of SEQ ID No. 4 of the Sequence Listing or a part thereof.

The twelfth aspect of the present invention relates to a method of the tenth or eleventh aspect characterized in that the antibody which specifically binds to a polypeptide consisting of the amino acid sequence encoded by any one of the above-mentioned nucleotide sequences a) to e) or a part thereof is an antibody which specifically recognizes a polypeptide which consists of the amino acid sequence shown in amino acid numbers 1-13 of SEQ ID No. 9 of the Sequence Listing or amino acid numbers 1-14 of SEQ ID No. 10.

The thirteenth aspect of the present invention relates to a method of any one of the tenth to the twelfth aspects characterized in that the method for detecting the amount of production of polypeptide having an amino acid sequence encoded by any one of the above mentioned nucleotide sequences a) to e) or a part thereof, using an antibody which specifically recognizes the polypeptide, is Western blotting, a dot blotting or slot blotting.

The fourteenth aspect of the present invention relates to a method of any one of the tenth to the twelfth aspects characterized in that the method for detecting the amount of production of polypeptide having an amino acid sequence encoded by any one of the above mentioned nucleotide sequences a) to e) or a part thereof, using an antibody which specifically binds to the polypeptide, is a solid-phase enzyme immunoassay (ELISA method), or a radioisotope immunoassay (RIA method).

The fifteenth aspect of the present invention relates to the method of the fourteenth aspect characterized in that a monoclonal antibody produced by mouse hybridoma 45B1 is used as the antibody immobilized on a plate in the ELISA method. The hybridoma 45B1 was deposited on March 13, 2002 in International Patent Organism Depositary, National Institute of Advanced Industrial Science and Technology, at Central 6, 1-1-1 Higashi, Tsukuba, Ibaraki, Japan, and was accorded the accession number FERM BP-7963.

The sixteenth aspect of the present invention relates to a kit for testing a therapeutic or preventive agent for one or more diseases selected from hyperlipidemia, arteriosclerosis, and hyperglycemia comprising the following antibodies i) and/or ii)
i) an antibody which specifically binds to a polypeptide consisting of an amino acid sequence encoded by a nucleotide sequence of any one of the following a) to e) or a part thereof
   a) the nucleotide sequence shown in nucleotide numbers 47-1411 of SEQ ID No. 1 of the Sequence Listing;
   b) the nucleotide sequence shown in nucleotide numbers 78-1457 of SEQ ID No. 3 of the Sequence Listing;
   c) the nucleotide sequence of DNA incorporated into a phagemid carried by transformed Escherichia coli E.coli pBK/m55-1 SANK72199 (FERM BP-6940);
   d) the nucleotide sequence of DNA incorporated into a phagemid carried by transformed Escherichia coli E.coli pTrip/h55-1 SANK72299 (FERM BP-6941);
   e) the nucleotide sequence which hybridizes to a polynucleotide consisting of an antisense sequence of a nucleotide sequence described in any of the above a) to d) under stringent conditions, and encodes a polypeptide having an activity of raising a concentration of neutral fat in blood;
ii) an antibody which specifically binds to a polypeptide which consists of the amino acid sequence shown in amino acid numbers 1-13 of SEQ ID No. 9 of the Sequence Listing or amino acid numbers 1-14 of SEQ ID No. 10.

The seventeenth aspect of the present invention relates to a composition, for gene therapy of one or more diseases selected from hyperlipidemia, arteriosclerosis, and hyperglycemia, which contains DNA or RNA which has a nucleotide sequence including an antisense sequence of a nucleotide sequence consisting of 15 or 30 continuous nucleotides from the nucleotide sequence shown in nucleotide numbers 78-1457 of SEQ ID No. 3 of the Sequence Listing as an active ingradient.

The eighteenth aspect of the present invention relates to a polypeptide having a continuous amino acid sequence contained in the amino acid sequence shown in SEQ ID No. 12 of the Sequence Listing wherein the amino terminal is the amino acid of the amino acid number 17, and the carboxyl terminal is any one of amino acid numbers 147 to 207.

The nineteenth aspect of the present invention relates to the polypeptide of the eighteenth aspect of the present invention characterized by consisting of an amino acid sequence shown in amino acid numbers 17 to 207 of SEQ ID No. 12 of the Sequence Listing.

The twentieth aspect of the present invention relates to the polypeptide of the eighteenth aspect of the present invention characterized by consisting of an amino acid sequence shown in amino acid numbers 17 to 195 of SEQ ID No. 12 of the Sequence Listing.

The 21st aspect of the present invention relates to the polypeptide of the eighteenth aspect of the present invention characterized by consisting of an amino acid sequence shown in amino acid numbers 17 to 147 of SEQ ID No. 12 of the Sequence Listing.

The 22nd aspect of the present invention relates to a polypeptide which has a continuous amino acid sequence contained in the amino acid sequence shown in SEQ ID No. 16 of the Sequence Listing wherein the amino terminal is amino acid number 26 and the carboxyl terminal is amino acid number 143.

The 23rd aspect of the present invention relates to a polypeptide which has a continuous amino acid sequence contained in the amino acid sequence shown in SEQ ID No. 16 of the Sequence Listing wherein the amino terminal is amino acid number 26 and the carboxyl terminal is any one of amino acid numbers 144 to 183.

The 24th aspect of the present invention relates to a polypeptide which has a continuous amino acid sequence contained in the amino acid sequence shown in SEQ ID No. 18 of the Sequence Listing wherein the amino terminal is amino acid number 22 and the carboxyl terminal is any one of amino acid numbers 202 to 276.

The 25th aspect of the present invention relates to a polypeptide which has a continuous amino acid sequence contained in the amino acid sequence shown in SEQ ID No. 20 of the Sequence Listing wherein the amino terminal is amino acid number 23 and the carboxyl terminal is any one of amino acid numbers 206 to 275.

The 26th aspect of the present invention relates to a polypeptide which has a continuous amino acid sequence contained in the amino acid sequence shown in SEQ ID No. 22 of the Sequence Listing wherein the amino terminal is amino acid number 21 and the carboxyl terminal is any one of amino acid numbers 185 to 258.

The 27th aspect of the present invention relates to a polypeptide which has a continuous amino acid sequence contained in the amino acid sequence shown in SEQ ID No. 24 of the Sequence Listing wherein the amino terminal is amino acid number 27 and the carboxyl terminal is any one of amino acid numbers 122 to 129.

The 28th aspect of the present invention relates to a polypeptide which consists of an amino acid sequence contained in the amino acid sequence shown in SEQ ID No. 16 of the Sequence Listing wherein the amino terminal is amino acid number 26 and the carboxyl terminal is amino acid number 406.

The 29th aspect of the present invention relates to a polypeptide which consists of an amino acid sequence contained in the amino acid sequence shown in SEQ ID No. 18 of the Sequence Listing wherein the amino terminal is amino acid number 22 and the carboxyl terminal is amino acid number 491.

The 30th aspect of the present invention relates to a polypeptide which consists of an amino acid sequence contained in the amino acid sequence shown in SEQ ID No. 20 of the Sequence Listing wherein the amino terminal is amino acid number 23 and the carboxyl terminal is amino acid number 493.

The 31st aspect of the present invention relates to a polypeptide which consists of an amino acid sequence contained in the amino acid sequence shown in SEQ ID No. 22 of the Sequence Listing wherein the amino terminal is amino acid number 21 and the carboxyl terminal is amino acid number 470.

The 32nd aspect of the present invention relates to a polypeptide which consists of an amino acid sequence contained in the amino acid sequence shown in SEQ ID No. 24 of the Sequence Listing wherein the amino terminal is amino acid number 27 and the carboxyl terminal is amino acid number 346.

The 33rd aspect of the present invention relates to a polypeptide consisting of an amino acid sequence shown in SEQ ID No. 26 of the Sequence Listing.

The 34th aspect of the present invention relates to an analogue of a polypeptide which has an activity of raising the neutral fat concentration in the blood of a mammal having an amino acid sequence wherein one or several amino acid residues are deleted, inserted, added or replaced in one or several sites of an amino acid sequence of any one of the polypeptides of the 18th to 33rd aspects.

The 35th aspect of the present invention relates to a DNA which codes for any one of the polypeptides of the 18th to 33rd aspects.

The 36th aspect of the present invention relates to a DNA consisting of a continuous sequence included in the nucleotide sequence shown in SEQ ID No. 11 of the Sequence Listing wherein nucleotide number 18 is the 5'-end and any one of nucleotide numbers 458 to 638 is the 3'-end;

The 37th aspect of the present invention relates to a DNA consisting of a continuous sequence included in the nucleotide sequence shown in SEQ ID No. 11 of the Sequence Listing wherein nucleotide number 18 is the 5'-end and any one of nucleotide numbers 458 to 602 is the 3'-end.

The 38th aspect of the present invention relates to DNA consisting of a continuous sequence included in the nucleotide sequence shown in SEQ ID No. 15 of the Sequence Listing wherein nucleotide number 173 is the 5'-end and nucleotide number 601 is the 3'-end.

The 39th aspect of the present invention relates to a DNA consisting of a continuous sequence included in the nucleotide sequence shown in SEQ ID No. 15 of the Sequence Listing wherein nucleotide number 173 is the 5'-end and nucleotide number 1393 is the 3'-end.

The 40th aspect of the present invention relates to a DNA consisting of a continuous sequence included in the nucleotide sequence shown in SEQ ID No. 17 of the Sequence Listing wherein nucleotide number 434 is the 5'-end and any one of nucleotide numbers 1039 to 1261 is the 3'-end.

The 41st aspect of the present invention relates to a DNA consisting of a continuous sequence included in the nucleotide sequence shown in SEQ ID No. 17 of the Sequence Listing wherein nucleotide number 434 is the 5'-end and nucleotide number 1909 is the 3'-end.

The 42nd aspect of the present invention relates to a DNA consisting of a continuous sequence included in the nucleotide sequence shown in SEQ ID No. 19 of the Sequence Listing wherein nucleotide number 22 is the 5'-end and any one of nucleotide numbers 639 to 846 is the 3'-end.

The 43rd aspect of the present invention relates to a DNA consisting of a continuous sequence included in the nucleotide sequence shown in SEQ ID No. 19 of the Sequence Listing wherein nucleotide number 22 is the 5'-end and nucleotide number 1503 is the 3'-end.

The 44th aspect of the present invention relates to a DNA consisting of a continuous sequence included in the nucleotide sequence shown in SEQ ID No. 21 of the Sequence Listing wherein nucleotide number 242 is the 5'-end and any one of nucleotide numbers 796 to 1015 is the 3'-end.

The 45th aspect of the present invention relates to a DNA consisting of a continuous sequence included in the nucleotide sequence shown in SEQ ID No. 21 of the Sequence Listing wherein nucleotide number 242 is the 5'-end and nucleotide number 1654 is the 3'-end.

The 46th aspect of the present invention relates to a DNA consisting of a continuous sequence included in the nucleotide sequence shown in SEQ ID No. 23 of the Sequence Listing wherein nucleotide number 86 is the 5'-end and any one of nucleotide numbers 373 to 394 is the 3'-end.

The 47th aspect of the present invention relates to a DNA consisting of a continuous sequence included in the nucleotide sequence shown in SEQ ID No. 23 of the Sequence Listing wherein nucleotide number 86 is the 5'-end and nucleotide number 1048 is the 3'-end.

The 48th aspect of the present invention relates to DNA which consists of a nucleotide sequence shown in nucleotide numbers 18 to 62 of SEQ ID No. 25 of the Sequence Listing.

The 49th aspect of the present invention relates to a recombinant vector containing a DNA of any one of the 35th to the 48th aspects.

The 50th aspect of the present invention relates to the recombinant vector of the 49th aspect characterized in that it is an adenovirus vector.

The 51st aspect of the present invention relates to a host cell transformed by a recombinant vector of the 49th or 50th aspect.

The 52nd aspect of the present invention relates to a method for producing a polypeptide having an activity of raising neutral fat concentration in the blood of a mammal characterized by culturing a host cell according to the 51st aspect, and subsequently collecting from the culture the polypeptides which have an activity of raising neutral fat concentration in the blood of a mammal.

The 53rd aspect of the present invention relates to a method for testing a therapeutic or preventive agent for one or more diseases selected from hyperlipidemia, arteriosclerosis, and hyperglycemia comprising the following steps:
1) administering to a non-human mammal one or more polypeptide selected from a polypeptide according to the 18th to 34th aspects; and
2) measuring a concentration of neutral fat in the blood of the animal of 1) above.

The 54th aspect of the present invention relates to a method for testing a therapeutic or preventive agent for one or more diseases selected from hyperlipidemia, arteriosclerosis, and hyperglycemia comprising the following steps:
1) infecting a non-human mammal with adenovirus carrying a recombinant adenovirus vector according to the 50th aspect;
2) administrating a test substance to the animal obtained in 1); and
3) measuring a concentration of neutral-fat in the blood of the animal obtained in 2).

The 55th aspect of the present invention relates to a method for testing a therapeutic or preventive agent for one or more diseases selected from hyperlipidemia, arteriosclerosis, and hyperglycemia characterized in that a polypeptide consisting of an amino acid sequence shown in amino acid numbers 17 to 460 of SEQ ID No. 4 of the Sequence Listing, a polypeptide wherein 1 to 253 amino acid residues at the carboxyl terminal are absent from the polypeptide, or any one of the polypeptides of the 18th to 34th aspects, are allowed to exist together with the test substance in the system, and in which a lipoprotein lipase (hereinafter referred to as "LPL") and/or hepatic triacylglycerol lipase (hereinafter referred to as "HTGL") are allowed to react with the substrates thereof, and the test substance is investigated for an effect of suppressing inhibition of the LPL and/or HTGL activity of the polypeptide.

The 56th aspect of the present invention relates to a method for testing a substance for regulating LPL activity comprising the following steps:
1) preparing a mixture wherein the substance described in the following i-a to iii and the substance to be tested are allowed to exist together:
   i-a material containing LPL,
   ii-a material containing the substrate of LPL,
   iii a polypeptide consisting of an amino acid sequence shown in amino acid numbers 17 to 460 of SEQ ID No. 4 of the Sequence Listing, a polypeptide wherein any length of from 1 to 253 amino acid residues at the carboxyl terminal is absent from the polypeptide, or any one of the polypeptides of the 18th to 34th aspects.
2) preparing a mixture consisting of only the material i-a to iii of 1) above, independently from the mixture 1).
3) measuring an amount of fatty acid isolated from the substrate in the mixtures of 1 and 2 above respectively, and comparing the measured values.

The 57th aspect of the present invention relates to the method according to the 56th aspect characterized in that the material containing LPL is a culture supernatant of a fat cell culture originating from a mammal.

The 58th aspect of the present invention relates to the method according to the 56th or 57th aspect characterized in that the material containing LPL is a culture supernatant of a fat cell culture originating from a rodent or a primate.

The 59th aspect of the present invention relates to the method according to any one of the 56th to 58th aspects characterized in that the material containing LPL is a glycerol trioleic acid.

The 60th aspect of the present invention relates to a method for testing a substance for regulating LPL and/or HTGL activity comprising the following steps:
1) preparing a mixture wherein the substance described in the following i-b to iii and the substance to be tested are allowed to exist together:
   i-b material containing LPL and/or HTGL,
   ii-b material containing the substrate of LPL and/or HTGL, iii polypeptide consisting of an amino acid sequence shown in amino acid numbers 17 to 460 of SEQ ID No. 4 of the Sequence Listing, a polypeptide wherein any length of from 1 to 253 amino acid residues at the carboxyl terminal is absent from the polypeptide, or any one of the polypeptides according to the 18th to 34th aspects.
2) preparing the mixture consisting of only the material i-b to iii of 1) above, independently from the mixture 1).
3) measuring an amount of fatty acid isolated from the substrate in the mixtures of 1 and 2 above respectively, and comparing the measured values.

The 61st aspect of the present invention relates to the method according to the 60th aspect characterized in that the material containing LPL and/or HTGL is a plasma originating from a mammal to which heparin has been administered intravenously.

The 62nd aspect of the present invention relates to the method according to the 61st or 62nd aspect characterized in that the material containing the substrate of LPL and/or HTGL is a glycerol trioleic acid.

The 63rd aspect of the present invention relates to the method according to the 59th aspect characterized in that the material containing LPL and/or HTGL is a culture supernatant of a cultured fat cell originating from a mammal or plasma originating from a mammal to which heparin has been administered intravenously.

The 64th aspect of the present invention relates to the method according to the 59th to the 63rd aspect characterized in that the material containing LPL and/or HTGL is a culture supernatant of a fat cell culture originating from a rodent or a primate.

The 65th aspect of the present invention relates to the method according to any one of the 59th, the 63rd, or the 64th aspect characterized in that the material containing the substrate of LPL and/or HTGL is a glycerol trioleic acid.

The 66th aspect of the present invention relates to a DNA of any one of 1) to 3)
1) DNA which consists of a nucleotide sequence shown in SEQ ID No. 27 of the Sequence Listing wherein the 5'-end is nucleotide number 1 and the 3'-end is any one of the nucleotides shown as nucleotide number 510 to 526;
2) DNA which hybridizes to the DNA in 1) above under stringent conditions, and which has a promoter activity in a cell originating from mammalian liver;
3) DNA which includes a nucleotide sequence having 95% or more nucleotide sequence identity with DNA in 1 above), and which has a promoter activity in a cell originating from mammalian liver.

The 67th aspect of the present invention relates to a DNA including a nucleotide sequence shown as nucleotide numbers 1-510 of SEQ ID No. 27 of the Sequence Listing.

The 68th aspect of the present invention relates to a DNA which consists of a nucleotide sequence shown as nucleotide numbers 1-510 of SEQ ID No. 27 of the Sequence Listing.

The 69th aspect of the present invention relates to a DNA which consists of a nucleotide sequence shown as nucleotide numbers 1-526 of SEQ ID No. 27 of the Sequence Listing.

The 70th aspect of the present invention relates to a DNA shown in any one of the following 1) to 3)
1) DNA which consists of a nucleotide sequence shown in SEQ ID No. 28 of the Sequence Listing wherein the 5'-end is nucleotide number 1 and the 3'-end is any one of the nucleotides shown as nucleotide number 188 to 260;
2) DNA which hybridizes to the DNA of 1) above under stringent conditions, and which has a promoter activity in a cell originating from mammalian liver;
3) DNA which includes a nucleotide sequence having 95% or more nucleotide-sequence identity with DNA of 1) above, and which has a promoter activity in a cell originating from mammalian liver.

The 71st aspect of the present invention relates to a DNA including a nucleotide sequence shown as nucleotide numbers 1-188 of SEQ ID No. 28 of the Sequence Listing, and which has a promoter activity in a cell originating from a mammal.

The 72nd aspect of the present invention relates to a DNA which consists of a nucleotide sequence shown as nucleotide numbers 1-188 of SEQ ID No. 28 of the Sequence Listing.

The 73rd aspect of the present invention relates to an expression vector containing a DNA according to any one of the 66th to the 72nd aspects.

The 74th aspect of the present invention relates to an expression vector wherein a DNA according to any one of the 66th to the 72nd aspects is connected to the upstream side of a foreign gene in the same transcriptional direction as that of the sequence of the foreign gene and which can express the foreign gene in a mammalian cell.

The 75th aspect of the present invention relates to an expression vector according to the 74th aspect characterized in that the sequence of the foreign gene is a nucleotide sequence coding for a protein selected from the group consisting of luciferase, horseradish peroxidase, alkaline phosphatase, beta-galactosidase, and green fluorescent protein.

The 76th aspect of the present invention relates to an expression vector according to the 74th aspect characterized in that the sequence of the foreign gene is a nucleotide sequence coding for luciferase.

The 77th aspect of the present invention relates to a recombinant plasmid pGL 8-3 (FERM BP-7627).

The 78th aspect of the present invention relates to an expression vector pGL 11-4.

The 79th aspect of the present invention relates to a host cell transformed with an expression vector according to any one of the 73rd to 78th aspects.

The 80th aspect of the present invention is a method for testing a therapeutic or preventive effect of a test substance for one or more diseases selected from hyperlipidemia, arteriosclerosis, and hyperglycemia comprising the following steps:
1) culturing a host cell transformed with an expression vector according to any one of the 73rd to 78th aspects in the presence or absence of the test substance;
2) detecting or measuring the amount of production of a protein which is encoded by the foreign gene in the expression vector according to any one of the 73rd to 78th aspects;
3) comparing the result of the detection or measuring in the above step 2) between the cells cultured in the presence of the substance and the cells cultured in the absence of the substance.

The 81st aspect of the present invention relates to a method for identifying cDNA coding for a protein having a therapeutic or preventive effect in one or more diseases selected from hyperlipidemia, arteriosclerosis, and hyperglycemia comprising the following steps:
i) individually culturing a cell selected from the group consisting of:
   1) a host cell transformed with an expression vector according to any one of the 73rd to the 78th aspects (hereinafter referred to as "cell A");
   2) a cell which is obtained by transforming a cell A with a recombinant plasmid in which the cDNA to be tested is incorporated into a mammalian expression vector and which expresses the cDNA to be tested transiently or stably (hereinafter referred to "cell B");
   3) a cell obtained by transforming a cell A with a second recombinant plasmid, which does not express the cDNA to be tested (hereinafter referred to as "cell C");
ii) measuring the amount of production of the protein which is encoded by the foreign gene in the expression vector according to any one of the 73rd to 78th aspects in the cell A, the cell B and the cell C described in i), and comparing the results.

The 82nd aspect of the present invention relates to a method for detecting a protein which regulates "an angiopoietin-related protein 3 " gene promoter activity characterized in that a sample containing the protein is brought into contact with a DNA according to any one of the 65th to the 69th aspects and then binding of the protein to the DNA is detected.

The 83rd aspect of the present invention relates to a method for testing a substance having an activity of regulating "an angiopoietin-related protein 3 " gene promoter activity which comprises the following steps:
i) preparing a sample wherein a nuclear extract of a mouse cell and a test substance are added to a DNA according to any one of the 65th to the 69th aspects which is radio labeled, and preparing a sample wherein only a nuclear extract of a mouse cell is added to the DNA.
ii) subjecting each of the sample mixtures described in i) above to electrophoresis, and comparing the migration rate of the band found in the sample to which only the nuclear extract of a mouse cell is added, with that of the band found in the sample to which the test substance is also added.

The 84th aspect of the present invention relates to a nucleic acid which consists of a nucleotide sequence including DNA which consists of at least 10 continuous nucleotides in the nucleotide sequence shown by nucleotide numbers 1-526 of SEQ ID No. 27 of the Sequence Listing, the antisense sequence thereof, or a derivative thereof.

The 85th aspect of the present invention relates to a method for detecting a protein having an activity of regulating "an angiopoietin related protein 4" gene promoter activity characterized in that a sample containing the protein is brought into contact with a DNA according to any one of the 70th to the 72nd aspects and then binding of the protein to the DNA is detected.

The 86th aspect of the present invention relates to a method for testing a substance having an activity of regulating "an angiopoietin related protein 4" gene promoter activity which comprises the following steps:
i) preparing a sample wherein a nuclear extract of a mouse cell and a test substance are added to a DNA according to any one of the 70th to the 72nd aspects which is radio labeled, and preparing a sample wherein only a nuclear extract of a mouse cell is added to the DNA.
ii) subjecting each of the sample mixtures described in i) above to electrophoresis, and comparing the migration rate of the band found in the sample to which only the nuclear extract of a mouse cell is added, with that of the band found in the sample to which the test substance is also added.

The 87th aspect of the present invention relates to a nucleic acid which consists of a nucleotide sequence including DNA which consists of at least 10 continuous nucleotides in the nucleotide sequence shown by nucleotide numbers 1-260 of SEQ ID No. 28 of the Sequence Listing, the antisense sequence thereof, or a derivative thereof.

In the present invention, the word "hybridizes under stringent conditions" means that it hybridizes at 68 °C in a commercially available hybridization solution ExpressHyb Hybridization Solution (manufactured by Clontech), or it hybridizes under conditions equivalent thereto.

### I. Method using expression of a gene as an index

Specifically, one of the methods of the present invention comprises: measuring an amount of expression of a gene having the nucleotide sequence shown in nucleotide numbers 47-1411 of SEQ ID No. 1 of the Sequence Listing, the nucleotide sequence shown in nucleotide numbers 78-1457 of SEQ ID No. 3 of the Sequence Listing, the nucleotide sequence shown in nucleotide numbers 173-1390 of SEQ ID No. 15 of the Sequence Listing, the nucleotide sequence shown in nucleotide numbers 434-1909 of SEQ ID No. 17 of the Sequence Listing, the nucleotide sequence shown in nucleotide numbers 22-1500 of SEQ ID No. 19 of the Sequence Listing, the nucleotide sequence shown in nucleotide numbers 242-1652 of SEQ ID No. 21 of the Sequence Listing or the nucleotide sequence shown in nucleotide numbers 8-1045 of SEQ ID No. 23 of the Sequence Listing, or a gene encoding a polypeptide having the same activity of increasing lipid concentration in blood as that of the polypeptide encoded by a gene above, by detecting specifically the nucleic acid (mRNA) or the polypeptide; and selecting a test substance, which reduces the amount of expression of the gene, as a candidate substance for a therapeutic or preventive agent for one or more diseases selected from hyperlipidemia, arteriosclerosis, and hyperglycemia. Embodiments of detection of a nucleic acid and embodiments of detection of a polypeptide will be explained below separately.

### (A) Detection of a nucleic acid

### 1) Probe

In embodiments of the method in which nucleic acid hybridization is used for detecting a nucleic acid, the probe used is DNA or RNA, wherein the nucleotide sequence of the nucleic acid may be any one of the following sequences a) to e) (t in the sequence is read as u):
a) the nucleotide sequence shown in nucleotide numbers 47-1411 of SEQ ID No. 1 of the Sequence Listing;
b) the nucleotide sequence shown in nucleotide numbers 78-1457 of SEQ ID No. 3 of the Sequence Listing;
c) the nucleotide sequence of the DNA fragment incorporated into a phagemid carried by a transformed Escherichia coli E.coli pBK/m55-1 SANK72199 (FERM BP-6940);
d) the nucleotide sequence of the DNA fragment incorporated into a phagemid carried by a transformed Escherichia coli E.coli pTrip/h55-1 SANK72299 (FERM BP-6941);
e) the nucleotide sequence which hybridizes to a polynucleotide having an antisense sequence of the nucleotide sequence described in any one of a) to d) above under stringent conditions, and codes for a polypeptide having an activity of raising a concentration of neutral fat in blood. For example, there can be used any probe that hybridizes to a polyribonucleotide having the nucleotide sequence described in any one of a) to e) above under stringent conditions, to enable the polyribonucleotide to be detected, such as a polynucleotide having an antisense sequence of a nucleotide sequence described in any one of a) to e) above, a polynucleotide having a partial sequence consisting of at least 15 continuous nucleotides of the antisense sequence, a modified sequence of the antisense nucleotide or the like. Among them, the above-mentioned polynucleotide which has an antisense sequence of the nucleotide sequence described in a) above can be obtained as a labeled probe by directly labeling, according to a well known method, a cDNA which is cloned from a cDNA library originating from a mouse liver based on the nucleotide sequence information shown in SEQ ID No. 1 of the Sequence Listing according to a well known method such as a plaque-hybridization method, a colony-hybridization method, or a PCR method, or by labeling in a replication or transcription reaction by a polymerase reaction using a cDNA clone as a template. The polynucleotide which has an antisense sequence of a nucleotide sequence described in b) can be obtained as a labeled probe from a cDNA clone by performing the same operation based on the nucleotide-sequence information shown in SEQ ID No. 3 of the Sequence Listing from a cDNA library originating from a human liver. On the other hand, a polynucleotide having an antisense sequence of the nucleotide sequence shown in the above c) or d) can be obtained from a recombinant phagemid carried by any one of transformed Escherichia coli E.coli pBK/m55-1 SANK 72199 and E.coli pTrip/h55-1 SANK 72299, which were internationally deposited on Nov. 19, 1999 in Kogyo Gijutsuin Seimei-Kogaku Kogyo Gijutsu Kenkyujo (International Patent Organism Depositary, National Institute of Advanced Industrial Science and Technology, at Central 6, 1-1-1 Higashi, Tsukuba, Ibaraki, Japan) and were accorded the accession numbers FERM BP-6940 and FERM BP-6941 respectively.

Furthermore, a polynucleotide which has an antisense sequence of the nucleotide sequence in e) above can be obtained from a cDNA clone isolated by cloning according to a plaque hybridization method or a colony hybridization method from a cDNA library originating from any suitable mammalian source (preferably a liver) using a polynucleotide, which has the antisense sequence of any one of the nucleotide sequences shown in a) to d) obtained as mentioned above, as a probe.

A polynucleotide having a partial sequence consisting of at least 15 continuous nucleotides of the antisense sequence of a nucleotide sequence of any one of a) to e) above can be obtained by chemosynthesis if the polynucleotide consists of a few dozens of nucleotides. Alternatively, it can be obtained by subcloning any one of the partial sequences in a cDNA clone which has a nucleotide sequence of any one of a) to e) above obtained as mentioned above by PCR or the like, and then prepared as a probe having an antisense sequence by the same method as described above.

For example, a polynucleotide having a partial sequence which consists of continuous dozens of nucleotides in the antisense sequence of the nucleotide sequence shown in SEQ ID No. 1 of the Sequence Listing wherein several nucleotides are added, deleted, and/or added can also be used in the method of the present invention, as long as it hybridizes to the polyribonucleotide described in any one of a) to e) above under stringent conditions. Such a polynucleotide can be produced according to a chemosynthesis method or a variation introducing method using an enzyme reaction which is well-known in the technical field of the present invention, such as polymerase chain reaction (hereinafter referred to as "PCR").

Moreover, the probe used in the method of the present invention is not limited to a single nucleotide sequence. That is, in the method of the present invention, for example, a mixture of two or more kinds of nucleotide sequences which satisfy the above-mentioned requirements, may be used as a probe, or a multiplex detection using two or more of these kinds of nucleotide sequences individually, may be performed.

### 2) Primer for RT-PCR

Another embodiment of detection of the nucleic acids in the present invention is a method of amplifying a DNA fragment specifically by PCR after performing a reverse transcriptase reaction using mRNA as a template, so-called RT-PCR. In this method, in order to amplify the target nucleotide sequence specifically, an antisense primer complementary to the specific partial sequence of the intended mRNA and a sense primer complementary to the specific partial sequence of the sequence of cDNA generated with reverse transcriptase from the antisense primer are used.

The antisense primer used for both a reverse transcriptase reaction and PCR essentially consists of a continuous sequence of at least 18 nucleotides, preferably at least 23 nucleotides in an antisense sequence of the above-mentioned nucleotide sequence shown in any one of the above a) to e).

On the other hand, the sequence of the sense primer used in PCR consists of any partial sequence of at least 18 nucleotides, preferably at least 21 nucleotides, of the sequence on the 5'-end side of the 5'-most end of the sequence corresponding to the complementary strand of the above-mentioned antisense primer in a nucleotide sequence in any one of a) to e) above. However, if there are complementary sequences in a sense primer and an antisense primer respectively, there is a possibility of a nonspecific sequence being amplified as a result of the fact that the primers are annealed to each other, thus impeding detection of the specific gene. Therefore, it is preferable to design the primers so that such a combination may be avoided.

A nucleotide sequence which does not have a relationship with a nucleotide sequence of any one of a) to e) above may be added to these antisense primers and sense primers as a linker at the 5'-end of the nucleotide sequences specified above. However, the linker is preferably one which will not anneal non-specifically to a nucleic acid in a reaction mixture during reaction so that it may not impede detection of the specific gene.

### 3) Cells or animals for detection of gene expression

Cultured cells used in methods of the present invention should be a mammalian cell in which a gene is expressed having a nucleotide sequence of any one of a) to e) above. Cultured cells originating from mammalian liver (preferably primary-culture hepatocytes) are preferred, but artificially transformed cells can also be used, for example, cells to which is introduced a gene having a nucleotide sequence of any one of a) to e) above together with a promoter domain thereof (for example, CHO cells). As the mammalian source, a human, a mouse, a rat, or a hamster is preferred, and a human or a mouse is more preferred. Moreover, primary-culture hepatocytes of KK mouse (available from Nihon Kurea) which is a hyperlipidemic mouse are still more preferable, without being limited thereto. Moreover, when it is considered that it is more preferable than use of culture cells, it is also possible to adopt a method wherein a test substance is administered to a mammal, and expression of a gene having a nucleotide sequence of any one of a) to e) above in the organs or tissue cells extracted from the animal is measured. The organs or tissue in which expression of the gene should be detected may be those wherein the gene having a nucleotide sequence of any one of a) to e) above can be expressed, and is preferably liver. A preferred mammal in this embodiment may be a human, a mouse, a rat, or a hamster, and human or a mouse is more preferred. For example, the mouse used is preferably the above-mentioned KK mouse, but the present invention is not limited thereto.

The cultured cells used in a method of the present invention can be cultured under any of the conditions wherein a gene having a nucleotide sequence of any one of a) to e) can be expressed when the test substance is not added. For example, established culture conditions are known for cell culture, and the cells may be cultured under conditions in which the cells can express the gene having a nucleotide sequence of any one of a) to e) above.

### 4) Addition of test substance

A test substance is added to a culture medium during culturing of the above-mentioned cells, and the cells are cultured for a certain period. Examples of a test substance may include: a compound, a microbial metabolite, an extract of a plant or an animal tissue, derivatives thereof, or mixtures thereof or the like. Alternatively, there can also be used as a test substance a nucleic acid or a derivative thereof designed so as to suppress expression of a gene having a nucleotide sequence of any one of the a) to e) above (including an antisense nucleotide, a ribozyme, RNAi or the like). The dose and concentration of the test substance can be suitably determined. Alternatively, for example, a dilution series is prepared and it is used as two or more sorts of dose. The period of culturing in the presence of the test substance may also be suitably determined, and it is preferably from 30 minutes to 24 hours. When the test substance is administrated to a mammal, a dosage form such as oral administration, intravenous injection, intraperitoneal injection, transdermal administration, and a hypodermic injection can be suitably used depending on the physical properties of the test substance or the like.

### 5) Preparation of sample

It is preferable to extract RNA from cells cultured as mentioned above by dissolving the cells directly in a solvent for RNA extraction (for example, those containing a component which has an activity of inactivating ribonuclease, such as phenol), immediately after the end of culturing. Alternatively, the cells are recovered by a method of scraping the cells carefully with a scraper so that the cells may not be destroyed, or a method of gently dissociating the cells from culture medium material using proteases, such as trypsin, or the like. Then, they are transferred promptly to an RNA extraction process.

As a method of extracting RNA, there can be adopted a thiocyanic acid guanidine caesium chloride ultracentrifugal method, a thiocyanic acid guanidine hot phenol process, a guanidine hydrochloric acid method, an acidic thiocyanic acid guanidine-phenol-chloroform method (Chomczynski, P. and Sacchi, N., (1987), Anal. Biochem., 162, 156-159), or the like. An acidic thiocyanic acid guanidine phenol chloroform method is preferred.

A method of further purifying mRNA from the obtained RNA will be explained below. Since it is known that many mRNAs existing in the cytoplasm of an eukaryote have a poly (A) sequence at the 3'-end, mRNA can be purified using this property, by adsorbing mRNA to a biotinylated oligo (dT) probe, catching the mRNA on a paramagnetic bead to which streptavidin is fixed using binding between biotin/streptavidin, and eluting the mRNA after a washing operation. Moreover, there can also be used a method for purification comprising adhering mRNA to an oligo (dT) cellulose column, and then eluting it. Furthermore, the mRNA can also be further fractionalized by sucrose density gradient centrifugation or the like. However, for the method of the present invention, these mRNA purifying processes are not indispensable, and total RNA can also be used for a subsequent process as long as expression of the gene having the nucleotide sequence of any one of a) to e) above can be detected.

### 6) Immobilization of sample

When detection is carried out according to nucleic acid hybridization, in order to detect specifically a gene in the RNA sample obtained as mentioned above, the RNA sample is subjected to agarose electrophoresis, and then transferred to a membrane for a hybridization experiment (hereinafter referred to as "membrane") (Northern blotting), or immobilized on a membrane by a so-called dot blot method or a slot blot method wherein a sample is directly applied to a membrane. As the membrane, there can be used nitrocellulose membrane (for example, High bond-C pure (manufactured by Amersham Pharmacia) or the like), a positively charged nylon membrane (for example, High bond-N+ (manufactured by Amersham Pharmacia) or the like), or hydrophilic nylon membranes (for example, High bond-N/NX (manufactured by Amersham Pharmacia) or the like).

An agarose electrophoresis method for Northern blotting can be an agarose formamide gel electrophoresis method, a method of treating a sample with glyoxal and dimethyl sulfoxide to denature and migrating the sample in agarose gel produced with phosphoric acid buffer solution, an agarose gel methylmercury electrophoresis method (Maniatis, T. et al. (1982) in "Molecular Cloning A Laboratory Manual" Cold Spring Harbor Laboratory, NY.), or the like, but is not limited thereto.

As a so-called blotting method for transferring RNA from gel after electrophoresis to a membrane, there can be adopted a capillary transferring method (Maniatis, T. et al. (1982) in "Molecular Cloning A Laboratory Manual" Cold Spring Harbor Laboratory, NY.), a vacuum method, an electrophoresis method (Maniatis and T. et al. (1989) in "Molecular Cloning A Laboratory Manual" 2nd ed. Cold Spring Harbor Laboratory, NY.), or the like. Equipment for the dot blot method or the slot blot method are also commercially available (for example, Biodot (manufactured by Bio-Rad) or the like).

After blotting, the RNA transferred to the membrane is fixed thereto (the fixing operation may be different depending on the material of the membrane, and the operation for fixing is not necessary for some products).

### 7) Labeling of probe

In performing detection by nucleic acid hybridization, a method for labeling a probe for detecting a specific mRNA in a RNA sample immobilized as mentioned above and a detection method are described below:

### i) Radioisotope labeling

A labeled DNA probe is prepared by nick translation using a DNA fragment or a vector carrying it or the like as a material or a template, (for example, using a nick-translation kit (manufactured by Amersham Pharmacia), or the like), a random prime method (for example, using a multi-prime DNA labeling system (manufactured by Amersham Pharmacia) or the like), an end-labeling method (for example, using Megalabel (TAKARA SHUZO CO., LTD.), 3'-end labeling kit (manufactured by Amersham Pharmacia), or the like). Alternatively, a labeled RNA probe is prepared according to an in vitro transcription method using SP6 promoter or T7 promoter in a vector in which the DNA to be a template is subcloned. The probes can be detected by autoradiography using a X-ray film or an imaging plate, and quantification can also be conducted using densitometry (for example, a GS-700 imaging densitometer (manufactured by Bio-Rad) is used) in the case of the X-ray film, or the BAS2000II (manufactured by Fuji film) system in the case of the imaging plate, respectively.

### ii) Enzyme labeling

The DNA or RNA fragment is directly labeled with enzyme. Examples of the enzyme used for labeling include: alkaline phosphatase (AlkPhos Direct system for chemiluminescence (manufactured by Amersham Pharmacia) or the like is used) and Western horseradish peroxidase (ECL direct nucleic acid labeling and detection system (manufactured by Amersham Pharmacia), or the like are used). Detection of the probe is carried out by immersing the membrane in an enzyme reaction buffer solution containing a substrate which can make a catalytic reaction of the enzyme used for labeling detectable, for example, a substrate generating a colored substance, or a substrate emitting light as a result of the catalytic reaction. When a coloring substrate is used, detection can be visually conducted. When an emitting substrate is used, it is detected by autoradiography using a X-ray film or an imaging plate or by photography using an instant camera in a similar way to the case of a radioisotope labeling. Furthermore, when an emitting substrate is used, quantification can be carried out using a densitometry or a BAS2000II system.

### iii) Labeling with other molecules

Fluorescein labeling: The DNA fragment is labeled by a nick translation method, a random prime method, or a 3'-end labeling method (ECL 3'-oligo labeling system available from Amersham Pharmacia). Alternatively, RNA is labeled by in vitro transcription using an SP6 or T7 promoter; Biotin labeling: The 5'-end of the DNA is labeled (using an oligonucleotide biotin labeling kit available from Amersham Pharmacia), or the DNA fragment is labeled by a nick translation method, a random prime method, or the like; Digoxigenin modified dUTP labeling: The DNA fragment is labeled according to a nick translation method, a random prime method, or the like.

Detection of these labeled molecules comprises the operation of binding a molecule that specifically binds to the molecule which has been labeled with a radioisotope or an enzyme to a probe. In the case of fluorescein or digoxigenin, the molecule specifically binding is an anti-fluorescein antibody or an anti-digoxigenin antibody. In the case of the biotin, it is avidin or streptavidin. After they are bound to the probe, detection can be conducted using the labeled radioisotope or the enzyme in according to the method as described in the i) or ii) above.

### 8) Hybridization

Hybridization can be carried out by a well-known method in the technical field of the present invention. The relation between a composition of a hybridization solution or a washing solution and a hybridization temperature or a washing temperature in the present invention can be defined as described in the reference (baiojikken illustrated 4, p148, Shujunsha), but preferable conditions are as follows:
Hybridization solution: ExpressHyb Hybridization Solution (manufactured by Clontech);
Final concentration of the probe (in the case of a radio-labeled probe): 1 to 2 x 10⁶ cpm/ml (preferably 2 x 10⁶ cpm/ml);
Hybridization temperature and time: 68 °C, 1 to 24 hours. Membrane washing conditions:
i) Shaking operation is conducted in 0.1 to 5 x SSC (most preferably 2x SSC) 0.05 to 0.1 % (most preferably 0.05 %) sodium dodecyl sulfate (hereinafter referred to as "SDS"), at room temperature to 42 °C (most preferably room temperature) for 20 to 60 minutes (most preferably for 20 minutes), two to six times (most suitably 3 times), with changes of the washing solution.
ii) After the operation i), shaking operation is conducted in 0.1 x SSC, 0.1 % SDS, at 50 to 65 °C for 20 to 60 minutes two to six times, changing the washing solution. Alternatively, the shaking operation is in 0.2 to 0.5 x SSC, 0.1 % SDS, at 62 to 65 °C for 20 to 60 minutes two to six times, with changes of the washing solution. Most preferably, shaking in 0.1 x SSC and 0.1 % SDS at 50 °C for 20 minutes is performed three times, with changes of the washing solution.

After washing, detection and quantification appropriate for the method with which the probe was labeled are conducted as described in 7) above. Furthermore, the level of expression of a gene wherein it is known that an expression level per cell is stable (for example, probes for detecting gene expression of 23 kDa highly basic protein, a-tubulin, glyceraldehyde-3-dehydrogenase, hypoxanthine guanine phosphoribosyltransferase, phospholipase A2, ribosomal protein S9, and ubiquitin or the like are commercially available) in each sample is measured simultaneously in order to correct differences resulting from a difference in the amount of RNA between each sample or the like, and then the relative value of the expression level of the gene to be detected on the basis of the expression level of this stable expression gene is compared between the cell population to which the test substance is administered and the cell population to which it is not administered. Thereby, a more precise evaluation can be performed.

As a result, a test substance which lowers an expression level of a gene having the nucleotide sequence shown in any one of a) to e) above can be used as a therapeutic or preventive agent of one or more diseases selected from hyperlipidemia, arteriosclerosis, and hyperglycemia.

### 9) RT-PCR Reaction

The conditions of each reaction in the embodiment wherein a nucleic acid is detected by RT-PCR are shown below. Usually, a sample for detection by RT-PCR does not need to be purified to be Poly (A)⁺ RNA.

### i) Reverse transcriptase reaction

Example of composition of a reaction mixture (total amount of 20 µl):
Total RNA: an adequate amount;
Magnesium chloride: 2.5 to 5 mM (preferably 5 mM);
1 x RNA PCR buffer solution (10 mM of Tris hydrochloride (pH 8.3 to 9.0 at 25 °C (preferably 8.3)), 50 mM of potassium chloride);
dNTPs: 0.5 to 1 mM (preferably 1 mM);
Antisense primer: 1 µM (2.5 µM of a commercial random primer or oligo (dT) primer (12-20 nucleotides) an also be added as a substitute for an antisense primer);
Reverse transcriptase: 0.25 to 1 unit/µl (preferably 0.25 unit /µl);
adjusted to 20 µl with sterilized water.
Reaction temperature conditions:
after keeping at 30 °C for 10 minutes (only when using random primers), keep at 42 to 60 °C (preferably 42 °C) for 15 to 30 minutes (preferably for 30 minutes), and then heat at 99 °C for 5 minutes to deactivate the enzyme, and then cool at 4 to 5 °C (preferably 5 °C) for 5 minutes.

### ii) PCR

Example of reaction mixture composition:
Magnesium chloride: 2 to 2.5 mM (preferably 2.5 mM);
1 x PCR buffer solution (10 mM of Tris hydrochloride (pH 8.3 to 9.0 at 25 °C (preferably 8.3)), 50 mM of potassium chloride);
dNTPs: 0.2 to 0.25 mM (preferably 0.25 mM);
Antisense primer and sense primer: 0.2 to 0.5 µM (preferably 0.2 µM);
Taq polymerase: 1 to 2.5 units (preferably 2.5 units);
The PCR mixture is adjusted to 80 µl with sterilized water, and the PCR mixture is added to the reverse transcription reaction mixture wherein the reverse transcription reaction has been terminated, and then PCR is initiated.
Reaction temperature conditions: heating at 94 °C for 2 minutes first, heating at 90 to 95 °C (preferably 94 °C) for 30 seconds, and then 28 or 50 cycles (preferably 28 cycles) of a temperature cycle which comprises heating at 40 to 60 °C (preferably at a temperature within the range from a dissociation temperature (Tm) calculated from characteristics of the primer to a temperature 20 °C lower than it) for 30 seconds and at 70 to 75 °C (preferably 72 °C) for 1.5 minutes, and then cooled at 4 °C

After completion of PCR, the reaction mixture is subjected to electrophoresis and if a band of the intended size is amplified, this is detected. In order to perform quantitative detection, PCR is carried out under the same conditions using a cDNA clone previously diluted stepwise as a standard template DNA, and the number of temperature cycles which enables quantitative detection is as previously defined, or a part of the reaction mixture is sampled every 5 cycles, and each sample is subjected to electrophoresis. Moreover, for example, if radio-labeled dCTPs are used for the PCR reaction, quantification can be performed using radioactivity incorporated in the band as an index.

The detection results are compared between the sample originating from the cells cultured in the presence of the test substance and the sample originating from the cells cultured in the absence of the test substance. A test substance in which the expression level of the gene having the nucleotide sequence shown in any one of a) to e) above is reduced may serve as a therapeutic or preventive agent for one or more of diseases selected from hyperlipidemia, arteriosclerosis, and hyperglycemia.

### 10) Other methods

Other methods of measuring the expression level of a gene having a nucleotide sequence shown in any one of the a) to e) above are mentioned below.

### i) Ribonuclease protection assay (RNase protection assay):

When a labeled probe is hybridized only to mRNA having the nucleotide sequence shown in any one of a) to e) above (however, t in the sequence is read as u) to form a double stranded polynucleotide, and then a ribonuclease is added to the sample and incubated, mRNA to which the probe is hybridized will not be digested by the ribonuclease since a double strand is formed, and other RNA will be digested. Thereby, only a double stranded polynucleotide remains (if a probe is shorter than the mRNA to be detected, the double stranded polynucleotide corresponding to the chain length of the probe will remain). The expression level of the target gene is measured by quantifying the double stranded polynucleotide. Specifically, it is conducted, for example, according to the method described below.

To facilitate quantification, it is preferable to digest excess labeled probe with a ribonuclease in order to separate efficiently the labeled probe which remained without forming a double strand with the double stranded polynucleotide. However, if a ribonuclease which can also digest single stranded DNA is used, either DNA or RNA can be used as the labeled probe. The method for preparing the labeled probe is similar to the method described above in 1) to 7). The length of the probe used in this method is preferably about 50 to 500 nucleotides.

For example, a RNA probe is, for example, prepared according to the following method. A template DNA is first incorporated in a plasmid vector (for example, pGEM-T (manufactured by Promega) or the like) having a promoter of a bacteriophage (T7, SP6, T3 promoter, or the like). Then, the resultant recombinant plasmid vector is digested with a restriction enzyme at a position just downstream of the insert so that only one site is cut. In vitro transcription reaction is performed using the resultant linearised DNA as a template in the presence of the radio-labeled ribonucleotides. Enzymes such as T7, SP6, or T3 polymerase or the like, are used for this reaction depending on the promoter in the vector. The operation described above can be conducted, for example, using Riboprobe system-T7 and -SP6 or -T3 (all of which are manufactured by Promega).

The steps up to preparation of the RNA sample are the same as 3) to 5) above. A ribonuclease protection assay is carried out using an amount equivalent to 10 to 20 µg of the prepared total RNA samples and an excess amount corresponding to 5 x 10⁵ cpm of the labeled probe. This operation can be carried out using a commercial kit (HybSpeed RPA Kit, manufactured by Anbion). After the resultant sample digested with ribonuclease is subjected to electrophoresis using 4 to 12% polyacrylamide gel containing 8M urea, the gel is dried and subjected to radioautography using a X-ray film. By the above operations, the band of the double-stranded polynucleotide which is not digested with a ribonuclease can be detected, and quantified according to the method described in the above i) of 7). Furthermore, if the expression amount of the β-actin gene is measured simultaneously in order to correct differences resulting from differences in the amount of RNA between each sample or the like, more precise evaluation can be performed, as in the case of Northern blotting analysis.

Thus, the results of detection are compared between the sample originating from the cells cultured in the presence of the test substance, and the sample originating from the cells cultured in the absence of the test substance. A test substance in which an expression level of the gene having the nucleotide sequence shown in any one of a) to e) above is reduced can be used as a therapeutic or preventive agent for one or more of diseases selected from hyperlipidemia, arteriosclerosis, and hyperglycemia.

### ii) Run-on assay (See Run-on assay, Greenberg, M. E. and Ziff, E. B. (1984) Nature 311, 433-438 and Groudine, M. et al. (1981) Mol. Cell Biol. 1, 281-288):

This method is a method of isolating a nucleus from a cell and measuring the transcriptional activity of a target gene. Although it is not a method of detecting mRNA in a cell as described above, it is included in "a method of detecting an expression level of a gene" in the present invention. If a transcription reaction is performed within a test tube using an isolated cell nucleus, only reactions wherein transcription has already started before isolation of the nucleus and the generated mRNA chain elongates will progress. The transcriptional activity of the target gene at the time of isolation of a nucleus can be measured by adding radio-labeled ribonucleotide during the reaction to label the elongating mRNA, and detecting mRNA hybridized to a non-labeled probe. Since the time at which the influence of the test substance is most significantly expressed is decisive, the assay can be carried out on nuclei isolated at, for example, 30 minutes, 1 hour, 2 hours, 4 hours, 8 hours, and 24 hours after the addition of the test substance to the culture cells. The specific operating method is approximately the same as those described in the above-mentioned reference, except that the non-labeled probe is prepared in a similar way to the description of the above 1). Thus, the results of detection are compared between the sample originating from the cell cultured in the presence of the test substance, and the sample originating from the cell cultured in the absence of the test substance, and the test substance which reduces the transcriptional activity of a gene having a nucleotide sequence shown in any one of a) to e) above may serve as a therapeutic or preventive agent for one or more diseases selected from hyperlipidemia, arteriosclerosis, and hyperglycemia.

### iii) DNA chip analysis, DNA micro array analysis

### [1] Preparation of a sample for obtaining a probe

First, mRNAs of the sample originating from the cells cultured in the presence of the test substance, and the sample originating from the cells cultured in the absence of the test substance are extracted and purified. The steps up to RNA sample preparation are the same as those described in 3) to 5) above.

### [2] Labeling of a probe

Although total RNA that is not purified can also be used as a starting material for producing a probe for DNA chip analysis or DNA micro array analysis, it is more preferable to use Poly (A)⁺ RNA purified by the method described in 5) above.

A method for labeling a probe and a method for detecting it by detection according to a nucleic acid hybridization will be explained below.

A probe for analysis using a DNA chip manufactured by Affymetrix: a cDNA probe labeled with biotin is used according to a protocol provided with the DNA chip manufactured by Affymetrix.

A probe for analysis using a DNA micro array: a cDNA is fluorescently labeled by adding d-UTP labeled with fluorochromes (for example, Cy3, Cy5, or the like) or the like when cDNA is prepared from Poly (A)⁺ RNA according to a reverse transcription reaction. At this time, if the sample originating from the cells cultured in the presence of the test substance, and the sample originating from the cells cultured in the absence of the test substance are labeled with pigments different from each other, they can be used as a mixture in the later hybridization process.

A probe for analysis using a membrane filter: when cDNA is prepared from Poly (A)⁺ RNA according to a reverse transcription reaction, the probe is labeled by adding d-CTP labeled with a radioisotope (for example, ³²P, ³³P) or the like.

### [3] Immobilized sample

An immobilized sample hybridization to the labeled probe obtained in the above-mentioned process [2] can be exemplified as follows.

A gene chip on which is immobilized an antisense oligonucleotide synthesized based on an EST (expressed sequence tag) sequence or an mRNA sequence from a database (manufactured by Affymetrix) (Lipshutz, R. J. et al. (1999) Nature genet. 21, supplement, 20-24):

The above-mentioned EST sequences or mRNA sequences are most preferably those originating from an animal of the same species as the cells used for preparation of the probe, but are not limited thereto. However, a gene having a nucleotide sequence shown in any one of a) to e) above, a gene disclosed on the Genbank database as a nucleotide sequence encoding "angiopoietin-related protein 3 ", or a sequence including a partial sequence of any one of them should be immobilized.

A DNA micro array or a membrane filter on which a cDNA or a RT-PCR product produced from mRNA obtained from cells isolated from an internal organ tissue (preferably liver) of an animal which is the same as or closely related to the animal used for the preparation of the probe is immobilized:

The cDNA or the RT-PCR product is cloned by carrying out a reverse transcriptase reaction or PCR using the primer produced based on the sequence information of an EST database of the animal used as the material for the mRNA or the like. As a material for preparing a sample, there can be used the cells (preferably originating from liver) expressing a gene having a nucleotide sequence shown in any one of a) to e) above, or a gene disclosed on the Genbank database as a nucleotide sequence encoding "angiopoietin-related protein 3 ". The cDNA or the RT-PCR products also include those prepared by previously selecting mRNA having a different expression level using the subtraction method (Diatchenko, L. et al. (1996) Proc. Natl. Acad. Sci. U.S.A. 93, 6025-6030), the differential display method (Kato, K. (1995) Nucleic Acids Res. 23, 3685-3690) or the like. Moreover, as the DNA micro array and the filter, there can be used a commercially available one comprising a gene to be detected, namely a gene disclosed on the Genbank database as a nucleotide sequence encoding "angiopoietin-related protein 3 ". Alternatively, the DNA micro array or the filter on which the gene having the nucleotide sequence shown in any of a) to e) above is immobilized using a spotter can also be produced (for example, GMS417 arrayer manufactured by TAKARA SHUZO CO., LTD., or the like).

The probes prepared in the above [2] are allowed to hybridize to this immobilized sample separately under the same conditions, or simultaneously as a mixture (Brown, P. O. et al. (1999) Nature genet. 21, supplement, 33-37).

### [4] Analysis

Analysis using a DNA chip manufactured by Affymetrix: hybridization and analysis are carried out according to a protocol provided with a DNA chip manufactured by Affymetrix.

Analysis using a DNA micro array: for example, in the case that a commercial DNA micro array available from TAKARA SHUZO CO., LTD is used, hybridization and washing are performed according to a protocol of the company, and a fluorescent signal is detected with a fluorescence detection apparatus (for example, GMS418 array scanner manufactured by TAKARA SHUZO CO., LTD. or the like), and analysis is carried out.

Analysis using a filter: Hybridization may be performed by a well-known method in the technical field of the present invention. For example, hybridization and washing are carried out, and analysis is carried out using an analysis equipment (for example, Atlasimage (manufactured by Clontech)).

In any of the cases described above, the probe in the sample originating from the cells cultured in the presence of the test substance, and the probe in the sample originating from the cells cultured in the absence of the test substance are allowed to hybridize to an immobilized sample from the same origin. At this time, the conditions of hybridization other than the probe to be used are to be the same. As indicated in the above [2], when each probe is labeled with a different fluorochrome, a mixture of both probes can be hybridized to one immobilized sample (Brown, P. O. et al. (1999) Nature genet. 21, supplement, 33-37).

As a result of the analysis, there are measured the amounts of the probes which hybridize to the target gene of an immobilized sample, i.e., a gene having a nucleotide sequence shown in any one of a) to e) above, or a gene having a nucleotide sequence encoding "angiopoietin-related protein 3 ", with the probe in the sample originating from the cells cultured in the presence of the test substance and the probe in the sample originating from the cells cultured in the absence of the test substance. As a result, if the amount of the probe hybridized to the target gene in the sample originating from the cells cultured in the presence of the test substance is less than the amount of the probe hybridized to the target gene in the sample originating from the cells cultured in the absence of the test substance, the substance suppresses the expression of the gene having a nucleotide sequence shown in any one of the above-mentioned a) to e), and thus it can be used as a therapeutic or preventive agent for one or more of diseases selected from hyperlipidemia, arteriosclerosis, and hyperglycemia.

### iv) Others

The following methods can be mentioned as methods of detecting a test substance which reduces the amount of expression of a gene having the nucleotide sequence shown in any one of a) to e) above compared with that in the sample originating from the cells cultured in the absence of the test substance.

Namely, there can be used a technique known as "Taqman" wherein PCR and hybridization probing (hereinafter referred to as "probing") are combined in a single reaction (Holland, P. M. et al. (1991) Proc. Natl. Acad. Sci. USA 88, 7276-7280) or a technique wherein PCR and probing are combined in a single reaction (Higuchi et al., Biotechnology, 10, 413-417 (1992)). In the latter method, ethidium bromide which is a nucleic acid detection reagent emitting fluorescence when excited by ultraviolet rays is added to a PCR reaction mixture. Since the fluorescence of ethidium bromide increases in the presence of double stranded DNA, an increase in the fluorescence detected when an excitation light is irradiated may be correlated with an accumulation of a double stranded PCR product.

Furthermore, a method described in European patent application publication No. 0601889 can also be used as a method wherein amplification by PCR and probing are combined. Furthermore, it is also possible to quantify the amount of mRNA(s) using the Light cycler system (manufactured by Roche Diagnostics, see Japanese patent application laid-open publication (KOKAI) No. 2000-312600).

### (B) Detection of polypeptide

As another embodiment of the method of the present invention there is a method of detecting a polypeptide encoded by the gene which is to be detected in the above-mentioned embodiment for detecting gene expression. In this embodiment, a polypeptide in a sample is immobilized to the bottom of a well of 96 well plate, a membrane, or the like, and then detection using an antibody specifically recognizing the target polypeptide is performed. Among them, the method using a 96 well plate is generally a method called solid-phase enzyme immunoassay (ELISA method) or a method called radioisotope immunoassay (RIA method). As a method for immobilizing to a membrane, there is a method of transferring a polypeptide to a membrane by polyacrylamide gel electrophoresis of a sample (Western blotting) or a so-called dot blot method and a slot blot method wherein a sample or its diluent is directly adsorbed onto a membrane.

### 1) Preparation of sample

Conditions for the kind of cultured cells used in the above-mentioned embodiment of detecting a polypeptide are the same as those in the case of the above-mentioned embodiment for detecting gene expression. Moreover, there can also be adopted a method wherein a test substance is administrated to a mammal, and serum extracted from the animal is used as the sample. The preferable mammal in this case is a human, a mouse, a rat, or a hamster, and more preferably a human or a mouse. For example, although it is preferred that a KK mouse which is a hyperlipidemic mouse is used as the mouse, the present invention is not limited thereto. As for the conditions for culturing cells, the methods of administrating the test substance are also the same as those in the case of the embodiment of detecting gene expression. A test substance the activity of which is to be tested as a therapeutic or preventive agent for hyperlipidemia can be a compound, a microbial metabolite, an extract from a plant or animal tissue, derivatives thereof, or mixtures thereof.

As a material for preparing the sample for this embodiment, there can be used a culture supernatant or a cytoplasmic fraction of cells cultured in the presence or absence of the test substance; preferably a culture supernatant is used. The culture supernatant is collected after completion of culturing, subjected to filter filtration sterilization treatment, and then used for preparation of a sample for ELISA/RIA or Western blotting.

As a sample for ELISA/RIA, there can be used, for example a collected culture supernatant as it is, or suitably diluted with a buffer solution.

A preparation method of a sample for Western blotting (for electrophoresis) is as follows. First, protein is precipitated, for example by subjecting a culture supernatant to trichloroacetic-acid treatment, and the precipitate is obtained by centrifugal separation. The precipitate is washed with acetone cooled with ice, air-dried and dissolved in a sample buffer solution containing 2-mercaptoethanol for SDS-polyacrylamide gel electrophoresis (manufactured by Bio-Rad or the like).

In the case of a dot/slot blot method, the collected culture supernatant itself or suitably diluted with a buffer solution is directly adsorbed on a membrane, for example using blotting equipment.

### 2) Immobilization of sample

The sample is immobilized in order to detect specifically a polypeptide in the sample obtained as mentioned above. As a membrane used for Western blotting, the dot blot method or the slot blot method, there can be used nitrocellulose membranes (for example, manufactured by Bio-Rad or the like), nylon membranes (for example, High bond-ECL (Amersham Pharmacia) or the like), cotton membranes (for example, Blot absorbent filter (manufactured by Bio-Rad) or the like), or poly vinylidene difluoride (PVDF) membranes (for example, manufactured by Bio-Rad or the like).

As a so-called blotting method which is a method of transferring a polypeptide from a gel to a membrane after electrophoresis, there can be used a wet blotting method (CURRENT PROTOCOLS IN IMMUNOLOGY volume 2 ed by J. E. Coligan, A. M. Kruisbeek, D. H. Margulies, E. M. Shevach, and W. Strober), a semi-dry blotting method (see the above-mentioned CURRENT PROTOCOLS IN IMMUNOLOGY volume 2), or the like. Equipment for the dot blot method or the slot blot method is also commercially available (for example, Bio dot (manufactured by Bio-Rad) or the like).

In order to perform detection and quantification by the ELISA method/the RIA method, a sample or a dilution thereof (for example, diluted with phosphate buffered physiological saline (hereinafter referred to as "PBS") which contains 0.05% sodium azide) is put into a single use 96 well plate (for example, Immunopiate maxi soap (manufactured by Nunc) or the like), and left to stand at 4 °C to room temperature overnight or at 37 °C for 1 to 3 hours, to immobilize the polypeptide on the bottom of the well.

### 3) Antibody

An antibody used in this embodiment is one that specifically recognizes a polypeptide produced when a gene having a nucleotide sequence described in a) to e) above of (A) is expressed in animal cells, preferably a polypeptide which consists of an amino acid sequence shown in amino acid number 17-455 (preferably 19-455) of SEQ ID No. 2 of the Sequence Listing or a part thereof, or a polypeptide which consists of an amino acid sequence shown in amino acid number 17-460 of SEQ ID No. 4 or a part thereof. These antibodies are preferably, for example, an antibody which binds to any of the polypeptides which consist of an amino acid sequence shown as amino acid number 17-455 (preferably 19-455) of SEQ ID No. 2 of the Sequence Listing, or a polypeptide which consists of an amino acid sequence shown as amino acid number 17-460 of SEQ ID No. 4, but does not bind to any other protein originating from mice or human.

The antibody for this embodiment can be obtained by immunizing an animal with the protein that is to be the antigen or any suitable polypeptide having a sequence which is selected from the amino acid sequence thereof using a conventional method (for example, Shin-seikagaku jikkenkouza 1, protein 1, p.389-397, 1992), and then extracting and purifying the antibody produced in the body. Moreover, a monoclonal antibody can also be obtained by preparing a hybridoma by cell fusion of an antibody-producing cell which produces the antibody to the protein of the present invention and a myeloma cell according to a known method (for example, Kohler and Milstein, Nature 256, 495-497, 1975, and Kennet, R. ed., Monoclonal Antibody p.365-367, 1980, Prenum Press, N.Y.).

Generally, preparation of a monoclonal antibody involves the following steps:
a) purification of a biopolymer for use as the antigen;
b) preparation of antibody producing cells consisting of immunizing a mouse by injections of the antigen, collecting blood thereof and assaying for determining when the spleen is removed;
c) preparation of myeloma cells;
d) removing the spleen and fusing the spleen cells and myeloma cells;
e) selecting a hybridoma producing an antibody of interest;
f) preparing a single cell clone (cloning);
g) optionally, culturing the hybridoma cells, or growing the mouse into which the hybridoma cells have been transplanted, for large scale preparation of the monoclonal antibody; and
h) testing the biological activities, or assaying the properties as a labeling agent, of the monoclonal antibody thus prepared.

The monoclonal antibody of the present invention can also be produced in line with the above described steps. However, the method for preparing the antibody is not limited thereto. Antibody producing cells other than spleen cells, i.e. other myeloma and other mammalian antibody producing cells can also be used.

### (a) Preparation of antigen

Any one of the polypeptides according to Claims 10 to 12 can be used as the antigen. For example, a fused protein obtained by introducing DNA of the present invention into Escherichia coli and expression thereof, followed by purification, a protein purified from a supernatant of a serum free culture obtained by transfecting COS-1 cells or CHO cells with DNA of the present invention and the like are effective as an antigen.

### (b) Preparation of antibody producing cells

The protein purified from a supernatant of a serum free culture obtained by transfecting COS-1 cells or CHO cells with DNA of the present invention is mixed with an adjuvant, such as Freund's complete or incomplete adjuvant and alum, and an experimental animal is immunized therewith. A suitable experimental animal may be a BALB/c mouse, since all commonly used myelomas originating from mice originate from BALB/c mice, and the characteristics thereof have been studied in detail, and further it has an advantage that the resulting hybridoma can be proliferated in the abdomen of a BALB/c mouse, when both the antibody producing cell and myeloma originate from a BALB/c mouse, so that monoclonal antibody can be obtained from ascites without a complicated operation. However, the present invention is not limited thereto.

Suitable administration routes of the antigen for immunizing include the subcutaneous, intraperitoneal, intravenous, intradermal and intramuscular injection routes, with subcutaneous and intraperitoneal injections being preferred.

Immunization can be by a single dose or, by several repeated doses at appropriate intervals (preferably 1 to 5 weeks). Immunized animals are monitored for antibody titer in their sera, and an animal with a sufficiently high antibody titer is selected as the source of antibody producing cells. Selecting an animal with a high titer makes the subsequent process more efficient. Cells for the subsequent fusion are generally harvested from the animal 3 to 5 days after the final immunization.

Methods for assaying antibody titer include various well known techniques such as radioimmunoassay (hereinafter, referred to as RIA), solid-phase enzyme immunoassay (hereinafter, referred to as ELISA), fluorescent antibody assay and passive hemagglutination assay, with RIA and ELISA preferred for reasons of detection sensitivity, rapidity, accuracy and potential for automation.

Determination of antibody titer may be performed, for example, by ELISA, as follows. First, the antigen is adsorbed onto the surface of a solid phase, followed by blocking any remaining surface, to which antigen has not been bound, with a protein unrelated to the antigen, such as bovine serum albumin (hereinafter referred to as BSA). After washing, the well surfaces are contacted with serially diluted samples of the first antibody (for example, mouse serum) to enable binding of the antibody in the samples to the antigen. An enzyme-labeled, anti-mouse antibody, as the secondary antibody, is added to be bound to the mouse antibody. After washing, the substrate for the enzyme is added, and an antibody titer can then be estimated by determining absorbance change due to color development caused by the decomposed substrate or the like.

### (c) Preparation of myeloma cells

In general, cells from established mouse cell lines serve as the source of myeloma cells, for example, 8-azaguanine resistant mouse (derived from BALB/c) myeloma strains P3-x63Ag8-U1 (P3-U1) [Current Topics in Microbiology and Immunology, 81, 1-7, (1978)], P3/NSI/1-Ag4.1(NS-1) [European J. Immunology, 6, 511-519 (1976)], Sp2/O-Ag14 (SP-2) [Nature, 276. 269-270 (1978)], P3x63Ag8.653 (653) [Kearney, J. F., et al., J. Immunology, 123, 1548-1550 (1979)] and P3-x63Ag8 (x63) [Nature, 256, 495-497 (1975)].

The cell line selected is serially transferred into an appropriate medium, such as 8-azaguanine medium [RPMI-1640 medium supplemented with glutamine (1.5 mM), 2-mercaptoethanol (5x10⁻⁵ M), gentamicin (10 µg/ml), fetal calf serum (hereinafter referred to as FCS) (10 %), and 8-azaguanine], Iscove's Modified Dulbecco's Medium (hereinafter referred to as IMDM) or Dulbecco's, Modified Eagle Medium (hereinafter referred to as DMEM). The cells are then transferred to TIL medium (a medium wherein 500 ml of TIL Media I (Mennekiseibutsukenkyujo) to which 5 ml of PENICILLIN-STREPTOMYCIN SOLUTION (Sigma) and fetal calf serum (FCS 10 %) is added) 3 to 4 days prior to cell fusion, in order to ensure that at least 2 x 10⁷ cells are collected on the day of fusion.

### (d) Cell fusion

The antibody producing cells to be used are plasma cells and lymphocytes which are their precursor cells, which may be obtained from any suitable part of the animal. Typical areas are spleen, lymph nodes, peripheral blood, or any appropriate combination thereof, spleen cells most commonly being used.

After the last booster injection, tissue in which antibody producing cells are present, such as the spleen, is removed from a mouse having the predetermined antibody titer to prepare antibody producing cells such as spleen cells. The currently favored technique for fusion of the spleen cells with the myeloma cells prepared in step c), employs polyethylene glycol, which has relatively low cytotoxicity and the fusion procedure using it is simple. An example of this technique is as follows.

The spleen and myeloma cells are washed well with the medium or phosphate buffered saline (hereinafter referred to as PBS), and then mixed, so that the number ratio of spleen cells to myeloma cells is approximately between 5 : 1 and 10 : 1 , and then centrifuged. After the supernatant has been discarded and the pelleted cells sufficiently loosened, a mixture of polyethylene glycol (m.w. 1,000 to 4,000) and a medium is added dropwise with mixing, and then the mixture is centrifuged after several minutes. The supernatant is discarded again, and the pelleted cells are suspended in TIL medium, and then dispensed into the wells of culture plates. If proliferation of the cells is confirmed in each well, the cells are transferred to HAT medium (a medium wherein are 10⁻⁶ to 10⁻³ M of hypoxanthine, 10⁻⁸ to 10⁻⁷ M of aminopterin and 10⁻⁶ to 10⁻⁴ of thymidine added to TIL medium)

### e) Selection of hybridomas

The culture plate is incubated in a CO₂ incubator at 35 to 40 °C. Thereafter it is incubated in a CO₂ incubator at 35 to 40 °C for 14 days with the supplementary addition of HAT medium in an amount corresponding to a half amount every one to three days.

The myeloma strain used is resistant to 8-azaguanine, i.e., it is deficient in the hypoxanthine guanine phosphoribosyl transferase (HGPRT) enzyme, any unfused myeloma cells and any myeloma-myeloma fusions are unable to survive in HAT medium. On the other hand, fusions of antibody producing cells with each other, as well as hybridomas of antibody producing cells with myeloma cells can survive, the former only having a limited life. Accordingly, continued incubation in HAT medium results in selection of only the desired hybridomas.

The resulting hybridomas grown up into colonies are then transferred into HAT medium lacking aminopterin (hereinafter referred to as HT medium). Thereafter, aliquots of the culture supernatant are removed to determine anti-Fas antibody titer by, for example, ELISA.

Although the above selection procedure is exemplified using an 8-azaguanine resistant cell line, it will be appreciated that other cell lines may be used with appropriate modifications to the media used.

### (f) Cloning

Hybridomas which have been shown to produce specific antibodies, using a method similar to that described in step b) to determine antibody titer, are then transferred to another plate for cloning. Suitable cloning methods include: the limiting dilution method, in which hybridomas are diluted to one cell per well of a plate and then cultured; the soft agar method in which colonies are recovered after culturing in soft agar medium; a method of using a micromanipulator to separate a single cell for culture; and "sort-a-clone", in which single cells are separated by a cell sorter. Limiting dilution is generally the most simple and is commonly used.

The cloning procedure according to, for example, the limiting dilution method is repeated 2 to 4 times for each well demonstrating an antibody titer, and clones having stable antibody titers are selected as a monoclonal antibody producing hybridomas of the present invention.

### (g) Culture of hybridoma to prepare monoclonal antibody

The hybridoma obtained by the cloning is then cultured in normal medium, not in HT medium. Large-scale culture can be performed by roller bottle culture, using large culture bottles, or by spinner culture. The supernatant from the large-scale culture is purified by a suitable method, such as gel filtration, which is well known to those skilled in the art, to obtain a monoclonal antibody that specifically binds to the polypeptide of the present invention. The hybridoma may also be grown intraperitoneally in a syngeneic mouse, such as a BALB/c mouse or a Nu/Nu mouse, to obtain a large quantity of ascites containing the monoclonal antibody of the present invention.

An easy method using commercially available monoclonal antibody purification kits (for example, MAbTrap GII Kit; Pharmacia) of the like can also be used.

### (h) Assay of monoclonal antibody

Determination of the isotype and the subclass of the monoclonal antibody thus obtained may be performed as follows. Suitable identification methods include the Ouchterlony method, ELISA and RIA. The Ouchterlony method is simple, but requires concentration of the solution when the concentration of the monoclonal antibody is low. When ELISA or RIA is used, the culture supernatant can be reacted directly with an antigen adsorbed on a solid phase, and secondary antibodies corresponding to IgG subclasses can be used. There is an easier method wherein a commercial kit for identification, such as a Mouse Monoclonal antibody isotoping Kit (manufactured by Amersham) is used.

Quantification of protein may be performed by the Folin-Lowry method, or by calculation based on the absorbance at 280 nm [1.4 (OD280) = Immunoglobulin 1 mg/ml].

In addition, a hybridoma clone which produced a monoclonal antibody of the present invention stably in high concentration was named 45B1 and was deposited on March 13, 2002 in International Patent Organism Depositary, National Institute of Advanced Industrial Science and Technology, and was accorded the accession number FERM BP-7963.

Since the monoclonal antibody thus obtained has a high specificity for the protein of the present invention, and is produced uniformly by culturing the above-mentioned hybridoma, it can be used for isolation and purification of the protein of the present invention. For example, the protein of the present invention can be isolated and purified with this monoclonal antibody according to the immunoprecipitation method using an antigen-antibody reaction. That is, this monoclonal antibody is added to a solution containing the protein of the present invention, and kept at room temperature, and a protein G Sepharose (manufactured by Pharmacia) is added, mixed, and further kept at room temperature. The precipitate is obtained by centrifugation, washed several times with PBS containing 0.1 % Tween 20 or the like, and subjected to centrifugation again. The resultant precipitate is a complex consisting of the protein, the monoclonal antibody, and the protein G Sepharose. The precipitate is suspended in a suitable buffer solution and subjected to heat treatment, then subjected to centrifugation, and then the supernatant is collected. The protein can be isolated and purified easily from this supernatant by molecular sieve chromatography (gel filtration) or the like to dissociate the complex into each component.

Moreover, this monoclonal antibody can be used also as a ligand in an affinity-chromatography method for isolation and purification of the protein. The affinity-chromatography method is a very effective procedure to isolate and purify only the substance specifically combined with a carrier from a mixture and can decrease the required purification steps significantly as compared with a gel filtration of a mixture. In this affinity chromatography, a useful fixed monoclonal antibody can be produced by a method similar to that for fixing an enzyme. For example, a method using CNBr activated carrier can be generally used. Preferred examples of the carrier may include various materials generally used for chromatography such as a cellulose, agarose, a cross linked dextran, polyacrylamide, porous glass or those wherein a spacer is introduced into these carriers. Sepharose 4B (manufactured by Pharmacia), Affigel 10 (manufactured by Bio Rad), or the like are especially preferable. Actual purification of protein using fixed monoclonal antibody can be conducted by filling up a column with the monoclonal antibody, and flowing a solution which contains the protein through it. The protein is adsorbed to the column by this operation. The adsorbed protein is eluted in high purity by elution with a solvent, for example, glycine hydrochloride buffer solution (pH 2.5), a solution of sodium chloride, a solution of propionic acid, dioxane, ethylene glycol, a chaotropic salt, guanidine hydrochloride, or urea.

The monoclonal antibody of the present invention is not restricted to those originating from a mouse but can be any antibody originated from any other mammals, provided that it has an affinity with the protein of the present invention. Moreover, a monoclonal antibody of the present invention is not limited to those produced by a hybridoma, but can be those produced by genetic engineering. For example, a monoclonal antibody of the present invention may include a chimeric antibody, a humanized antibody, provided that it has an affinity with a protein of the present invention. Such an antibody can be produced according to a similar method to the method by Man Sung Co et al (Man Sung Co, et al. (1992) J. Immunol. 148, 1149-1154) or the like.

An antigen for producing an antibody used for this embodiment can be a polypeptide which consists of amino acid numbers 17-455 (preferably 19-455) of SEQ ID No. 2 of the Sequence Listing or a polypeptide consisting of a partial sequence of at least six continuous amino acids thereof, a polypeptide which consists of an amino acid sequence shown in amino acid numbers 17-460 of SEQ ID No. 4 or a polypeptide which consists of a partial sequence of at least six continuous amino acids thereof, a polypeptide which consists of an amino acid sequence shown in amino acid numbers 26-406 of SEQ ID No. 16 or a polypeptide which consists of a partial sequence of at least six continuous amino acids thereof, a polypeptide which consists of an amino acid sequence shown in amino acid numbers 22-491 of SEQ ID No. 18 or a polypeptide which consists of a partial sequence of at least six continuous amino acids thereof, a polypeptide which consists of an amino acid sequence shown in amino acid numbers 23-493 of SEQ ID No. 20 or a polypeptide which consists of a partial sequence of at least six continuous amino acids thereof, a polypeptide which consists of an amino acid sequence shown in amino acid numbers 21-470 of SEQ ID No. 22 or a polypeptide which consists of a partial sequence of at least six continuous amino acids thereof, a polypeptide which consists of an amino acid sequence shown in amino acid numbers 27-346 of SEQ ID No. 24 or a polypeptide which consists of a partial sequence of at least six continuous amino acids thereof, or a derivative wherein any suitable amino acid sequence or carrier is added thereto. Preferably, it is one obtained by fusing Keyhole limpet hemocyanin as a carrier to the C-terminal of a polypeptide which consists of amino acid numbers 1-14 of SEQ ID No. 9 of the Sequence Listing or to the N-terminal of a polypeptide which consists of the amino acid sequence shown in amino acid numbers 1-14 of SEQ ID No. 10.

A polypeptide which consists of an amino acid sequence shown in amino acid numbers 17-455 (preferably 19-455) of SEQ ID No. 2 of the Sequence Listing, or a polypeptide which consists of an amino acid sequence shown in amino acid numbers 17-460 of SEQ ID No. 4 can be obtained by making a host cell produce the polypeptide encoded by the nucleotide sequence shown in nucleotide numbers 47-1411 of SEQ ID No. 1 of the Sequence Listing or the nucleotide sequence shown in nucleotide numbers 78-1457 of SEQ ID No. 3 of the Sequence Listing by genetic manipulation. Specifically, the host cell of other prokaryotes or eukaryotes can be transformed by incorporating DNA having the above-mentioned nucleotide sequence into suitable vector DNA. It is possible to express the gene in each host by introducing into these vectors a suitable promoter and a sequence inducing gene expression.

Examples of a prokaryotic cell for use as a host include Escherichia coli, Bacillus subtilis or the like. In order to transform these host cells with the target gene, the host cell is transformed with a plasmid vector containing a replicon, namely a replication origin, originating from a species compatible with the host and a regulatory sequence. As the vector, a vector comprising a sequence affording selectivity of phenotype to a transformed cell is preferable.

For example, K12 or the like is a commonly used Escherichia coli strain and the plasmid pBR322 and pUC system is generally used as a vector, but the system used is not limited thereto, and other known strains and vectors can be used.

As a promoter in Escherichia coli, there can be used a tryptophan (trp) promoter, a lactose (lac) promoter, a tryptophan lactose (tac) promoter, a lipoprotein (lpp) promoter, a polypeptide-chain tension factor Tu (tufb) promoter, or the like. Any one of these promoters can be used for production of the target polypeptide.

As a Bacillus subtilis, for example, the 207-25 strain is preferable, and pTUB228 (Ohmura, K. et al. (1984) J. Biochem. 95, 87-93) or the like is used as a vector, but the system used is not limited thereto. Extracellular secretion expression can be attained by connecting a DNA sequence encoding a signal peptide sequence of the α-amylase of Bacillus subtilis.

Examples of eukaryotic host cells include: cells of a vertebrate, an insect, and yeast or the like. Examples of the cells of a vertebrate cell include: COS cells (Gluzman, Y. (1981) Cell 23, 175-182, ATCC CRL-1650) which are the cells of an ape, a dihydrofolic acid reductase deficient strain of a Chinese hamster ovary cell (CHO cell, ATCC CCL-61), or the like, but eukaryotic cells are not limited thereto (Urlaub, G. and Chasin, L. A. (1980) Proc. Natl. Acad. Sci. USA 77, 4126-4220).

Expression promoters for vertebrate cells can be those having a promoter upstream of the gene to be expressed, an RNA splicing site, a polyadenylation site and a transcription termination site, and furthermore having a replication origin if desired. Examples of the expression vector include pSV2dhfr having an initial promoter of SV40 (Subramani, S. et al. (1981) Mol. Cell. Biol. 1, 854-864) or the like, but the expression vector is not limited thereto.

When COS cells are used as host cells, expression vectors suitably comprise the SV40 replication origin, enabling autonomous replication in COS cells, a transcription promoter, a transcription termination signal and an RNA splicing site. The expression vectors can be used to transform the COS cells by a known method, such as a diethylaminoethyl (DEAE)-dextran method [cf. Luthman. H, and Magnusson. G. (1983), Nucleic Acids Res., 11, 1295-1308], a phosphate calcium-DNA co-precipitation method [Graham, F. L. and Van der Eb, A. J., (1973), Virology, 52, 456-457] and an electric pulse electroporation method [cf. Neumann, E., et. al., (1982), EMBO J, 1, 841-845] or the like, and thereby the intended transformed cells can be obtained. In the case that a CHO cell is used as the host cell, transformed cells stably producing the protein of the present invention can be produced by co-transfecting with an expression vector and a vector which can express the neo gene which functions as an antibiotic G418 resistance marker, for example, pRSVneo (Sambrook, J. et al. (1989): "Molecular Cloning A Laboratory Manual" Cold Spring Harbor Laboratory, NY), pSV2-neo (Southern, P. J. and Berg, P. (1982) J. Mol. Appl. Genet. 1, 327-341) or the like, and selecting G-418 resistant colonies.

In the case that an insect cell is used as a host cell, a strain cell line (Sf-9 or Sf-21) originating from the ovarian-cells of Spodoptera frugiperda of the Lepidoptera Phalaenidae, High Five cells originating from the ootid of Trichoplusia ni (Wickham, T. J. et al. (1992) Biotechnol. Prog. I: 391-396 or the like) are often used as a host cell. pVL1392/1393 using a polyhedrin protein promoter of autograph polyhedron virus (AcNPV) is often used as baculovirus transfer vector (Kidd, I. M. and V. C. Emery (1993). The use of baculoviruses as expression vectors, Applied Biochemistry and Biotechnology 42, 137-159). Furthermore, a vector using a promoter of baculovirus P10 or a basic protein can also be used. Furthermore, it is possible to express recombinant protein as a secretory protein by fusing the secretion-signal sequence of the envelope surface protein GP67 of AcNPV to the N-terminus of the target protein (Zhe-mei Wang, et al. (1998) Biol. Chem., 379, 167-174).

As an expression system using a eukaryotic microorganism as a host cell, yeast is generally known, and Saccharomyces yeasts, for example, baker's yeast Saccharomyces cerevisiae and the petroleum yeast Pichia pastoris are preferable. As an expression vector for eukaryotic microorganisms such as yeasts, the promoter of an alcohol dehydrogenase gene (Bennetzen, J. L. and Hall, B. D. (1982) J. Biol. Chem. 257, 3018-3025), the promoter of an acid-phosphatase gene (Miyanohara, A. et al. (1983) Proc. Natl. Acad. Sci. USA 80, 1-5), or the like can be used preferably. In order to express a secretory protein, it is possible to express a recombinant protein which has a secretion signal sequence and the cleavage site of the mature endogenous protease of the host cell, or a known N-terminal protease. For example, it is known that active form tryptase will be secreted in a medium by fusing the secretion-signal sequence of α factor of yeast and the cleavage site of KEX2 protease of petroleum yeast at the N-terminal end and then expressing them in a system where human mast cell tryptase of a trypsin type serine protease is expressed in petroleum yeast (Andrew, L. Niles, et al. (1998) Biotechnol. Appl. Biochem. 28, 125-131).

Transformants obtained by the above methods can be cultured using conventional methods, and thereby protein of the present invention can be produced either intra- or extracellularly. Suitable culture media include various commonly used media, depending on the host chosen. For example, for COS cells, there can be used RPMI-1640 and Dulbecco's Modified Eagle's Medium (hereinafter referred to as DMEM), which can be supplemented with, as desired, serum component such as fetal bovine serum.

The recombinant protein expressed intra- or extracellularly by the transformants as described above may be isolated and purified by various well known methods of separation according to the physical and chemical properties of the protein. Suitable specific methods of separation include: treatment with commonly used precipitating agents for protein; various liquid chromatography such as ultrafiltration, molecular sieve chromatography (gel filtration), adsorption chromatography, ion exchange chromatography, affinity chromatography and high performance liquid chromatography (HPLC), dialysis and combinations thereof. Moreover, it can be purified efficiently in a nickel affinity column by fusing six histidine residues with the expressed recombinant protein. The polypeptide of the present invention can be easily manufactured in large quantities in high yield and high purity by combining the above-mentioned methods.

The antibody obtained as mentioned above can be used for various immunoassays such as the RIA method, the ELISA method, a fluorescent antibody technique, and the passive-hemagglutination reacting method, immuno tissue dyeing, or the like.

### 4) Detection

The antibody obtained by the method of the above 3) is directly labeled, or used for detection as a primary antibody in cooperation with a labeled secondary antibody which recognizes this primary antibody specifically (recognizes an antibody of the animal used for production of the antibody).

Although the preferred substance for labeling is an enzyme (alkaline phosphatase or horseradish peroxidase) or a biotin (however, the operation of binding enzyme-labeled streptavidin to the biotin as a secondary antibody is added further), the labelling substance is not limited thereto. Previously labeled antibodies (or streptavidin) for the method of using a labeled secondary antibody (or labeled streptavidin) are commercially available. In the case of RIA, measurement is performed by a liquid scintillation counter or the like using antibodies labeled with a radioisotope such as I¹²⁵ or the like.

The quantity of the polypeptide which is an antigen is measured by detecting the activity of the enzymes used for labeling. As for alkaline phosphatase or horseradish peroxidase, substrates which are colored or emit light following catalytic action of these enzymes are commercially available.

When a substrate which is colored is used, it can be detected visually in a Western blot technique or a dot/slot blot method. In the ELISA method, a quantification is preferably carried out by measuring the extinction coefficient (wavelength for measurement depends on the substrate) in each well using a commercial microplate reader. Moreover, it is possible to quantify the antigen concentration in other samples by preparing a dilution series of the antigen preferably used in the above 3) for antibody production, and using it as a standard antigen sample and detecting it together with other samples at the same time to make a standard curve wherein the standard antigen concentration and the measured value are plotted.

On the other hand, when a substrate which emits light is used, detection can be conducted by radioautography using an X-ray film or an imaging plate or photography using an instant camera in a Western blot technique or a dot/slot blot method, and quantification by densitometry or BAS2000II system is also possible. Moreover, when using a light-emitting substrate in the ELISA method, enzyme activity is measured using light-emission micro plate readers (for example, manufactured by Bio-Rad or the like).

### 5) Measurement Operation

### i) In the case of Western blotting, a dot blot method, or a slot blot method

First, in order to prevent non-specific adsorption of an antibody, a membrane is immersed in a buffer solution containing a substance which inhibits non-specific adsorption (skimmed milk, bovine serum albumin, gelatin, polyvinylpyrrolidone, or the like) for a certain time (blocking) in advance. As the blocking solution, for example, phosphate buffered saline (PBS) or Tris buffered saline (TBS) containing 5% of skimmed milk and 0.05 to 0.1% of Tween 20 is used. Instead of skimmed milk, 1 to 10% of bovine serum albumin, 0.5 to 3% of gelatin, or 1% of polyvinylpyrrolidone or the like can be used. The blocking time is 16 to 24 hours at 4 °C, or 1 to 3 hours at room temperature.

Then, after washing the membrane with PBS or TBS containing 0.05 to 0.1% of Tween 20 (hereinafter referred to as "washing solution") to remove any excess blocking solution, the antibody produced by the method of the above 3) is incubated for a certain time in the solution diluted suitably with the washing solution to allow the antibody to bind with the antigen on the membrane. The dilution rate of the antibody at this time can be determined by conducting a preliminary Western-blotting experiment using the stepwise diluted recombinant antigen described in the above 3) as a sample. This antibody reaction operation is preferably performed at room temperature for one hour. The membrane is washed with a washing solution after completion of the antibody reaction operation. When a labeled antibody is used, the detection operation can be performed immediately at this time. When a non-labeled antibody is used, a second-antibody reaction is performed thereafter. The labeled second antibody is diluted 2000 or 20000 times by the washing solution, if it is a commercially available second antibody and if a suitable dilution rate is indicated in instructions provided, the antibody should be diluted in accordance with the instructions. The membrane from which the primary antibody is removed by washing is soaked in a solution of the secondary antibody at room temperature for one to 3 hours, and detection depending on the labeling method is conducted following washing with the washing solution. The washing is conducted by incubating the membrane in the washing solution for 15 minutes, changing the washing solution, incubating it for 5 minutes, and changing the washing solution again, and then incubating it for 5 minutes. If necessary, the washing solution is then changed and further washing takes place.

### ii) ELISA/RIA

First, in order to prevent nonspecific adsorption of the antibody to the bottom of a well of a plate on which the sample is immobilized by the method of the above 2), blocking is conducted in advance as in the case of Western blotting. The conditions of blocking are described in the paragraph concerning Western blotting.

Then, after washing the inside of the well with PBS or TBS containing 0.05 to 0.1% of Tween 20 (hereinafter referred to as "washing solution") to remove any excess blocking solution, a solution obtained by suitably diluting the antibody produced by the method of the above 3) with the washing solution is added into the wells, and incubated for a certain time, and thereby the antibody is bound to the antigen. The dilution of the antibody at this time can be determined by conducting a preliminary ELISA experiment using a recombinant antigen of the above 3) which is stepwise diluted as a sample. This antibody reaction step is preferably performed at room temperature for about 1 hour. The membrane is washed with the washing solution after completion of the antibody reaction step. When the antibody is labeled, the detection operation can be performed immediately at this time. When the antibody is not labeled, a second-antibody reaction is performed thereafter. The labeled second antibody should be diluted 2000 or 20000 times in the washing solution, in the case that it is a commercially available antibody and if a suitable dilution rate is indicated in attached instructions, it should be diluted in accordance with the instructions. The membrane from which the primary antibody is removed by washing is immersed in a solution of the secondary antibody at room temperature for 1 to 3 hours, and detection which depends on the labeling method used is conducted following washing with the washing solution. The washing is conducted by incubating the membrane in the washing solution for 15 minutes, changing the washing solution, incubating it for 5 minutes, and changing the washing solution again, and then incubating it for 5 minutes. If necessary, the washing solution is then changed and further washing takes place.

In the present invention, a so-called sandwich ELISA can be carried out by the method indicated below. First, in any one of the amino acid sequences shown in amino acid numbers 17-455 (preferably 19-455) of SEQ ID No. 2 of the Sequence Listing, and the amino acid sequences shown in amino acid numbers 17-460 of SEQ ID No. 4, two highly hydrophilic domains are chosen, and partial peptides consisting of six or more amino acid residues of each domain are synthesized, and then two kinds of antibodies for these partial peptides as antigen are obtained. One of these antibodies is labeled as described in 4) above. The antibody which is not labeled is immobilized on the bottom of a well of the 96 well plate for ELISA according to the method described in 2) above. After blocking, the sample liquid is put into the well and incubated at room temperature for one hour. After washing the inside of the well, the dilution of the labeled antibody is poured into each well. After washing the inside of the well again, a detection operation which depends on the labeling method used is conducted.

### 6) Evaluation

The detection results obtained by the method described above are compared between the sample originating from the cell cultured in the presence of the test substance, and the sample originating from the cell cultured in the absence of the test substance. As a result, the test substance for which the level of production of the polypeptide to which the antibody binds specifically is reduced may be a therapeutic or preventive agent for one or more diseases selected from hyperlipidemia, arteriosclerosis, and hyperglycemia. Moreover, a kit for investigating a therapeutic or preventive agent for hyperlipidemia can be provided by packaging the antibody produced by the method described in the above 3), and the reagents used for a series of the above-mentioned methods.

The polypeptide to be detected in the method of the present invention can be obtained according to the above-mentioned method of obtaining the antigen for preparation of the antibody production, or obtained by making animal cells to produce it using an adenovirus vector in which DNA having the nucleotide sequence shown in nucleotide numbers 47-1411 of SEQ ID No. 1 of the Sequence Listing, or the nucleotide sequence shown in nucleotide numbers 78-1457 of SEQ ID No. 3 of the Sequence Listing is incorporated. The method of constructing such a recombinant adenovirus vector can be a method using a commercial kit (for example, an adenovirus expression vector kit, TAKARA SHUZO CO., LTD.). The fact that the gene to be detected in the method of the present invention correlates closely with the neutral fat concentration in the blood of mammals can be proved by the fact that elevation of neutral fat concentration in blood is observed when a mammal, for example, a mouse is injected with a recombinant adenovirus having the recombinant adenovirus vector obtained as mentioned above, and the gene carried by the recombinant adenovirus vector is then expressed, and by the fact that the degree of expression of the nucleotide sequence shown in nucleotide numbers 47-1411 of SEQ ID No. 1 of the Sequence Listing in a congenitally hypolipidemic mouse is lower than in a hyperlipidemic mouse.

Moreover, the present invention also relates to a method for testing a therapeutic or preventive agent for hyperlipidemia using a non-human animal to which a foreign gene containing the nucleotide sequence described in any one of a) to e) above is introduced by genetic manipulation so that the gene can be expressed highly. The animal used for the method may be, for example, a KK/Snk mouse infected with the above-mentioned recombinant adenovirus, or a transgenic mouse obtained by introducing into the mouse DNA having the nucleotide sequence shown in nucleotide numbers 47-1411 of SEQ ID No. 1 of the Sequence Listing or nucleotide numbers 78-1457 of SEQ ID No. 3 of the Sequence Listing, but it is not limited thereto.

A transgenic animal is obtained by taking a fertilized ovum from an animal, introducing a gene into it, and transplanting it to a false-pregnant animal and making it develop to an individual mouse. An already-established method can be followed [See "Hasseikougaku mannual", edited by Tatsuji Nomura and Motonari Katsuki, 1987 annual publications], "Manipulating the Mouse Embryo and A Laboratory Manual" B. Hogan, F. Costantini and E. Lacy, translated by Kazuya Yamauchi, Hiroshi Toyoda, Hiroatu Mori, Yoichiro Iwakura, 1989, and Japanese patent publication (Kokoku) No.5-48093]. Specifically, in the case of a mouse, for example, an ovulation inducing agent is administrated to a female mouse (a mouse in which the neutral-fat concentration in the blood is lower than usual is preferable, for example, a KK/Snk mouse is preferable, but the mouse is not limited thereto), and crossed with a male of the same line, and then a pronuclear fertilized ovum is extracted from the oviduct of the female mouse on the next day. Subsequently, a DNA fragment solution is introduced into the pronucleus of the fertilized ovum using a minute glass tube (microinjection). Any regulatory genes for expressing the gene in the animal cell such as a promoter and an enhancer can be used, as long as they function in the cell of the introduced animal. The fertilized ovum to which DNA is introduced is transplanted to an oviduct of a false pregnant female mouse as an adoptive mother (Slc:ICR or the like), and progeny are delivered by natural birth or by cesarean section after about 20 days. A method of checking whether the thus-obtained animal has the introduced gene may be a method of extracting DNA from the tail of this animal or the like and performing PCR using a specific sense primer and antisense primer to the introduced gene and using the above DNA as a template, a method of digesting this DNA with several sorts of restriction enzymes followed by electrophoresis and blotting of the DNA on the gel onto a nitrocellulose membrane, a nylon film, or the like, and then performing a Southern blot analysis using all or a part of the labeled gene which is the same as the introduced gene as a probe. Moreover, it can be checked whether the introduced gene is actually expressed in the animal body by measuring the neutral fat concentration in the peripheral blood. When the introduced gene is actually expressed in the animal's body, the neutral fat concentration in the blood becomes higher than an animal into which the gene is not introduced.

The test substance is administrated to the transgenic animal thus obtained, and the substance which reduces neutral fat concentration in blood is chosen (preferably, the test substance is also administered to the animal to which the gene is not introduced, and the results are compared, and the substance which notably reduces the neutral fat concentration in blood in the transgenic animal is chosen). According to this method, not only can substances that suppress or inhibit expression of the introduced gene be found, but also substances which inhibit any of the vital reactions connected with elevation of the neutral fat concentration in blood by an action of a polypeptide, such as a factor contributing to expression of a function of the polypeptide itself or a function of interacting with this polypeptide to express the function of the polypeptide (for example, a receptor). Such a substance may also serve as a therapeutic or preventive agent for one or more diseases selected from hyperlipidemia, arteriosclerosis, and hyperglycemia.

The present invention relates to a polypeptide other than an antibody namely a receptor which specifically binds to the polypeptide to be detected by the method of the present invention (hereinafter referred to as "polypeptide to be detected"). When the receptor is a protein which exists in a cell membrane, the receptor can be cloned according to the following method. First, a recombinant vector by which DNA consisting of a nucleotide sequence shown as nucleotide numbers 47-1411 of SEQ ID No. 1 of the Sequence Listing or the nucleotide sequence shown as nucleotide numbers 78-1457 of SEQ ID No. 3 of the Sequence Listing can be expressed in a mammals cell is constructed, then introduced into COS-1, and culture supernatants are collected followed by purification of the recombinant polypeptide of the polypeptide to be detected. The obtained recombinant polypeptide is labeled with fluorescein isothiocyanate (hereinafter referred to as "FITC"). Then, the labeled recombinant polypeptide is added to cultured cells originating from various mammalian sources, and the cells wherein the labeled recombinant polypeptide specifically binds to a cell membrane thereof, namely the cells which express a receptor are identified. A cDNA library originating from the identified cells is incorporated into vectors which can be expressed in a mammalian cell, and expressed in cells which do not express the receptor (preferably a COS-1 or a CHO cell, more preferably a CHO cell). The recombinant polypeptide labeled with FITC is added to the cells in which the cDNA library is expressed, and the cells are collected after culturing for a certain time, and then the cells to which the recombinant polypeptide labeled with FITC binds are selected using a cell sorter. The introduced cDNA is cloned from the obtained cells by PCR or the like. If necessary, the above operation is repeated, and finally, cDNA encoding the receptor specifically binding to the FITC-labeled recombinant polypeptide is cloned. Moreover, the receptor itself is recoverable from the cell cloned as above.

Thus, if cDNA which encodes the receptor of the polypeptide to be detected is obtained, the cells used as the material for the cDNA library are further used for screening of substances which inhibit the interaction between the polypeptide to be detected and the receptor, and substances which bind to the receptor and have the same function as the polypeptide to be detected or substances which inhibit the signal transfer initiated by the interaction between the polypeptide to be detected and the receptor. Specifically, mouse genomic DNA is suitably fragmented by restriction enzyme digestion or the like, and cloned into a vector so that DNA encoding green-fluorescent protein is connected to the end of the cloned frgment (pEGFP-1 (manufactured by Clontech) is marketed as a vector for reporter assay) and this vector is introduced into the cells used as the material for the above-mentioned cDNA library. During culturing of the cells, the recombinant polypeptide (which is not labeled) of the polypeptide to be detected is added, and cells which produce green-fluorescent protein are sorted out by the cell sorter. The selected cells produce green-fluorescent protein in the presence of the recombinant polypeptide. The cells are cultured so that the number of cells per well becomes roughly equivalent in each well of a 96 well culturing plate, and after culturing for a certain period after addition of the test substance alone, or after addition of the recombinant polypeptide and the test substance together, the amount of the green-fluorescent protein produced is measured using a fluorescence plate reader or the like. If production of green-fluorescent protein occurs when culturing only with the test substance, the substance is considered to be an agonist of the polypeptide to be detected. On the other hand, when the amount of the green-fluorescent protein produced in the well to which the recombinant polypeptide and the test substance are added together is lower than the amount thereof in the well to which only the recombinant polypeptide is added, this substance is an antagonist of the polypeptide to be detected, or a signal transfer repressor of this polypeptide, and is considered to be useful as a therapeutic or preventive agent for one or more diseases selected from hyperlipidemia, arteriosclerosis, and hyperglycemia.

When the antagonist thus obtained is a protein or a peptide, the polynucleotide which has a nucleotide sequence encoding the protein or the peptide can be used for gene therapy of one or more diseases selected from hyperlipidemia, arteriosclerosis, and hyperglycemia. Such a polynucleotide can be obtained, for example, by analyzing the amino acid sequence of the identified antagonist protein or the polypeptide, and synthesizing an oligonucleotide probe which consists of a nucleotide sequence encoding the amino acid sequence, and performing screening of various cDNA libraries or genomic libraries. Moreover, when the peptide having an antagonist activity originates from an artificial peptide library synthesized at random, DNA consisting of a nucleotide sequence encoding the amino acid sequence of the peptide is chemically synthesized. In gene therapy, the polynucleotide which encodes the antagonist obtained as above is incorporated, for example, into a virus vector, and a patient is infected with the virus (attenuated) which is a recombinant virus vector. In the patient's body, an antagonist is produced, and the function of the polypeptide which consists of an amino acid sequence shown in SEQ ID No. 4 of the Sequence Listing is inhibited, and therefore the neutral fat concentration in the blood can be reduced.

As a method of introducing a gene therapy agent in a cell, either a transgenic method using a virus vector or a non-viral transgenic method (Nikkei science, April, 1994, 20-45 pages, an experimental-medicine special number, 12 (15) and (1994), an experimental-medicine separate volume "the basic technology of gene therapy", Youdosha (1996)) is applicable.

Examples of a method of introducing a gene into a cell with a virus vector include a method of incorporating DNA which encodes TR4 or variant TR4 into a DNA virus or RNA virus such as a retrovirus, adenovirus, an adenovirus related virus, Herpesvirus, vaccinia virus, a poxvirus, poliomyelitis virus, and Sindbis virus. Among them, methods using a retrovirus, adenovirus, an adenovirus related virus, and the vaccinia virus are especially preferable. As a non-viral transgenic method, a method of administering an expression plasmid to muscles directly (DNA vaccine method), a liposome method, a lipofection method, a microinjection method, a calcium phosphate method, an electroporation method or the like are suitable; the DNA vaccine method and the liposome method are especially preferred.

Moreover, to make a gene therapy agent act as a therapeutic agent, there is an in vivo method which introduces DNA into the body directly and an ex vivo method wherein a certain kind of cell is taken out from a human, DNA is introduced into a cell outside the body, and the cell is then returned into the body (Nikkei science, April 1994, 20-45 pages, Gekkanyakuji, 36 (1), 23-48 (1994), Jikkenigaku zoukan, 12 (15), (1994)).

For example, when a gene therapy agent is administered to a patient by the in vivo method, the medicine is administrated to the patient by a suitable administration pathway, such as by an intravenous, intra-arterial, hypodermical, intradermical, or intramuscular route, depending on the disease, the symptoms, or the like. Moreover, although this gene therapy agent is generally formulated as an injection or the like when it is administered to a patient by the in vivo method, a conventionally used carrier may be added if necessary. Moreover, when it is formulated in the form of a liposome or a membrane-fusion liposome (Sendai-virus liposome or the like), it can be formulated as a liposome preparation, such as a suspension, cryogen, or centrifugal-separation concentrated cryogen or the like.

A nucleotide sequence complementary to the partial sequence of the nucleotide sequence shown in the nucleotide numbers 78-1457 of SEQ ID No. 3 of the Sequence Listing can be used for a so-called antisense treatment. An antisense molecule may be used as DNA which usually consists of a 15 or 30 mer complementary to a part of the nucleotide sequence shown as nucleotide numbers 78-1457 of SEQ ID No. 3 of the Sequence Listing, or a stable DNA derivative such as a phosphorothioate, methyl phosphonate, or a morpholine derivative or the like, or a stable RNA derivative such as 2'-O-alkyl RNA. Such an antisense molecule can be introduced into a cell by a method well known in the technical field of the present invention, such as microinjection, liposome capsulation, or expression using a vector having an antisense sequence or the like. Such an antisense therapy is useful for a disease wherein it is useful to reduce the activity of the protein encoded by the nucleotide sequence shown as nucleotide numbers 78-1457 of SEQ ID No. 3 of the Sequence Listing, especially for treatment of one or more diseases selected from hyperlipidemia, arteriosclerosis, and hyperglycemia.

A composition useful as a medicine containing the above-mentioned antisense oligonucleotide may be manufactured by well known methods, such as mixing with a carrier permissible as a medicine. Examples of such carriers and manufacture methods are described in Applied Antisense Oligonucleotide Technology (1988) Wiley-Liss, Inc.). A sufficient amount of the medicine for the treatment of the hyperlipidemia wherein expression of the gene containing the nucleotide sequence shown as nucleotide numbers 78-1457 of SEQ ID No. 3 or the activity of the gene product is abnormal is administered. The effective dose may be varied due to various factors such as condition, weight, sex, and age, and due to difference in the administration method such as hypodermic, local, oral and intramuscular. For example, it is 0.02 to 0.2 mg/kg/hour for 2 hours when administered by an intravenous injection, and 1 to 200 mg/m²/day in the case of hypodermic administration.

### II A method of testing using a polypeptide

The polypeptide of the present invention can be obtained by obtaining any one of the following (a) to (1):
(a) DNA comprising a continuous sequence included in the nucleotide sequence shown in SEQ ID No. 11 of the Sequence Listing wherein nucleotide number 18 is the 5'-end and any one of nucleotide numbers 458 to 638 is the 3'-end;
(b) DNA comprising a continuous sequence included in the nucleotide sequence shown in SEQ ID No. 11 of the Sequence Listing wherein nucleotide number 18 is the 5'-end and any one of nucleotide numbers 458 to 602 is the 3'-end;
(c) DNA comprising a continuous sequence included in the nucleotide sequence shown in SEQ ID No. 15 of the Sequence Listing wherein nucleotide number 173 is the 5'-end and nucleotide number 601 is the 3'-end;
(d) DNA comprising a continuous sequence included in the nucleotide sequence shown in SEQ ID No. 15 of the Sequence Listing wherein nucleotide number 173 is the 5'-end and nucleotide number 1393 is the 3'-end;
(e) DNA comprising a continuous sequence included in the nucleotide sequence shown in SEQ ID No. 17 of the Sequence Listing wherein nucleotide number 434 is the 5'-end and any one of nucleotide numbers 1039 to 1261 is the 3'-end;
(f) DNA comprising a continuous sequence included in the nucleotide sequence shown in SEQ ID No. 17 of the Sequence Listing wherein nucleotide number 434 is the 5'-end and nucleotide number 1909 is the 3'-end;
(g) DNA comprising a continuous sequence included in the nucleotide sequence shown in SEQ ID No. 19 of the Sequence Listing wherein nucleotide number 22 is the 5'-end and any one of nucleotide numbers 639 to 846 is the 3'-end;
(h) DNA comprising a continuous sequence included in the nucleotide sequence shown in SEQ ID No. 19 of the Sequence Listing wherein nucleotide number 22 is the 5'-end and nucleotide number 1503 is the 3'-end;
(i) DNA comprising a continuous sequence included in the nucleotide sequence shown in SEQ ID No. 21 of the Sequence Listing wherein nucleotide number 242 is the 5'-end and any one of the nucleotide numbers 796 to 1015 is the 3'-end;
(j) DNA comprising a continuous sequence included in the nucleotide sequence shown in SEQ ID No. 21 of the Sequence Listing wherein nucleotide number 242 is the 5'-end and nucleotide number 1654 is the 3'-end;
(k) DNA comprising a continuous sequence included in the nucleotide sequence shown in SEQ ID No. 23 of the Sequence Listing wherein nucleotide number 86 is the 5'-end and any one of nucleotide numbers 373 to 394 is the 3'-end;
(1) DNA comprising a continuous sequence included in the nucleotide sequence shown in SEQ ID No. 23 of the Sequence Listing wherein nucleotide number 86 is the 5'-end and nucleotide number 1048 is the 3'-end; and subsequently making a host cell to produce the polyeptide encoded by the DNA according to a conventional genetic engineering method.

The amino acids shown as amino acid numbers 1 to 16 of the amino acid sequence encoded by the nucleotide sequence shown in SEQ ID No. 11 of the Sequence Listing, i.e., the amino acid sequence of SEQ ID No. 12 of the Sequence Listing is removed as a signal peptide after translation in a mammalian cell (Conklin, D. et al. (1999) Genomics 62,477-482). Accordingly, when the host cell originates from a mammalian cell in the above-mentioned method for production procedure, there can be obtained a polypeptide having a sequence of SEQ ID No. 12 of the Sequence Listing wherein the amino terminus is amino acid number 17. In a similar way, the amino acids shown as amino acid numbers 1 to 25 in the amino acid sequence encoded by the nucleotide sequence shown in SEQ ID No. 15 of the Sequence Listing, i.e., the amino acid sequence of SEQ ID No. 16 of the Sequence Listing, are removed as a signal peptide after translation in a mammalian cell, and therefore, when a host cell originates from a mammalian cell in the above-mentioned method for production procedure, there can be obtained a polypeptide having a sequence of SEQ ID No. 16 of the Sequence Listing wherein the amino terminus is amino acid number 26. In a similar way, the amino acids shown as amino acid numbers 1 to 21 of the amino acid sequence encoded by the nucleotide sequence shown in SEQ ID No. 17 of the Sequence Listing, i.e., the amino acid sequence of SEQ ID No. 18 of the Sequence Listing are removed as a signal peptide after translation in a mammalian cell, and therefore, when a host cell originates from a mammalian cell in the above-mentioned method for production procedure, there can be obtained a polypeptide having a sequence of SEQ ID No. 18 of the Sequence Listing wherein the amino terminus is amino acid number 22. In a similar way, the amino acids shown as amino acid numbers 1 to 22 of the amino acid sequence encoded by the nucleotide sequence shown in SEQ ID No. 19 of the Sequence Listing, i.e., the amino acid sequence of SEQ ID No. 20 of the Sequence Listing, are removed as a signal peptide after translation in a mammalian cell, and therefore, when a host cell originates from a mammalian cell in the above-mentioned method for production procedure, there can be obtained a polypeptide having a sequence of SEQ ID No. 20 of the Sequence Listing wherein the amino terminal is amino acid number 23. In a similar way, the amino acids shown as amino acid numbers 1 to 20 of the amino acid sequence encoded by the nucleotide sequence shown in SEQ ID No. 21 of the Sequence Listing, i.e., the amino acid sequence of SEQ ID No. 22 of the Sequence Listing, are removed as a signal peptide after translation in a mammalian cell, and therefore, when a host cell originates from a mammalian cell in the above-mentioned method for production procedure, there can be obtained a polypeptide having a sequence of SEQ ID No. 22 of the Sequence Listing wherein the amino terminus is amino acid number 21. In a similar way, the amino acids shown as amino acid numbers 1 to 26 in the amino acid sequence encoded by the nucleotide sequence shown in SEQ ID No. 23 of the Sequence Listing, i.e., the amino acid sequence of SEQ ID No. 24 of the Sequence Listing, are removed as a signal peptide after translation in a mammalian cell, and therefore, when a host cell originates from a mammalian cell in the above-mentioned method for production procedure, there can be obtained a polypeptide having a sequence of SEQ ID No. 24 of the Sequence Listing wherein the amino terminus is amino acid number 27.

A method for producing a polypeptide of the present invention using a host which, unlike a mammal, does not have a function to remove a signal peptide of a protein, such as a prokaryotic cell, may be, for example, a method which comprises:
making Escherichia coli which can produce a fusion protein having an amino acid sequence wherein to the amino acid sequence of any one of the following amino acid sequences (a) to (1) is connected to an amino acid sequence which can be removed specifically by a protease which does not cut the above amino acid sequences (a) to (1), and an amino acid sequence of a protein which has a specific affinity with a certain compound is connected to the amino terminal thereof:
(a) a polypeptide having a continuous amino acid sequence contained in the amino acid sequence shown in SEQ ID No. 12 of the Sequence Listing wherein the amino terminal is amino acid number 17, and the carboxyl terminal is any one of amino acid numbers 147 to 207;
(b) a polypeptide which has a continuous amino acid sequence contained in the amino acid sequence shown in SEQ ID No. 16 of the Sequence Listing wherein the amino terminal is amino acid number 26 and the carboxyl terminal is amino acid number 143;
(c) a polypeptide which has a continuous amino acid sequence contained in the amino acid sequence shown in SEQ ID No. 16 of the Sequence Listing wherein the amino terminal is amino acid number 26 and the carboxyl terminal is any one of amino acid numbers 144 to 183;
(d) a polypeptide which has a continuous amino acid sequence contained in the amino acid sequence shown in SEQ ID No. 18 of the Sequence Listing wherein the amino terminal is amino acid number 22 and the carboxyl terminal is any one of amino acid numbers 202 to 276;
(e) a polypeptide which has a continuous amino acid sequence contained in the amino acid sequence shown in SEQ ID No. 20 of the Sequence Listing wherein the amino terminal is amino acid number 23 and the carboxyl terminal is any one of amino acid numbers 206 to 275;
(f) a polypeptide which has a continuous amino acid sequence contained in the amino acid sequence shown in SEQ ID No. 22 of the Sequence Listing wherein the amino terminal is amino acid number 21 and the carboxyl terminal is any one of amino acid numbers 185 to 258;
(g) a polypeptide which has a continuous amino acid sequence contained in the amino acid sequence shown in SEQ ID No. 24 of the Sequence Listing wherein the amino terminal is amino acid number 27 and the carboxyl terminal is any one of amino acid numbers 122 to 129;
(h) a polypeptide which has a continuous amino acid sequence contained in the amino acid sequence shown in SEQ ID No. 16 of the Sequence Listing wherein the amino terminal is amino acid number 26 and the carboxyl terminal is amino acid number 406;
(i) a polypeptide which has a continuous amino acid sequence contained in the amino acid sequence shown in SEQ ID No. 18 of the Sequence Listing wherein the amino terminal is amino acid number 22 and the carboxyl terminal is amino acid number 491;
(j) a polypeptide which has a continuous amino acid sequence contained in the amino acid sequence shown in SEQ ID No. 20 of the Sequence Listing, wherein the amino terminal is amino acid number 23 and the carboxyl terminal is amino acid number 493.
(k) a polypeptide which has a continuous amino acid sequence contained in the amino acid sequence shown in SEQ ID No. 22 of the Sequence Listing, wherein the amino terminal is amino acid number 21 and the carboxyl terminal is amino acid number 470.
(1) a polypeptide which has a continuous amino acid sequence contained in the amino acid sequence shown in SEQ ID No. 24 of the Sequence Listing, wherein the amino terminal is amino acid number 27 and the carboxyl terminal is amino acid number 346.

Examples of such a purification using an affinity to a certain compound may include a method wherein Escherichia coli is made to produce a fusion protein to which an amino acid sequence of glutathione S-transferase (hereinafter referred to as "GST") is connected, and the fusion protein is subsequently purified using affinity of glutathione with GST, and then the sequence of GST is removed by a protease. However, even if the polypeptide of the present invention is a fusion protein wherein GST is not removed, the function is not lost, as shown by the example in this specification.

Moreover, the function of increasing the concentration of a neutral fat in blood is not lost in a polypeptide wherein a histidine tag for purification is inserted into the amino-terminal side of the "angiopoietin related protein" (in a signal peptide) and a fibrinogen binding domain at the carboxyl terminal end is deleted. For example, a polypeptide such as shown in SEQ ID No. 24 of the Sequence Listing wherein a histidine tag for purification is inserted into the amino-terminal side of the "angiopoietin related protein" (in a signal peptide) and 253 residues at the carboxyl terminal side are deleted also has the function.

Namely, the present invention may also include a polypeptide having an activity of increasing concentration of a neutral fat in blood wherein an arbitrary amino acid sequence is connected to any one of the following amino acid sequences (a) to (l):
(a) a polypeptide having a continuous amino acid sequence contained in the amino acid sequence shown in SEQ ID No. 12 of the Sequence Listing wherein the amino terminal is the amino acid of amino acid number 17, and the carboxyl terminal is any one of amino acid numbers 147 to 207;
(b) a polypeptide which has a continuous amino acid sequence contained in the amino acid sequence shown in SEQ ID No. 16 of the Sequence Listing wherein the amino terminal is amino acid number 17 and the carboxyl terminal is any one of amino acid numbers 147 to 195;
(c) a polypeptide which has a continuous amino acid sequence contained in the amino acid sequence shown in SEQ ID No. 16 of the Sequence Listing wherein the amino terminal is amino acid number 26 and the carboxyl terminal is amino acid number 143;
(d) a polypeptide which has a continuous amino acid sequence contained in the amino acid sequence shown in SEQ ID No. 18 of the Sequence Listing wherein the amino terminal is amino acid number 22 and the carboxyl terminal is any one of amino acid numbers 202 to 276;
(e) a polypeptide which has a continuous amino acid sequence contained in the amino acid sequence shown in SEQ ID No. 20 of the Sequence Listing wherein the amino terminal is amino acid number 23 and the carboxyl terminal is any one of amino acid numbers 206 to 275;
(f) a polypeptide which has a continuous amino acid sequence contained in the amino acid sequence shown in SEQ ID No. 22 of the Sequence Listing wherein the amino terminal is amino acid number 21 and the carboxyl terminal is any one of amino acid numbers 185 to 258;
(g) a polypeptide which has a continuous amino acid sequence contained in the amino acid sequence shown in SEQ ID No. 24 of the Sequence Listing wherein the amino terminal is amino acid number 27 and the carboxyl terminal is any one of amino acid numbers 122 to 129;
(h) a polypeptide which has a continuous amino acid sequence contained in the amino acid sequence shown in SEQ ID No. 16 of the Sequence Listing wherein the amino terminal is amino acid number 26 and the carboxyl terminal is amino acid number 406;
(i) a polypeptide which has a continuous amino acid sequence contained in the amino acid sequence shown in SEQ ID No. 18 of the Sequence Listing wherein the amino terminal is amino acid number 22 and the carboxyl terminal is amino acid number 491;
(j) a polypeptide which has a continuous amino acid sequence contained in the amino acid sequence shown in SEQ ID No. 20 of the Sequence Listing, and consists of an amino acid sequence wherein the amino terminal is amino acid number 23 and the carboxyl terminal is amino acid number 493.
(k) a polypeptide which has a continuous amino acid sequence contained in the amino acid sequence shown in SEQ ID No. 22 of the Sequence Listing, and consists of an amino acid sequence wherein the amino terminal is amino acid number 21 and the carboxyl terminal is amino acid number 470.
(1) a polypeptide which has a continuous amino acid sequence contained in the amino acid sequence shown in SEQ ID No. 24 of the Sequence Listing, and consists of an amino acid sequence wherein the amino terminal is amino acid number 27 and the carboxyl terminal is amino acid number 346. In that case, a continuous amino acid sequence consisting of 18 or more residues originating from an amino acid sequence of each Human - "angiopoietin related protein" may not be included in the arbitrary amino acid sequence to be connected.

Information on the nucleotide sequence of cDNA of "angiopoietin related protein" can be acquired from the database (GeneBank) of the National Center for Biotechnology Information. The "angiopoietin related protein 1" is registered as the accession number XM_001720. The "angiopoietin related protein 2" is registered as the accession number NM_012098. The "angiopoietin-related protein 3 " is registered as the accession number AF152562. The "angiopoietin related protein 4" is registered as the accession number AF202636. The angiopoietin related protein 5" is registered as the accession number NM031917. The angiopoietin related protein 6" is registered as the accession number Y16132.

A nucleotide consisting of a continuous sequence included in the nucleotide sequence shown in SEQ ID No. 11 of the Sequence Listing wherein nucleotide number 18 is the 5'-end and any one of nucleotide numbers 458 to 638 is the 3' end can be obtained, for example, by performing a polymerase chain reaction (henceforth "PCR") using as a template cDNA coding for human "angiopoietin-related protein 3 " cloned from human liver cDNA library or a recombinant phagemid carried by a transformed Escherichia coli E.coli pTrip/h55-1 SANK 72299 (FERM BP-6941), which was internationally deposited in International Patent Organism Depositary, National Institute of Advanced Industrial Science and Technology, and using an oligonucleotide primer designed so that DNA consisting of a desired nucleotide sequence may be amplified. Alternatively, it can also be obtained by site specific artificial mutation by which the nucleotide sequence which serves as a stop codon on the same reading frame as the open reading frame of this cDNA is inserted in the desired position in the nucleotide sequence of cDNA inserted in the transformed phagemid maintained in a transformed Escherichia coli E.coli pTrip/h55-1 SANK 72299 (FERM BP-6941).

The following nucleotides (a) to (h) can be obtained by PCR using as a template cDNA coding for human "angiopoietin 1, 2, 4, 5, 6" cloned from cDNA library originating from an appropriate human organ or a commercially available cDNA clone (available, for example, from I.M.A.G.E Consortium) and using an oligonucleotide primer designed so that DNA consisting of a desired nucleotide sequence may be amplified.
(a) a nucleotide consisting of a continuous sequence included in the nucleotide sequence shown in SEQ ID No. 17 of the Sequence Listing wherein nucleotide number 434 is the 5'-end and any one of nucleotide numbers 1039 to 1261 is the 3'-end.
(b) a nucleotide consisting of a continuous sequence included in the nucleotide sequence shown in SEQ ID No. 17 of the Sequence Listing wherein nucleotide number 434 is the 5'-end and nucleotide number 1909 is the 3'-end.
(c) a nucleotide consisting of a continuous sequence included in the nucleotide sequence shown in SEQ ID No. 19 of the Sequence Listing wherein nucleotide number 22 is the 5'-end and any one of nucleotide numbers 639 to 846 is the 3'-end.
(d) a nucleotide consisting of a continuous sequence included in the nucleotide sequence shown in SEQ ID No. 19 of the Sequence Listing wherein nucleotide number 22 is the 5'-end and nucleotide number 1503 is the 3'-end.
(e) a nucleotide comprising a continuous sequence included in the nucleotide sequence shown in SEQ ID No. 21 of the Sequence Listing wherein nucleotide number 242 is the 5'-end and any one of nucleotide numbers 796 to 1015 is the 3'-end.
(f) a nucleotide consisting of a continuous sequence included in the nucleotide sequence shown in SEQ ID No. 21 of the Sequence Listing wherein nucleotide number 242 is the 5'-end and nucleotide number 1654 is the 3'-end.
(g) a nucleotide consisting of a continuous sequence included in the nucleotide sequence shown in SEQ ID No. 23 of the Sequence Listing wherein nucleotide number 86 is the 5'-end and any one of nucleotide numbers 373 to 394 is the 3'-end.
(h) a nucleotide consisting of a continuous sequence included in the nucleotide sequence shown in SEQ ID No. 23 of the Sequence Listing wherein nucleotide number 86 is the 5'-end and nucleotide number 1048 is the 3'-end.

However, DNA coding for a polypeptide of the present invention is not limited thereto. For example, there can be included a polynucleotide modified by considering codon frequency of a host used in the above-mentioned biotechnological method, or those obtained by connecting to an artificial DNA, as far as it has a nucleotide sequence enabling production of any one of the following polypeptides:
(a) a polypeptide having a continuous amino acid sequence contained in the amino acid sequence shown in SEQ ID No. 12 of the Sequence Listing wherein the amino terminal is the amino acid of amino acid number 17, and the carboxyl terminal is any one of amino acid numbers 147 to 207;
(b) a polypeptide which has a continuous amino acid sequence contained in the amino acid sequence shown in SEQ ID No. 16 of the Sequence Listing wherein the amino terminal is amino acid number 26 and the carboxyl terminal is amino acid number 143;
(c) a polypeptide which has a continuous amino acid sequence contained in the amino acid sequence shown in SEQ ID No. 16 of the Sequence Listing wherein the amino terminal is amino acid number 26 and the carboxyl terminal is any one of amino acid numbers 144 to 183;
(d) a polypeptide which has a continuous amino acid sequence contained in the amino acid sequence shown in SEQ ID No. 18 of the Sequence Listing wherein the amino terminal is amino acid number 22 and the carboxyl terminal is any one of amino acid numbers 202 to 276;
(e) a polypeptide which has a continuous amino acid sequence contained in the amino acid sequence shown in SEQ ID No. 20 of the Sequence Listing wherein the amino terminal is amino acid number 23 and the carboxyl terminal is any one of amino acid numbers 206 to 275;
(f) a polypeptide which has a continuous amino acid sequence contained in the amino acid sequence shown in SEQ ID No. 22 of the Sequence Listing wherein the amino terminal is amino acid number 21 and the carboxyl terminal is any one of amino acid numbers 185 to 258;
(g) a polypeptide which has a continuous amino acid sequence contained in the amino acid sequence shown in SEQ ID No. 24 of the Sequence Listing wherein the amino terminal is amino acid number 27 and the carboxyl terminal is any one of amino acid numbers 122 to 129;
(h) a polypeptide which has a continuous amino acid sequence contained in the amino acid sequence shown in SEQ ID No. 16 of the Sequence Listing wherein the amino terminal is amino acid number 26 and the carboxyl terminal is amino acid number 406;
(i) a polypeptide which has a continuous amino acid sequence contained in the amino acid sequence shown in SEQ ID No. 18 of the Sequence Listing wherein the amino terminal is amino acid number 22 and the carboxyl terminal is amino acid number 491;
(j) a polypeptide which has a continuous amino acid sequence contained in the amino acid sequence shown in SEQ ID No. 20 of the Sequence Listing, and consists of an amino acid sequence wherein the amino terminal is amino acid number 23 and the carboxyl terminal is amino acid number 493.
(k) a polypeptide which has a continuous amino acid sequence contained in the amino acid sequence shown in SEQ ID No. 22 of the Sequence Listing, and consists of an amino acid sequence wherein the amino terminal is amino acid number 21 and the carboxyl terminal is amino acid number 470.
(1) a polypeptide which has a continuous amino acid sequence contained in the amino acid sequence shown in SEQ ID No. 24 of the Sequence Listing, and consists of an amino acid sequence wherein the amino terminal is amino acid number 27 and the carboxyl terminal is amino acid number 346.

A host cell of a prokaryotic or eukaryotic organism can be transformed with a recombinant vector obtained by inserting DNA which has a nucleotide sequence coding for a polypeptide of the present invention in a suitable vector DNA. The introduced gene can be expressed by introducing into the vector a suitable promoter and sequences involved in gene expression.

Examples of a prokaryotic cell for use as a host include Escherichia coli, Bacillus subtilis or the like. In order to transform these host cells with the target gene, the host cell is transformed with a plasmid vector containing a replicon, namely a replication origin, originating from a species compatible with the host and a regulatory sequence. As the vector, a vector comprising a sequence affording selectivity of phenotype to a transformed cell is preferable.

For example, K12, JM109 or the like is commonly used as Escherichia coli and the plasmid pBR322 and pUC system is commonly used as the vector, but these are not limiting, and other known strains and vectors can be used.

When a protein originating from another organism is expressed in Escherichia coli, translation efficiency may be restricted by the difference in codon frequency, depending on the kinds of organism. The reason for this is variation in the amount of tRNA(s) in a cell of Escherichia coli, some kinds of tRNA(s) may be exhausted. In this situation, expression using a strain transformed so as to express a large amount of tRNA which is normally present at relatively low levels in Escherichia coli may succeed in some cases. A suitable Escherichia coli is BL21 Codon Plus (DE3)-RIL. In this strain the copy number of tRNA genes (arginine isoleucine leucine), which is relatively low in Escherichia coli, is increased.

As a promoter in Escherichia coli, there can be used a tryptophan (trp) promoter, a lactose (lac) promoter, a tryptophan lactose (tac) promoter, a lipoprotein (lpp) promoter, a polypeptide-chain elongation factor Tu (tufb) promoter, or the like. Any one of these promoters can be used for production of the target polypeptide.

As a Bacillus subtilis, for example, the 207-25 strain is preferred, and pTUB228 (Ohmura, K. et al.(1984) J. Biochem. 95, 87-93) or the like is used as a vector, but this is not limiting. Extracellular secretory expression can be attained by connecting the DNA sequence encoding a signal peptide sequence of the α-amylase of Bacillus subtilis.

Examples of a eukaryotic host cell include: cells of a vertebrate, an insect, and yeast or the like. Examples of the cells of a vertebrate cell include: COS cells (Gluzman, Y. (1981) Cell 23, 175-182, ATCC CRL-1650) which are the cells of an ape, a dihydrofolic acid reductase deficient strain of a Chinese hamster ovary cell (CHO cell, ATCC CCL-61), or the like, but are not limited thereto (Urlaub, G. and Chasin, L. A. (1980) Proc. Natl. Acad. Sci. USA 77, 4126-4220).

Expression vectors for vertebrate cells can be those having a promoter upstream of the gene to be expressed, an RNA splicing site, a polyadenylation site, a termination site of transcription and furthermore having a replication origin if desired. Examples of the expression vector include pSV2dhfr (Subramani, S. et al. (1981) Mol. Cell. Biol. 1, 854-864) or the like, but the expression vector is not limited thereto.

When COS cells are used as the host cell, expression vectors suitably comprise the SV40 replication origin enabling autonomous replication in COS cells, a transcription promoter, a transcription termination signal and an RNA splicing site. The expression vectors can be used to transform the COS cells by a known method, such as a diethylaminoethyl (DEAE)-dextran method [cf. Luthman. H, and Magnusson. G. (1983), Nucleic Acids Res., 11, 1295-1308], a phosphate calcium-DNA co-precipitation method [Graham, F. L. and Van der Eb, A. J., (1973), Virology, 52, 456-457] and an electric pulse electroporation method [cf. Neumann, E., et. al., (1982), EMBO J, 1, 841-845] or the like. In the case that a CHO cell is used as the host cell, transformed cells stably producing the protein of the present invention can be produced by co-transfecting with an expression vector and a vector which can express the neo gene which functions as a G418 antibiotic resistance marker, for example, pRSVneo (Sambrook, J. et al. (1989): "Molecular Cloning A Laboratory Manual" Cold Spring Harbor Laboratory, NY), pSV2-neo (Southern, P. J. and Berg, P. (1982) J. Mol. Appl. Genet. 1, 327-341) or the like, and selecting G-418 resistant colonies.

In the case that an insect cell is used as a host cell, a cell line (Sf-9 or Sf-21) originating from ovarian-cells of Spodoptera frugiperda of the Lepidoptera Phalaenidae, and High Five cells originating from the ootid of Trichoplusia ni (Wickham, T. J. et al. (1992) Biotechnol. Prog. I: 391-396 or the like) are often used as host cells. pVL1392/1393 using a polyhedrin protein promoter of autograph polyhedron virus (AcNPV) is often used as baculovirus transfer vector (Kidd, I. M. and V. C. Emery (1993). The use of baculoviruses as expression vectors, Applied Biochemistry and Biotechnology 42, 137-159). Furthermore, a vector using a promoter of baculovirus P10 or a basic protein can also be used. Furthermore, it is possible to express recombinant protein as a secretory protein by fusing the secretion signal sequence of the envelope surface protein GP67 of AcNPV to the N-terminal of the target protein (Zhe-mei Wang, et al. (1998) Biol. Chem., 379, 167-174).

As an expression system using a eukaryotic microorganism as the host cell, yeast is generally known; Saccharomyces yeasts, for example, baker's yeast Saccharomyces cerevisiae and the petroleum yeast Pichia pastoris are preferred. As an expression vector for eukaryotic microorganisms such as yeasts, the promoter of an alcohol dehydrogenase gene (Bennetzen, J. L. and Hall, B. D. (1982) J. Biol. Chem. 257, 3018-3025), the promoter of an acid-phosphatase gene (Miyanohara, A. et al. (1983) Proc. Natl. Acad. Sci. USA 80, 1-5), or the like are preferably used. In order to express as a secretory protein, it is possible to express a recombinant protein which has a secretion signal sequence and the cleavage site of a mature endogenous protease of the host cell or a known protease at the N-terminus. For example, it is known that active form tryptase will be secreted into a medium by fusing the secretion-signal sequence of alpha factor of yeast and the cleavage site of KEX2 protease of petroleum yeast at the N-terminal end and then expressing them in a system where human mast cell tryptase of a trypsin type serine protease is expressed in petroleum yeast (Andrew, L. Niles, et al. (1998) Biotechnol. Appl. Biochem. 28, 125-131).

The transformant obtained as mentioned above can be cultured according to a conventional method, and the target polypeptide produced intracellularly, or secreted extra cellularly. Various kinds of commonly used media can be used for cell culture depending on the host cell used. When the above-mentioned COS cells are used, for example, RPMI1640 medium and Dulbecco's, Modified Eagle Medium (hereinafter referred to as DMEM) to which blood serum components, such as fetal bovine serum are added can be used.

The recombinant protein produced intracellularly or secreted extracellularly by a transformant by the above-mentioned culture process can be separated and purified according to various well-known separation methods using a physical property, a chemical property or the like of this protein. Specific examples of the methods include: protein precipitation using various agents, various liquid chromatography methods such as ultrafiltration, molecular sieve chromatography (gel filtration), adsorption chromatography, ion exchange chromatography, affinity chromatography and high performance liquid chromatography (HPLC), dialysis and combinations thereof. Moreover, it can be purified efficiently by using a nickel affinity column and by fusing six histidine residues with the expressed recombinant protein. The polypeptide of the present invention can be easily manufactured in large quantities in high yield and high purity by combining the above-mentioned methods.

The polypeptide of the present invention can be obtained according to the above-mentioned procedure, and can also be produced in an animal cell using an adenovirus vector in which DNA which has the nucleotide sequence encoding the polypeptide is incorporated.

Examples of a method of constructing such a recombinant adenovirus vector may include: a method using a commercial kit (for example, Adenovirus Expression vector kit manufactured by TAKARA SHUZO CO., LTD.). The fact that the polypeptide of the present invention closely correlates with a concentration of neutral fat in blood of mammals can be supported by the fact that if the gene carried by the recombinant adenovirus is expressed by following infection of a mammal, for example, by a mouse infected with the recombinant adenovirus vector obtained as mentioned above, an increase in the concentration of neutral fat (triglyceride) in blood will be observed.

If "angiopoietin related protein" is administrated into the blood of a mouse, a polypeptide from which the peptides at a carboxyl terminal is removed will be generated. For example, when "angiopoietin-related protein 3 " is administered into blood, two kinds of polypeptides can be obtained, a polypeptide cut between amino acid numbers 221 and 222 of the amino acid of SEQ ID No. 52 and a polypeptide cut between amino acid numbers 224 and 225 of the amino acid of SEQ ID No.52. That is, for an increase in concentration of lipid in blood, either "angiopoietin-related protein" or a variant polypeptide modified so that only the helix domain is included in the "angiopoietin-related protein" can be administrated.

The polypeptide of the present invention obtained by the above-mentioned method can be used to test a therapeutic or preventive agent for one or more diseases selected from hyperlipidemia, arteriosclerosis, and hyperglycemia which are, for example, those described below.

A preferred non-human animal used in the method is, for example, the KK/Snk mouse which has a mutant gene which does not normally produce the "angiopoietin-related protein" (for example, the "angiopoietin-related protein 3" and/or the "angiopoietin related protein 4"), the knockout mouse is genetically manipulated so that the "angiopoietin-related protein" (for example, the "angiopoietin-related protein 3 " and/or the "angiopoietin related protein 4") gene may not be expressed. However, use of this preferred mouse is not limiting.

The polypeptide to be administered may be
1) For the KK/Snk mouse having a mutant gene which does not produce normally the "angiopoietin-related protein 3 ", for example, a polypeptide having an amino acid sequence which is a continuous amino acid sequence shown in SEQ ID No. 12 of Sequence Listing wherein amino acid number 17 is the amino terminus and any one of amino acid numbers 147 to 207 is the carboxyl terminus.
2) For the KK/Snk mouse having a mutant gene which does not produce normally the "angiopoietin related protein 4", for example, a polypeptide having an amino acid sequence which is a continuous amino acid sequence shown in SEQ ID No. 16 of Sequence Listing wherein amino acid number 26 is the amino terminus and any one of amino acid numbers 143 to 183 is the carboxyl terminus. However, as long as an increase in concentration of neutral fat in blood can be confirmed, the polypeptide is not limited to these examples. The polypeptide of the present invention can be used solely or in combination of two or more kinds of polypeptide.
   A method for administering the polypeptide of the present invention to the above-mentioned animal can be administration into a blood vessel, oral administration or the like. It is not limited thereto, as long as an increase in the concentration of neutral fat in blood can be confirmed. The dose administered to the above-mentioned animal of the polypeptide of the present invention is not restricted provided that an increase in the concentration of fat in blood can be confirmed; it can be administered in an amount of, for example 0.01 µg to 10 mg per 1 g mouse weight, when administering to a tail vein of the mouse.
   When the polypeptide of the present invention is administered to the above-mentioned animal, an increase in the concentration of neutral fat (triglyceride) in blood will be observed.
   In this experimental system, the polypeptide of the present invention and test substance are administered to an experimental animal, and the substance by which the concentration of neutral fat in blood is lowered is selected. More preferably, the results are compared between 1) an animal to which only the polypeptide of the present invention is administered;
2) an animal to which only the test substance is administered and
3) an animal to which both of the polypeptide and test substance are administered,
   and the results are compared. When the substance does not affect the concentration of neutral fat in blood in the animal of 2) and lowers the concentration of neutral fat in blood significantly in the animal of 3) as compared with the animal of 1), it is selected as a substance which suppresses specifically the function of the polypeptide of the present invention. In this method, there can be found a substance which inhibits either of the vital reactions relating to raising the concentration of neutral fat in blood by action of these polypeptides, such as a function of the polypeptide of the present invention itself or a factor which interacts with the polypeptide and contributes to the functional expression of this polypeptide (for example, receptor). Such a substance can be a therapeutic or preventive agent for one or more diseases selected from hyperlipidemia, arteriosclerosis, and hyperglycemia.

Since the polypeptide of the present invention has the effect of inhibiting LPL and/or HTGL activity in vitro, it can be used in a test method for a substance that regulates LPL and/or HTGL activity as described below.

This test method can be carried out by providing both the polypeptide of the present invention and the substance which is to be tested for regulating LPL and/or HTGL activity (hereinafter referred to as a test substance), together, during the reaction of the LPL and/or HTGL in a method of measuring activity of LPL and or HTGL. More specifically, in a system for testing LPL activity wherein the material of 1-a) to 3) or 1-b) to 3) are provided in combination, and the change of LPL activity after adding a test substance is investigated.

### 1-a) Material containing LPL

As material containing LPL, the culture supernatant of a cell strain originating from a mammalian fat cell or a precursor cell of a fat cell which is producing LPL, can be used. When the cell is from an undifferentiated strain it is desirable to prepare material after making the fat cell differentiate. As a cell strain which is producing LPL, the mouse cell-strain 3T3-L1 (commercially available from Dainippon Pharmaceutical Co., LTD.), the mouse cell strain 3T3-F442A, and the mouse cell strain Ob17 or the like can be used.

Alternatively, a cell obtained by differentiating a rat white fat precursor cell (primary-culture cell) to a fat cell can also be used suitably. However, the present invention is not limited thereto.

Moreover, purified LPL or a recombinant LPL (see Methods Eyzymol (1996) 263, 319-26) can also be used as the material containing LPL. For example, it is possible to use LPL originating from cows' milk (commercially available from Sigma) for the procedure of the present invention.

### 1-b) Material containing LPL and HTGL

As material containing LPL and HTGL, plasma of peripheral blood origin extracted from a mammal 10 to 15 minutes after administration of 10 to 100 units of heparin per 1 kg weight by intravenous injection is suitable.

### 2) Material containing substrate of LPL and/or HTGL

As material containing the substrate of LPL and/or HTGL, there can be used a substance which is known to release fatty acid by catalytic action of these lipases and which does not contain a component which inhibits the activity of these lipases or inactivates them. For example, glycerol tri [9,10(n)-³H] oleic acid, a compound wherein the fatty acid to be released according to the catalytic action of LPL is labeled with radioisotope, and a compound labeled with a substance which emits fluorescence under specific excitation wavelengths similarly are especially suitable. Moreover, when measuring an amount of a free fatty acid according to a biochemical procedure, a substrate (for example, glycerol trioleic acid) which is not labeled may also be used.

### 3) the polypeptide of the present invention

As the polypeptide of the present invention, there can be used a polypeptide which consists of an amino acid sequence shown in the amino acid numbers 17 to 460 of SEQ ID No. 4 of Sequence Listing, a polypeptide lacking any length of 1 to 253 residues at the carboxyl terminus thereof, or a polypeptide according to Claims 18 to 34. However, the polypeptide of the present invention is not limited thereto.

The test substance in a method of the present invention is not limited. There can be used an extract from a culture of a microorganism, a tissue of animals or plants, an extract from a culture of cultured cells, a synthesized inorganic or organic compound, a recombinant protein, an antibody, a fragment thereof, or a composition wherein two or more kinds of substances are mixed.

In addition, a buffer for pH adjustment and a protein component, such as albumin, may be added to the reaction mixture if needed.

The above-mentioned materials are mixed and subjected to the LPL reaction under the following conditions:
temperature: 27 to 37 °C, preferably 37 °C
suitable pH of the reaction mixture: 8.0 to 8.5, preferably 8.0
suitable concentration of sodium chloride: 0.1 to 0.15 M, preferably 0.15 M
reaction-time; 15 to 120 minutes, preferably 120 minutes

When using material containing both LPL and HTGL such as plasma originating from a mammal to which heparin has been administered, the total lipase activity of LPL and HTGL can be measured under the above-mentioned conditions.

If, the same conditions are used, with the exception that sodium chloride concentration is 1 M, then only HTGL activity can be measured. The lipase reaction may progress while LPL is inactivated. In that case, LPL activity can be calculated by deducting HTGL activity from the total lipase activity.

After completion of the reaction, the layer containing a free fatty acid and the layer containing unreacted substrate are separated by adding an organic solvent to the reaction mixture and mixing it; the amount of the free fatty acid collected in the former layer is measured. Moreover, an example of the test method in the case of using the unlabeled substrate will be shown below (Clin. Chem. 30. 748 (1984)).

After incubating 0.5 ml of the substrate solutions (7.5 µmol of glycerol trioleic acid, 22.5 mg of gum arabic, 100 µmol of Tris-hydrochloride (pH 8.2), 50 µmol of sodium chloride, 25 mg of albumin, 140 µl of human plasma) for 80 minutes at 37 °C, a fat cell culture supernatant is added thereto, and polypeptide of the present invention and a test substance are added together, and they are allowed to react for 60 minutes at 28 °C. Next, 2.5 ml of isopropanol : heptane : sulfuric acid (2.5 mol/L) = 40 : 10 : 2 (v/v) is added to the reaction mixture, and mixed for 10 minutes, and left at a room temperature for 40 minutes. Then, one ml of the organic layers is separated, and dried up with nitrogen. The resulting material is dissolved in 5 % Triton-X 100 solution, and the amount of fatty acid is measured using a commercial fatty acid measurement kit (for example, NEFA-Test Wako manufactured by Wako Pure Chemical Industries, Ltd.) or the like.

As a result of performing a test according to the above-mentioned method, a test substance which suppresses inhibition of LPL and/or HTGL by the polypeptide of the present invention, and a test substance which raises LPL and/or HTGL activity as a result can be selected as a new therapeutic or preventive agent for one or more diseases selected from hyperlipidemia, arteriosclerosis, and hyperglycemia, which lowers a concentration of neutral fat in blood.

### III Test method using a promoter DNA

DNA of the present invention can be isolated from a gene library created from the mouse genome, for example, using the 5'-end side of the nucleotide sequence of Mouse "angiopoietin-related protein 3 " cDNA (Comklin, D. et al. Genomics 62, 477-482 (1999)) or the nucleotide sequence of Mouse "angiopoietin related protein 4" cDNA (Kersten, S., et al. (2000) J. Biol. Chem. 275, 28488-28493; Yoon, J. C., et al. (2000) Mol. Cell. Biol. 20, 5343-5349). Alternatively, DNA of the present invention can be amplified directly by the polymerase chain reaction (henceforth "PCR") using mouse genomic DNA as the template, without conducting the process of screening of a library, based on the information on this sequence.

In addition, a recombinant plasmid pGL 8-3 in which DNA of the present invention is inserted was internationally deposited on June 7, 2001 in International Patent Organism Depositary, National Institute of Advanced Industrial Science and Technology at Central 6, 1-1-1 Higashi, Tsukuba, Ibaraki, Japan, and was accorded the accession number FERM BP-7627. Therefore, DNA of the present invention can also be obtained from the plasmid.

In addition, those skilled in the art to which the present invention belongs, can prepare DNA which has a promoter activity equivalent to the wild type promoter DNA by modifying a part of the nucleotide sequence of the wild type promoter DNA, by alteration such as a substitution, deletion, addition of nucleotides or the like. DNA having a nucleotide sequence in which nucleotides are substituted, deleted, or added to the wild type nucleotide sequence, and which has promoter activity equivalent to the wild type promoter DNA is also included in DNA of the present invention. The alteration of a nucleotide sequence can be performed by introducing a deletion using a restriction enzyme or a DNA exonuclease, by introducing mutation using a site-directed-mutagenesis method, and by modification of the promoter sequence using a PCR method using a mutagenic primer, by the direct introduction of a synthetic mutant DNA, or the like. Preferable DNAs among them include: DNA containing a nucleotide sequence shown in nucleotide numbers 1-510 of SEQ ID No. 27 of the Sequence Listing or a nucleotide sequence shown in nucleotide numbers 1-260 of SEQ ID No. 2 of Sequence Listing, and more preferably DNA which consists of a nucleotide sequence shown in nucleotide numbers 1-526 of SEQ ID No. 27 of the Sequence Listing or a nucleotide sequence shown in nucleotide numbers 1-188 of SEQ ID No. 28 of the Sequence Listing.

Moreover, examples of DNA of the present invention may include: DNA which contains DNA which can hybridize under the stringent conditions to DNA consisting of a nucleotide sequence shown in nucleotide numbers 1 to 526 of SEQ ID No. 27 of Sequence Listing, a nucleotide sequence shown in nucleotide numbers 1 to 260 of SEQ ID No. 28 of Sequence Listing. Such DNA which can hybridize may be DNA which has a promoter activity. Such DNA has generally 70 % or more, preferably 80 % or more and more preferably 95 % or more nucleotide sequence identity with the nucleotide sequence shown as nucleotide numbers 1-526 of SEQ ID No. 27 of the Sequence Listing or a nucleotide sequence shown as nucleotide numbers 1-260 of SEQ ID No. 28 of the Sequence Listing. Such DNA may include a variant (mutant) type gene discovered in a nature, an artificial mutant gene, a homologous gene originating from a different species of organism, or the like.

In the present invention, hybridization under stringent conditions is as follows: a normal stringency reaction (low stringent conditions) is carried out by hybridizing in 5 x SSC (0.75 M sodium chloride, 0.075M sodium citrate) or a hybridization solution which has a salt concentration equivalent thereto at 37 to 42 °C for about 12 hours, then conducting a washing operation with 5 x SSC or a solution which has a salt concentration equivalent thereto if necessary and then conducting a washing operation in 1 x SSC or a solution which has a salt concentration equivalent thereto. A higher stringency reaction (high stringent conditions) is carried out in the same manner as above except that the washing operation involves 0.1 x SSC or a solution which has a salt concentration equivalent thereto.

Accordingly, whether DNA of the present invention thus obtained has a promoter activity can be confirmed by transformating a mammalian cell with a plasmid wherein a marker gene such as a luciferase gene is connected downstream (hereinafter referred to as a "reporter gene"), and detecting whether expression of the marker gene in the transformed cell is inhibited as described below.

DNA of the present invention is connected on the upstream side of a foreign gene in the same transcriptional direction as the foreign gene sequence in the expression vector of the present invention used, the foreign gene can be expressed in a mammalian animal cell. Since DNA of the present invention is connected upstream of the foreign gene, a foreign gene can be expressed by the expression vector under control of the promoter for the DNA of the present invention. The expression vectors of the present invention may be in any form, provided that it can be replicated when introduced into a mammalian cell, for example, it can be circular or linear.

Examples of methods for introducing a gene into a cell with a virus vector include a method of incorporating DNA which encodes TR4 or variant TR4 into a DNA virus or RNA virus such as a retrovirus, adenovirus, an adenovirus-related virus, Herpesvirus, vaccinia virus, a poxvirus, poliomyelitis virus, and Sindbis virus. Amongst these, methods using a retrovirus, adenovirus, an adenovirus-related virus, and the vaccinia virus are especially preferred.

As the non-viral transgenic method, a method of administering an expression plasmid to muscles directly (DNA vaccine method), a liposome method, a lipofection method, a microinjection method, a calcium phosphate method, an electroporation method or the like are suggested; the DNA vaccine method and the liposome method are especially preferred.

The expression vector can be introduced into a COS cell by, for example, a diethyl amino ethyl (DEAE)-dextran method (Luthmann, H. and Manusson, G. (1983) Nucleic Acids Res, 11, 1295-1308), a calcium phosphate DNA co-precipitation method (Graham F. L. and van der Eb, A. J. (1973) Virology 52, 456-457), an electric-pulse terebration method (Neumann, E. et al. (1982) EMBO J. 1, 841 - 845) and a lipofection method (Lopata et al. (1984) Nucl. Acids Res. 12, 5707 - 5717, Sussman and Milman (1984) Mol. Cell. Biol. 4, 1641-1643) or the like. A desired transformed cell can be obtained in this way. However, when a cultured-cell strain is a so-called suspension cell, it is desirable to use a method other than a calcium phosphate DNA co-precipitation method. In any of the methods used, the transfection conditions are optimized depending on the cell used.

Moreover, a transgenic animal obtained by taking a fertilized egg from an animal, transplanting it to a false pregnant animal and generating transgenic progeny can also be used. A well-known procedure can be followed, (See Hassei Kougaku Jikken manual, supervised by Tatsuji Nomura, edited by Motoya Katsuki, and Japanese Patent Application Laid-open No. (KOKAI) Hei 5-48093). Specifically, in the case of a mouse, an ovulation induction agent is administrated to a female mouse, which is then crossed with a male mouse of the same line; a pronuclear fertilized egg is taken from the oviduct of the female mouse on the next day. Subsequently, the DNA fragment solution which is to be introduced is injected into the pronucleus of the fertilized egg using a minute glass tube (microinjection). The regulatory genes, such as an enhancer, for expressing the introduced gene in an animal cell which has DNA of the present invention as a promoter can be anything which can function as such in the animal cell in which the gene is introduced. The fertilized egg into which the DNA is injected is transplanted into the oviduct of the false pregnant female mouse (Slc:ICR or the like), and is born by natural birth or cesarean section after about 20 days.

Examples of the method for confirming that the animal thus obtained has the introduced gene may include: a method of extracting DNA from the tail of the above-mentioned animal or the like, and carrying out PCR amplification of the DNA using a sense primer and an antisense primer specific thereto, a method of subjecting the DNA to gel electrophoresis, blotting the DNA in the gel on a nylon film or the like, and conducting Southern blotting analysis using all or a part of the labeled introduced gene as a probe.

Examples of a foreign gene include: a gene encoding a protein for treating a disease, a marker gene, or the like.

Hereafter, a method for using DNA of the present invention will be described.

### 1) Use as an inducing type expression vector

When it is found that DNA of the present invention has a promoter activity induced by a certain specific stimulus, a vector wherein DNA of the present invention is inserted upstream of the desired gene is produced, and introduced into a somatic cell. The desired gene can be expressed inducibly by adding the stimulus.

### 2) Regulation of a promoter activity of DNA using competitive inhibition by DNA

The present invention relates to DNA containing at least part of the above-mentioned promoter DNA (including a derivative thereof, hereinafter referred to as "the partial sequence"). It is possible to conduct a method for competitive inhibition of the binding the above-mentioned promoter DNA with a protein which can bind to it (for example, a transcription factor), whether or not the partial sequence itself has promoter activity. For example, when this partial sequence corresponds to the binding site of the protein inhibiting a promoter activity on the DNA sequence of the present invention, the promoter activity can be accelerated by the method. On the contrary, when it corresponds to a binding part of the protein accelerating promoter activity (including a transcription factor), promoter activity can be inhibited. The partial sequence DNA used for competitive inhibition usually has a length of at least 6 nucleotides, preferably 10 nucleotides or more. Examples of the kind of partial sequence selected to be used for competitive inhibition, include the sequence containing a binding consensus sequence of a transcription factor on a promoter. Moreover, examples of the derivative of such a partial sequence DNA may include those consisting of several joined partial sequences, those wherein it was inserted into an adenovirus vector or the like to enable gene transfer to an animal cell, those wherein biotin is connected to the 3'-end or 5'-end of DNA, or the like.

It is clear from the following examples that induced expression of "angiopoietin-related protein 3 " and "angiopoietin related protein 4" in a hypolipidemia mouse may lead to an increase in a concentration of neutral fat in blood. It is observed that the influence of the mutant gene by which "angiopoietin-related protein 3 " is not normally produced may significantly lower lipid concentration in blood, suppress progress of arteriosclerosis, and improve hyperglycemia. Since it is believed that the same phenomenon is observed for "angiopoietin-related protein 4", the partial sequence which functions to control a promoter activity of DNA of the present invention, or a derivative thereof, is useful as an active substance in a pharmaceutical composition for treatment or prevention of one or more diseases selected from hyperlipidemia, arteriosclerosis, and hyperglycemia.

### 3) Screening of a protein which regulates a promoter activity

In order to regulate a promoter activity of DNA of the present invention, there can be used not only competitive inhibition which uses a partial sequence or the like as described above, but also a method using a protein which regulates the promoter activity of the DNA. The DNA of the present invention can be used for screening a protein which regulates the promoter activity. Therefore, the present invention relates to a method for screening a protein regulating the promoter activity of the DNA, as shown below. Examples of the protein may include not only protein which binds directly to the DNA of the present invention but also a protein which acts on a cell membrane receptor or a protein in a cell to indirectly promote or inhibit the promoter activity of the DNA of the present invention.

### 3-1) Screening of protein binding to a promoter DNA

The method comprises contacting a protein to be tested with the DNA of the present invention to select a protein binding to the DNA of the present invention. Examples of such a method include affinity purification of the protein which binds thereto. One example of a specific method comprises biotinylating the promoter DNA of the present invention, and allowing it to bind to a magnetic bead to which streptavidin is bound to produce DNA affinity beads. Then, incubating with a nuclear extract of a cell, to isolate a protein from the nuclear extract which specifically binds to DNA of the present invention, to enable determination of the structure thereof. Thereby, it is possible to isolate protein which binds to DNA of the present invention and protein which binds to DNA of the present invention by forming a protein complex as a subunit although it does not by itself have an activity of binding to the DNA. (Gabrielsen O. S. et al., Nucleic acid Research 17, 6253-6267 (1989), Savoysky E et al., Oncogene 9, 1839-1846 (1994)).

### 3-2) Screening a protein using a promoter activity as an index

The method comprises introducing the DNA to be tested into a cell carrying a marker gene connected downstream of the DNA of the present invention (described in detail in 5-2) below) and selecting for test DNA that encodes an expressed product that regulates expression of the marker gene. Examples of such a method include: the one-hybrid method using, for example, yeast or animal cells. More specifically, the reporter gene in which the DNA of the present invention and a marker gene are inserted is stably introduced into a cell, subsequently a gene library is introduced therein, a clone which shows increased expression or decreased expression of the reporter gene is selected, and the protein binding to the DNA of the present invention is selected. According to this method, there can be obtained not only the protein which binds to the DNA of the present invention directly but also a protein which regulates indirectly the promoter activity of the DNA of the present invention by acting on an endogenous protein in a cell other than the protein combined directly with DNA of the present invention. For one-hybrid method (Li J. J. and Herskowitz I., Science 262, 1870 - 1873 (1993)), "Matchmaker system (Crontech)" or the like is commercially available as a kit.

Another embodiment comprises a step bringing a sample to be tested into contact with a cell carrying a marker gene connected downstream of the DNA of the present invention, and selecting a protein which regulates expression of the marker gene. In one example of a specific procedure, the cells in which the reporter gene in which the DNA of the present invention and a marker gene are inserted is introduced stably are incubated with the test sample (for example, culture supernatant of cells in which a gene library has been introduced), and a protein which promotes or inhibits an expression of a marker gene is selected. According to this method, a protein which affects promoter activity of the DNA of the present invention indirectly through a cell membrane receptor or the like can be obtained.

### 4) Screening method for cDNA encoding a protein which regulates a promoter activity

DNA encoding a protein which regulates promoter activity can also be isolated directly using DNA of the present invention. The present invention also relates to a method for screening for cDNA encoding a protein which regulates promoter activity of the DNA of the present invention. One embodiment of this screening method comprises a step of bringing the expression product of the DNA to be tested into contact with the DNA of the present invention, and selecting cDNA encoding the protein binding to the DNA of the present invention. Such method includes a South Western method. Specifically, each protein is expressed in Escherichia coli into which a gene library has been introduced, the cells are transferred onto a filter film, and the DNA of the present invention is directly blotted as a probe; a clone in which a protein binding to the DNA probe is expressed is selected, and then a gene encoding it is isolated. According to the method, the gene encoding a protein having a activity of binding to the DNA of the present invention can be obtained (See Jikken Igaku Bessatu Bio manual series "transcription factor approach" published by Yodosha, page 177-188).

Another embodiment comprises a step of introducing cDNA in a cell carrying a marker gene connected downstream of DNA of the present invention, and selecting cDNA encoding an expression product which regulates expression of a marker gene. Such a method include the above-mentioned one-hybrid method using yeast and animal cells.

Namely, the cells (hereinafter referred to as "cell B" obtained by introducing a recombinant plasmid, wherein cDNA to be tested is introduced into an expression vector for a mammalian cell, into a cell in which a reporter gene in which the DNA of the present invention and a marker gene are inserted is introduced stably (hereinafter referred to as "Cell A") are cultured. As a control, cell A is cultured as it is, or the cell (herein after referred to as "cell C") obtained by introducing an expression vector for a mammalian cell which does not contain cDNA to be tested in the cell A or by introducing a recombinant plasmid wherein cDNA to be tested is contained but is not expressed is cultured. The expression level of the marker gene is compared between the "cell B" and "cell C" or "cell A", and cDNA clone which promotes or inhibits expression of the marker gene is selected. According to this method, not only cDNA encoding a protein which binds directly to the DNA of the present invention but also cDNA encoding a protein which regulates promoter activity of the DNA of the present invention indirectly by acting endogenous protein in a cell can be detected.

Another embodiment comprises a step of bringing an expression product of the cDNA to be tested in contact with a cell carrying a maker gene connected downstream of the DNA of the present invention and selecting cDNA encoding an expression product which regulates expression of the marker gene. In one specific example, a cell in which the reporter gene in which the DNA of the present invention and a marker gene are inserted is stably introduced and an expression product of cDNA to be tested (for example, culture supernatant of the cell in which the cDNA library is introduced) are incubated, and a protein which upregulates or inhibits expression of the marker gene or the cDNA encoding it is isolated. According to this method, cDNA encoding the protein which acts on the promoter activity of the DNA of the present invention indirectly through a cell membrane receptor or the like can be obtained.

### 5) Screening method for a compound regulating the promoter activity

Using DNA of the present invention, a compound which regulates the promoter activity thereof can also be screened. Namely, the present invention relates to a method for screening a compound which regulates the promoter activity of DNA as shown below.

### 5-1) Screening using binding of a promoter and protein as an index

This method comprises a step of bringing a test sample into contact with DNA of the present invention in the presence of a compound to be tested, and a step of selecting a compound that promotes or inhibits binding of the DNA of the present invention and a protein in the test sample. Specifically, a nuclear extract of a cell is brought into contact with a probe obtained by labeling the DNA of the present invention with a radioactive isotope or the like, and a band of a complex of the protein from the nuclear extract and the DNA of the present invention is produced by polyacrylamide gel electrophoresis according to a gel shift method (Jikken Igaku Bessatsu Bio manual series "transcription factor approach" published by Yodosha, pages 107-112). When the DNA probe is added, the test compound is also added and a compound which promotes or inhibits formation of the band of the complex of the protein from the nuclear extract and the DNA of the present invention is selected. According to this method, there can be obtained a compound which directly acts on the DNA of the present invention and a compound which acts on the protein which binds to the DNA of the present invention. For example, when the protein which binds to the DNA of the present invention inhibits the promoter activity of the DNA of the present invention in vivo, it is considered that the compound which inhibits binding of this protein and the DNA of the present invention can promote the promoter activity of the DNA of the present invention. If isolation of the protein which binds to the DNA of the present invention has already been carried out, it is also possible to substitute a recombinant protein derived from this protein for the nuclear extract of a cell.

### 5-2) Screening using a promoter activity as an index

The method comprises a step of bringing the test compound into contact with a cell carrying the marker gene connected to the 3'-end side of the DNA of the present invention, and a step of selecting the compound which regulates expression of the marker gene. According to the method, there can be obtained a compound which regulates the promoter activity of the DNA of the present invention, either directly or indirectly.

In this method, the marker protein encoded by the marker gene connected to the 3'-end side of the DNA of the present invention can be any protein, as far as it can be distinguished from any other protein produced by the above-mentioned host cell in the course of the method of the present invention (preferably the above-mentioned cell before transformation does not have a gene encoding a protein which is the same as, or similar to, the marker protein). For example, even when the marker protein is toxic to the cell, or provides resistance to antibiotics to which the cell is sensitive, existence of expression of the marker gene can be judged by the survival ratio of a cell. However, a more desirable marker gene for use in the present invention is a structural gene, wherein the expression level can be detected specifically and quantitatively (for example, those wherein an antibody specific to the protein encoded by the marker gene has been obtained). A further more preferable marker gene is one encoding an enzyme which can be reacted specifically with a foreign substrate to produce a metabolic product which can be easily measured quantitatively. Examples include a gene encoding one of the following proteins, without being limited thereto:
Chloramphenicol acetyltransferase: adding an acetyl group to a chloramphenicol. Detection is possible by the so-called "CAT assay" or the like. pCAT3-Basic vector (manufactured by Promega) as a vector which can be a vector for reporter assays only by incorporating a promoter is commercially available; Firefly luciferase: quantified by measuring a bioluminescence produced when luciferin is metabolized. pGL3-Basic vector (manufactured by Promega) as a vector which can be a vector for reporter assays only by incorporating a promoter is commercially available;
β-galactosidase: the substrate for this enzyme can be measured by a color reaction, fluorescence, or chemoluminescence, respectively. Pβgal-Basic (manufactured by Promega) as a vector for reporter assays is commercially available;
Secretor alkaline phosphatase: the substrate for this enzyme can be measured by a color reaction, bioluminescence, or chemoluminescence, respectively. pSEAP2-Basic (manufactured by Clontech) as a vector for reporter assays is commercially available;
Green fluorescence protein (green-fluorescent protein): is not an enzyme, but can be directly quantified since it emits fluorescence. pEGFP-1 (manufactured by Clontech) as a vector for reporter assays is commercially available.

Examples of a method for introducing an expression plasmid into a cultured cell strain may include: DEAE-dextran method {Luthmann H. and Magnusson, G. (1983) Nucleic Acids Res. 11, 1295-1308}, a calcium phosphate DNA co-precipitation method (Graham, F. L. and van der Eb, A. J. (1973) Virology 52, 456-457), an electric-pulse terebration method (Neumann, E. et al. (1982) EMBO J. 1. 841-845), a lipofection method (Lopata et al. (1984) Nucl. Acids Res. 12, 5707-5717, Sussman and Milman (1984) Mol. Cell. Biol. 4, 1641-1643) or the like. However, the method for introduction is not limited thereto, and other methods which are commonly used in the technical field to which the present invention belongs can also be used. However, when a cultured cell strain is a so-called "suspension cell", it is desirable to use a method other than a calcium phosphate DNA co-precipitation method. In any of the above methods, the transfection conditions used are optimized depending on the cell to be used.

If a cell transfected with an expression vector wherein the marker gene was connected to the 3'-end side of the DNA of the present invention as described above is cultured, the transcription of the marker gene is promoted. In culturing under conditions wherein a marker gene can be expressed, the level of expression of a marker gene can be measured after culturing in defined conditions wherein a test substance is added to the medium, and wherein it is not added, and it can be determined if there is a change in the expression level of the marker gene following addition of the test substance. "Conditions wherein a marker gene can be expressed" can be conditions under which a protein can be produced. Preferably, culture is carried out using a medium (blood serum components such as a fetal bovine serum can be added thereto) which is suitable for the cell strain to be used, at 36 to 38 °C (most suitably 37 °C) for two or three days (most suitably for two days) in an atmosphere containing 4 to 6 % (most suitably 5 %) carbon dioxide.

A test substance which suppresses the expression of the reporter gene when the expression vector wherein the marker gene is connected to the 3'-end side of the DNA of the present invention is introduced can be selected as a candidate substance useful as a therapeutic or preventive agent for one or more diseases selected from hyperlipidemia, arteriosclerosis, and hyperglycemia.

In the case that a protein which regulates the promoter activity of the DNA of the present invention has already been obtained, it is possible to bring the protein (or a derivative thereof) into contact with the DNA of the present invention in the presence of a compound to be tested, select a compound which promotes or inhibits binding of the protein (or a derivative thereof) and the DNA of the present invention, and thereby screen for a compound which regulates the promoter activity of the DNA of the present invention. Specifically, the protein binding to the DNA of the present invention which is fused with glutathione S-transferase can be purified (it can be only a domain binding to DNA), fixed on a micro plate covered with an anti-glutathione S-transferase antibody, and brought into contact with the biotinylated DNA of the present invention. Then, the binding of the protein and the DNA of the present invention can be detected using a streptavidinized alkaline phosphatase. When the DNA of the present invention is added, a compound to be tested is also added, and a compound which promotes or inhibits binding of the protein and the DNA of the present invention is selected. According to this method, a compound which acts directly on the DNA of the present invention and a compound which acts on a protein binding to the DNA of the present invention can be obtained. For example, when a protein binding to the DNA of the present invention inhibits the promoter activity of the DNA of the present invention in vivo, it is considered that a compound which inhibits binding of this protein and the DNA of the present invention can promote the promoter activity of the DNA of the present invention.

If the expression of the "angiopoietin-related protein 3" and the "angiopoietin-related protein 4" are inducibly expressed in a hypolipidemic mouse, the concentration of neutral fat in the blood will rise, as clearly shown in the following examples. Under the influence of the mutant gene by which "angiopoietin-related protein 3 " is not normally produced, significant lowering of lipid concentration in blood, suppression of progress of arteriosclerosis, and improvement of hyperglycemia are observed, and it is considered that the same phenomenon may also be observed as for "angiopoietin-related protein 4". Accordingly, a partial sequence having a function which suppresses the promoter activity of the DNA of the present invention or a derivative thereof can act as a therapeutic or preventive agent for one or more diseases selected from hyperlipidemia, arteriosclerosis, and hyperglycemia.

### BRIEF DESCRIPTION OF THE DRAWINGS

FIG. 1 shows the result of a Northern blot analysis of samples originating from the organs of a KK mouse. The numbers on the right-hand side of the lanes (28S and 18S) show the sedimentation coefficients of marker RNAs.
FIG. 2 shows the result of a Western blot analysis using a transfected COS-1 cell culture supernatant as a sample. The numbers on the left-hand side of the lanes show the molecular weights (KDa) of molecular weight markers.
FIG. 3 shows the result of a Western blot analysis using transfected HeLa-cell culture supernatant as a sample. The numbers on the left-hand side of the lanes show the molecular weight (KDa) of molecular weight markers.
FIG. 4 is a graph showing the results of measurement of a concentration of neutral fat in the peripheral blood of a KK/Snk mouse infected with a recombinant adenovirus.
FIG. 5 shows the result of the Northern blot analysis for a sample originating from the liver of a KK mouse and a KK/Snk mouse infected with a recombinant adenovirus. The numbers (28S and 18S) on the left-hand side of the lanes show the sedimentation coefficients of marker RNAs.
Fig. 6 shows a calibration curve of a purified "angiopoietin-related protein 3" by ELISA.
Fig. 7 shows the structure of "angiopoietin-related proteins 1 or 5." The left-hand side corresponds to the amino terminus and the right-hand side corresponds to the carboxyl terminus. The angiopoietin-related proteins consist of a signal peptide, a helix domain, and a fibrinogen-like domain from an amino terminus.
Fig. 8 shows the influence of a fusion protein on concentration of neutral fat in blood in the KK/Snk mouse.
Fig. 9 shows the result of the Western blot analysis using plasma of a mouse infected with a recombinant adenovirus as a sample. The numbers on the left-hand side of the lanes show the molecular weight (KDa) of molecular weight markers.
Fig. 10 shows a results of measurement of concentration of neutral fat in the peripheral blood of a mouse infected with a recombinant adenovirus.
Fig. 11 shows an effect on LPL activity of angiopoietin-related protein 3 wherein a mutation is introduced into the heparin binding site.

### EXAMPLES

The present invention will be explained below in more detail with reference to examples, but the present invention is not limited thereto. In addition, in the following examples, genetic manipulation was conducted according to the method described in 1989 "Molecular Cloning" [Sambrook, J., Fritsch, E. F. and Maniatis, T., Cold Spring Harbor Laboratory Press], unless otherwise indicated. Alternatively, if a commercial reagent and a commercial kit were used, it was conducted according to directions therein.

### Reference Example 1: Cloning of cDNA

A cDNA having the nucleotide sequence shown in SEQ ID No. 1 of the Sequence Listing was obtained using mouse liver as a material according to the following method.

### a) Extraction of mRNA from mouse liver

Two 9 week old KK mice (male, obtained from the Animal experiment institution attached to Hamamatsu University School of Medicine) were dissected, and their livers were extracted and put into liquid nitrogen promptly for quick freezing. The weight was measured and 3.1 g of liver was ground in a mortar in the presence of liquid nitrogen. Thereto was added 5.5 M guanidine thiocyanate buffer solution (hereinafter referred to as GT) (5.5 M guanidine thiocyanate, 25 mM sodium citrate (pH 7.0), 0.5% sarkosyl, 0.2 M β-mercaptoethanol) (30 ml). Then, the sample was crushed with a pestle, then GT buffer solution (10 ml) was newly added, and crushed with the pestle, and the solution was recovered. Then, the mortar was washed with GT buffer solution (20 ml) and the solution was also recovered. After this, 36 ml of the recovered solution was centrifuged at 3000 rpm, at 10 °C for 10 minutes, the supernatant was transferred to a new tube, and the supernatant was drawn up and expelled 20 times using an 18 gauge injection needle. Then, total RNA was separated by density-gradient centrifugation using cesium trifluoroacetic acid (CsTFA). CsTFA undiluted solution (19 ml) was diluted with ribonuclease-free distilled water (18.924 ml), and the diluent (6.18 ml) was put into six 13PA tubes (manufactured by Beckmann), and the sample (5.2 ml per one tube) collected previously was layered thereon. After centrifugation at 30000 rpm (about 125000 g), at 20 °C with an ultracentrifuge (Hitachi SCP70H type) for 20.5 hours using a swing rotor (Hitachi Koki CO., LTD. P40ST), the pellet obtained by removing the supernatant was suspended in 3.3 ml of a buffer solution for extraction using an mRNA purifying kit (Quick prep mRNA purifying kit manufactured by Amersham Pharmacia). The mRNA was purified using the purifying kit according to the protocol provided. A lambda-phage cDNA library was produced, using 5 µg of mRNA thus obtained as a template with a cDNA library production kit (ZAP express, cDNA Giga pack III gold cloning kit manufactured by Stratagene) according to the protocol provided.

### b) Primary Screening of a cDNA Library

Escherichia coli infected with the lambda-phage cDNA library obtained by the above-mentioned method was spread on an agar plate (NZY culture medium: 0.5 % sodium chloride, 0.2 % magnesium sulfate heptahydrate, 0.5 % yeast extract, 1 % casein hydrolysate and 1.5 % agar) prepared in a culture laboratory dish with a diameter of 9 cm so that 1.8 x 10⁵ plaques per plate may be formed, and cultured at 37 °C for 8 hours. At 14 places on the agar on which plaque was formed, the agar including the plaque was sampled using the tip of a large diameter 250 µl pipette (manufactured by RAININ), and the agar samples were each put into a plastic centrifugation vessel containing 100 µl of SM buffer solution (0.1 M of sodium chloride, 8 mM of magnesium sulfate, 50 mM of Tris-hydrochloride (pH 7.5), and 0.01 % of gelatin), agitated using a vortex mixer until cloudy and then left at 4 °C for 1 to 2 hours. Then, the supernatants were recovered by centrifugation at 12000 x g for 5 minutes, and used as a phage suspension.

As a primer used for PCR, oligonucleotides having the following nucleotide sequences were synthesized using an automatic DNA-synthesis machine (model 394: product manufactured by Perkin-Elmer Japan, Applied Biotechnology Systems Operation Division) according to a phosphoramidite method (Matteucci, M. D., and Caruthers, M. H. (1981) J. Am. Chem. Soc. 103, 3185-3191).
Primer 1: 5'-gactgatcaa atatgttgag ctt-3' (SEQ ID No. 5 of the Sequence Listing);
Primer 2: 5'-tgcatccaga gtggatccag a-3' (SEQ ID No. 6 of the Sequence Listing).

5 µl of the thus obtained phage suspension were mixed with 2.5 µl of a buffer solution for 10xPCR (provided with Taq polymerase, manufactured by TAKARA SHUZO CO., LTD.), 4 µl of a dNTP mixture (2.5 mM each, provided with Taq polymerase, manufactured by TAKARA SHUZO CO., LTD.), 1 µl each of the above-mentioned primers 1 and 2 adjusted to 7.5 µM, 0.25 µl of Taq polymerase (manufactured by TAKARA SHUZO CO., LTD.) and 11.25 µl of sterilized water, and then the mixture was heated first at 94 °C for 5 minutes, a cycle of 30 seconds at 94 °C, 30 seconds at 55 °C, and 30 seconds at 72 °C was repeated 30 times, then the mixture was finally kept for 7 minutes at 72 °C was repeated 30 times, and it was then stored at 4 °C. The reaction mixture was subjected to electrophoresis on a 4 % agarose gel (NuSieve 3:1 agarose (manufactured by FMC bioProducts)), and analyzed to detect amplification of a specific fragment. As a result of the above screening of 14 phage suspensions, two positive samples in which the intended cDNA fragment was amplified were obtained.

### c) Secondary Screening

The DNA fragments amplified by performing PCR under the conditions described in b) above using 100 ng of a mouse genomic DNA (manufactured by Clontech) as a template were collected by performing agarose electrophoresis. DNA fragments labeled with ³²P were produced with a multi-prime DNA labeling system (manufactured by Amersham Pharmacia) using these DNA fragments as templates, and the reaction mixture was poured into a nick column (manufactured by Amersham Pharmacia). 400 µl of TE (10 mM Tris-hydrochloride (pH 7.5), 1 mM EDTA) were passed through the column once for washing, and a further 400 µl of TE were passed through, and the eluates were collected. All of the eluted fractions were used for the following secondary screening as a labeled probe.

Alternatively, the phage suspension judged to be positive by b) above was diluted with SM buffer solution 100 times, Escherichia coli infected with 2 µl of the phage was spread on an agar plate prepared on a laboratory culture dish with a diameter of 9 cm and cultured at 37 °C for 8 hours. On this agar on which the plaques were formed, a circular nylon membrane (manufactured by Amersham Pharmacia, High bond N+) fitted to the inside diameter of the laboratory dish was placed, and the plaques were transferred by leaving the membrane for 5 minutes at 4 °C. At three places, the membrane was punctured to the agar using an 18 G injection needle to mark the position, and then the membrane was removed, dipped in an alkali solution (1.5 M sodium chloride, 0.5 M sodium hydroxide) for 2 minutes, then in a neutralization solution (1.5 M sodium chloride, 0.5 M Tris-hydrochloride (pH 8.0)) for 5 minutes, and further in a solution containing 2 x SSC, 0.2 M Tris-hydrochloric acid (pH 7.5) for 30 seconds, and subsequently air-dried completely at room temperature.

After incubating (pre-hybridization) the membrane in 20 ml of a hybridization solution (Express Hyb hybridization solution, manufactured by Clontech) at 68 °C for 1 hour, the solution was replaced by 8 ml of a hybridization solution containing a labeled probe, and incubated at 68 °C for 6 hours. Then, the membrane was washed three times with a solution containing 2 x SSC, 0.05 % of SDS at room temperature for 15 minutes, then the membrane was washed three times with a solution containing 2 x SSC, 0.05 % of SDS with gentle shaking for 15 minutes, and further the membrane was washed 3 times with a solution containing 0.1 x SSC, 0.1 % of SDS at 50 °C for 30 minutes.

The membrane after washing was subjected to autoradiography, and the original plaques at the position recognized to be positive were collected from the agar, and the phage suspension subjected to PCR under the same conditions indicated in b) above. Specific amplification of the DNA fragment was recognized in six cases among ten positive plaque samples.

The phage suspension from the sample with the greatest amount of amplification, was subjected to in vivo excision using a helper phage and a host bacterium which were provided with a ZAP express cDNA Giga pack III gold cloning kit (manufactured by Stratagene) according to the protocol provided with the kit, to make Escherichia coli colonies which contain a phagemid on the agar. These colonies were isolated, and the phagemids were extracted from each colony, and subjected to PCR by the method described in b) above. As a result, colonies in which specific amplification of the DNA fragment was recognized were chosen and cultured, to isolate transformed Escherichia coli which carried phagemid #55-1 having the cDNA insert of 1.6 kbp, E.coli pBK/m55-1 SANK72199.

The entire nucleotide sequence of the cDNA incorporated in phagemid #55-1 thus obtained was analyzed. As a result, it was revealed that the sequence shown in SEQ ID No. 1 of the Sequence Listing (however, the nucleotide number 1-8 of SEQ ID No. 1 of the Sequence Listing is the adapter sequence originating from the vector) was incorporated in the phagemid. This sequence is identical to that of a sequence (registration number: AF162224) registered into the GenBank database as a mouse "angiopoietin-related protein 3 ". In addition, transformed Escherichia coli, E.coli pBK/m55-1 SANK72199 carrying the phagemid #55-1 was internationally deposited on Nov. 19, 1999 with the Kogyo Gijutsuin Seimei-Kogaku Kogyo Gijutsu Kenkyujo (International Patent Organism Depositary, National Institute of Advanced Industrial Science and Technology at Central 6, 1-1-1 Higashi, Tsukuba, Ibaraki, Japan) and was accorded the accession number FERM BP-6940.

### Example 1: Northern Blotting Analysis

### a) Extraction of Total RNA from Mouse Organs

Northern blot analysis was carried out to determine the organ in which cDNA obtained in the Example 1 was expressed. First, total RNA was extracted from the testis, spleen, kidney, small intestine, liver, and brain of KK mouse (hyperlipidemic mouse) and a KK/Snk mouse (hypolipidemic mutant mouse, Shiraki et al., the 7th diabetes animal study group (1993)). The 18 week old KK mouse and the KK/Snk mouse were dissected, then each of the organs thereof was extracted, and then put in liquid nitrogen promptly to be cooled rapidly, and stored at-80 °C. About 15 ml of TRIzol reagent (manufactured by Gibco BRL) was added to 0.5 g each of the organs, and homogenized on ice using an ultra high-speed homogenizer Polytoron (manufactured by Ckinematica) (dial 6, for 2 minutes). After incubation for 5 minutes at room temperature, 3 ml chloroform were added thereto and it was vigorously mixed by hand for 15 seconds. After incubation for a further 3 minutes at room temperature, the samples were centrifuged at 12000 x g and at 4 °C for 15 minutes. The upper layer was collected after centrifugation, and 0.8 volume of ribonuclease-free isopropyl alcohol was added and mixed. After incubation at room temperature for 10 minutes and then centrifugation at 12000 x g at 4 °C for 10 minutes, the supernatant was removed and ribonuclease-free 70 % ethanol was added thereto. After centrifugation at 12000 x g at 4 °C for 10 minutes, the supernatant was removed and the precipitate was dried and then stored at -80 °C

### b) Electrophoresis and Blotting of Total RNA

The total RNA from each collected organ was made up to 4 µg/µl with ribonuclease-free distilled water, and then 5 µl of the RNA solution and 16 µl of a RNA sample buffer solution (1.15xMOPS buffer solution (1 x MOPS buffer solution contains 20 mM of MOPS, 5 mM of sodium acetate and 1 mM of ethylenediamine tetraacetic acid (hereinafter referred to as "EDTA")), 2.4 M of formaldehyde, 57 % of formamide, 7 % of glycerol, 18 µg/ml of Bromophenol Blue, 18 µg/ml of xylene cyanol, and 0.18 mM of EDTA) were mixed, kept at 65 °C for 10 minutes, and then left on ice for 5 minutes. Each entire sample solution was applied into one well on the agarose gel for electrophoresis (1 x MOPS buffer solution, 1.17% agarose (high strength, for analysis, manufactured by Bio-Rad), 0.66 M formaldehyde) containing 1.17% formalin, and subjected to electrophoresis. Electrophoresis was performed at 50 V for about 1 hour, and then at 100 V for about 1.5 hours, in the submerged electrophoresis apparatus filled with 1 x MOPS buffer solution containing 500 ng/ml ethidium bromide. After electrophoresis, the RNA in the agarose gel was transferred to a nylon membrane (High bond N+, manufactured by Amersham Pharmacia) overnight (20x SSC was used as the solution for transferring) according to the capillary transfer method (Maniatis, T. et al. (1982) in "Molecular Cloning A Laboratory Manual" Cold Spring Harbor Laboratory, NY). The membrane was washed with 2x SSC for 5 minutes and air dried, and then irradiated with ultraviolet rays using an ultraviolet ray irradiating apparatus to crosslink (Spectrolinker XL-1000, manufactured by Tomy seiko) (1200 J/cm²), and thereby the RNA was fixed.

### c) Preparation of a Probe

PCR was performed under the following conditions using a thermal cycler (Gene Amplifier PCR System 9600, manufactured by Perkin-Elmer Japan Applied Biotechnology Systems Operation Division) using the primer synthesized in b) of Reference Example 1. After adding sterilized water to the primer (final-concentration 0.5 µM each) and Tween 20 (manufactured by Sigma, final concentration of 0.1%) to give 7.5 µl, 7.5 µl of 2 x PCR solution premix Taq (manufactured by TAKARA SHUZO CO., LTD.: 0.05 unit/µl Taq polymerase, 0.4 mM dNTPs, 20 mM Tris-hydrochloride (pH 8.3), 100 mM potassium chloride, and 3 mM magnesium chloride) were added. Furthermore, 1 µl (an equivalent to 100 ng) of mouse genomic DNA (manufactured by Clontech) was added thereto, and thereby the reaction mixture was prepared. After heating the reaction mixture for 3 minutes at 94 °C first, a cycle of heating at 94 °C for 30 seconds, at 55 °C for 1 minute and at 72 °C for 45 seconds was repeated 35 times, and then the mixture was kept at 4 °C.

One µl of the reaction mixture after PCR was taken, and the amplified DNA fragment was cloned into a plasmid vector using a TA cloning kit (Dual promoter version A, manufactured by Invitrogen) according to the protocol provided with the TA cloning kit. A competent strain of Escherichia coli was transformed with the recombinant plasmid vector, and cultured on LB agar containing 50 µg/ml ampicillin. The Escherichia coli colonies showing ampicillin resistance that grew as a result were chosen, and cultured at 37 °C overnight in 4 ml of liquid LB culture medium containing 50 µg/ml of ampicillin. From 3.5 ml of this liquid culture, plasmid DNA was collected using a plasmid automatic extractor (PI-50, manufactured by Kurabo Industries, Ltd.). The nucleotide sequence of the plasmid DNA obtained was analyzed, and the plasmid in which the target PCR product was incorporated was used for the following operations.

After digesting 8 µg of the selected plasmid DNA with the restriction enzyme EcoRI, phenol/chloroform extraction and ethanol precipitation were performed. The precipitate obtained was dissolved in 10 µl of sterilized water. To the solution was added 2 µl of a dye solution (0.25% Bromophenol Blue, 0.25% xylene cyanol, 15% Ficoll (Type 400)), and then subjected to polyacrylamide gel electrophoresis (8% gel concentration, 100V, at room temperature, for 3 hours). After electrophoresis, the gel was stained with ethidium bromide, a piece of the gel at the band equivalent to the target DNA (about 200 bp(s), SEQ ID No. 11 of the Sequencing Listing) under ultraviolet irradiation was cut out with a razor blade, and transferred to a microdose centrifuge tube and broken up. Thereto was added 300 µl of an elution buffer solution (0.5 M ammonium acetate, 10 mM EDTA (pH 8.0), 0.1% SDS), and this was kept at 37 °C overnight, and then phenol/chloroform extraction was performed twice, and ethanol precipitation was performed once, and the precipitate was dissolved in 20 µl of sterilized water.

Using 5 µl of the DNA solution obtained, a probe (400 µl) labeled with ³²P was prepared by method of c) of Reference Example 1.

### d) Hybridization

After putting the membrane prepared in the above b) into 20 ml of hybridization solution (Express Hyb hybridization solution, manufactured by Clontech) and carrying out an incubation at 68 °C for 1 hour (pre-hybridization), incubation was carried out at 68 °C in 20 ml of a hybridization solution containing a ³²P labeled probe overnight. Then, the membrane was washed three times with a solution which contains 2x SSC and 0.05 % SDS at room temperature for 20 minutes and 3 times with a solution containing 0.1 x SSC and 0.1 % SDS at 50 °C for 20 minutes, and thereafter autoradiography was performed.

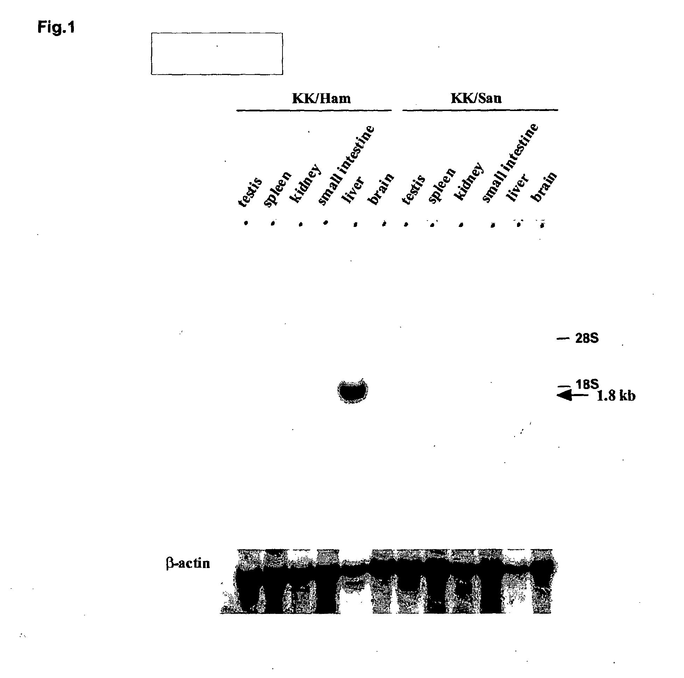

Consequently, expression of the detected gene was seen only in the liver, and it became clear that the expression level thereof was significantly reduced in the KK/Snk mouse (hypolipidemic mouse) compared with in the KK mouse (hyperlipidemic mouse) (FIG. 1).

In the above-mentioned experimental system, the effect of the test susbtance as a therapeutic or preventive agent for hyperlipidemia can be investigated by preparing an RNA sample from primary culture hepatocytes of KK mouse cultured in the presence or absence of the test substance, and performing the same operation as above. Test substances which reduce the expression level of the gene detected in this experiment may serve as a therapeutic or preventive agent for one or more diseases selected from hyperlipidemia, arteriosclerosis, and hyperglycemia. When performing a high throughput screening, electrophoresis can be omitted and a dot blot or a slot blot can be performed.

### Reference Example 2: Cloning of Human cDNA

### 1) Preparation of a Probe

In order to obtain human cDNA corresponding to the mouse cDNA shown in SEQ ID No. 1 of the Sequence Listing, oligonucleotide primers having the following nucleotide sequence were synthesized: 5'-tcctctagtt atttcctcca g-3' (SEQ ID No. 7 of the Sequencing Listing); and 5'-tggtttgcca gcgatagatc-3' (SEQ ID No. 8 of the Sequence Listing).

Then, one µl of human genome DNA (manufactured by Boehringer Mannheim, 200 mg/ml), one µl of Taq polymerase (rTaq, manufactured by TAKARA SHUZO CO., LTD., five units/µl), 10 µl of 10 x buffer solution for PCR (manufactured by TAKARA SHUZO CO., LTD.), 16 µl of dNTP mixed solution (2.5 mM each), 2 µl each of 20 µM primer, and 68 µl of sterilized water were mixed. The reaction mixture was heated at 94 °C for 5 minutes, and then a temperature cycle of heating at 94 °C for 30 seconds, at 55 °C for 30 seconds and at 72 °C for 30 seconds was repeated 30 times, and then it was finally heated at 72 °C for 10 minutes, and then stored at 4 °C. The reaction solution was subjected to electrophoresis on a 2% agarose gel, and an amplified DNA band on the gel part was excised and purified. The DNA thus obtained was labeled with ³²P using a DNA labeling kit (BcaBest labeling kit, manufactured TAKARA SHUZO CO., LTD.), and used as a probe in the following 2).

### 2) Primary Screening of cDNA Library

1 x 10⁶ plaques of DNA from commercial cDNA libraries originating from human liver (Human Liver 5'-STRETCH cDNA Library, manufactured by Clontech) were fixed to a nylon membrane. Namely, Escherichia coli infected with the cDNA library was spread on 20 agar plates which were created on laboratory culture dishes with a diameter of 9 cm so that 5 x 10⁴ plaques per dish were formed, and then cultured at 37 °C for 8 hours. On the agar on which plaque formation had been carried out, a circular nylon membrane (manufactured by Amersham Pharmacia, High bond N+) fitted to the inside diameter of the laboratory dish was placed thereon, and the plaques were transferred thereto by incubation for 5 minutes at 4 °C. The membrane was pierced at three places and the agar thus was marked using an 18G injection needle to mark the position, and then the membrane was removed, dipped in an alkali solution (1.5 M sodium chloride, 0.5 M sodium hydroxide) for 2 minutes, then in a neutralization solution (1.5 M sodium chloride, 0.5 M Tris-hydrochloride (pH 8.0)) for 5 minutes, and then in a solution containing 2x SSC and 0.2 M Tris-hydrochloride (pH 7.5) for 30 seconds, and subsequently air-dried completely at room temperature. Then, using UV irradiation apparatus (Spectro-linker XL-1000, manufactured by Tomy seiko) (1200 J/cm²), the DNA was fixed.

The membrane thus prepared was incubated at 65 °C overnight in a hybridization solution (Express Hyb hybridization solution, manufactured by Clontech). Then, the membrane was washed 3 times with a solution containing 2 x and 0.05% of SDS for 15 minutes, and a further 3 times with a solution containing 0.1 x SSC and 0.1% of SDS at 50 °C for 30 minutes, and then subjected to autoradiography.

The plaques at the position of the positive signal identified as a result were collected including the culture medium from the above-mentioned agar plate, and put into 100 µl of a SM buffer solution (0.1 M sodium chloride, 8 mM magnesium sulfate, 50 mM Tris hydrochloride (pH 7.5), 0.01% gelatin) respectively, suspended therein and incubated at 4 °C for 2 hours. The supernatant was collected by centrifugation at 12000 x g for 5 minutes.

Escherichia coli infected with the thus obtained primary positive phage liquid was cultured on agar medium produced on a laboratory culture dish with a diameter of 9 cm so that 500 plaques per laboratory dish were formed, and secondary screening was performed by repeating the above-mentioned operation. Escherichia coli strain BM25.8 (manufactured by Clontech) infected with the obtained secondary positive clone phage was cultured at 37 °C on the agar medium to form Escherichia coli colonies containing the phagemid. The colonies were isolated, cultured in a small amount of the liquid medium, and thereby phagemid was extracted. The cloned insert was analyzed using restriction enzyme digestion, and Escherichia coli, E.coli pTrip/h55-1 SANK72299, comprising the clone #h5-1 having a 1.6 kbp insert was isolated. The nucleotide sequence of the insert of this clone was analyzed, and it was confirmed that it was the same as the cDNA sequence registered in GenBank as human "angiopoietin-related protein 3" (registration number: AF152562) (SEQ ID No. 3 of the Sequence Listing. However, the nucleotide number 1-14 of SEQ ID No. 3 of the Sequence Listing is the adapter sequence originating from the vector.) In addition, the transformed Escherichia coli, E.coli pTrip/h55-1 SANK72299, carrying the phagemid #h5-1 was internationally deposited on Nov. 19, 1999 with the Kogyo Gijutsuin Seimei-Kogaku Kogyo Gijutsu Kenkyujo (International Patent Organism Depositary, National Institute of Advanced Industrial Science and Technology at Central 6, 1-1-1 Higashi, Tsukuba, Ibaraki,Japan) and was accorded the accession number FERM BP-6941.

### Example 2: Production of a Polyclonal Antibody

A peptides having two kinds of amino acid sequences selected from the domain conserved between the polypeptides of mice and humans as an antigen in order to produce an antibody which recognizes each polypeptide, which has the amino acid sequence encoded by the nucleotide sequence shown in SEQ ID No. 1 and SEQ ID No. 3 of the Sequence Listing, namely the amino acid sequence shown in SEQ ID No. 2 of the Sequence Listing and SEQ ID No. 4 of the Sequence Listing: Glu-Pro-Lys-Ser-Arg-Phe-Ala-Met-Leu-Asp-Asp-Val-Lys-Cys (55-1-N1, SEQ ID No. 9 of the Sequence Listing) and Cys-Gly-Glu-Asn-Asn-Leu-Asn-Gly-Lys-Tyr-Asn-Lys-Pro-Arg (55-1-C1, SEQ ID No. 10 of the Sequence Listing) were chemically synthesized (the apparatus used: Perkin-Elmer Japan model 433). However, a cysteine residue was added to the C terminus of the original amino acid sequence in the amino acid sequence of 55-1-N1 to which a Keyhole limpet hemocyanin (hereinafter referred to as "KLH") as a carrier would bind later. On the other hand, the cysteine at the N terminus of 55-1-C1 to which KLH binds originates from the original amino acid sequence.

Then, 11.1 mg of synthetic peptide 55-1-N1 and 21.5 mg of KLH, or 10.2 mg of 55-1-C1 and 21.2 mg of KLH were condensed using N-(6-maleimidecaproyloxy) succinimide (EMCS, manufactured by Dojinkagakukenkyusho) respectively (it was kept at room temperature for 15 hours using 0.02 M phosphate buffer solution (pH 7.5) which contains 8 M urea and 0.9% sodium chloride as a condensation reaction solvent). This reaction mixture was put into 8 M urea solution, dialyzed to running water and dialyzed to purified water, and then freeze-dried, to provide a KLH-bound peptide antigen. One ml of physiological saline was added to about 10 mg of these peptide antigens, and then converted into a fine suspension using an ultrasonic oscillation machine (sonicator), a vortex mixer, a glass rod or the like. Then, the whole amount was made up to 7.5 ml with a physiological salt solution, and one ml each thereof was subdivided to a vial, and then frozen and stored.

When immunizing, the antigen solution in one of the above-mentioned vials was dissolved, mixed with an equal amount of adjuvant, and then injected into the back of two rabbits hypodermically or intradermally, respectively. As the adjuvant, complete Freund's adjuvant was used. For the 2nd or later immunization, incomplete Freund's adjuvant was used. Immunization was performed 4 times every two weeks, after the 2nd immunization, test blood was collected and the antibody titer in the serum was investigated by an enzyme immunoassay (ELISA), a solid phase method. Each antigen peptide was coated on a 96 well plate for ELISA (manufactured by Sumitomo Bakelite CO., LTD., 96 well H type), and a horseradish-peroxidase labeled anti-rabbit IgG antibody was used as a second antibody. The exsanguination was performed 13 days after the 4th immunization.

The antibody was purified after blood collection using an affinity column. Namely, the peptide (55-1-N1: 7.82 mg, 55-1-C1: 8.07 mg) was combined with the carrier EMC-agarose (about 5 ml) which was activated by reaction of N-(6-maleimidecaproyloxy succinimide (EMCS, manufactured by Dojinkagakukenkyusho) with an aminoalkyl agarose (manufactured by Bio-Rad, Affigel 102). The inactivation of the unreacted EMC group was carried out by treatment with 0.1 M mercaptoethylamine hydrochloride (5 mM EDTA being included). 85 ml of antiserum were doubly diluted with PBS (containing 0.02 M phosphate buffer solution (pH 7.0), 0.9% sodium chloride), a precipitate was obtained by ammonium sulfate precipitation (final concentration 40%), this precipitate was dissolved in PBS, and then dialyzed with PBS after desalting. The dialysis liquid was used as the rough IgG fraction. Chromatography with an affinity column was carried out in three steps. Namely, 1/3 quantity of the rough IgG fraction was charged (adsorbed) to the affinity column, and re-charging a flow-through fraction into the column was repeated 3 times. 40 ml of the combined solution of the flow-through fraction and the washing solution were collected as a non-adsorbing fraction. In order to remove substances binding non-specifically to the column, it was washed with PBS containing 1M sodium chloride, and then 4 M magnesium chloride solution, 3.5 M potassium thiocyanate solution, and 0.1 M glycine hydrochloric acid buffer solution (pH 2.3) were applied in the column one by one, and the antibody specifically binding to the peptide fixed on the column was eluted as an affinity-purified antibody. Since the target antibody was contained in the eluate of 4 M magnesium chloride solution and 3.5 M potassium thiocyanate solution, each eluate dialyzed to PBS was used as an antibody in the following sxperiments.

### Example 3: Expression and Western blot Analysis in COS-1 Cell

#h5-1 phagemid DNA obtained in Reference example 2 was digested with restriction enzymes EcoRI and XbaI, and subjected to 8 % polyacrylamide gel electrophoresis, and about 1.6 kb of the fragment containing cDNA was isolated and purified by the method described in c) of Example 1. At the same time, a high expression vector pME18S (Hara, T. et al. (1992) EMBO. J. 11, 1875-, edited by Takashi Yokota, Kennichi Arai, The Biotechnology Manual Series 3, The Gene-Cloning Experimental Method, Yodosha, p18-20) was similarly digested with EcoRI and XbaI, and the ends were dephosphorylated, and ligated to the above-mentioned cDNA fragments using a DNA ligation kit (Version 2, manufactured by TAKARA SHUZO CO., LTD.). Escherichia coli was transformed with the DNA,and the plasmid carried by the resultant transformant was analyzed with the restriction enzyme. The strain having the 1.6 kb DNA fragment was chosen, and designated as pMEh55-1.

Then, the transformed Escherichia coli carrying pMEh55-1 was cultured at 37 °C overnight in 100 ml of liquid LB culture medium containing 50 µg/ml ampicillin. From this medium, pMEh55-1 DNA was collected using a plasmid purifying kit (Wizard purefection plasmid DNA purifying system, manufactured by Promega), and purified by a cesium chloride method.

COS-1 cells were transfected with the thus obtained plasmid pMEh55-1. The transfection into COS-1 cells was performed according to an electroporation method using transgenics equipment GTE-1 manufactured by Shimadzu Corp. Namely, from the flask in which COS-1 cells were proliferated until they became semi-cofluent, the cells were recovered by trypsin-EDTA treatment, and washed with PBS (-) buffer solution (TAKARA SHUZO CO., LTD.). Next, the cells were suspended at 6 x 10⁷ cells/ml in PBS (-) buffer solution. The plasmid DNA (pMEh55-1) collected by the above-mentioned method was diluted to 200 µg/ml with a PBS (-) buffer solution. 20 µl each of the cell suspension and the DNA solution were mixed, put into a chamber with 2 mm spaced electrodes, and pulses of 600V-30 µsec were given twice at intervals of 1 second. After cooling the chamber for 5 minutes at 4 °C, the cell-DNA mixture was added to 10 ml of DMEM which contained 10 % fetal bovine serum, transferred to a laboratory dish, and was cultured under 5% CO₂ at 37 °C overnight. Then, the culture supernatant was removed and the cells were washed with serum-free medium (DMEM), and then 10 ml of DMEM were added, and cultured for three days.

The culture supernatant was collected from the thus-obtained cell culture. 0.3 ml each of the serum-free culture supernatants of COS-1 cells transfected with negative control plasmid pME18S or pMEh55-1 were treated with trichloroacetic acid (hereinafter referred to as "TCA") to precipitate protein, and the precipitate was obtained by centrifugation. The precipitate was washed with acetone cooled on ice, air-dried, dissolved in a sample buffer solution (manufactured by Bio-Rad) containing 2-mercaptoethanol for SDS-polyacrylamide gel electrophoresis (SDS-PAGE). Then, SDS-PAGE was performed under reducing conditions using 4-20 % polyacrylamide density-gradient gel (multi-gel 4/20, manufactured by Daiichikagaku).

After electrophoresis, the band was transferred to a nitrocellulose membrane (manufactured by Bio-Rad) in a transfer buffer solution (192 mM glycine, 20 % methanol, 25 mM Tris) from the polyacrylamide gel using 200 mA conditions for 90 minutes at 4 °C using gel membrane transfer equipment (manufactured by Marisol, NP7513).

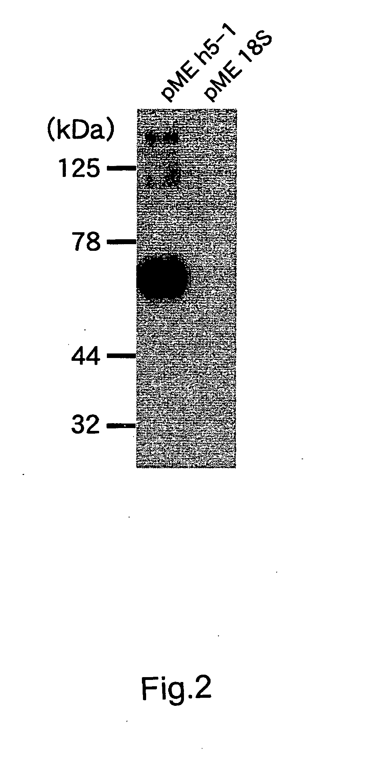

As for the nitrocellulose membrane after transfer, Western blot analysis using the antibody (hereinafter referred to as "55-1-N1 antibody" or "55-1-C1 antibody") obtained in Example 2 was performed. Namely, the nitrocellulose membrane is first washed with PBS containing 0.05% of Tween 20 (hereinafter referred to as "0.05 % Tween 20-PBS") (at room temperature for 15 minutes once and then for 5 minutes twice). Then, it was put into a plastic bag (brand name hybribag, manufactured by Cosmobio), and 20 ml of 0.05% Tween 20-PBS containing 5% skimmed milk (manufactured Snow Brand Milk Products CO., LTD.) were added, and incubated with shaking at room temperature for 1 hour. After one hour, the membrane was taken out, and washed for 15 minutes once, subsequently for 5 minutes twice with 0.05 % Tween 20-PBS. After washing, the membrane was transferred to a new plastic bag, and incubated with shaking after adding 20 ml of 0.05 % Tween 20-PBS containing 55-1-N1 antibody or 55-1-C1 antibody (100 times dilution) and 1% bovine serum albumin (hereinafter referred to as "BSA", manufactured by Sigma). One hour later, the membrane was taken out, washed for 15 minutes once, subsequently for 5 minutes twice with 0.05 % Tween 20-PBS. Then, the membrane was transferred to a new plastic bag, to which was added 20 ml of a solution obtained by diluting 2000-fold horseradish peroxidase labeled anti-rabbit IgG antibody (manufactured by Amersham Pharmacia) with 0.05% Tween 20-PBS containing 1% BSA, and incubated with agitation at a room temperature for 1 hour. One hour later, the membrane was taken out, and washed with a 0.05% Tween 20-PBS solution for 15 minutes once, 5 minutes four times. After washing, the membrane was placed on a wrap film, and the band to which 55-1-N1 antibody or 55-1-C1 antibody binds was detected using ECL Western-blotting detection solution (manufactured by Amersham Pharmacia) leaving it at room temperature for 1 hour, after placing the membrane on a wrap film and dipping it in an ECL Western-blotting detection solution for 1 minute. Thereby, the background was attenuated, and the X-ray film was exposed to it (for 3 seconds)). Consequently, the specific band was detected with both antibodies in the culture supernatant of the COS-1 cells to which the pMEh55-1 plasmid DNA was introduced (FIG. 2).

The same experiment can be conducted also as for the COS-1 cell culture supernatant wherein the mouse cDNA obtained in Reference Example 1 is expressed. Namely, the insert DNA fragment of about 1.6 kb(s) obtained by digesting phagemid #55-1 DNA obtained in Reference example 1 with restriction enzymes EcoRI and XbaI is incorporated in pME18S to construct a clone (pME55-1), which is then introduced into COS-1 cells by the same method as above, and the culture supernatant is collected. When the sample prepared from this culture supernatant was analyzed by Western blotting using 55-1-N1 antibody and 55-1-C1 antibody, a specific band was detected in the culture supernatant of the COS-1 cells to which the pME55-1 plasmid DNA was introduced, using any one of two antibodies for detection.

In the above-mentioned experimental system, the effect of a test substance as a therapeutic or preventive agent for one or more diseases selected from hyperlipidemia, arteriosclerosis, and hyperglycemia can be investigated by preparing a sample from the culture supernatant of a KK mouse primary culture hepatocyte cultured in the presence or absence of the test substance, and performing the same operation described in Example 3. A test substance which reduces the amount of detected antigen in this experiment may serve as a therapeutic or preventive agent for one or more diseases selected from hyperlipidemia, arteriosclerosis, and hyperglycemia. When performing a high throughput screening, the electrophoresis can be omitted and a dot blot and a slot blot can be performed.

### Example 4: Preparation of Recombinant Adenovirus and

### Expression Thereof in Culture Cells

In order to carry out induced expression of the cDNA originating from the mouse obtained in Reference example 1, a recombinant adenovirus was produced using a commercial kit (an adenovirus expression vector kit, manufactured by TAKARA SHUZO CO., LTD.). Namely, the phagemid clone #55-1 obtained in Reference example 1 was digested with restriction enzymes EcoRI and NotI, and the ends of the obtained DNA fragment of about 1.7 kb(s) were blunted, and it was used for the following steps as an insert DNA fragment.

Moreover, a cosmid pAxCA/mAP5 wherein the insert DNA fragment was incorporated at the restriction enzyme SwaI recognition site of the cosmid vector pAxCAwt (provided with the adenovirus expression vector kit) was designed so that expression may be conducted using a cytomegalovirus enhancer and a chiken β-actin promoter. pAxCA/mAP5 DNA and an end protein joint virus DNA (attached to DNA-TPC, adenovirus expression vector kit) were co-transfected to the 293 cells (ATCC CRL 1573) using a calcium phosphate transfection system (manufactured by Lifetech), to isolate a recombinant adenovirus Ad/mAP-5 amplified in 293 cells. Moreover, adenovirus Ad/LacZ carrying a recombinant adenovirus vector into which the LacZ gene cut out from a control cosmid pAxCAiLacZ was incorporated was produced as well, and amplified in the 293 cells. The amplified virus was recovered from the 293 cells by conducting ultrasonication four times for 30 seconds (B-1200, manufactured by Branson) on 293 cells infected with the virus, and then repeating purification by cesium chloride density-gradient centrifugation twice. The obtained virus solution was dialyzed at 4 °C with PBS containing 10% glycerol, frozen and saved at -70 °C or lower until it was used.

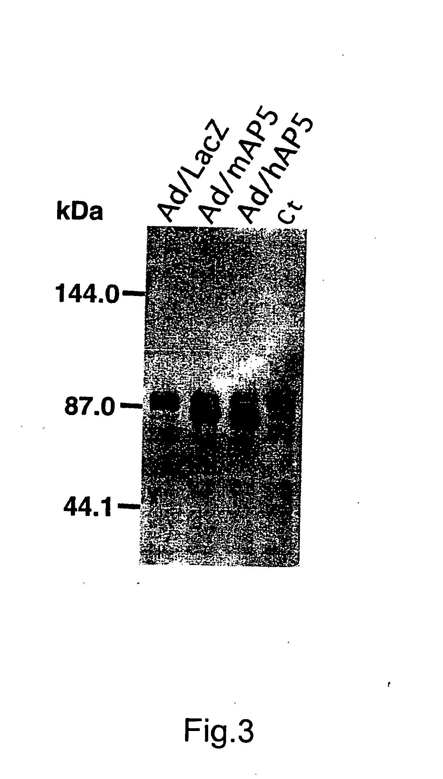

HeLa cells (ATCC CCL2) were infected with the thus obtained recombinant adenovirus at about 5 m.o.i. (multiplicity of infection), cultured in a serum-free medium (Dulbecco's modified Eagle culture medium (DMEM)) for three to four days, and then the culture supernatant was collected. One ml of the supernatant was put into a 1.5 ml volume Eppendorf tube, to which 100 µl of trichloroacetic acid were added, and then left at a room temperature for 3 minutes, and then centrifuged at 15000 rpm with a desk centrifuge apparatus for 5 minutes. After removing the supernatant, 0.5 ml of acetone cooled with ice were added, and stirred well, and it was then centrifuged again at 15000 rpm using a desk centrifuge apparatus for 2 minutes. After removing the supernatant, 0.5 ml of acetone cooled with ice were added again, agitated and centrifuged for 2 minutes at 15000 rpm with the desk centrifuge apparatus. Then, the supernatant was removed, and the precipitate was dried using vacuum evaporation. The precipitate was dissolved in 10 µl of distilled water, mixed with SDS-PAGE sample buffer solution (manufactured by Bio-Rad) containing 2-mercaptoethanol, heated for 5 minutes at 99 °C, and thereby the sample for electrophoresis was prepared. The sample was subjected to electrophoresis through a polyacrylamide density gradient gel at a gel concentration of 4-20 % (buffer solution for electrophoresis: 25 mM Tris, 192 mM glycine, and 0.1% SDS), and then transferred to a nitrocellulose membrane in a transfer buffer solution at 200 mA and 4 °C for 1 hour. The transferred membrane was blocked at 4 °C overnight with PBS to which 0.5% of skimmed milk was added, and then washed three times with a washing solution (0.05% Tween 20-PBS). Subsequently, after putting the membrane into the reaction mixture obtained by mixing the antiserum of 55-1-N1 antibody before purification and the antiserum of 55-1-C1 antibody before purification, which was then diluted 10000 times with 0.05% Tween 20-PBS which contained 5% fetal bovine serum, the membrane was incubated at room temperature for 1 hour, followed by washing 3 times with the washing solution. Furthermore, the membrane was incubated in a reaction mixture obtained by diluting 10000 times horseradish-peroxidase labeled goat anti-rabbit IgG (H+L) (manufactured by Bio-Rad) with 0.05 % Tween 20-PBS which contained 5% fetal bovine serum at room temperature for 1 hour, and then washed 5 times with the washing solution. The membrane was placed on a wrap film, and immersed in an ECL Western-blotting detection solution for 1 minute, then left at room temperature for 1 hour to attenuate the background, and then an X-ray film was exposed to it (for 3 seconds). Consequently, the specific band in the lane of the sample originating from a HeLa cell culture supernatant infected with a recombinant adenovirus Ad/mAP-5 was detected (FIG. 3).

Alternatively, a recombinant adenovirus (Ad/hAP5) which has an adenovirus vector in which cDNA carried by the phagemid clone #h5-1 was incorporated was prepared, and subjected to a similar experiment. In Western blot analysis of the culture supernatant expressed in HeLa cells, a specific band was also detected. (FIG. 3).

### Example 5: Expression in vivo using a Recombinant Adenovirus

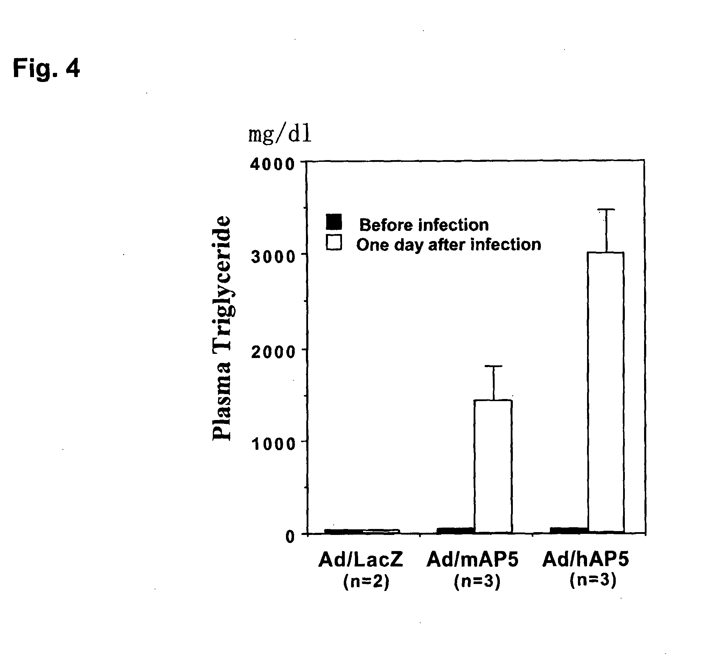

The recombinant adenovirus Ad/mAP-5 or Ad/LacZ purified as mentioned above was diluted with PBS containing 10 % glycerol to give 2 x 10¹⁰ pfu (plaque forming units)/ml, and 100 µl (2 x 10⁹ pfu)/mouse of the diluted solution was inoculated into three (Ad/mAP-5) or two (Ad/LacZ) 13-14-week old male KK/Snk mice by intravenous injection into the tail. One day after inoculation, blood was collected from the eyegrounds of each mouse with a hematocrit pipe, centrifuged at 5200 rpm(s) using a desk centrifuge apparatus for 15 minutes to separate plasma. Then, the concentration of neutral fat was measured using a kit for neutral fat measurement (triglyceride E-test Wako, manufactured by Wako Pure Chemical Industries, Ltd.). In the Ad/LacZ inoculated mouse group, there was no significant difference in the concentration of neutral fat in blood, whereas a significant difference in the concentration of neutral fat in blood (FIG. 4) was detected between the Ad/mAP-5 inoculated mouse group and the other two groups. (FIG. 4).

As for the recombinant adenovirus (Ad/hAP5) which has an adenovirus vector in which cDNA carried by the phagemid clone #h5-1 is incorporated, the same experiment was conducted. Namely, it was expressed in a male KK/Snk mouse. As a result, significant elevation of a concentration of neutral fat in blood was seen, and it became clear that the human type molecule also functions in a mouse (FIG. 4).

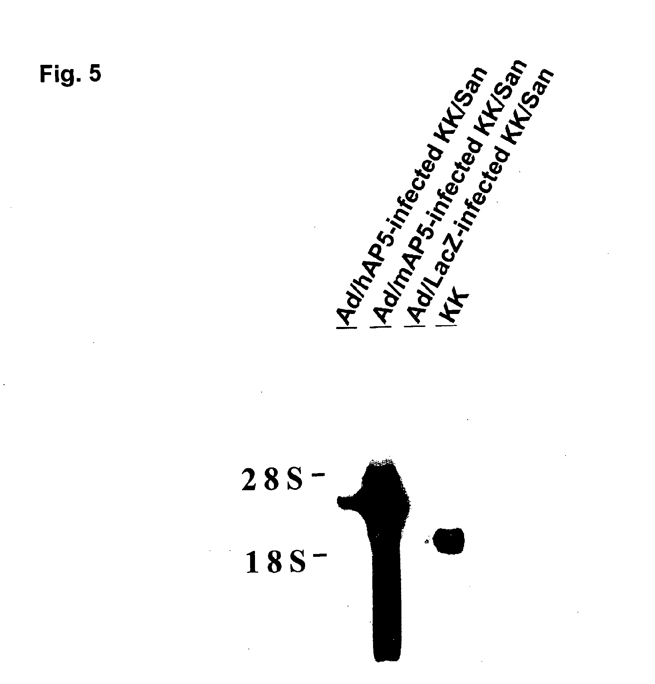

Moreover, when total RNA was collected from the liver of the mouse group infected with the adenovirus in which significant difference was detected in the concentration of neutral fat in blood, and subjected to Northern blot analysis according to the method described in Example 1, it was confirmed that the introduced gene was highly expressed (FIG. 5). Although the band detected in the KK/Snk mouse group infected with the adenovirus is larger than the band detected in the KK mouse which is not genetically manipulated, it is considered that an effective transcription initiation site or the (Poly A) addition signal in a recombinant adenovirus vector are different from those of the endogeneous gene. The difference in the size of this mRNA does not influence the amino acid sequence translated, since the translation termination codon exists just to the 5'-end side of the first translation initiation codon in cDNA incorporated in the recombinant adenovirus vector in the same reading frame.

### Example 6 Purification of Recombinant Protein and Determination of N-terminal Amino Acid Sequence

According to the following method, transformation of the animal cell was carried out using the expression vector pMEh55-1 which was created in Example 3 and in which the human cDNA having the nucleotide sequence shown in SEQ ID No. 3 of the Sequence Listing was incorporated, the recombinant protein secreted in the culture supernatant of the transformed cell was purified, and the N-terminal amino acid sequence thereof was determined.

### (1) Acquisition of a transformant, and preparation of a culture supernatant

Dyhydrofolic acid reductase deficient CHO cells (ATCC CRL-9096) were proliferated in αMEM (manufactured by Gibco BRL) containing 10 % FCS (manufactured by Gibco BRL), 10 units/ml of penicillin and 10 µg/ml of streptomycin (product manufactured by the Gibco BRL), and then transfected with pMEh55-1 plasmid at a rate of 1 µg/10⁶ cells using a transfection reagent (FuGENE6: manufactured by Roche Diagnostics). Specifically, the cells were cultured at 1.5 x 10⁷ cells/laboratory dish on 200 laboratory dishes for cell cultures (150 mm φ, manufactured by Corning). For transfection, 15 µg of pMEh55-1 plasmid per laboratory dish were used. After transfection, the cells were cultured for 24 hours in the culture medium containing the above FCS, and the medium was replaced by serum-free α-MEM (30 ml/laboratory dish). Three more days after exchanging the culture medium, 6 1 of serum-free supernatant were collected.

### (2) Purification of recombinant protein

1) 600 ml of the culture supernatant obtained above in part 1) was applied to a column (stream line C-100: manufactured by Amersham Pharmacia Biotech) which was packed with 1.6 1 of SEPHADEX G25 (manufactured by Amersham Pharmacia Biotech) (flow rate: 20 ml/min). Subsequently, an elution buffer solution (20 mM Tris hydrochloride (pH 7.5), 0.01% sodium azide (manufactured by Sigma), 0.05% protease-inhibitor mixture (manufactured by Sigma), 0.05% Tween 20 (manufactured by Sigma))(hereinafter referred to as "Liquid A") was passed through the column at a flow rate of 50 ml/min, and 1500 ml of the first eluates were collected. The operation was carried out 10 times, and thereby desalting and removal of low weight molecules from the 6 1 of the culture supernatant obtained in
1) above were performed.
2) Next, the eluate obtained in part 1) above was fractionated using FPLC equipment (Biopilot system, manufactured by Amersham Pharmacia Biotech) by ion exchange chromatography under the following conditions.
   Column: XK50/100 column (manufactured by Amersham Pharmacia Biotech) was packed with 100 ml of Q Sepharose fastflow (manufactured by Amersham Pharmacia Biotech).
   Elution buffer solution composition:
   [Liquid A] see the above description
   [Liquid B] 20 mM Tris hydrochloride, 1 M sodium chloride (pH 7.5), 0.01% sodium azide, 0.05% protease inhibitor mixture, 0.05% Tween 20
   Flow Rate: 10 ml/mm
   Fractionation: 10 ml/tube
   Temperature: 4 °C
   Elution conditions: linear gradient from Liquid A 100% to Liquid B 100% (for 60 minutes), the fraction eluted at a sodium chloride concentration of 0.3-0.4 M was collected.
3) The fraction collected by ion exchange chromatography in 2) above was subjected to group specific affinity chromatography under the following conditions using FPLC equipment.
   Column: XK16/40 column (manufactured by Amersham Pharmacia Biotech) was filled up with 10 ml of Affigel blue (manufactured by Bio-Rad).
   Elution buffer solution composition:
   [Liquid C] 20 mM Tris hydrochloride, 0.5M sodium chloride (pH 7.5), 0.01% sodium azide, 0.05% protease inhibitor mixture, 0.05% Tween 20
   [Liquid D] 20 mM Tris hydrochloride, 1M sodium chloride (pH 7.5), 0.01% sodium azide, 0.05% protease inhibitor mixture, 0.05% Tween 20
   Flow Rate: 1.5 ml/min
   Temperature: 4 °C
   After passing the fraction collected in 2) above through the column, 60 ml of Liquid C was passed through to wash, 100 ml of Liquid D was applied, and the eluate with liquid D was collected.
4) An equal amount (100 ml) of Liquid A was added to the eluate obtained in 3) above. It was subjected to group specific affinity chromatography under the conditions indicated below using FPLC equipment.
   Column: XK16/40 column was packed with 10 ml of lentil lectin Sepharose 4B (manufactured by Amersham Pharmacia Biotech). Elution buffer solution composition:
   [C liquid] see the above description
   [F liquid] 20 mM Tris hydrochloride, 0.5 M sodium chloride, 0.3 M methyl mannopyranoside (pH 7.5), 0.01 % sodium azide, 0.05 % protease inhibitor mixture, 0.05 % Tween 20
   Flow Rate: 1 ml/min
   Temperature: 4 °C

After passing the sample through the column, 50 ml of Liquid C were passed to wash, 50 ml of Liquid F were passed and the eluate with Liquid F was collected.

The eluate was transferred to a dialysis tube (exclusion limit molecular weight 10 kDa: manufactured by Gibco BRL), and it was dialyzed at 4 °C overnight to 2 1 of Dulbecco's modified PBS(-) (manufactured by NISSUI PHARMACEUTICAL CO., LTD.) containing 0.01 % sodium azide and 0.1% protease inhibitor mixture. Then, the solution in the dialysis tube was collected. To 4 ml of it was added 1/10 volume (0.4 ml) of TCA containing 4 mg/ml of sodium deoxycholate. The precipitate obtained was collected, and the precipitate obtained by adding acetone was collected, which was then dissolved in 50 µl of sterilized ultrapure water.

### (3) Determination of the N terminal amino acid sequence

PNGaseF (produced by New England Biolab) was added to the sample purified in (2) above, and the N binding type sugar chain was cut. Specifically, 6 µl of 10 x denaturation buffer (attached to the above PNGaseF reagent) were added to 50 µl of the sample, then agitated, and heated in boiling water for 10 minutes. After cooling to room temperature, 6 µl of 10% Nonidet P-40 and 6 µl of 10 x G7 buffer (both provided with the above PNGaseF reagent) were added in order, and the mixture was agitated. 3 µl of the PNGaseF were added and stirred, and then the mixture was kept in a water bath at 37 °C for 2 hours or more.

As for the reaction mixture after this N binding type sugar chain cutting reaction, SDS-PAGE was carried out under reducing conditions using a 4-20 % concentration gradient acrylamide gel (Multi gel 4/20, manufactured by Daiichikagakuyakuhin) and mini-slab electrophoresis equipment (manufactured by Nihoneido). After electrophoresis, the protein separated on the gel was transferred to a poly vinylidene difluoride (PVDF) membrane (manufactured by Bio-Rad) having a pore size of 0.2 µM (2 mA/cm², at 4 °C, for 2 hours) using gel membrane transfer equipment (manufactured by Marisol). After transfer, the membrane was soaked in 100 % methanol (manufactured Wako Pure Chem) for 10 seconds, washed for 2 minutes with ultra pure water, stained with a Coomasie staining solution (manufactured by Bio-Rad) for 5 minutes, and then the membrane was immersed in methanol for two hours for decolorizing. As a result, a band corresponding to about 50 KDa was seen. A part of the band on the membrane was excised and the N terminal sequence was analyzed in a protein sequencer (PPSQ-10, manufactured by Shimadzu Seisakusho).

Consequently, the N terminal amino acid sequence of the above-mentioned band of about 50 kDa was as follows: Ser-Arg-Ile-Asp-Gln-Asp-Asn-Ser-Ser-Phe-Asp (amino acid numbers 17 to 27 of SEQ ID No. 4 of the Sequence Listing)

Therefore, protein encoded by the nucleotide sequence shown in nucleotide numbers 78-1457 of SEQ ID No. 3 of the Sequence Listing was secreted, in the mammalian cell, after 16 residues from the N terminal (amino acid numbers 1-16 of SEQ ID No. 4 of the Sequence Listing) were cleaved, as a mature protein in which the N terminal is serine residue.

As described above, according to the present invention, it was confirmed that genes consisting of the nucleotide sequence shown in the nucleotide numbers 47-1411 of SEQ ID No. 1 of the Sequence Listing or the nucleotide sequence shown in the nucleotide numbers 78-1457 of SEQ ID No. 3 of the Sequence Listing are responsible for diseases which raise the concentration of neutral fat in blood, and therefore substances which control the level of expression of these genes may serve as a therapeutic or preventive agent for one or more diseases selected from hyperlipidemia, arteriosclerosis, and hyperglycemia. Namely, the method of the present invention, the polynucleotide used as a probe or a primer in the method which detects a nucleic acid among the methods, and the antibody used in the method which similarly detects the polypeptide are useful for treatment of hyperlipidemia, or tosearch for a therapeutic or preventive agent for one or more diseases selected from hyperlipidemia, arteriosclerosis, and hyperglycemia.

### Reference Example 3. Purification of a recombinant protein and administration to a mouse

300 µg of the recombinant protein purified in Example 6 was administrated to the tail vein of four KK/Snk male mice, at 15 weeks. To a further four mice PBS was administered (manufactured by Gibco BRL) as a control (0.5 ml/mouse). At 1, 3, 6 and 24 hours after administration, eyegrounds blood was collected from each mouse with a capillary blood collecting pipe, treated with 75 µl heparin (manufactured by Funakoshi) and subjected to centrifugation at 5200 rpm at 4 °C for 15 minutes (using TH-1 rotor manufactured by Tomy Seiko). Then, plasma was separated and collected. Similarly, the plasma was separated from the eyegrounds blood collected before administration of the purified protein, as a sample to provide the value before administration.

Measurement of a concentration of neutral fat in blood is conducted using Triglyceride E test Wako (manufactured by Wako Pure Chemical Industries, Ltd.).

Namely, 2 µl each of the collected plasma was added to a 96 well plate (manufactured by Corning), and 200 µl of a color reagent were added thereto, followed by stirring, and incubation at room temperature for 10 minutes. After completion of the step, the absorbance at a dominant wavelength of 600 nm, and at a complementary wavelength of 700 nm was measured with a micro plate reader. The concentration of neutral fat was calculated from the value obtained by deducting the value measured at a complementary wavelength from the value measured at a dominant wave.

The results are shown in Table 1.

It was shown that administration of the recombinant protein raised the concentration of neutral fat in blood.

**[Table 1]**

| (unit: mg/dl) | | |
|---|---|---|
| | PBS | recombinant protein |
| Before administration | 34.4 | 33.8 |
| 1 hour after administration | 30.5 | 142.8 |
| 3 hours after administration | 35.1 | 99.9 |
| 6 hours after administration | 32.4 | 44.3 |
| 24 hours after administration | 28.6 | 35.1 |

### Reference Example 4. Influence by administration of bezafibrate

For seven days, the antilipemic bezafibrate (manufactured by Sigma) mixed with F2 powder feed (manufactured by Funabashi Noujou) at 0.3% of concentration was administrated to two Zucker fatty rats (purchased from Nihon Charles River) of age 11 weeks.

To the two rats in the control group only F2 powder feed was given. After collecting blood samples from each of the rats under both conditions, the liver thereof was extracted, put in liquid nitrogen immediately to be frozen rapidly, and kept at -80 °C. 5 ml of TRIzol reagent (manufactured by Gibco BRL) were added to 0.1 g of the liver tissue obtained, and it was homogenized on ice using an ultra high-speed homogenizer (Polytoron, manufactured by ckinematica) (at dial 10 for 2 minutes). After incubation for 5 minutes at a room temperature, 1 ml of chloroform was added and mixed vigorously for 15 seconds.

After incubation for 10 minutes at room temperature again, it was subjected to centrifugation at 12000 x g at room temperature for 10 minutes.

Then the upper layer was collected, and 2.5 ml of ribonuclease-free isopropyl alcohol were added, and mixed. After incubation 10 minutes at room temperature, it was subjected to centrifugation at 12000 x g at room temperature for 10 minutes. The supernatant was removed and 70% ribonuclease-free ethanol was added thereto. It was then subjected to centrifugation at 12000 x g at room temperature for 5 minutes, the supernatant was removed, and the precipitate was dried, and kept at -80 °C.

The pellet obtained was dissolved in ribonuclease-free purified water and was used as a total RNA solution.

A single stranded cDNA was produced according to the following procedures. 1 µg of the obtained total RNA was made up to 6 µl with ribonuclease-free purified water. Thereto were added 1 µl of 10 x DNAase reaction buffer solution (attached to DNaseI manufactured by Gibco BRL) and 1 µl of DNaseI (Amp grade, manufactured by Gibco BRL), and the mixture was incubated at room temperature for 15 minutes. Then, 1 µl of 25 mM EDTA (provided with DNaseI) was added, and kept at 65 °C for 10 minutes. To the resultant solution were added 1 µl of a random hexamer primer attached to a single-strand cDNA synthesis kit (Superscript first-strand synthesis system for RT-PCR, manufactured by Gibco BRL), 1 µl of 10 mM dNTP mix, and the mixture was incubated at 65 °C for 5 minutes, and was rapidly cooled to 4 °C, to be thermally denatured. Subsequently, thereto were added 2 µl of 10x reverse transcription reaction buffer provided with the cDNA synthesis kit, 4 µl of 25 mM magnesium chloride, 2 µl of 0.1 M dithiothreitol solution and 1 µl of a ribonuclease inhibitor, incubation was at 25 °C for 2 minutes, and then 1 µl of the reverse transcriptase provided was added. The sample was kept at 25 °C for 10 minutes, and then at 42 °C for 50 minutes. After heating at 70 °C for 15 minutes, it was cooled to 4 °C rapidly, to be thermally denatured. Then, 1 µl of the ribonuclease H provided was added, and the mixture was incubated at 37 °C for 20 minutes to degrade the RNA, and the mixture was stored at -20 °C. After completion of the reaction, 29 µl of ribonuclease-free purified water was added to each of the samples, and diluted with ribonuclease-free purified water a further 5 times for use in the following quantification. The single-stranded cDNA solution for creation of a calibration-curve was prepared by producing a 5-times serial dilution with ribonuclease-free purified water from the solution for quantification derived from one control sample.

Addionally, oligonucleotides having the following sequence:
5'-caaagcttga aagtctactg gagg-3' (SEQ ID No. 30 of the Sequence Listing); and
5'-ggttctgaac caagctggtc a-3' (SEQ ID No. 31 of the Sequence Listing)
were synthesized as primers used for TaqMan PCR (ordered from Amersham Pharmacia).
Furthermore, as a probe for TaqMan PCR, there was synthesized an oligonucleotide with the following sequence
5'-aagatggcgc tccaacacag agtcag-3' (SEQ ID No. 32 of the Sequence Listing), wherein a reporter pigment Fam was bound to 5'-end thereof and a reporter quenching substance Tamura was bound to 3'-end thereof (TaqMan fluorescent probe, manufactured by Applied Biosystems Japan). Moreover, rat 18s ribosome RNA was quantified in order to achieve standardization of the level of expression of "angiopoietin-related protein 3", using TaqMan ribosomal RNA control reagent (manufactured by Applied Biosystems Japan).

The quantification of the level of expression of the rat "angiopoietin-related protein 3" gene was performed using these materials according to the TaqMan PCR method. The PCR reaction was performed in a 96 well reaction plate (Microamp optical 96 well reaction plate, manufactured by Applied Biosystems Japan). The composition of a reaction solution in one well was as follows: 5 µl of single-stranded cDNA solution obtained by the above-mentioned procedure, 0.2 µl of primer for TaqMan PCR (50 pmol/µl) either forward primer or reverse primer (the final concentration being 200 nM), 0.75 µl of a probe for TaqManPCR (6.6 µM) (the final concentration being 100 nM), 25 µl of a mixture for PCR amplification (TaqMan Universal PCR Master Mix, manufactured by Applied Biosystems Japan), and 18.85 µl of ultra pure water. Under the present circumstances, a concentration of the undiluted solution of the single-stranded cDNA solution for creation of a calibration curve was set to "625" for convenience, a 5 times dilution was repeated to prepare the reaction mixtures of concentration "625", "125", "25", "5", and "1". A PCR reaction was carried out using a thermal cycler and a detector for exclusive use for TaqMan PCR(s), (ABI7700, manufactured by Applied Biosystems Japan.) The reaction was carried out at 50 °C for 2 minutes, at 95 °C for 10 minutes, and a cycle of 95 °C for 15 seconds and 60 °C for 1 minute was repeated 40 times. The amount of fluorescence of the reporter pigment was measured for every cycle. The amplification curve of the 18S ribosome and the "angiopoietin-related protein 3" was created from the amount of fluorescence of the reporter pigment for every cycle. From the amplification curve of the serial dilution of the single-stranded cDNA solution for calibration curve creation, a calibration curve wherein a concentration is shown along the horizontal axis, and the number of cycles is shown along the vertical axis was created. As for each sample for expression, the number of cycles at which the amount of fluorescence is beyond the arbitrarily defined value set in logarithmic phase were plotted on the calibration curve, and the relative level of expression was calculated. The level of expression of the "angiopoietin-related protein 3" gene was corrected with the value of the level of expression of an 18S ribosome in the same sample.

Consequently, it was shown that the level of expression of a rat "angiopoietin-related protein 3" gene is decreased by 73% in the bezafibrate-administered group compared to the control group. In addition, the concentration of neutral fat in blood of the rat of the bezafibrate-administered group used at this time was reduced by 50% compared to the control group. Reference-Example 5. Influence on arteriosclerosis and the blood sugar level

The hypolipidemia mouse KK/Snk has a mutant type gene encoding a polypeptide which consists of an amino acid sequence shown in the SEQ ID No. 2 of the Sequence Listing (hereinafter referred to as the "hypolipidemia mutant gene") in homozygous configuration and the amount of the polypeptide produced is hereditarily low. According to the procedure described below, the influence of the hypolipidemia mutant gene in an arteriosclerosis mouse model or a hyperglycemia mouse model was considered.

### 1) Production of a congenic mouse

### a) Crossing method

A female KK/Snk mouse was crossed with a male C57BL/6J mouse(received from an animal experiment institution attached to Hamamatsu Medical College), and the 1st generation (N1 generation) back-crossing was made. Subsequently, an N1 female was again crossed with a C57BL/6J male, and an N2 generation mouse was obtained. Genomic DNA was extracted from the tail of this N2 mouse, and subjected to PCR according to the procedure described below, and a mouse having the hypolipidemia variant gene in heterozygous configuration was selected. The selected female was further crossed with a C57BL/6J male, and an N3 generation was made. Then, selection hybridization was continuously carried out up to the N10 generation by the same procedure. Subsequently, an N10 generation male and female in heterozygous configuration were crossed, and the genome extracted from the progeny obtained was subjected to PCR according to the procedure described in c) below, and a male and a female which have the hypolipidemia mutant gene in homozygous configuration were selected. Then, the breeding was carried out by sibling mating, and a hypolipidemia mutant congenic mouse (C57BL/6J1 hypolipidemia) model of C57BL/6J background was obtained. All animals were bred inside at a room temperature of 23 ± 1 °C, humidity of 55 ± 10 %, and in 14-hour illumination; a CMF pellet feed (manufactured by Oriental Yeast Industry) and the 7 ppm chlorine water were given freely.

### b) Method of extraction of a genomic DNA

A tail (1.5cm) extracted from a mouse (four-week age) used for selection in a) above was immersed in 840 µl of a solution (containing 720 µl of 1 x SSC, 80 µl of 10 % SDS, and 40 µl of 10 mg/ml proteinase K), and incubated overnight, with shaking, at 50 °C. Then, 20 µl of 1 mg/ml ribonuclease A were add thereto, and the mixture was kept at 50 °C for 1 hour.

Subsequently, phenol/chloroform extraction was carried out twice followed by ethanol precipitation once. And then, the precipitate was dissolved in 150 µl of a buffer solution containing 10 mM Tris-hydrochloride (pH 7.5) and 1 mM EDTA. Then, the absorbance at a wavelength of 260 nm was measured with a spectrophotometer (U-3000, manufactured by Hitachiseisakusho). Then, sterilized water was added thereto so that the concentration was adjusted to 25 ng/µl, to provide a sample of genomic DNA.

### c) Judgment of a genotype

PCR was performed under the following condition with a thermal cycler (PTC-100, manufactured MJ research company) using two kinds of primer sets for microsatellite marker detection (D4Mit15 and D4Mit219, manufactured by Research Genetics). Sterilized water was added to 1.5 µl of 10 x PCR buffer (containing 100 mM Tris-hydrochloride (pH 8.3), 15 mM of magnesium chloride, and 500 mM of potassium chloride, manufactured by Roche Diagnotics), 2.4 µl of 1.25 mM dNTPs (manufactured by Takara Shuzo CO., LTD.), 6.7 µM primer of 0.45 µl each, and 0.35 U of the TaqDNA polymerase (manufactured by Roche Diagnotics) so that the total amount was 11 µl. And 4 µl of the genomic DNA prepared in b) above were added thereto to prepare the reaction mixture. The reaction mixuture was heated at 94 °C for 3 minutes, and then the heat cycle of 94 °C for 30 seconds, 55 °C for 1 minute and 72 °C for 45 seconds was repeated 35 times, and then it was further heated at 72 °C for 3 minutes, and was kept at 4 °C.

2 µl of a dye solution (0.15% Bromophenol Blue, 50% glycerol) were added to the reaction mixture after the above PCR, and 5 µl were subjected to electrophoresis at 150 V at room temperature for 1.5 hours to 2 hours on a 4% agarose gel (prepared with NuSieve 3:1 agarose (manufactured by FMC Bioproduts)). The gel after electrophoresis was stained with ethidium bromide, the band which is equivalent to the target DNA was confirmed under a ultraviolet irradiation. A mouse which has genomic DNA wherein two kinds of fragments, about 280 bp and about 300 bp, were amplified by the primer set D4Mit15, and two kinds of fragments, about 110 bp and about 120 bp, were amplified by D4Mit219 was defined as a "hypolipidemia mutant gene heterozygote", and a mouse which has genomic DNA wherein only a fragment of about 300 bp was amplified by D4Mit15, and only a fragment of about 120 bp was amplified by D4Mit219 was defined as a "hypolipidemia mutant gene homozygote."

### 2) Production of a hypolipidemia variant gene homozygote/ApoE homozygote deficient mouse

### a) Selection method

The 1st generation (F1 generation) of cross-breed was produced from a C57BL/6J-hypolipidemia mouse which was obtained in 1) a) above, and the apolipoprotein E (hereinafter referred to as "ApoE") homozygote deficient mouse (Zhang, S.H. et al. (1992) Science 25, 468-471, which was obtained from the medical department organic morbidity medicine classroom of Tokyo University. Subsequently, male and female mice of the F1 generation were crossed to provide an F2 generation. Among F2 generations, a mouse with a deficient ApoE homozygote and which has the hypolipidemia variant gene homozygote (hereinafter referred to as "ApoE KO-hypolipidemia mouse") and a mouse with a deficient ApoE homozygote and which does not have a hypolipidemia mutant gene (hereinafter referred to as "ApoE KO mouse") were selected by judging the genotype according to the following procedure. All animals were bred according to the procedure in 1) a) above.

### b) Method for judgement of genotype

Genomic DNA was extracted from the tail of a mouse according to the method for judgment described in 1) b) above. Oligonucleotide primers for detecting size polymorphorizm in a mutant allele and a wild-type allele were synthesized in order to select a hypolipidemia mutant gene
5'-ggctaaatag taaaaccctg gcg-3' (SEQ ID No. 33 of the Sequence Listing)
5'-gtgcttgctg tctttccagt ctt-3' (SEQ ID No. 34 of the Sequence Listing).

When PCR was performed using this primer set, the fragment of about 240 bp was amplified as for a hypolipidemia mutant gene, and a fragment of about 230 bp was amplified as for the normal gene which does not have mutation.

Moreover, the oligonucleotide primers which consist of the following sequences were synthesised to amplify a genomic sequence in which ApoE gene is deficient (and replaced by neomycin resistance gene) in order to detect ApoE gene deficiency.
5'-aggatctcgt cgtgacccat ggcga-3'(SEQ ID No. 35 of the Sequence Listing ) and
5'-gagcggcgat accgtaaagc acgagg -3'(SEQ ID No. 36 of the Sequence Listing).
(See Gaw. A. et al. (1995) Lab. Anim. 29, 447-449)

When PCR is carried out using this primer set, only when an ApoE deficient variation is in the gene used as a template, is the fragment of 200 bp amplified.

Furthermore, oligonucleotide primers which consist of the following sequences for amplifying wild-type alleles having no ApoE deficiency were synthesized specifically:
5'-tcccaagtca cacaagaact gac -3' (SEQ ID No. 37 of the Sequence Listing); and
5'-catccagaag gctaaagaag gca -3' (SEQ ID No. 38 of the Sequence Listing).

When PCR is carried out using this primer set, only when there is no ApoE deficient variation in the gene used as the template, is the fragment of 174 bp amplified.

PCR and electrophoresis were carried out using the three sets of the above-mentioned primers as described in 1) c) above and the amplified fragment was investigated. As a result, a mouse wherein the fragments of about 240 bp and 200bp were detected was used as the "ApoE KO-hypolipidemia mouse", and a mouse wherein the fragments of about 230 bp and 200bp were detected was selected as an "ApoE KO mouse."

### 3) Production of a hypolipidemia variant gene homozygote / leptin variant gene homozygote mouse

### a) Selection method

C57BL / 6J-hypolipidemia mice obtained by the 1) a) above, and C57BL / 6 J-Lep^{ob} mice with the leptin mutant gene (Lepob) (Colemam, D. L. and Hummel, K. P. (1973) Diabetologia 9:287-293, obtained from the animal experiment institution attached to Hamamatsu Medical College) were crossed, and the 1st generation (F1 generation) back-cross was made. Subsequently, an F1 female and male were crossed to produce an F2 generation. In the F2 generation, the mouse which has both a leptin mutant gene and a hypolipidemia mutant gene homozygote (hereinafter referred to as an "ob-hypolipidemia mouse") and the leptin mutant homozygote which does not have a hypolipidemia variant gene (hereinafter referred to as an "ob mouse") were selected by judging the genotype according to the following procedure.

In addition, all animals were bred by the method described in the above 1) a).

### b) Method for judgment of a genotype

Genomic DNA was extracted from the tail of a mouse by the method described in 1) b) above.

Oligonucleotide primers consisting of the following sequences for amplifying a mutant allele were synthesized specifically in order to detect leptin mutation:
5'-tgacctggag aatctct-3' (SEQ ID No. 39 of the Sequence Listing), and
5'-catccaggct ctctggc-3' (SEQ ID No. 40 of the Sequence Listing):
   (See Namae, M. et al. (1998) Lab. Anim. Sci. 48, 103 - 104)

When PCR is carried out using this primer set, only when a mutant allele is the gene used as a template, is the fragment of about 100 bp amplified.

Furthermore, oligonucleotide primers consisting of the following sequence for amplifying specifically the wild-type allele having no leptin mutation were synthesized:
5'-tgacctggag aatctcc-3' (SEQ ID No. 41 of the Sequence Listing): and
5'-catccaggct ctctggc-3' (the above, SEQ ID No. 40 of the Sequence Listing)

When PCR is carried out using this primer set, only when the gene having no leptin mutation is used as a template, is the fragment of about 100 bp amplified.

PCR and electrophoresis were carried out using the two sets of the above-mentioned primers and one set of the primers for selecting the hypolipidemia mutant gene described in 2) b) above, according to the method described in 1) c) above, and the amplified fragment was investigated. As a result, the mouse wherein fragments of 100 bp (leptin variation type) and about 240 bp were detected was used as the "ob-hypolipidemia mouse", and the mouse wherein fragments of 100 bp (leptin variation type) and about 230 bp were detected was selected as a "ob mouse."

### 4) Measurement a serum lipid and arteriosclerosis lesion area

After fasting for 17 hours each ApoE KO mouse (three males, 22 -23-week age) and ApoEKO-hypolipidemia mouse (three males, 19 -23-week age), had blood taken from the belly vena cava thereof under anesthesia. Subsequently, the blood was left for 30 minutes, and then subjected to centrifugation at 2000 x g at room temperature for 10 minutes, and the serum was collected. The concentration of neutral fat in serum was measured using Triglyceride E-test Wako (manufactured by Wako Pure Chemical Industries, Ltd.), a concentration of serum cholesterol was measured using Cholesterol C-test Wako (manufactured by Wako Pure Chemical Industries, Ltd.).

Moreover, the thorax of each mouse was cut open under anesthesia, and washed with a physiological salt solution which contains one unit/ml of heparin from the ventriclus sinister. The part from the heart to the aortic-arch was taken out, and the fat was removed until the branching point of the heart and an aortic-valve part appeared. After removal of the fat, the part directly above the aorta was cut, and immersed in a 4 % neutral formalin solution for 24 hours to seven days to be fixed. According to a conventional method, a paraffin block was produced after fixation. This paraffin block was sliced with a microtome from the aortic-arch part side, and after the valve appeared for the first time in the section, five sections were continuously taken, in a thickness of 5 µM, and the next five sections were discarded. This operation was defined as one unit, and slicing was continued to a full length of 500 µM according to the same procedure. The obtained sections were subjected to Elastica Masson staining.

The transection image of the aortic-valve part subjected to Elastica Masson staining was projected on a television monitor, under an optical microscope (BX-50, manufactured by Olympus Optical Industrial company) in which a CCD camera (HC-2000, manufactured by Fuji Photographic-film company) was installed, the border of a lesion was traced using a image analysis program (QWin, manufactured by lyca), and the lesion area was measured.

For each sample, lesion area was measured for ten sections in total at an interval of 50 µM in thickness, and the total calculated.
The result was shown in Table 2.

**[Table 2]**

| | ApoE KO | ApoE KO-hypolipidemia |
|---|---|---|
| Concentration of neutral-fat (mg/dl) | 611.7 ± 223.8 | 178.7 ± 47.4 |
| Total cholesterol (mg/dl) | 1227.3 ± 24.7 | 610.7 ± 163.2 |
| Total of arteriosclerosis lesion area(x 10⁵ µm²) | 10.81 ± 5.48 | 2.55 ± 1.07 |

It was shown from the above result that the mutation of the gene encoding the polypeptide which consists of an amino acid sequence shown in SEQ ID No. 2 of the Sequence Listing controls progress of an arteriosclerosis lesion by reduction of serum lipid.

### 5) Measurement of the blood sugar level

ApoEKO mice (nine males, 24-week age) and ApoEKO-hypolipidemia mice (ten males, 24-week age), ob mice (12 males, 17 -18-week age), and ob 1 hypolipidemia mice (14 males, 17-19-week age) were fasted for 17 hours, then blood was collected from the belly vena cava under anesthesia. Subsequently, after leaving the blood for 30 minutes, centrifugation was performed at 2000 G at room temperature for 10 minutes, and sera were collected.

The blood sugar level was measured using glucose B-test Wako (manufactured by Wako Pure Chemical Industries, Ltd.).

The results as for ApoE KO and ApoE KO-hypolipidemia mice are shown in Table 3, and the results for the ob mice and ob-hypolipidemia mice are shown in Table 4.

**[Table 3]**

| | ApoE KO | ApoE KO-hypolipidemia |
|---|---|---|
| Blood sugar level (mg/dl) | 276.8 ± 69.3 | 121.4 ± 30.0** |

| | | |
|---|---|---|
| **P<0.00.1 | | |

**[Table 4]**

| | ob | ob-hypolipidemia |
|---|---|---|
| Blood sugar level (mg/dl) | 407.2 ± 195.1 | 260.1 ± 146.6* |

| | | |
|---|---|---|
| *P < 0.05 | | |

It was shown from the above result that the variation of the gene encoding the polypeptide which consists of an amino acid sequence shown in SEQ ID No. 2 of the Sequence Listing specifically reduces hyperglycemia due to the ApoE deficient or leptin mutations.

### Example 7 Preparation of monoclonal antibody

### (a) Preparation of antigen protein

### 1) Production of an antigen producing CHO cell strain

The expression vector wherein human angiopoietin-related protein 3 cDNA is located downstream of a pSRα promoter (pME18S plasmid) was produced. This expression vector and pSV2-dhfr (ATCC) were co-introduced into a dihydrofolic acid reductase deficient CHO cell (ATCC) in a 10 % FCS (manufactured by Gibco BRL) - MEMα (manufactured by Gibco BRL) - 10 units/ml penicillin 10 µg/ml streptomycin (manufactured by Gibco BRL) medium, and a stable strain was obtained in a nucleic acid free medium. Then, from the stable strain, a resistant strain which can be grown in the presence of methotrexate at 0.08 µM concentration was produced, and the strain wherein an increase in the level of expression by gene amplification was confirmed was produced. In a similar way, a strain which can be grown in nucleic acid free MEM α medium containing 1.28 and 30 or 60 µM of methotrexate were produced, and finally angiopoietin-related protein 3 producing CHO cell strain which has the ability to produce 5-10 mg/l/3 days in the flask for cell culture in confluent serum-free condition was produced.

The angiopoietin-related protein 3 producing CHO cell strain produced as described above was cultured until it became confluent, and then the medium was replaced with serum-free MEM α medium (manufactured by Gibco BRL), followed by culturing for three days, and thereby a serum-free culture supernatant was obtained.

### 2) Desalting and removing of small molecules

For desalting and elimination of small molecules from the sample, 600 ml of the sample were processed with 1.6 1 of a Sephadex G25 gel (manufactured by Amersham Pharmacia Biotech) at a rate of 20 ml/min in 10 steps. The sample was fractionated by ion exchange chromatography using FPLC (Biopilot system, manufactured by Amersham Pharmacia Biotech).

### 3) Fractionation Step I

Column: Qsepharose Fast Flow (manufactured by Amersham Pharmacia Biotech) was packed with 100 ml of XK50 (manufactured by Amersham Pharmacia Biotech).
Elution buffer solution:
A) 20 mM Tris-HCl (pH 7.5) - 0.01 % NaN₃ (manufactured by SIGMA) - 0.005 % Protease inhibitor cocktail(manufactured by SIGMA) - 0.005 % Tween 20 (manufactured by SIGMA)
B) 20 mM Tris-HCl-1 M NaCl (pH 7.5) - 0.01 % NaN₃ (manufactured by SIGMA) - 0.005 % Protease inhibitor cocktail (manufactured by SIGMA) - 0.005 % Tween 20 (manufactured by SIGMA)
   Flow rate: 10 ml/min
   Fraction solution: 10 ml/tube
   Temperature: 4 °C
   Elution conditions: the eluent A- the eluent B (a linear gradient, 60 minutes).
   The fractions eluted with 0.3 - 0.45 M NaCl concentration were collected and combined into one. Group specific affinity chromatography was carried out under the following condition using FPLC (manufactured by a Amersham Pharmacia Biotech), using the combined solution as a sample.

### 4) Fractionation Step II

Column: KX16 (manufactured by a Amersham Pharmacia Biotech) was packed with 10 ml of Affi Gel Blue (BioRad).
Elution buffer solution:
C) 20 mM Tris-HCl-0.5M NaCl(pH 7.5) - 0.01 % NaN₃ (manufactured by SIGMA)-0.005% Protease inhibitor cocktail(manufactured by SIGMA) - 0.005 % Tween 20 (manufactured by SIGMA)
D) 20 mM Tris - HCl - 1M NaCl(pH 7.5) - 0.01 % NaN₃ (manufactured by SIGMA)-0.005 % Protease inhibitor cocktail (manufactured by SIGMA)-0.005 % Tween 20 (manufactured by SIGMA)
The rate of flow: 1.5 ml/min
Temperature: 4 °C

After charging the sample, 60 ml of C solution were applied.
Elution conditions: 100 ml of solution D were used and this was collected as one solution. The group specific affinity chromatography using FPLC (manufactured by Amersham Pharmacia Biotech) was carried out under the following conditions, using 200 ml of a solution obtained by mixing the collected solution with equal volume (100 ml) of A solution as a sample.

### 5) Fractionation Step III

Column: KX16 (manufactured by Amersham Pharmacia Biotech) was packed with 10 ml of Lentil lectin Sepharose™ (manufactured by Amersham Pharmacia Biotech).
Elution buffer solution:
C) 20 mM Tris-HCl-0.5 M NaCl(pH 7.5) - 0.01 % NaN₃ (manufactured by SIGMA) - 0.005 % Protease inhibitor cocktail(manufactured by SIGMA) - 0.005 % Tween 20 (manufactured by SIGMA)
F) 20 mM Tris - HCl-0.5 M NaCl - 0.3 M Methylmannopyranoside (pH 7.5) - 0.01 % NaN₃ (manufactured by SIGMA) - 0.005 % Protease inhibitor cocktail (manufactured by SlGMA) - 0.005 % Tween 20 (manufactured by SIGMA)
Rate of flow: 1 ml/min
Temperature: 4 °C

After charging the sample, 50 ml of C solution were applied.
Elution conditions: 50 ml of solution F were applied and this was collected as one solution.

### 6) Dialysis

The collected solution was transferred to Dialysis tubing - 1 - 7/8 in Diameter (manufactured by GibcoBRL), and dialyzed at 4 °C overnight against 2 l of a solution of Dulbecco's PBS (-) (Nissui Pharmaceutical company), 0.01 % NaN₃ (manufactured by SIGMA) - 0.1 % Protease inhibitor cocktail (manufactured by SIGMA).

### 7) Confirmation of the sample

After dialysis, concentration of the collected sample was measured using a DC protein assay kit (manufactured by Bio Rad), western blot analysis, and SDS-PAGE, and the amount of "angiopoietin-related protein 3" protein was measured. Endotoxin concentration was measured with an endotoxin measurement kit (Seikagakukogyo), and it was confirmed to be 20 mg/mg or less. The purified protein was used as an antigen.

### (b) Preparation of immunized mouse splenic cells

0.5 ml of Freund's complete adjuvant (manufactured by Difico) to 0.5 ml (corresponding to 100 µg) of the angiopoietin-related protein 3 purified in (a), and an emulsion were produced in an injection syringe equipped with a connected needle. Three eight week age BALB/c male mice were immunized by subcutaneous injection of the emulsion as an adjuvant. In the 2nd or later immunization, Freund's incomplete adjuvant (manufactured by Difico) was used as an adjuvant. After the 2nd immunization, immunization was performed 4 times every two weeks. The blood was collected from the eyeground venous plexus just before carrying out immunization, and the anti-"angiopoietin-related protein 3 " antibody titer in the serum was investigated by enzyme immunoassay (the ELISA method) according to the solid phase method described below.

### [Solid-phase enzyme immunoassay]

50 ng of the purified "angiopoietin-related protein 3" were dispensed to each well of a 96 well plate for ELISA (Maxisoap, manufactured by Nunc), and left at 4 °C overnight. Thereby, the antigen coated on the bottom of the plate well. After washing the plate three times with PBS containing 0.1 % (v/v) of Tween 20, 10 µg/ml BSA solution in PBS was added at 100 µl/well on the above antigen-coated plate, and left at the room temperature for 1 hour. It was washed three times with PBS containing 0.1 % (v/v) Tween 20, the sample (mouse serum) which was diluted serially was added as the first antibody at 30-100 µl/well, and was incubated at room temperature for 1 hour. Then, it was washed three times with PBS containing 0.1 % (v/v) of Tween 20, peroxidase-labeled goat anti-mouse IgG (manufactured by Bio-Rad) diluted 3000 times was added as a second antibody at 100 µl/well, and left at room temperature for 1 to 2 hours. It was washed three times with PBS containing 0.1 % (v/v) Tween 20; TMB peroxidase substrate (TMB peroxidase kit, manufactured by Bio-Rad) was added at 100 µl/well, and left at room temperature for 30 minutes. Then, 1N sulfuric acid was added at 100 µl/well to stop the peroxidase reaction, absorbance at 450 nm was measured with a micro plate reader (manufactured by Bio-Rad), and the antibody titer was calculated.

### (C) Preparation of a mouse myeloma cell

8-azaguanine resistant mouse-myeloma cell P3 - X63-Ag8.653(653) was cultured in the TIL medium, and 2 x 10⁷ or more cells were obtained.

### (d) Production of a hybridoma

1.5 x 10⁷ of mouse-myeloma cells P3-X63-Ag8.653(653) were mixed with 1.4 x 10⁸ immunized mouse splenic cells which were washed well with DMEM (manufactured by Nissui pharmaceutical), and centrifuged at 800 rpm for 6 minutes. After resuspending the precipitated cell mixture comprising of the splenic cells and P3-X63-Ag8.6531653, polyethylene glycol 4000 (PEG 4000) solution previously prepared to 50% with DMEM was added dropwise with stirring so that it might be 2 ml/min for 1 minute. Then, DMEM was added dropwise for 1 minute at 2 ml/min, and this operation was repeated once again. Then, 16 ml or more of DMEM was added in 3 minutes, and centrifuged at 800 rpm for 6 minutes. The supernatant after centrifugation was discarded and the pellet was suspended in 35 ml of TIL medium.

### (e) Selection of a hybridoma group

The suspension was dispensed at 100 µl/well on 96 well plate (Coaster), and cultured at 37 °C in 7.5 % of CO₂ incubator. HAT medium was added at 50 µl/well seven days later. The same amount of HAT medium was similarly added thereto a further four days later. A sample of the culture supernatant in the well in which the fusion cells were grown in the shape of a colony was extracted three days later, and the anti-"angiopoietin-related protein 3" antibody titer was measured by the same solid-phase enzyme immunoassay as (b) above, and was transferred to the HT medium at the same time.

### (f) Cloning

For the well in which the antibody titer was recognized, cloning was repeated three times by limiting dilution. Then, the clone wherein an antibody titer was stably recognized was selected as the anti-"angiopoietin-related protein 3" monoclonal antibody production hybridoma strain. One of the hybridoma clones obtained was named 45B1, and was deposited on March 13, 2002 in International Patent Organism Depositary, National Institute of Advanced Industrial Science and Technology and was accorded the accession number FERM BP-7963.

### (g) Purification of the monoclonal antibody

The culture supernatants of anti- "angiopoietin-related protein 3" monoclonal antibody production hybridoma 45B1 strain was collected, and then sterilized by filtration through a 0.22 µm filter (manufactured by Milipore). Thereafater, the antibody was purified using MAbTrapGII (manufactured by Pharmacia). The anti-"angiopoietin-related protein 3" monoclonal antibody MAb 45B1 was obtained through the above process.

### (h) Antigen specificity of the monoclonal antibody

By ELISA using the serum-free culture supernatant of the angiopoietin-related protein 3 producing CHO cell strain produced in 1) of (a) and the serum-free culture supernatant of CHO cells respectively as an adsorption antigen, it was confirmed that Mab 45B1 was specific to the "angiopoietin-related protein 3". Namely, 100 µl each of the serum-free culture supernatant of the "angiopoietin-related protein 3 " producing CHO cell strain and the serum-free culture supernatant of the CHO cell were dispensed into each well of the 96 well plate for ELISA (Maxisoap, manufactured by Nunc), and incubated at 4 °C overnight. Thereby, the antibody coated onto the bottom of the plate well. After washing the plate three times with PBS, 200 µl/well of PBS containing 3 % (w/v) of BSA was added thereto, and incubated at 4 °C overnight. Each of the wells was washed twice with PBS containing 0.05 % (v/v) Tween 20 (hereinafter referred to as PBST), the culture supernatant of the antibody producing hybridoma which was serially diluted with PBS containing 1 % (w/v) of BSA was added at 100 µl/well, and was incubated at room temperature for 1 hour. Then, it was washed five times with PBS containing 0.05 % (v/v) of Tween 20, the HRP-labeled anti-mouse IgG antibody (manufactured by Amersham Bioscience) diluted 5000 times with PBS containing 1 % (w/v) of BSA was added thereto at 100 µl/well, and kept at 37 °C for 1 hour. It was washed five times with PBS containing 0.05 % (v/v) Tween 20, the ABTS peroxidase substrate (manufactured by Roche Diagnotics) was added thereto at 100 µl/well, and kept at room temperature for 30 minutes. Then, absorbance at 450 nm was measured. High absorbance was seen as for the well in which the serum-free culture supernatant of the "angiopoietin-related protein 3" producing CHO cell strain was adsorbed, and the absorbance decreased with dilution of the antibody. Low absorbance was seen for the well in which the serum-free culture supernatant of a CHO cell was adsorbed regardless of the dilution of the antibody.

### (i) Isotyping of a monoclonal antibody

As a result of analyzing using the mouse monoclonal antibody isotyping kit (manufactured by Amersham), the antibody was identified to be an IgG1 subclass antibody. Example 8 "Angiopoietin-related protein 3" quantification system using MAb 45B1.

### (a) production of rabbit anti- "angiopoietin-related protein 3" polyclonal antibody

0.5 ml of Freund's complete adjuvant (manufactured by Difco) as adjuvant were added to 0.5 ml (an equivalent for 100 µg) of "angiopoietin-related protein 3" purified in (a) of Example 7 to form an emulsion in a injection syringe equipped with a needle, which was then hypodermically inoculated into a male rabbit. For the 2nd or later immunization, incomplete Freund's adjuvant (manufactured by Difco) was used as an adjuvant. Immunization was carried out 8 times every two weeks. After inoculation, partial blood collection was carried out over time, and elevation of antibody titer was investigated. The exsanguination was carried out under anesthetization seven days after the 8th immunization, and 53.5 ml of immune serum were obtained. 20 ml of the serum were purified using the MabTrap GII kit, and 115 ml of 1.64 mg/ml IgG was obtained.

The rabbit anti-"angiopoietin-related protein 3" polyclonal antibody (hereinafter referred to as "No.1 antibody") was obtained through the above process.

### (b) Preparation of HRP labeled Fab' polyclonal antibody

### 1) Preparation of F(ab')₂

30 ml of 1.64 mg/ml No.1 antibody were dialyzed with 0.1 M sodium-acetate buffer solution (pH 4.5, containing 0.1 M NaCl), and condensed using CENTR1CON-30 (manufactured by Amicon). The concentration was adjusted to 6 mg/ml with 1 M sodium acetate buffer solution (pH 4.5, containing 0.1 M NaCl). 20 µl (0.2 mg) of Swine stomach pepsin (manufactured by Sigma) dissolved in 1M sodium acetate buffer solution (pH 4.5, containing 0.1 M NaCl) so as to be 10 mg/ml were added to 0.5 ml of 6 mg/ml No.1 antibody, and allowed to react at 37 °C for 6 hours. After dialyzing the reaction mixture with 0.1 M sodium phosphate buffer solution (pH 7.0), gel filtration was carried out using an Ultrogel AcA 44 (manufactured by Invitrogen) column (1.5 cm x 45 cm) - 0.1 M sodium-phosphate buffer solution (pH 7.0), at a flow rate of 0.35 ml/min, and at 0.5-1.0 ml per one fraction. No.1 antibody F(ab')₂ F fraction was confirmed by the absorbance at 280 nm, and condensed using CENTRICON-30 (manufactured by Amicon). These operations were repeated and finally 11.2 ml of 2.5 mg/ml No.1 F(ab')2 antibody were obtained.

### 2) Preparation of Fab'

11.2 ml of 2.5 mg/ml No.1 F(ab')2 antibody were dialyzed with 0.1 M sodium phosphate buffer solution (pH 6.0), and condensed using a CENTRlCON-30 (manufactured by Amicon). The concentration was adjusted to 5 mg/ml with 1 M sodium phosphate buffer solution (pH 6.0). 50 µl of 0.1 M sodium phosphate buffer solution (pH 6.0) containing 0.1 M 2-mercapotethylamine and 50 mM EDTA were added to 0.45 ml of 5 mg/ml No.1 F(ab')2 antibody, and allowed to react for 90 minutes at 37 °C. The reaction mixture was subjected to gel filtration using an Ultrogel AcA 44 (manufactured by Invitrogen) column (1.5 x 45cm) -0.1 M sodium phosphate buffer solution (pH 6.0, containing 0.5 mM EDTA), at flow rate of 0.35 ml/min, and at 0.5-1.0 ml per fraction. After confirming the existence of a No.1 Fab' antibody by absorbance at 280 nm, it was condensed using a CENTRICON-30 (manufactured by Amicon). These operations were repeated and, finally 11.4 ml of 1.4 mg/ml No.1 Fab' antibody were obtained.

### 3) Preparation of maleimide peroxidase

36.8 mg of horseradish peroxidase (manufactured by Sigma) were dissolved in 0.1 M sodium-phosphate buffer solution (pH 7.0) so as to be 6.66 mg/ml. N-succinimidyl-4-(N-maleimidomethyl) cyclohexane-1-lcarboxylate (Sigma) was dissolved in N,N-dimethylformamide, and the concentration was adjusted to 70 mM. 30 µl of 70 mM N-succinimidy 1-4-(N-maleimidomethyl) cyclohexane-1-lcarboxylate were added to 300 µl of 6.66 mg/ml horseradish peroxidase, and allowed to react at 30 °C for 30 minutes. After the precipitate was removed from the reaction mixture by centrifugation, gel filtration was carried out using a Sephadex G-25 column (1 x 30 cm) -0.1 M sodium phosphate buffer solution (pH 6.0) at a flow rate of 0.4 - 0.6 ml/min, at 0.5-1.0 ml per fraction. The fraction containing peroxidase was confirmed by absorbance at 403 nm, and condensed using a CENTRICON-30 (manufactured by Amicon). These operations were repeated, and finally 5.2 ml of 6.5mg/ml maleimide peroxidase were obtained.

### 4) Reaction of maleimideized peroxidase and Fab'

0.28 ml of 6.5 mg/ml maleimideized peroxidase were added to 0.71 ml of 1.4mg/ml No.1 Fab-antibody, and allowed to react at 4 °C for 20 hours. After the reaction, 10 µl of 50 mM N-ethylmaleimide were added thereto. The mixture was then subjected to gel filtration using an Ultrogel AcA 44 (manufactured by Invitrogen) column (1.5 x 45-100 cm) - 0.1M sodium phosphate buffer solution (pH 6.5), at a flow rate of 0.35 ml/min, and at 1.0 ml per fraction. The absorbance at 280 nm and 403 nm of each fraction was measured, and the peroxidase activity of each fraction was measured further. To each fraction, was added 0.2 % (w/v) of thimerosal (manufactured by Sigma) to 0.002 % final concentration and 10% (w/v) bovine serum albumin to 0.1 % final concentration, and the mixture was stored at 4 °C. The concentration of Fab' labeled with peroxidase was calculated from the peroxidase activity or the absorbance at 403 nm, and the mixture was stored at a concentration of 10 µg/ml or more. These operations were repeated, and finally 8.6 ml of 0.78 mg/ml HRP-labeled No.1 Fab-antibody was obtained, and used for the following experiments.

### (c) Preparation of "angiopoietin-related protein 3" quantification system

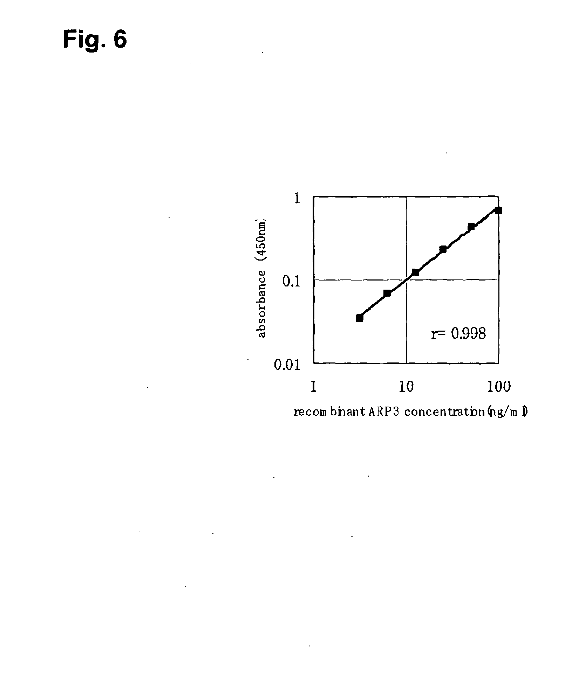

The purified MAb 45B1 obtained from the culture supernatant of (g) of Example 7 was diluted with 0.1 M carbonic acid buffer solution (pH 9.6) to 2 µg/ml, and 100 µl were dipensed into each well of a 96 well plate for ELISA (maxisoap, manufactured by Nunc), and kept at 4 °C overnight. The antibody coated the bottom of the well. After washing each of the wells 3 times with PBS, PBS containing 0.1% (w/v) BSA was added at 200 µl/well, and was kept at 4 °C overnight. Each of the wells was washed twice with PBS containing 0.05 % (v/v) of Tween 20(hereinafter referred to as "PBST"), the sample (the purified "angiopoietin-related protein 3 " or human plasma) which was serially diluted with PBS containing 0.1 % (w/v) BSA and 0.05 % (v/v) Tween 20 was dispensed at 100 µl/well, and was kept at 4 °C overnight. Each of the wells was washed 7 times with PBS containing 0.05 % (v/v) of Tween 20, the HRP labeled No.1 Fab-antibody diluted with PBS which contains 1 % (w/v) BSA and 0.05 % (v/v) of Tween 20 to 0.5 µg/ml was added thereto at 100 µl/well, and allowed to react for 30 minutes at 37 °C. Each of the wells was washed 9 times with PBS containing 0.05 % (v/v) of Tween 20, and TMB peroxidase substrate solution (manufactured Bio-Rad, TMB peroxidase kit) was added thereto at 100 µl/well, then kept at room temperature for 30 minutes, and then 1 N sulfuric acid was added thereto at 100 µl/well to stop the peroxidase reaction. Thereafter, absorbance at 450 nm was measured with a micro plate reader (manufactured by Bio-Rad). As a result, because the concentration of the purified "angiopoietin-related protein 3" and the measurement result were in direct proportion, it was revealed that quantification of "angiopoietin-related protein 3 " was possible with sufficient linearity using this ELISA system (refer to Fig. 6). When human plasma was used as a sample, it could be detected using this ELISA system.

### Example 9. Production of the recombinant polypeptide using the adenovirus vector

### (1) Preparation of DNA

### 1) Insertion of Cossack sequence

In order to obtain the cDNA clone which was cloned in order to detect the expression level of the gene in the method for testing a therapeutic or preventive agent for hyperlipidemia described in Reference Example 2 to which a Cossack's consensus sequence was added, there were synthesized oligonucleotide primers consisting of the following nucleotide sequence:
5'-gccaccatggtcacaattaagctccttctttttattg-3' (SEQ ID No. 42 of the Sequence Listing) and the nucleotide sequence to which the specific recognition sequence of a restriction enzyme XhoI was added:
5'-tctagagcctcgttcattcaaagctttctgaatctg-3' (SEQ ID No. 43 of the Sequence Listing). A part of the primer shown in the SEQ ID No. 42 corresponds to nucleotide numbers 25-55 of cDNA of the "angiopoietin-related protein 3" shown in the SEQ ID No. 53 of the Sequence Listing, and a part of primer shown in the SEQ ID No. 43 corresponds to the anti-sense of nucleotide numbers 1385-1415 of the SEQ ID No. 53 of the Sequence Listing. Then, PCR was performed using as a template about 0.4 µg/ml of a human cDNA inserted in a recombinant phagemid pTrip/h55-1 (disclosed in the Genbank database as the nucleotide sequence encoding "the angiopoietin-related protein 3") obtained by the method described in Reference Example 2, in the presence of the above-mentioned primers (respectively 0.4 mM), Tbr EXT DNA polymerase (manufactured by Finzyme), 1 x PCR reaction buffer solution (1/10 volume of 10 x buffer solution provided with the DNA polymerase), 2 mM magnesium chloride, and 0.4 mM each of dNTPs. PCR was performed as follows: the above-mentioned sample was heated at 94 °C for 3 minutes, and subjected to 30 heating cycles of 94 °C for 30 seconds, at 55 °C for 30 seconds, and at 72 °C for 1 minute and then kept at 68 °C for 2 minutes.

The amplified fragment (about 1.4 kbp) obtained as a result of PCR was inserted in the plasmid vector pCR2.1 provided with the TA cloning kit (manufactured by Invitrogen), and the plasmid clone (pCR/KhAP5) containing the insert was isolated. It was confirmed that mutation was not generated in the insert during PCR by analyzing the sequence of the insert with the DNA sequencer (ABI PRISM 3700, manufactured by Applied Biosystems).

Next, the recombinant plasmid pME18S/KhAP5 was obtained by inserting the fragment containing cDNA encoding human-"angiopoietin-related protein 3" from the fragments produced by digesting pCR/KhAP5 with the restriction enzymes EcoRI and XbaI into a mammalian cell expression vector pME18S digested by restriction enzymes EcoRI and XbaI beforehand.

### 2) Production of a partially deleted mutant

### a) Mutant wherein a carboxyl-terminal side domain is deleted

The plasmid pME18S/KhAP5 CC wherein a stop codon (tag) was inserted after the codon (atc) encoding the 207th amino acid residue (serine) from the amino terminus in the amino acid sequence containing the signal peptide of human "angiopoietin-related protein 3" was constructed by digesting the plasmid pME18S/KhAP5 obtained by 1) above with the restriction enzyme SpeI, and the cut end was blunted with T4 DNA polymerase, a DNA ligase reaction was performed to connect both ends again. The nucleotide sequence of the insert in this recombinant plasmid is as shown as SEQ ID No. 11 of the Sequence Listing.

### b) Mutant wherein an amino-terminal side domain is deleted

The plasmid pME18S/KhAP5 FLD, wherein a nucleotide sequence encoding the 21st to 206th amino acid residues from the amino terminus in the amino acid sequence containing the signal peptide of human "angiopoietin-related protein 3" was substituted by a codon encoding proline, was constructed by digesting the plasmid pME18S/KhAP5 obtained by 1) above with restriction enzymes BclI and SpeI, removing only the nucleotide sequence encoding the amino acid sequence of the 207th amino acid residue (serine) from the amino terminus in the amino acid sequence containing the signal peptide of human - "angiopoietin-related protein 3", and then blunting the cut end with T4 DNA polymerase, and performing a DNA ligase reaction to connect both ends again. The nucleotide sequence of the insert in this recombinant plasmid is shown as SEQ ID No. 13 of the Sequence Listing.

### (2) Preparation of a recombinant adenovirus

A recombinant adenovirus, in which the gene encoding the partially deleted variant of the "angiopoietin-related protein 3" gene which exists in the plasmid pME18S/KhAP5 CC and pME18S/KhAP5FLD obtained in (1) above was inserted, was prepared using an adenovirus expression vector kit (manufactured by Takara Shuzo CO., LTD.) according to the following method.

First, plasmid pME18S/KhAP5 CC and pME18S/KhAP5 FLD obtained by (1) above were digested with the restriction enzymes EcoRI and XbaI respectively, and fragments of about 1.4 kb DNA and about 0.9 kb DNA thus obtained were collected, and the cut ends thereof were blunted. Next, these fragments were inserted into the SwaI cleavage site of the cosmid vector pAxCAwt designed so that an inserted gene can be expressed from a cytomegalovirus enhancer and the chicken β-actin promoter respectively, and thereby the recombinant cosmids pAxCA/KhAP5 CC and pAxCA/KhAP5 FLD were prepared.

The recombinant cosmid DNAs and an end protein jointing virus DNA (DNA-TPC, provided with an adenovirus expression vector kit) were co-transfected to a 293 cell (ATCC CRL1573) using a calcium phosphate transfection system (manufactured by Lifetech), and the resultant recombinant adenoviruses Ad/KhAP5 CC and Ad/KhAP5 FLD were isolated, and further amplified in the 293 cell. Collection of the amplified virus from the 293 cell was performed by subjecting viral infected 293 cells to ultrasonication for 30 second x 4 times (using B-1200 manufactured by Branson), or by freezing and thawing 3 times, and thereafter repeating purification by cesium chloride density gradient centrifugation twice. The obtained virus solution was dialyzed at 4 °C to PBS to which 10 % glycerol was added, and stored at -70 °C or lower until used.

### (3) Confirming expression of the target protein by the recombinant adenovirus

HeLa cells were infected with the recombinant adenovirus obtained by the procedure of (2) above, and expression of the target protein was confirmed.

First, HeLa cells (ATCC CCL2) were infected with each of the recombinant adenoviruses obtained by the procedure described in 2) above at about 5 m.o.i. (multiplicity of infection), and the culture supernatants after culture in the serum free medium (Dulbecco's modified Eagle medium (DMEM) for three to four days were collected. One ml of culture supernatant was put into a 1.5 ml Eppendorf tube, and 100 µl of trichloroacetic acid solution (1 g/ml of trichloroacetic acid, 4 mg/ml of deoxycholic acid) were added, and allowed to sit for three minutes at room temperature, followed by centrifugation at 15000 rpm with a desk centrifuge apparatus for 5 minutes. After the supernatant was removed, 0.5 ml of acetone cooled on ice were added thereto, and then the mixture was centrifuged at 15000 rpm with a desk centrifuge apparatus for 2 minutes. After the supernatant was removed, 0.5 ml of acetone cooled with ice were added again to the precipitate and agitated, followed by centrifugation at 15000 rpm with a desk centrifuge apparatus for 2 minutes. The supernatant was removed, and the precipitate was subjected to vacuum evaporation. Each of the precipitates was dissolved in 10 µl of distilled water, and mixed with a SDS-PAGE sample buffer solution (manufactured by Bio-Rad) containing 2-mercaptoethanol, and then heated for 5 minutes at 99 °C, to provide a sample for electrophoresis. After these samples were subjected to electrophoresis in a polyacrylamide density-gradient gel (buffer solution for electrophoresis: 25 mM of Tris, 192 mM of glycine, 0.1 % SDS) at a gel concentration of 4-20 %, they were transferred to a nitrocellulose membrane under the conditions of 200 mA, 4 °C and for one hour in a transcription buffer solution (25 mM of Tris, 192 mM of glycine, 20% methanol).

The transferred membrane was dipped into a PBS solution containing 0.5 % of skimmed milk to be blocked at 4 °C overnight, and was washed 3 times with a washing solution (0.05 % Tween 20-PBS). Subsequently, the membrane was incubated at room temperature for one hour in 0.05 % Tween 20-PBS solution containing about 270 ng/ml of 55-1-N1 antibody or about 1 µg/ml of 55-1-C1 antibody, and 5 % fetal bovine serum, and washed 3 times with a washing solution. Each of these antibodies was obtained by the method described in Example 2. The 55-1-N1 antibody is an antibody which specifically binds to the amino terminal side of the human "angiopoietin-related protein 3", and 55-1-C1 antibody is an antibody which specifically binds to the carboxyl terminal side of the same.

Furthermore, after carrying out the incubation of the membrane at room temperature for 1 hour in the reaction mixture wherein horseradish peroxidase labeled goat anti-rabbit IgG (H+L) (manufactured by Bio-Rad) was diluted 2000 times with 0.05 % Tween 20-PBS which contains a 5 % fetal bovine serum, and was washed 5 times with the washing solution. This membrane was placed on a wrap film and dipped in a photogenesis reagent solution (ECL Western detection reagent manufactured by Amersham Pharmacia Biotech) for 1 minute, Then it was left at room temperature for 1 hour to attenuated the background, and then exposed to X-ray film (for 10 seconds). As a result of developing this X-ray film, it was confirmed that the target polypeptides were produced, respectively in the HeLa cells infected with the recombinant adenoviruses.

### (4) Concentration of neutral fat in blood of the KK/Snk mouse inoculated with the recombinant adenovirus

The recombinant adenoviruses Ad/KhAP5 CC and Ad/KhAP5 FLD purified by the above procedure were diluted with 10 % glycerol in PBS to 2.0 x 10¹⁰ pfu/ml and 1.0 x 10¹⁰ pfu/ml, and 300 µl of each dilution was administered by tail intravenous injection to three 19-week old male KK/Snk mice respectively. As a negative control, PBS containing 10 % glycerol was administered by tail intravenous injection to three 19-week old male KK/Snk mice.

One day after the inoculation, blood was collected from the eyegrounds of each mouse with a hematocrit pipe, and centrifuged for 15 minutes at 5200 rpm with a desk centrifuge apparatus to separate the plasma. The concentration of neutral fat was measured using a kit (triglyceride E-test, manufactured by Wako Pure Chemical).

As a result, there was no significant difference in the concentration of neutral fat in blood between the Ad/KhAP5 FLD inoculated mouse group and the 10% glycerol in PBS inoculated mouse group (all being 50 mg/dL or less), and a remarkable rise in the concentration of neutral fat in blood was seen in the Ad/KhAP5cc inoculation mouse group (over 3000 mg/dL).

In the procedure of this Example, the test for a therapeutic or preventive agent for one or more diseases selected from hyperlipidemia, arteriosclerosis, and hyperglycemia or for two diseases can be performed, for example, by administering a test substance to a mouse inoculated with Ad/KhAP5CC, and investigating the change in concentration of neutral fat in the blood.

### Example 10. Evaluation of activity of the fused protein

### (1) Preparation of the transformed Escherichia coli expressing the fused protein

There were produced according to the method described below fused proteins wherein glutathione S transferase (hereinafeter referred to as "GST") was fused to the amino terminal of the polypeptide which consists of an amino acid sequence shown in amino acid numbers 17-207 of the SEQ ID No. 12 of the Sequence Listing, or amino acid numbers 17-195 of the same, or amino acid numbers 17-147 of the same (hereinafter referred to as "GST-CC", "GST-CCdel", and "GST-C1" respectively.)

First, oligonucleotides having the following nucleotide sequence were synthesized as PCR primers:
5'-cggaattccctccagaattgatcaag-3' (the sense primer commonly used for GST-CC, GST-CCdel, and GST-C1, SEQ ID No. 44 of the Sequence Listing);
5'-ccgctcgagactagtccttctgagctg-3' (the antisense primer for GST-CC, SEQ ID No. 45 of the Sequence Listing), and
5'-ccgctcgagttgactatgctgttggtt-3' (the antisense primer for GST-CC del, SEQ ID No. 46 of the Sequence Listing), and
5'-ccgctcgaggttagttagttgctcttc-3' (the antisense primer for GST-C1, SEQ ID No. 47 of the Sequence Listing).

Then, PCR was carried out using cDNA (pMEh55-1) described in Example 3 as a template in the presence of the sense primer and the antisense primer (0.2 µM each), Tbr EXT DNA polymerase, 1 x PCR buffer solution (10 x buffer solution provided with the Tbr EXT DNA polymerase was diluted 10 times), 2mM of magnesium chloride and 0.4 M of each dNTPs. PCR was carried out under the following conditions: the above-mentioned sample was heated at 94 °C for 3 minutes, and a heat cycle of 94 °C for 30 seconds, 55 °C for 30 seconds, and 72 °C for one minute was repeated for 30 cycles, and finally, the sample was heated at 72 °C for 5 minutes.

By PCR, there were amplified respectively a DNA fragment of about 580 bp (using the antisense primer for GST-CC), a DNA fragment of about 540 bp (using the antisense primer for GST-CCdel), and a DNA fragment of about 400 bp (using the antisense primer for GST-C1). The amplified DNA fragments were collected, and each of them was digested with EcoRI and XhoI. Each of the fragments digested with EcoRI and XhoI was inserted in the plasmid vector pGEX-4T-2 (containing the gene encoding GST, Amersham Pharmacia Biotech) which was digested with EcoRI and XhoI and subjected to dephosphorylation of the cut ends using bovine small intestine alkaline phosphatase (manufactured by Takara Shuzo CO., LTD.), and three kinds of the plasmid clone containing three kinds of the inserted fragment respectively were isolated. The plasmid clone amplified using the antisense primer for GST-CC was named pGST-CC, the plasmid clone amplified using the antisense primer for GST-CCdel was named pGST-CCdel, the plasmid clone amplified using the antisense primer for GST-C1 was named pGST-Cl.

The Escherichia coli JM 109 was transformed with the recombinant plasmid clone pGST-CC, pGST-CCdel, pGST-C1 or the control vector pGEX-4T-2 for expressing only GST which does not contain the insert fragment, respectively. The transformation was performed by adding the plasmid to competent cells of Escherichia coli JM 109, leaving them for 30 minutes on ice, keeping them at 42 °C for 30 seconds and leaving them for 2 minutes on ice again, and thereafter culturing the cells at 37 °C for 1 hour. The culture medium after transformation was spread on LB agar plates to which ampicillin was added, the recombinant colonies which appeared were picked and subjected to liquid culture, and expression of

### (2) Preparation of fusion protein

### 1) Culture of the transformed Escherichia coli

The transformed Escherichia coli carrying the plasmid clone pGST-CC, pGST-CC del, pGST-C1 and the control vector pGEX-4T-2 were inoculated respectively to LB media containing ampicillin, and cultured with agitation at 37 °C for 18 hours. A sample of the culture medium was added to new media, and cultured with agitation at 37 °C. When the absorbance at 600 nm became 0.5 - 0.7, the isopropylthiogalactopyranoside (IPTG) was added to a final concentration of 1 mM and the culture was incubated with agitation for a further 4 hours.

### 2) Preparation of a cell lysate solution from the Escherichia coli carrying pGEX-4T-2

The culture medium of Escherichia coli transformed with the control vector pGEX-4T-2 was centrifuged at 4 °C, at 6000 rpm for 10 minutes, and the precipitated cells were collected. The collected cells were suspended in the buffer solution for ultrasonication (50 mM of Tris-hydrochloride (pH 8.0), 50 mM of sodium chloride, 1 mM of EDTA, 1 mM of DTT), and the suspension was centrifuged at 4 °C at 6000 rpm for 10 minutes. The precipitation was frozen at -80 °C, subsequently thawed at room temperature, and was suspended in the buffer solution for ultrasonication again. The suspension was treated for 5 minutes under the conditions that output =5 and duty =50 using the ultrasonic blender (UD-201 manufactured by Tomy Seiko). Triton X-100 was added to the lysate solution after ultrasonication to a final concentration of 1 %, and mixed therewith. Thereafter it was centrifuged at 4 °C at 10000 rpm for 30 minutes, and the supernatant was collected. The supernatant was passed through the GSTrap column (manufactured by Amersham Pharmacia Biotech).

### 3) Preparation of the cell lysate solution from the Escherichia coli carrying pGST-CC, pGST-CCdel, pGST-C1

Each of the media of the Escherichia coli transformed with the recombinant plasmid pGST-CC, pGST-CCdel, pGST-C1 was centrifuged at 4 °C at 5000 rpm for 15 minutes, and the precipitated cells were collected. The precipitated cells were suspended in the washing buffer solution (0.5% of Triton X-100, 1 mM of EDTA) and the centrifuged at 4 °C at 5000 rpm for 10 minutes three times, the cells obtained were dissolved in 8 M urea solution. After leaving this solution at room temperature for 1 hour, centrifugation at 4 °C at 13000 rpm for 30 minutes was performed, and the supernatant was collected. Three kinds of supernatant obtained from the culture originating from Escherichia coli transformed with pGST-CC, pGST-CCdel, pGST-C1 were dialyzed. The dialysis was performed against 4 M urea solution at 4 °C for 1 hour, to 2 M urea solution at 4 °C for 1 hour, to the buffer solution for ultrasonication at 4 °C for one hour and to the buffer solution for ultrasonication at 4 °C overnight, in that order. After dialysis, centrifugation was carried out at 4 °C at 12000 rpm for 30 minutes, and the solution was passed through the GSTrap column.

### 4) Purification of fused protein

PBS was passed through the GST column to which GST, the fused proteins GST-CC, GST-C1, or GST-CCdel was adsorbed, the inside of the column was washed to remove impurities. Subsequently, a 10 mM reduced glutathione solution was passed through it, and GST and the fused protein GST-CC, GST-C1, or GST-CCdel were eluted. A part of the eluate was separted by SDS-PAGE electrophoresis, and then the gel was stained by Coomassie staining to confirm the existence of the protein. Moreover, the elaute was dialyzed with PBS at 4 °C and overnight. The protein purified from the transformant carrying plasmid pGEX-4T-2 was GST, and the proteins originating from pGST-CC, pGST-C1, and pGST-CCdel were GST-CC, GST-C1, and GST-CCdel respectively.

### (3) The activity of increasing concentration of neutral fat in blood

After adjusting GST, GST-CC, GST-C1, GST-CCdel, and GST-C1 which were obtained in (2) above to a protein concentration of about 1 mg/ml with PBS, 300 µl of each of them were inoculated by tail intravenous injection to three, four, two and five 19-week old male KK/Snk mice respectively. Blood was collected before and 30 mins after inoculation from the eyegrounds of each mouse with a hematocrit pipe, and centrifuged for 15 minutes at 5200 rpm with a desk centrifuge apparatus to separate the plasma. The concentration of neutral fat (triacyl glycerol) in blood before and after inoculation was measured.

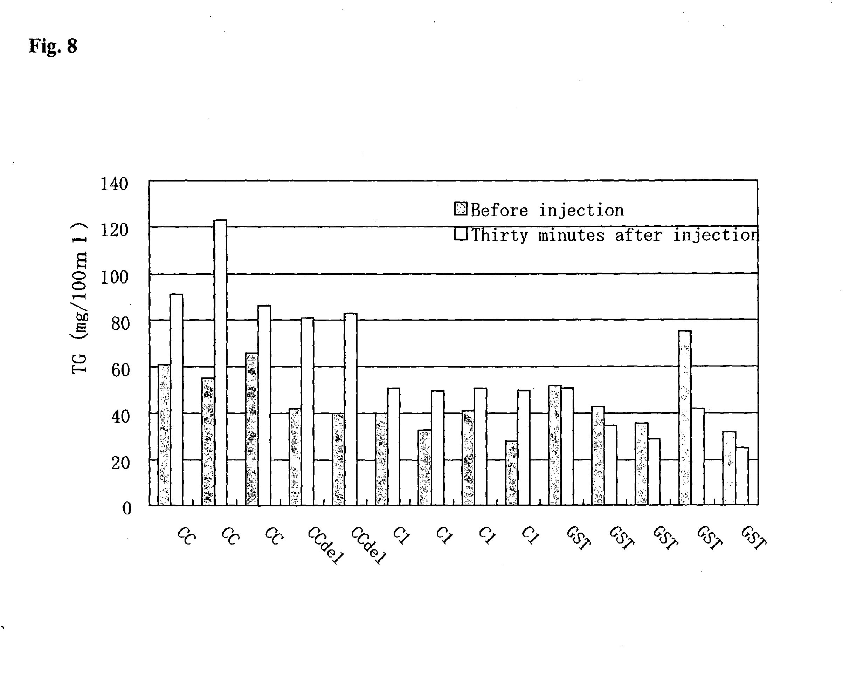

The results are shown in Fig.8. The vertical axis shows the amount of neutral fat (triacylglycerol) in 100 ml of plasma in the graph of Fig. 8. The horizontal axis shows each fused protein. No difference was found in samples taken before and after inoculation in the GST inoculated mouse group ("GST" in Fig. 8), the concentration of neutral fat in blood was raised somewhat in the GST-C1 inoculated mouse group ("C1" in Fig. 8). However, in the GST-CC-inoculated mouse group and the GST-CCdel inoculated mouse group ("CC" and "CCdel" in Fig. 8), the concentration of neutral fat in blood was increased significantly.

In the procedure of this Example, the test of a therapeutic or preventive agent for one or more diseases selected from hyperlipidemia, arteriosclerosis, and hyperglycemia, or two diseases, can be performed, for example, by administrating a test substance to a mouse inoculated with GST-CC or CCdel, and investigating the change in concentration of neutral fat in the blood.

### Example 11. LPL activity-measurement method

### (1) A method using LPL originated from a rat fat cell

### 1) Preparation of LPL sample

A rat white fat precursor cell (commercially available from Hokudo) was cultured at 37 °C for 24 hours in a cell culture flask (cultivation area of 25 cm²) containing a medium for proliferation [Dulbecco's modified Eagle culture medium (referred to as "DMEM") containing 10% fetal bovine serum (hereinafter referred to as "FCS"), 100 units/ml of penicillin, 100 µg/ml of streptomycin, 17 µM of pantothenic acid, 33 µM of (+)-biotin, 100 µM of ascorbic acid, 1 µM of octanoic acid and 50 nM of triiodothyronine]. Then, the medium was replaced with a medium for inducing differentiation (DMEM containing 10 % FCS, 100 units/ml of penicillin, 100 µg/ml of streptomycin, 17 µM of pantothenic acid, 33 M of (+)-biotin, 100 µM of ascorbic acid, 1 µM of octanoic acid, 50 nM of triiodothyronine, 10 µg/ml of insulin and 2.5 M of dexamethasone), and cultured at 37 °C for a further 48 hours. Then, the medium was replaced with a medium for maintaining fat cells (DMEM containing 10 % FCS, 100 U/ml of penicillin, 100 µg/ml of streptomycin, 17 µM of pantothenic acid, 33 M of (+)-biotin, 100 µM of ascorbic acid, 1 µM of octanoic acid, 50 nM of triiodothyronine and 10 µg/ml of insulin). After two or three days, 2 ml of DMEM medium containing 10 % FCS, 50 ng/ml insulin, and 10 units/ml of heparin sodium was added thereto, and the culture supernatant was collected after culturing for one hour, and used for measurement of LPL activity.

### 2) Measurement of LPL activity

The method for measurement is according to the method described in the literature (Nilsson-Ehle, P. and Schotz, M. C. (1976) J. Lipid Res. 17, 536-541). A polypeptide which consists of an amino acid sequence shown in amino acid numbers 17-460 of SEQ ID No. 4 of the Sequence Listing (the angiopoietin-related protein 3) was prepared according to the procedure described in Example 6.

First, 100 µl of the culture supernatant prepared by the procedure described in the above 1) were mixed with an equivalent amount of a substrate solution [2 mM glycerol, tri[9, 10(n)-³H] oleic acid (131 KBeq/µmol, manufactured by Amersham), 0.189 µg/ml or L α-phosphatidylcholine (manufactured by Sigma), 14 mg/ml of bovine serum albumin (manufactured by Sigma), 140 mM of Tris hydrochloride (pH 8.0), 15 % (v/v) of glycerol, 10 % (v/v) of an inactivated FCS]. To this was added a purified angiopoietin-related protein 3, and the mixture was kept at 37 °C for 120 minutes. Then, the following were added 1.05 ml of 0.1 M potassium carbonate/boric acid buffer solution (pH 10.5) and 3.25 ml of methanol:chloroform: hexane = 141 : 125 : 100 (v/v), to stop the reaction. After strong agitation, centrifugation was carried out at 3000 x g for 15 minutes. ³H count in a water/methanol layer was measured by using a solution scintillation counter. One unit of LPL activity was defined as an activity which generates one µmol of fatty acid in 1 minute. The result was shown in Table 5.

**[Table 5]**

| Polypeptide 1 (µg / ml) | relative value of LPL activity (%) |
|---|---|
| 0 | 100 ± 13 |
| 1 | 84 ± 9 |
| 2.5 | 64 ± 1 |
| 5 | 52 ± 3 |
| 10 | 29 ± 3 |

The angiopoietin-related protein 3 suppresses the LPL activity in a dose dependent manner. In this experimental system, if the test substance is added during the lipase reaction, it can be investigated whether the substance has an effect of regulating LPL activity.

### (2) Method using mouse plasma after administering heparin intravenously

Heparin sodium was administered intravenously to a tail of C57BL/6j mouse (100 units/kg). After 10 minutes, blood was collected from the ventral aorta, followed by centrifugation to prepare mouse plasma after administering heparin intravenously, which was used for measurement of the LPL activity. However, if measurement was performed under the conditions described in (1)2) above when using the plasma, the lipase activity of both HTGL and LPL contained in the plasma originating from liver can be measured. Accordingly, in order to measure them separately, the lipase reaction was first performed under the conditions described in (1) 2) above, to measure a lipase activity, and the result obtained was defined as the "total lipase activity." Additionally, the lipase reaction was performed under conditions whereby LPL was inactivated by adding sodium chloride to a reaction mixture to a final concentration of 1 M, and thus only the lipase activity of HTGL remaining in plasma was measured. The value obtained by deducting the HTGL activity from the total lipase activity was defined as the LPL activity. The results are shown in Table 6.

**[Table 6]**

| Polypeptide 1 (µg/ml) | relative value of HTGL activity (%) | relative value of LPL activity (%) |
|---|---|---|
| 0 | 100 ± 8 | 100 ± 0 |
| 1 | 109 ± 13 | 93 ± 2 |
| 2.5 | 94 ± 4 | 82 ± 5 |
| 5 | 80 ± 2 | 70 ± 5 |
| 10 | 64 ± 7 | 49 ± 5 |

It has been revealed that angiopoietin-related protein 3 inhibits in vitro the activity of both LPL and HTGL in mouse plasma after administration of heparin intravenously. In this experimental system, if the test substance is added during the lipase reaction for measuring the total lipase activity, it can be determined whether the substance has an activity of regulating the activity of either LPL or HTGL. In a similar way, if the test substance is added during the lipase reaction for measuring HTGL activity, it can be determined whether the substance has an activity of regulating the activity of HTGL. Example 12 Effect of inhibiting LPL activity of a fused protein

LPL activity was investigated using GST-CC, GST-CCdel or GST instead of the angiopoietin-related protein 3 , according to the method described in (1) of Example 11.

By defining the LPL activity when adding GST (10 µg/ml) as 100%, the relative LPL activity in the case of adding GST-CC or GST-CCdel was calculated. The results are shown in Table 7.

**[Table 7]**

| GST-CC (µg/ml) | relative value of LPL activity (%) |
|---|---|
| 1 | 97 ± 4 |
| 2.5 | 91 ± 2 |
| 5 | 81 ± 4 |
| 10 | 62 ± 1 |

| GST-Ccdel (µg/ml) | relative value of LPL activity (%) |
|---|---|
| 1 | 88 ± 2 |
| 2.5 | 84 ± 2 |
| 5 | 69 ± 7 |
| 10 | 47 ± 1 |

GST-CC and GST-CCdel suppressed the activity of LPL in a dose dependent manner. In this experimental system, if the test substance is added during the lipase reaction, it can be determined whether the substance has an activity of regulating the activity of LPL.

### Example 13. Preparation of a polypeptide to which a histidine tag was added and which has an activity of inhibiting LPL

### (1) Preparation of a polypeptide to which a histidine tag was added

KhAP5 CC/His wherein a histidine tag was inserted immediately after the codon encoding the 20th amino acid residue (aspartic acid) from the amino terminal in the amino acid sequence containing the signal peptide of the "angiopoietin-related protein 3", and wherein the amino acid sequence after the 208th amino acid from amino terminal was deleted, was prepared according to the procedure described below.

### 1) Insertion of a histidine tag

The plasmid pME18 S/KhAP5 obtained in 1) of (1) of Example 9 was modified so that a histidine tag (the amino acid sequence shown in amino acid numbers 21-28 of the SEQ ID No. 26 of the Sequence Listing was introduced into the carboxyl terminal side of the amino acid (aspartic acid) corresponding to the 20^{th} amino acid (equivalent to the 4th amino acid of a mature polypeptide) from the amino terminal of the amino acid sequence of the precursor of the "angiopoietin-related protein 3 ".

First, oligonucleotides which are partially complementary and consist of following nucleotide sequences were synthesized:
5'-gatccgcatcatcaccatcaccat-3' (SEQ ID No. 48 of the Sequence Listing) and
5'-gatcatggtgatggtgatgatgcg-3' (SEQ ID No. 49 of the Sequence Listing).
Then, phosphorylation of the 5'-end was performed using a synthetic DNA end phosphorylation reagent Phosphalink (manufactured by Applied Biosystem). The oligonucleotides were annealed, and inserted into a fragment of about 4.2 kb obtained by completely digesting pME18S/KhpAP5 with BclI. As a result, a plasmid clone, wherein the DNA wherein three units of the above-mentioned annealed oligonucleotides were connected was inserted in the BclI cleavage site, was obtained. Then, the plasmid pME18S/KhAP5/His+16 was obtained by inserting a fragment of about 180 bp(s), obtained by digesting pME18 S/KhAP5 completely with BclI, into the above-mentioned clone digested with BclI.

The plasmid pME18S/KhAP5/His+16 comprises three units of sequence encoding the histidine tag, two units were superfluous sequences which were connected in a reverse direction to that desired. In order to amplify by PCR the fragments from which these sequences are removed, an oligonucleotide primer used as both a sense primer and an antisense primer was synthesized which consists of the following nucleotide sequence:
5'-gaattgtcagcatcatcaccatcaccactc-3' (SEQ ID No. 50 of the Sequence Listing). Subsequently, PCR was carried out using pME18S/KhAP5/His+16 (0.4 µg/ml) as a template in the presence of, the above-mentioned primer (0.4 mM), Tbr EXT DNA polymerase (manufactured by Finzyme), 1 x PCR reaction buffer solution (1/10 volume of 10 X buffer solution attached to the DNA polymerase), 2 mM of magnesium chloride, and 0.4 mM of each dNTPs. PCR was performed under the following conditions: heating at 94 °C for 2 minutes, repeating, 30 times, a heating cycle of 94 °C for 30 seconds, 60 °C for 30 seconds and 72 °C for 30 seconds, and finally heating at 72 °C for 7 minutes.

The amplified fragment (268 bp) obtained as a result of PCR was digested with BclI, and was inserted into a fragment of about 4.2 kb(s) obtained by digesting pME18S/KhAP5 completely. According to the procedures described above, recombinant plasmid pME18S/KhAP5/His was obtained containing DNA in which the nucleotide sequence encoding the histidine tag (the nucleotide numbers 78-101 of the SEQ ID No. 25 of the Sequence Listing) was inserted upstream of the cleavage site between the two BclI cleavage sites existing in the cDNA sequence inserted in pME18S/KhAP5.

### 2) Preparation of a variant wherein a carboxyl terminal side is deleted

The plasmid pME18S/KhAP5/His obtained in 1) was digested with the restriction enzyme SpeI, and the cut ends blunted by T4 DNA polymerase, and then the ends were reconnected by a DNA ligase reaction. Thereby the recombinant plasmid pME18S/KhAP5 CC/His containing a DNA fragment (SEQ ID No.25 of the Sequence Listing) wherein a stop codon (tag) is inserted after a codon (atc) encoding the 207th amino acid residue (serine) from the amino terminal in the amino acid sequence containing a signal peptide of "angiopoietin-related protein 3 ", and a histidine tag was inserted immediately after the codon encoding the 20th amino acid residue (aspartic acid), was constructed.

### 3) Expression in COS-1 cell

To a laboratory dish with a diameter of 150 mm (manufactured by Corning) for culturing a tissue were added 30 ml of Dulbecco's modified Eagle medium (hereinafter referred to as "DMEM", manufactured by Gibco) containing 10% fetal bovine serum (hereinafter referred to as "FCS") in which COS-1 cells are suspended (0.5 x 10⁶ cell), and cultured for three days at 37 °C in the atmosphere of 5% carbon dioxide. Then, after the medium was removed, transfection was performed by adding to COS-1 cell a mixture obtained by adding 36 µg of pME18S/KhAP5 CC/His to a transfection reagent (a suspension of 180 µl of Lipofectamine 2000 and 3 ml of OPTIMEM-I solution (all are the products manufactured by Gibco)). After the transfection, the mixture was incubated at 37 °C in an atmosphere of 5% carbon dioxide. The procedures described above were performed as for COS-1 cells in 130 laboratory dishes, and 4.4 L of the culture supernatant in total were collected. The culture supernatant was subjected to suction filtration using a 0.22 µm bottle top filter made of cellulose acetate (manufactured by Corning), and the filtrate was collected.

### 4) Confirming expression level and concentration

SDS-PAGE was carried out for a sample of filtrate collected in 3) above and the "angiopoietin-related protein 3 " purified by the method described in Example 6; and the protein in the gel was transferred to a nitrocellulose membrane (manufactured by Bio-Rad) by a wet method (0.2 A, 90 minutes, 4 °C). The nitrocellulose membrane was soaked overnight in PBS-0.05% Tween 20 solution (hereinafter referred to as "PBS-T") containing 5% skimmed milk (manufactured by Snow Brand Milk Products Co., Ltd.) for blocking. Subsequently, the nitrocellulose membrane was washed with PBS-T, and then it was subjected to Western blotting. The 55-N1 antibody described in Example 2 was used as a primary antibody, and the anti-rabbit polyclonal IgG antibody labeled with horseradish peroxidase (manufactured by Amersham) was used as a secondary antibody. The ECL reagent (manufactured by Amersham) was used for detection of the band to which the 55-N1 antibody was bound specifically. As a result, it was found that about 1.0 to 1.5 mg/l (4.4 to 6.6 mg in all filtrates) of KhAP5 CC/His is contained in the filtrate obtained by 3) above.

### 5) Purification of KhAP5 CC/His

The total amount of filtrate obtained in 3) was subjected to extra concentration using ultrafiltration equipment (TIFF Laboscale and an ultrafiltration film having a pore size of 30 kDa, both are manufactured by Millipore) to 440 ml. Subsequently, ultrafiltration of the resultant sample was continued in an ultrafiltration apparatus, by gradually adding 4 1 of a buffer solution (pH 7.4) containing 20 mM of sodium phosphate, 0.5 M of sodium chloride, 0.1 % of sodium azide, and 0.05 % of protease inhibitor cocktail (manufactured by Sigma), to provide a sample wherein the buffer was substituted for the solvent (600 ml).

Additionally, nickel ions were bound to the carrier by passing 5 ml of 0.1 M nickel sulfate through a chelating column (HiTrap Chelating 5 ml column, manufactured by Amersham) at a flow rate of 5 ml/min. Ultra pure water was passed through the column for washing, installed in the HPLC solution-sending system (manufactured by Amersham), and affinity purification of the sample was performed under the conditions indicated below.
Solvent:
Liquid A: 20 mM of sodium phosphate, 0.5 M of sodium chloride (pH 7.4), 0.1 % of sodium azide, 0.05 % of protease inhibitor cocktail (manufactured by Sigma)
Liquid B: 20 mM of sodium-phosphate, 0.5 M of sodium chloride, 0.5 M of imidazole (pH 7.4), 0.1 % of sodium azide, and 0.05 % of protease inhibitor cocktail (manufactured by Sigma)
   Flow rate: 5 ml/min
   Purifying procedure: After passing 600 ml of the samples through the column, 50 ml of the buffer solution (equivalent to 0.1 M imidazole concentration) of 80 % solution A and 20 % solution B were passed through the column to remove the non-adsorbed substance. Then, 50 ml of B solution were passed through the column to elute the polypeptide which has a histidine tag, and the eluates were collected. 200 ml of solution A were added to this eluate to give an imidazole concentration of 0.1 M, and this was used as the sample for the second affinity purification.

The nickel ion was bound to the carrier by passing 5 ml of 0.1 M nickel sulfate through a small scale chelating column (HiTrap Chelating 1 ml column, manufactured by Amersham) at a flow rate of 5 ml/min. Ultra pure water was then passed through the column for washing, installed in the HPLC solution-sending system (manufactured by Amersham). Subsequently, 20 mM sodium phosphate, 0.5 M sodium chloride, 0.1 M imidazole (pH 7.4) buffer solution were manually passed through the column to remove the non-adsorbed substance. Then, 20 mM sodium phosphate, 0.5 M sodium chloride, 0.3 M imidazole (pH 7.4) buffer solution were similarly passed through at a flow rate of 1 ml/min. The eluates were fractionated and collected as five fractions of one ml each (a total of 5 ml). The collected eluates were put into a dialysis tube (manufactured by Gibco), respectively, and dialysis was performed to 500 ml of PBS (manufactured by Gibco). The PBS solution outside of the dialysis membrane was exchanged 3 times. A sample of the final solution from outside of the dialysis membrane was collected, and used as a negative control sample in LPL activity evaluation.

As a result of estimating the protein concentration in the collected sample using a protein assay (DC protein assay kit (manufactured by Bio-Rad) is used), SDS-PAGE, or the like, it was revealed that 1.8 mg in total of the KhAP5 CC/His was obtained. The combined solution of the 2nd and the 3rd fractions obtained in the 2nd affinity purification was used for the following LPL activity evaluation.

### (2) Measurement of LPL activity

The KhAP5 CC/His sample obtained in 5) of (1) was used instead of GST-CC, and LPL activity was measured by the procedure described in (1) of Example 11. The result is shown in Table 8.

**[Table 8]**

| KhAP5 CC/His (µg/ml) | relative value of LPL activity (%) |
|---|---|
| 0 | 100 ± 3 |
| 1 | 27 ± 5 |
| 2.5 | 21 ± 2 |
| 5 | 14 ± 2 |
| 10 | 11 ± 1 |

KhAP5 CC/His inhibited the LPL activity strongly. In this experimental system, if a test substance is added during the lipase reaction, it can be investigated whether the substance has an activity of regulating the activity of LPL. (3) An activity of reducing concentration of neutral fat in blood

According to the procedure described in (3) of Example 10, KhAP5 CC/His was administered to a tail vein of the KK/Snk mouse (100 µg/mouse). Peripheral blood was collected from the mouse before and after administration, and the concentration of neutral fat was measured. As a result, 30 minutes after administration of KhAP5 CC/His it was raised to 106.0 mg/dl, whereas a concentration of neutral fat in blood before administration was 40.5 mg/dl. A concentration of neutral fat in blood 30 minutes after administration in the control group to which only the solvent was administrated was 41.3 mg/dl. From the above result, it was confirmed that KhAP5 CC/His raised the concentration of neutral fat in blood significantly.

### Example 14. The effect of the angiopoietin-related protein 3 into which mutations were introduced at a heparin binding domain on the plasma triglyceride

### (1) Construction of the N terminal 207 amino acid protein fragment of the angiopoietin-related protein 3 (ARP3) in which mutations were introduced into the heparin binding motif

A fragment of about 1.4 kbp obtained by cutting the plasmid pME18S/KhAP5 CC/His described in 2) of Example 13 with EcoRI and XbaI was isolated, and it was inserted into a site in the plasmid pKF18k provided with the Mutan-Super Express Km kit (manufactured by Takara Shuzo CO., LTD.) cut with EcoRI/XbaI. Using a primer for introducing variation GTCATAAACACGAACGGCCAAATTAATGAC, mutations were introduced according to the instructions provided with the kit, and plasmid pKF18/KhAP5 CC(-HB)/His was obtained. It was confirmed that the desired mutations were introduced and mutations were not introduced in other parts by determining the sequence of the obtained plasmid clone. As a result, there was produced the DNA sequence encoding the protein wherein the 62nd histidine, the 63rd lysine and the 65th lysine of the SEQ ID No. 12 of the Sequence Listing were substituted respectively by isoleucine, asparagine, and asparagine. The obtained plasmid was cut with EcoRI and XbaI to isolate the fragment of about 1.4 kbp, and inserted into the EcoRI/XbaI restriction site of the high expression vector pME18S (Hara, T. et al. (1992) EMBO. J. 11 1875-, edited by Takashi Yokota, Kenichi Arai, The Biotechnology Manual Series 3, Gene-cloning Jikkennhou, published by Yodosha p.18-20). The resulting plasmid was named as pME18S/KhAP5 CC (-HB).

### (2) Examination of the activity of the N terminal 207 amino acid partial protein of the angiopoietin-related protein 3 (ARP3) in which mutations were introduced into the heparin binding motif

### 1) An activity of raising triglyceride concentration in mouse plasma

### A) Production of a recombinant adenovirus

In order to carry out inducible expression of the N terminal 207 amino acid partial protein of ARP3 (ARP3 CC) and the N terminal 207 amino acid partial protein of ARP3 in which variation was introduced into a heparin binding motif (ARP3 CC(-HB)), a recombinant adenovirus was produced using a commercial kit (adenovirus expression vector kit, manufactured by Takara Shuzo CO., LTD.). Namely, pME18S/KhAP5 CC/H or pKF18/KhAP5 CC(-HB)/His was cut by EcoRI and XbaI, and the ends of the DNA fragment of about 1.4 kb were blunted, and the fragment thus obtained was used as the DNA for insertion in the following procedures.

Moreover, cosmid vectors were produced pAxCA/KhAP5 CC/His or pAxCA/KhAP5 CC (-HB)/His were produced, wherein the DNA for insertion is inserted into the restriction enzyme SwaI recognition site of the cosmid vector pAxCAwt (provided with an adenovirus expression vector kit) designed so that the inserted gene can be expressed from a cytomegalovirus enhancer and a chicken β-actin promoter. 293 cells (ATCC CRL1573) were co-transfected with pAxCA/KhAP5 CC/His DNA or pAxCA/KhAP5 CC (-HB)/HisDNA and an end protein joint virus DNA (DNA-TPC provided with the adenovirus expression vector kit) using a calcium phosphate transfection system (manufactured by Lifetech), and the recombinant adenoviruses Ad/KhAP5 CC/His or Ad/KhAP5 CC(-HB)/His were isolated and amplified in the 293 cells. Amplified virus was collected from the 293 cells by subjecting viral infected 293 cells to ultrasonication for 30 second x 4 times (B-1200 manufactured by Branson), and repeating purification by cesium chloride density gradient centrifugation twice. The virus solution obtained was dialyzed at 4 °C to PBS to which 10 % glycerol had been added, and then subjected to cryopreservation at -70 °C or lower.

### B) Concentration of triglyceride in plasma after inoculation of a recombinant adenovirus to a mouse

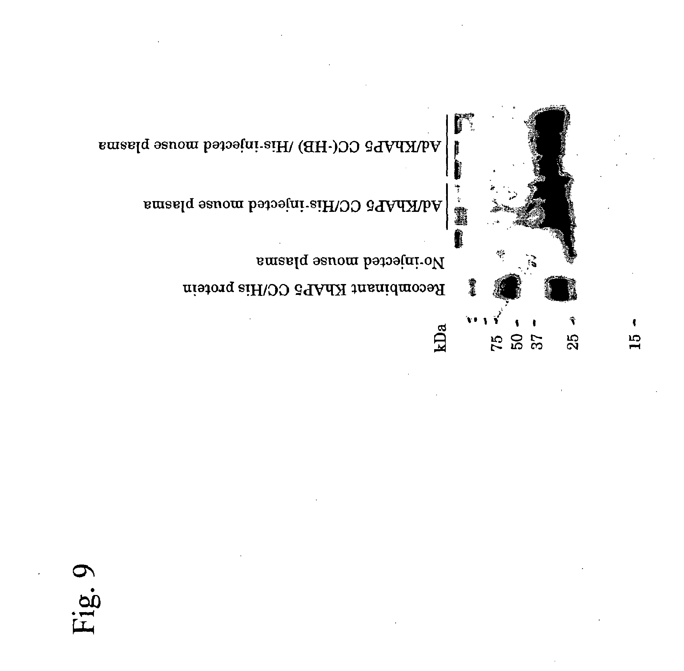

The adenoviruses Ad/KhAP5 CC/His and Ad/KhAP5 CC(-HB)/His purified as mentioned above were diluted with PBS, which contained 10% glycerol, to 2 x 10¹⁰ pfu/ml, and 200 µl (5 x 10⁹ pfu) was inoculated by tail intravenous injection to two (Ad/KhAP5 CC/His) or three male KK/Snk mice (Ad/KhAP5 CC(-HB)/His) of 20 - 21 weeks old, respectively. Blood was collected with a hematocrit pipe from the eyegrounds of each mouse before inoculation and at 1, 2, 4, 7, 11, and 14 days after inoculation, and the plasma was separated by centrifugation at 5200 rpm for 15 minutes with a desk centrifuge apparatus. One µl of the plasma one day after the inoculation was mixed with the SDS-PAGE sample buffer solution (manufactured by Bio-Rad) containing 2-mercaptoethanol, then heated at 99 °C for 5 minutes, to produce a sample for electrophoresis. The sample was subjected to electrophoresis using a polyacrylamide density gradient gel with a gel concentration of 4-20 % (a buffer solution for electrophoresis: 25 mM Tris, 192 mM glycine, 0.1 % SDS), and transferred to a nitrocellulose membrane at 4 °C, for one hour at 200 mA in a transfer buffer solution (25mM Tris, 192 mM glycine, 20 % methanol). The transferred membrane was blocked at 4 °C overnight with PBS solution to which 0.5 % skimmed milk had been added, and was washed 3 times with a washing solution (0.05 % Tween 20-PBS). Subsequently, the membrane was incubated at room temperature for 1 hour in the 0.05 % Tween 20-PBS solution containing 270 ng/ml of 55-1-N1 antibody described in Example 2 and 5 % fetal bovine serum, and thereafter washed 3 times with washing solution. Then, the membrane was incubated at a room temperature for 1 hour in a reaction mixture obtained by diluting horseradish peroxidase labeled goat anti-rabbit IgG(H+L) (manufactured by Amersham Bioscience) 2000 times with 0.05 % Tween 20-PBS which contains 5 % fetal bovine serum, and washed 5 times with washing solution. The membrane was placed on a wrap film, and dipped in an ECL Western blot detection solution for 1 minute, and exposed to X-ray film. As a result, a specific band was detected in the mouse plasma infected with the recombinant adenovirus Ad/KhAP5 CC/His and in the mouse plasma infected with the recombinant adenovirus Ad/KhAP5 CC(-HB)/His (Fig. 9).

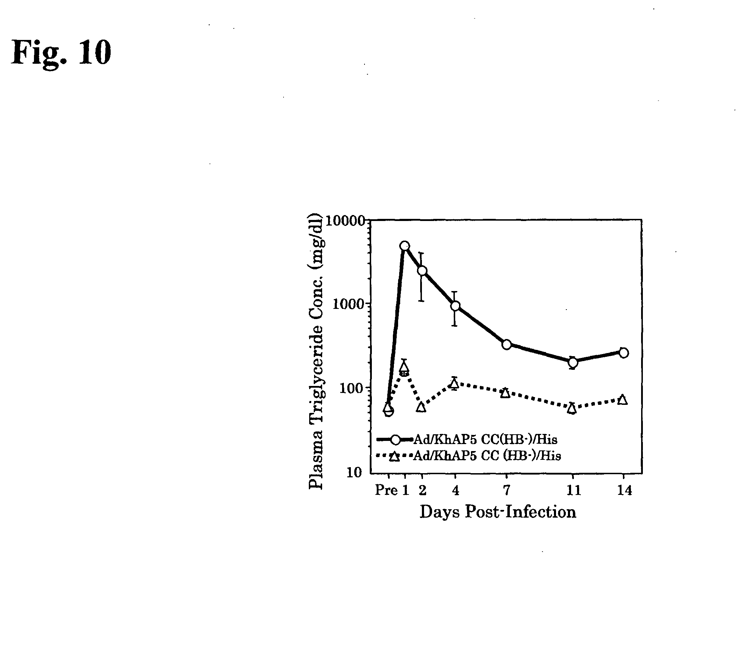

Subsequently, the concentration of neutral fat was measured using a kit for measurement of a neutral fat (triglyceride E test Wako, manufactured by Wako Pure Chemicals). The concentration of neutral fat in plasma was significantly raised in the Ad/KhAP5 CC/His inoculated mouse group, whereas the concentration of neutral fat in blood was hardly raised in the Ad/KhAP5 CC (-HB)/His inoculated mouse group (Fig. 10).

### 2) Lipoprotein lipase (LPL) inhibiting activity

### A) Preparation of a recombinant protein

pME18S/KhAP5 CC/His or pKF18/KhAP5 CC(-HB)/His was introduced into a COS cell (Gluzman, Y. (1981) Cell 23, 175-182, ATCC CRL-1650) using LipofectAMINE2000 reagent (manufactured by Invitrogen) and cultured for 4 days in Dulbecco's Modified Eagle's Medium to which the serum was not added, and then the culture supernatant was collected. The culture supernatant was condensed by ultra concentration to about 1/10 quantity using a 30K filter (manufactured by Millipore) with the TIFF Laboscale (manufactured by Millipore). The condensed sample was diluted by 10 times volume of 50 mM imidazole, 0.5 M of sodium chloride, and 0.02 M of sodium-phosphate buffer to replace the buffer composition, and condensed again by ultra concentration. The sample replaced with the buffer was beforehand treated with 0.5 ml of 0.1 M nickel sulfate, and then allowed to bind to the HiTrap Chelating column (manufactured by Amersham Bioscience) replaced with 50 mM imidazole, 0.5 M sodium chloride, and the 0.02 M sodium-phosphate buffer. Then, it was washed with 100 mM imidazole, 0.5 M sodium chloride and 0.02 M sodium phosphate buffer, and was eluted with 300 mM imidazole, 0.5 M sodium chloride, and 0.02 M sodium phosphate buffer. The eluted sample obtained was dialyzed 3 times with 1,000 times volume of PBS (-) (Sigma), to provide the recombinant proteins ARP3 CC and ARP3 CC (-HB), respectively.

### B) LPL inhibiting activity

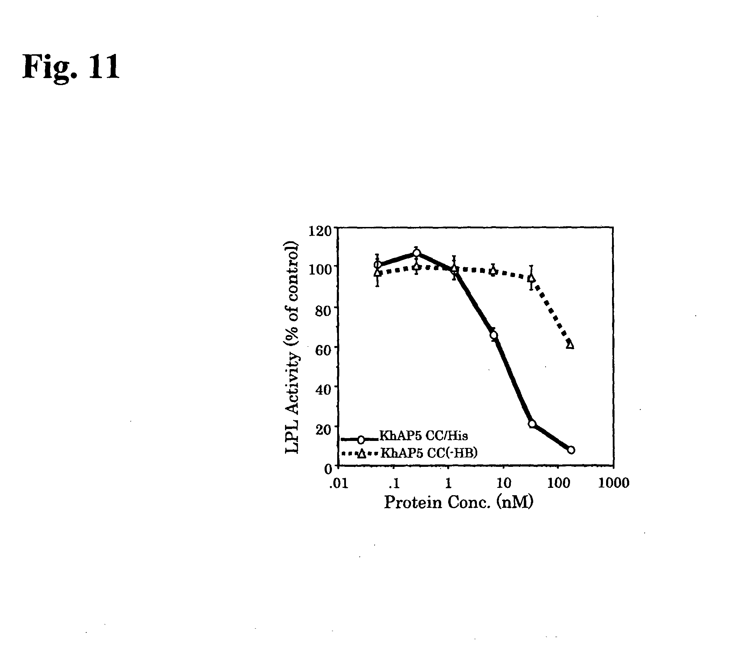

The LPL inhibiting activity of the recombinant protein obtained above was examined by the method described in (1) 2) of Example 11. The LPL inhibiting activity of ARP3 CC(-HB) was remarkably low compared with the LPL inhibiting activity of ARP3 CC (Fig. 11).

As described above, ARP3 CC(-HB) can be used as a negative control when screening for a functional inhibition agent for ARP3.

### Example 15. Preparation of plasma from a mouse in which adenovirus vector for the expression of the histidine tagged protein was inoculated

### (1) Preparation of the recombinant adenovirus

The recombinant adenovirus was produced using an adenovirus expression vector kit (manufactured by Takara Shuzo CO., LTD.) according to the following procedures. The recombination plasmid pME18S/KhAP5/His obtained in (1) 1) of Example 13 was digested with the restriction enzymes EcoRI and XbaI, DNA fragments of about 1.4 kb(s) were collected, and the cut ends were blunted. Then, these fragments were inserted in the SwaI cleavage site of the cosmid vector pAxCAwt designed so that an inserted gene may be expressed from a cytomegalovirus enhancer and the chiken β-actin promoter, to prepare the recombinant cosmid pAxCA/KhAP5/His.

293 cells (ATCC CRL1573) were co-transfected by the recombinant cosmid DNA and the end protein joint virus DNA (DNA-TPC, provided with the adenovirus expression vector kit) using a calcium phosphate transfection system (manufactured by Lifetech) to isolate the recombinant adenovirus Ad/KhAP5/His and amplify it within 293 cells. The amplified virus in the 293 cells was collected from the 293 cells by subjecting the 293 cells infected with the virus to ultrasonication for 30 second x 4 times (the product B-1200 manufactured by Branson), or by repeating freeze-thawing of the cells 3 times. Subsequently, the amplified virus was purified by subjecting to a cesium chloride density gradient centrifugation twice. The viral solution was dialyzed at 4 °C against PBS to which 10 % glycerol had been added, and was frozen and stored at -70 °C or lower until used.

### (2) Confirmation of expression of the target protein by a recombinant adenovirus

HeLa cells were infected with the recombinant adenovirus obtained by the procedure described in (1) above to confirm expression of the target protein.

First, the HeLa cells (ATCC CCL2) were infected at about 5 m.o.i. (multiplicity of infection) with the recombinant adenovirus obtained by the procedure described in (1) above, and were cultured in serum-free medium (Dulbecco's Modified Eagle's Medium (DMEM)) for three to four days, and then the culture supernatant was collected. One ml of the culture supernatant was put into an 1.5 ml Eppendorf tube, and 100 µl of trichloroacetic acid solution (1 g/ml of trichloroacetic acid, 4 mg/ml of deoxycholic acid) were added thereto, left for 3 minutes at room temperature, and then centrifuged at 15000 rpm with a desk centrifuge apparatus for 5 minutes. The supernatant was removed, and 0.5 ml of acetone which was cooled with ice were added and stirred, and then the mixture was centrifuged at 15000 rpm using the desk centrifuge apparatus for 2 minutes. After removing the supernatant, 0.5 ml of acetone which was cooled with ice were added again to the precipitate and agitated, then centrifuged at 15000 rpm with the desk centrifuge apparatus for 2 minutes; vacuum evaporation of the precipitate was carried out after removing the supernatant. The precipitate was dissolved in 10 µl of distilled water, and mixed with SDS-PAGE sample buffer solution (manufactured by Bio-Rad) containing 2-mercaptoethanol, and then heated for 5 minutes at 99 °C, to prepare the sample for electrophoresis. After the samples were subjected to electrophoresis through a polyacrylamide density gradient gel (buffer solution for electrophoresis: 25 mM Tris, 192 mM glycine, 0.1 % SDS) with a gel concentration of 4-20 %, they were transferred to a nitrocellulose membrane under the conditions of 200mA, at 4 °C for 1 hour in a transfer buffer solution (25 mM Tris, 192 mM glycine, 20 % methanol).

The transferred membrane was blocked at 4 °C overnight with the PBS solution to which 0.5 % skimmed milk had been added, and was washed 3 times with washing solution (0.05 % Tween 20-PBS). Subsequently, the membrane was incubated at room temperature for 1 hour in the 0.05 % Tween 20-PBS solution containing 270 ng/ml of the 55-1-N1 antibody described in Example 2 and 5 % fetal bovine serum, and thereafter washed 3 times with washing solution.

Then, the membrane was incubated at room temperature for 1 hour in a reaction mixture obtained by diluting horseradish peroxidase labeled goat anti-rabbit IgG(H+L) (manufactured by Amersham Bioscience) 2000 times with 0.05 % Tween 20-PBS which contains 5 % fetal bovine serum, and washed 5 times with washing solution. This membrane was placed on a wrap film and dipped in a light-emitting reagent solution (ECL Western detection reagent manufactured by Amersham Pharmacia Biotech) for 1 minute, Then it was left at a room temperature for 1 hour to attenuate the background, and then exposed to X-ray film (for 10 seconds). As a result of developing this X-ray film, it was confirmed that the target polypeptides were produced in HeLa cells infected with the recombinant adenovirus.

### (3) Preparation of plasma of KK/Snk mouse infected with the recombinant adenovirus

The recombinant adenovirus Ad/KhAP5/His purified in (1) above was diluted with PBS 10 % glycerol to 7.2 x 10⁹ pfu/ml, and five 18-week old male KK/Snk mice were each administered 300 µl of this dilution by tail intravenous injection.

Two days after the inoculation, blood was collected from the ventral aorta of each mouse using an injection needle (a 26 gauge hypodermic needle and 1 ml syringe manufactured by Terumo) into which 60 µl of 1 % EDTA had been put beforehand, transferred to a 1.5 ml Eppendorf tube, and centrifuged at 5200 rpm for 15 minutes using a desk centrifuge apparatus after mixing with turning to separate the plasma.

### (4) Purification of the target protein in KK/Snk mouse plasma

The obtained plasma was dialyzed against 10 mM imidazole/PBS at 4 °C for four days, and then added to a HiTrap Chelating HP column (manufactured by Amersham) and washed with 20 mM imidazole/PBS and 100 mM imidazole/PBS. Elution was conducted using 300 mM imidazole/PBS, and each fraction was subjected to electrophoresis through a polyacrylamide density gradient gel with a gel concentration of 4 - 20 % (buffer solution for electrophoresis: 25 mM Tris, 192 mM glycine, and 0.1 % SDS), and then GelCode staining (manufactured by Pierce) was performed and the existence of the target protein was confirmed.

### (5) Identification of a restriction site of the target protein in the plasma of KK/Snk mouse

After adding dithiothreitol (manufactured by Sigma. St. Louis, MO) to a final concentration of 10 mM to reduce the human "angiopoietin-related protein 3 " fraction obtained by His tag affinity purification, 55 mM of iodoacetamide (manufactured by Wako, Osaka, Japan) was added thereto to conduct an alkylation reaction. Then, digestion at 37 °C overnight was conducted by adding Lysyl Endopeptidase (manufactured by Wako, Osaka, Japan). Independently, sugar chain elimination treatment at 37 °C for 2 hours by adding PNGase F (manufactured by New England Biolabs, Beverly, MA), and digestion at 37 °C overnight by adding Sequencing Grade Endoproteinase Glu-C (manufactured by Promega, Madison, WI) were conducted. After the digestion reaction, 0.05 % formic acid/H₂O was added to provide a sample for LC/ESI MS/MS (liquid chromatography electrospray ionization tandem mass spectrometry) measurement. For LC, an electro spray needle with a carrier for reversed phase chromatography Inertsil ODS-2 (manufactured by GL Science, Tokyo, Japan) was used as a column and eluted with CapLC System (manufactured by Waters, Milford, MA). Using as elution solvent solution A: 0.05 % formic acid/H₂O, solution B: 0.05 % formic acid/acetonitril, gradient elution wherein a concentration of solution B was linearly increased from 0 % to 30 % in one hour was conducted. The eluates were directly introduced into Q-Tof2 (manufactured by Micromass, Manchester, UK) for ESE MS/MS measrement.
When peptide mapping of human "angiopoietin-related protein 3 " was performed using the MS spectrum obtained, there existed two peptides originating from human "angiopoietin-related protein 3 ":
EIENQLRRTSIQEPTEISLSSKPR (amino acid residue 198-221) and
EIENQLRRTSIQEPTEISLSSKPRAPR (198-224) in the sample digested with Lysyl Endopeptidase and two peptides ISLSSKPR (214-221) and ISLSSKPRAPR (214-224) in the sample digested by endoproteinase Glu-C. The carboxyl terminal of these peptides is not the substrate recognition site of two kinds of these enzymes. Therefore, two sites, between Arg221 and Ala222, and between Arg224 and Thr225, were confirmed as the cleavage sites of the human "angiopoietin-related protein 3 " in blood.

### Example 16. Evaluation of the activity of fused angiopoietin related protein 4

### (1) Preparation of the fused protein

The fused protein (hereinafter referred to as "GST-ARP4N") wherein a glutathione-S-transferase (hereinafter referred to as "GST") was connected to the amino terminus of the polypeptide which consists of the amino acid sequence shown in amino acid numbers 26-143 of SEQ ID No. 16 of the Sequence Listing was prepared according to the procedure indicated below.

First, oligonucleotides having the following nucleotide-sequence were synthesized as PCR primers:
5'-tcccccgggggacccgtgcagtccaag-3' (a sense primer for GST-ARP4N, SEQ ID No. 51 of the Sequence Listing); and
5'-ccgctcgagctggctttgcagatgctg-3' (antisense primer for GST-ARP4N, SEQ ID No. 52 of the Sequence Listing).

Then, PCR was carried out using as a template cDNA clone (clone 4149039) purchased from I.M.A.G.E.Consortium in the presence of the sense primer and the antisense primer (respectively 0.2 M), Tbr EXT DNA polymerase, 1 x PCR buffer solution (10 x buffer solution, provided with a Tbr EXT DNA polymerase, diluted 10 times), 2 mM of magnesium chloride and 0.4 mM of each dNTPs. PCR was carried out under the following conditions: heating the above-mentioned samples at 94 °C for 3 minutes, repeating a temperature cycle of 94 °C for 30 seconds, 55 °C for 30 seconds and 72 °C for 30 seconds 30 times and finally heating at 72 °C for 7 minutes.

DNA fragments of about 370 bp amplified by PCR were collected, and digested with SmaI and XhoI. The fragment was inserted in the plasmid vector pGEX-4T-2 (containing the gene encoding GST, manufactured by Amersham Pharmacia Biotech) which was digested with SmaI and XhoI and subjected to dephosphorylation of the cut ends using a bovine small intestine alkaline phosphatase (manufactured by Takara Shuzo CO., LTD.), and the plasmid clone containing the inserted fragment was isolated. This plasmid clone was named pGST-ARP4N.

Escherichia coli JM 109 was transformed with the recombinant plasmid clone pGST-ARP4N, or the control vector pGEX-4T-2 for expressing only GST which does not contain the insert fragment. Transformation was performed by adding plasmid to competent cells of Escherichia coli JM 109, incubating for 30 minutes on ice, keeping at 42 °C for 30 seconds and leaving for 2 minutes on ice again, and thereafter culturing the cells at 37 °C for 1 hour with shaking. The culture medium after transformation was spread on LB agar plates to which ampicillin was added, the recombinant colonies which appeared were picked and subjected to liquid culture, and expression of recombinant protein induced by IPTG was confirmed.

### (2) Preparation of fused protein

### 1) Culture of the transformed Escherichia coli

The transformed Escherichia coli carrying the plasmid clone pGST-ARP4N and the control vector pGEX-4T-2 were inoculated respectively into LB medium containing ampicillin, and cultured with shaking at 37 °C for 18 hours. A sample of the medium was added to a new medium, and cultured with shaking at 37 °C. When the absorbance at 600 nm became 0.5-0.7, the isopropylthiogalactopyranoside (IPTG) was added thereto to a final concentration of 1 mM, and culture with shaking was continued for further 4 hours.

### 2) Preparation of a cell lysate solution from Escherichia coli carrying pGEX-4T-2

Culture medium containing Escherichia coli transformed with the control vector pGEX-4T-2 was centrifuged at 4 °C, at 6000 rpm for 10 minutes, and the precipitated cells were collected. The collected cells were suspended in the buffer solution for ultrasonication (50 mM of Tris hydrochloride (pH 8.0), 50 mM sodium chloride, 1 mM EDTA, 1 mM DTT), and the suspension was centrifuged at 6000 rpm, at 4 °C for 10 minutes. The precipitate was frozen at -80 °C, subsequently thawed at room temperature, and was suspended in the buffer solution for ultrasonication again. The suspension was treated for 5 minutes under the conditions that output =5 and duty =50 using the ultrasonic blender (UD-201 manufactured by Tomy Seiko). Triton-X 100 was added to the lysate solution after ultrasonication so that the final concentration was 1 %, and mixed therewith. Thereafter it was centrifuged at 1 °C, at 10000 rpm, for 30 minutes, and the supernatant was collected. The supernatant was passed through the GSTrap column (manufactured by Amersham Pharmacia Biotech).

### 3) Preparation of the cell lysate solution from the Escherichia coli carrying pGST-ARP4N

Each sample of the media containing Escherichia coli transformed with the recombinant plasmid pGST-ARP4N was centrifuged at 4 °C, at 5000 rpm for 5 minutes, and the precipitated cells were collected. The precipitated cells were washed 3 times, repeating the suspension process in the washing buffer solution (0.5% Triton-X 100, 1 mM EDTA) and centrifugation at 4 °C, at 5000 rpm, for 10 minutes. The cells obtained were dissolved in an 8 M urea solution. After leaving this solution at room temperature for 1 hour, centrifugation at 4 °C, at 13000 rpm, for 30 minutes was performed, and the supernatant was collected. The supernatant obtained was dialyzed. Dialysis was performed against 4 M urea solution at 4 °C for 1 hour, against 2 M urea solution at 4 °C for 1 hour, against the buffer solution for ultrasonication at 4 °C for a hour, and against the buffer solution for ultrasonication at 4 °C overnight, in that order. After dialysis, centrifugation was carried out at 4 °C, at 12000 rpm, for 30 minutes, and the solution was passed through a GSTrap column.

### 4) Purification of fused protein

PBS was passed through a GST column to which GST, the fused protein with GST (hereinafter referred to as "GST-ARP4N") was adsorbed by the above-mentioned processes, in order to wash the inside of the column to remove impurities. Subsequently, 10 mM reduced glutathione solution was passed through the column to elute GST and GST-ARP4N. After separating a sample of eluate by SDS-PAGE electrophoresis, Coomassie staining of the gel was carried out to confirm the presence of the protein. Moreover, the eluate was dialyzed to PBS 4 °C overnight.

### (2) An activity of raising concentration of neutral fat in blood

GST and GST-ARP4N purified in (1) above were adjusted to a protein concentration of about 1 mg/ml with PBS, and inoculated by tail intravenous injection into two 19-week old male KK/Snk mice at about 300 µl each. Blood was collected before and 30 minutes after the inoculation. The blood was collected from the eyegrounds of each mouse with a hematocrit pipe, and was centrifuged at 5200 rpm using a desk centrifuge for 15 minutes to separate the plasma. A concentration of neutral fat in blood before and after the inoculation was measured. The results are shown in Table 9.

**[Table 9]**

| | TG value (mg/d1) | |
|---|---|---|
| | Pre | After 30 minutes |
| GST | 51 | 44 |
| GST | 38 | 44 |
| GST - ARP4N | 41 | 224 |
| GST - ARP4N | 23 | 216 |

There was no difference before and after inoculation in the GST inoculation mouse group, the concentration of neutral fat in blood was raised significantly in the GST-ARP4N inoculation mouse group.

Using the procedure of this Example, testing of a therapeutic or preventive agent for one or more diseases selected from hyperlipidemia, arteriosclerosis, and hyperglycemia, or two diseases, can be performed, for example, by administering a test substance to the mouse to which GST-ARP4N was inoculated, and investigating the change in concentration of neutral fat in the blood.

### Example 17. LPL activity inhibiting effect

### (1) Preparation of the LPL sample

A rat white fat precursor cell (commercially available from Hokudo) was cultured at 37 °C for 24 hours in a cell culture flask (cultivation area of 25 cm²) containing a medium for proliferation [Dulbecco's modified Eagle culture medium (referred to as "DMEM") containing 10% fetal bovine serum (hereinafter referred to as "FCS"), 100 units/ml of penicillin, 100 µg/ml of streptomycin, 17 µM of pantothenic acid, 33 µM of (+)-biotin, 100 µM of ascorbic acid, 1 µM of octanoic acid and 50 nM of triiodothyronine]. Then, the medium was replaced with a medium for inducing differentiation (DMEM containing 10 % FCS, 100 units/ml of penicillin, 100 µg/ml of streptomycin, 17 µM of pantothenic acid, a 33 µM of (+)-biotin, 100 µM of ascorbic acid, 1 µM of octanoic acid, 50 nM of triiodothyronine, 10 µg/ml of insulin and 2.5 µM of dexamethasone, and cultured at 37 °C for further 48 hours. Then, the medium was replaced with a medium for maintaining fat cells (DMEM containing 10 % FCS, 100 U/ml of penicillin, 100 µg/ml of streptomycin, 17 µM of pantothenic acid, 33 µM of (+)-biotin, 100 µM of ascorbic acid, 1 µM of octanoic acid, 50 nM of triiodothyronine and 10 µg/ml of insulin). Two or three days later, 2 ml of DMEM medium containing 10 % FCS, 50 ng/ml insulin, and 10 units/ml of heparin sodium were added thereto, and the culture supernatant was collected after culturing for one hour, and used for measurement of LPL activity.

### (2) Measurement of LPL activity

The method for measurement was according to the method described in the literature (Nilsson-Ehle, P. and Schotz, M. C. (1976) J. Lipid Res. 17, 536-541).
First, 100 µl of the culture supernatant prepared by the procedure described in 1) above were mixed with an equivalent amount of a substrate solution [2mM glycerol, tri[9, 10(n)-³H] oleic acid (131 KBeq/µmol, manufactured by Amersham), 0.189 µg/ml of L α-phosphatidylcholine (manufactured by Sigma), 14 mg/ml or a bovine serum albumin (manufactured by Sigma), 140 mM of Tris hydrochloride (pH 8.0), 15 % (v/v) of glycerol, 10 % (v/v) of inactivated FCS]. Thereto was added GST or GST-ARP4N obtained in Example 15, and the mixture was kept at 37 °C for 120 minutes. Then, thereto were added 1.05 ml of 0.1 M potassium carbonate/boric acid buffer solution (pH 10.5) and 3.25 ml of methanol: chloroform: hexane = 141 : 125 : 100 (v/v), to stop the reaction. After agitating strongly, centrifugation was carried out at 3000 x g for 15 minutes. ³H count in a water/methanol layer was measured using a solution scintillation counter. One unit of LPL activity was defined as an activity which generates one µmol of fatty acid per 1 minute. Furthermore, LPL activity when adding GST (10 µg/ml) was defined to be 100 %, and the relative LPL activity when adding GST-ARP4N was calculated. The results are shown in Table 10.

**[Table 10]**

| GST - ARP4N (µg/ml) | LPL activity (%) |
|---|---|
| 1 | 74 ± 6 |
| 2.5 | 61 ± 1 |
| 5 | 34 ± 4 |
| 10 | 16 ± 2 |

GST-ARP4N suppressed LPL activity in a dose-dependent manner. In this experimental system, if the test substance is added during the lipase reaction, it can be investigated whether the substance has an effect of regulating LPL activity. Example 18 Evaluation of the activity of the fused protein of angiopoietin-related protein 4 and glutathione-S-transferase

### (1) Preparation of the fused protein

The fused protein (hereinafter referred to as "GST-ARP4Full") wherein a glutathione-S-transferase (hereinafter referred to as "GST") was connected to the amino terminus of a polypeptide which consists of an amino acid sequence shown in amino acid numbers 26-406 of SEQ ID No. 16 of the Sequence Listing was prepared according to the procedure indicated below.

First, oligonucleotides having the following nucleotide-sequence were synthesized as PCR primers:
5'-tcccccgggggacccgtgcagtccaag-3' (a sense primer for GST-ARP4Full, SEQ ID No. 55 of the Sequence Listing); and
5'-ccgctcgagggaggctgcctctgctgc-3' (antisense primer for GST-ARP4Full, SEQ ID No. 56 of the Sequence Listing).

Then, PCR was carried out using as a template 10 ng of cDNA clone (clone 4149039) purchased from I.M.A.G.E. Consortium in the presence of the sense primer and the antisense primer (respectively 0.2 µM), Tbr EXT DNA polymerase, 1 x PCR buffer solution (10 x buffer solution provided with Tbr EXT DNA polymerase, diluted 10 times), 2 mM magnesium chloride and 0.4 mM each dNTPs. PCR was carried out under the following conditions: heating the above-mentioned sample at 94 °C for 3 minutes, repeating a temperature cycle of 94 °C for 30 seconds, 55 °C for 30 seconds and 72 °C for one minute and 30 seconds 35 times, and finally heating at 72 °C for 5 minutes.

DNA fragments of about 1140 bp amplified by PCR were collected, and digested with SmaI and XhoI. The fragment digested with SmaI and XhoI was inserted into the plasmid vector pGEX-4T-2 (containing the gene encoding GST, manufactured by Amersham Pharmacia Biotech) which was digested with SmaI and XhoI and subjected to dephosphorylation oxidation of the cut end using bovine small intestine alkaline phosphatase (manufactured by Takara Shuzo CO., LTD.), and the plasmid clone containing the inserted fragment was isolated. This plasmid clone was named pGST-ARP4Full.

The Escherichia coli BL21 CodonPlus (DE3)-RIL (manufactured by Stratagene) was transformed with the recombinant plasmid clone pGST-ARP4Full, or Escherichia coli JM 109 was transformed with the control vector pGEX-4T-2 for expressing only GST which does not contain the insert fragment.

Transformation was performed by adding 2 µl of XL10-Gold β-mercaptoethanol mix to 100 µl of competent cells Escherichia coli BL21 CodonPlus (DE3)-RIL, then leaving them for 10 minutes on ice, stirring every two minutes, then adding the recombinant or control plasmid, then leaving the mixture for 30 minutes on ice, then keeping the mixture at 42 °C for 30 seconds and leaving for 2 minutes on ice again, and thereafter culturing the cells in SOC medium with shaking at 37 °C for 1 hour. The culture medium after transformation was spread on LB agar plates to which ampicillin and chloramphenicol were added, the recombinant colonies which appeared were picked and subjected to liquid culture, and the expression of recombinant protein induced by IPTG was confirmed.

Additionally, transformation of Escherichia coli JM109 was performed by adding plasmid to the competent Escherichia coli JM 109 cells, leaving for 30 minutes on ice, keeping at 42 °C for 30 seconds and leaving for 2 minutes on ice again, and thereafter culturing the cells in SOC medium at 37 °C for 1 hour. The culture medium after transformation was spread on LB agar plates to which ampicillin was added, recombinant colonies which appeared were picked and subjected to liquid culture, and the expression of recombinant protein induced by IPTG was confirmed.

### (2) Preparation of fused protein

### 1) Culture of the transformed Escherichia coli

The transformed Escherichia coli carrying the plasmid clone pGST-ARP4Full and the control vector pGEX-4T-2 were inoculated respectively into LB media containing ampicillin, and cultured with shaking at 37 °C for 18 hours. A sample of the media was added to a new medium, and cultured with shaking at 37 °C. When the absorbance at 600 nm became 0.5 - 0.7, the isopropylthiogalactopyranoside (IPTG) was added thereto to a final concentration of 1 mM, and culture with shaking was continued for further 4 hours.

### 2) Preparation of a cell lysate solution from the Escherichia coli carrying pGEX-4T-2

Culture media containing Escherichia coli transformed with the control vector pGEX-4T-2 was centrifuged at 4 °C, at 6000 rpm for 10 minutes, and the precipitated cells were collected. The collected cells were suspended in ultrasonication buffer solution (50 mM of Tris hydrochloride (pH 8.0), 50 mM sodium chloride, 1 mM EDTA, 1 mM DTT), and the suspension was centrifuged at 4 °C, at 6000 rpm, for 10 minutes. The precipitate was frozen at -80 °C, subsequently thawed at room temperature, and was suspended in ultrasonication buffer solution again. The suspension was treated for 5 minutes under the conditions that output =5 and duty =50 using the ultrasonic blender (UD-201 manufactured by Tomy Seiko). Triton-X 100 was added to the lysate solution after ultrasonication to a final concentration of 1 %, and mixed therewith. Thereafter it was centrifuged at 4 °C, 10000 rpm, for 30 minutes, and the supernatant was collected. The supernatant was passed through a GSTrap column (manufactured by Amersham Pharmacia Biotech).

### 3) Preparation of the cell lysate solution from Escherichia coli carrying pGST-ARP4Full

Media containing Escherichia coli transformed with the recombinant plasmid pGST-ARP4Full was centrifuged at 4 °C, at 5000 rpm for 5 minutes, and the precipitated cells were collected. The precipitated cells were washed 3 times repeating the process of suspending in washing buffer solution (0.5% of Triton-X 100, 1 mM EDTA) and centrifugation at 4 °C, at 5000 rpm, for 10 minutes three times. The cells obtained were dissolved in an 8 M urea solution. After leaving this solution at room temperature for 1 hour, centrifugation at 4 °C, at 13000 rpm, for 30 minutes was performed, and the supernatant was collected. The supernatant obtained from Escherichia coli transformed with the recombinant plasmid pGST-ARP4Full was dialyzed. The dialysis was performed against 4 M urea solution at 4 °C for 1 hour, against 2 M urea solution at 4 °C for 1 hour, against ultrasonication buffer solution at 4 °C for 1 hour,and against ultrasonication buffer solution at 4 °C overnight, in that order. After dialysis, centrifugation was carried out at 4 °C, 12000 rpm, 30 minutes, and the solution was passed through a GSTrap column.

### 4) Purification of fused protein

PBS was passed through a GST column, to which GST, or the fused protein GST-ARP4Full were adsorbed by the above-mentioned processes, in order to wash the inside of the column to remove impurities. Subsequently, 10 mM reduced glutathione solution were passed through the column to elute GST or GST-ARP4Full. After separating a sample of the eluate by SDS-PAGE electrophoresis, Coomassie staining of the gel was carried out to confirm the presence of the protein. Moreover, the eluate was dialyzed against PBS at 4 °C overnight. The protein purified from the transformant carrying plasmid pGEX-4T-2 was GST, and the protein originating from the transformant carrying pGST-ARP4Full was GST-ARP4Full.

### (3) An activity of raising the concentration of neutral fat in blood

After adjusting GST and GST-ARP4Full which were obtained by (2) above to a protein concentration of about 0.5 mg/ml with PBS, 100 µl was inoculated by tail intravenous injection into two 19-week old male KK/Snk mice respectively. Blood was collected before and 30 minutes after inoculation. The blood was collected from the eyegrounds of each mouse with a hematocrit tube, and centrifuged for 15 minutes at 5200 rpm using a desk centrifuge apparatus to separate the plasma. The concentration of neutral fat (triacyl glycerol) in blood before and after inoculation was measured.

The results are shown in Fig.11. There was no difference before and after inoculation in the GST inoculated mouse group ("GST" in Table 11), the concentration of neutral fat in blood was significantly increased in the GST-ARP4Full inoculated mouse group ("GST-ARP4Full" in Table 11).

In the procedure of this Example, testing of a therapeutic or preventive agent for one or more diseases selected from hyperlipidemia, arteriosclerosis, and hyperglycemia can be performed, for example, by administering a test substance to a mouse inoculated with GST-ARP4Full, and investigating the change in concentration of neutral fat in the blood.

**[Table 11]**

| | TG value (mg/dl) | |
|---|---|---|
| | Pre | After 30 minutes |
| GST | 39 | 39 |
| GST | 62 | 67 |
| GST - ARP4Full | 47 | 291 |
| GST - ARP4Full | 35 | 249 |

### Example 19. Inhibitory effect of GST-ARP4Full on LPL activity (1) A method using LPL originated from a rat fat cell

100 µl of the culture supernatant of rat white fat precursor cell prepared according to the procedure described in (1) 1) of Example 11 was mixed with a equivalent amount of a substrate solution (2mM glycerol, tri[9, 10(n)-³H] oleic acid (131 KBeq/µmol, manufactured by Amersham), 0.189 µg/ml of L α-phosphatidylcholine (manufactured by Sigma), 14 mg/ml bovine serum albumin (manufactured by Sigma), 140 mM Tris hydrochloride (pH 8.0), 15 % (v/v) glycerol, 10 % (v/v) inactivated FCS). Thereto was added GST-ARP4Full or GST obtained in Example 18, and the samples were incubated at 37 °C for 120 minutes. Then, thereto were added 1.05 ml of 0.1 M potassium carbonate/boric acid buffer solution (pH 10.5) and 3.25 ml of methanol: chloroform: hexane = 141 : 125 : 100 (v/v), to stop the reaction. After agitating strongly, centrifugation was carried out at 3000 x g for 15 minutes. ³H count in a water/methanol layer was measured using a solution scintillation counter (manufactured by Reckman). One unit of LPL activity was defined as the activity which generates one µmol of fatty acid per 1 minute. Furthermore, LPL activity when adding GST (10 µg/ml) was defined to be 100 %, and the relative LPL activity when adding GST-ARP4Full was calculated. The results are shown in Table 12.

**[Table 12]**

| GST - ARP4Full (µg/ml) | relative value of LPL activity (%) |
|---|---|
| 1 | 67 ± 4 |
| 2.5 | 30 ± 1 |
| 5 | 16 ± 2 |
| 10 | 5 ± 1 |

GST-ARP4Full suppressed LPL activity in a dose-dependent manner. In this experimental system, if the test substance is added during the lipase reaction, it can be investigated whether the substance has an effect of regulating LPL activity. Example 20. Analysis of the mouse "angiopoietin-related protein 3 " gene

### a) Preparation of BAC (Bacterial artificial chromosome) DNA

Escherichia coli carrying the mouse BAC clone 355L-1 (commercially available from Research Genetics) was cultured at 37 °C overnight in 100 ml of LB medium which contained 12.5 µg/ml chloramphenicol, then 4 ml aliquots of the medium were dispensed into four tubes, and DNA was extracted using an automatic DNA extractor (PI-50, manufactured by Kurabo). Furthermore, this sample was purified by the cesium chloride method.

### b) Fragmentation of DNA

300 µl of 30 ng/µl BAC DNA was transferred to a 1.5 ml Eppendorf tube, sonication was carried out twice using a sonicator (Sonifier II 250, manufactured by Branson) and the special microchip (manufactured by Branson) under the conditions that duty cycle was "constant" and the output adjustment was 1 for 3 seconds, so that DNA was fragmented. The degree of the fragmentation of DNA was confirmed by performing agarose gel electrophoresis.

### c) End restoration of the DNA fragment

To the DNA fragmented as above, 10 units of T4 DNA polymerase (manufactured by Takara Shuzo CO., LTD.) was added in the presence of magnesium chloride (final concentration 5 mM) and 0.05 mM of dNTPs (manufactured by Takara Shuzo CO., LTD.), and kept at 25 °C for 15 minutes. Five units of Klenow fragment (manufactured by Takara Shuzo CO., LTD.) was added, and kept at 25 °C for 15 minutes, to blunt end the fragmented DNA. After purifying the DNA, thereto were added ATP (final concentration of 2 mM) (manufactured by Takara Shuzo CO., LTD.), one unit of the polynucleotide kinase (manufactured by Takara Shuzo CO., LTD.) and the polynucleotide kinase reaction buffer solution (provided with the polynucleotide kinase, the final concentrations were: 50 mM Tris hydrochloride (pH 8.0), 10 mM magnesium chloride, and 5mM dithiothreitol), the mixture was incubated at 37 °C for 30 minutes, and phosphorylation DNA ends was carried out. After completion of the reaction, the polynucleotide kinase was inactivated with TE saturated phenol/chloroform.

### d) Sucrose density gradient centrifugation

The 10 % sucrose solution was prepared mixing 10 ml 50 % sucrose solution, 0.25 ml of 20 x SSC, and 0.1 ml of 0.5 M ethylene diamine tetraacetic acid (hereinafter referred to as "EDTA") and adjusting the total amount to 50 ml with sterilized water. The 38 % sucrose solution was prepared by mixing 38 ml of 50 % sucrose solution, 0.25 ml 20 x SSC and 0.1 ml 0.5 M EDTA, and adjusting the total amount to 50 ml with sterilized water. After adding 38 % sucrose solution to a 13PA tube (manufactured by Hitachikoki), 5 ml of the 10 % sucrose solutions was added gently. Then, a sucrose density gradient was produced using a gradient former (Gradient Mate, manufactured by Cosmobio), under the conditions that velocity (SPEED) was 10, and time (TIME) was 2 minutes and 30 seconds, and the angle (ANGLE) was eight degrees. The DNA obtained in c) above was added to the sucrose density gradient solution obtained, and the mixture centrifuged at 150000 x g at 20 °C for 16 hours. Then, a hole was made at the lower part of the tube using a hypodermic needle (18G), and the solution in the tube was fractionated and collected, in 300 µl fractions from the lower layer. A sample of each fraction was subjected to electrophoresis in an 0.8 % agarose gel, the fraction containing the DNA fragment of about 2.0 kb was chosen, and the DNA was dissolved in 10 µl of TE after ethanol precipitation.

### e) preparation of vector and production of a library

The fragment of 2.0 kb originating from the BAC DNA described above was connected to 0.1 µg of pUC19 DNA/SmaI (manufactured by Fermentus) using a DNA ligation kit (Version 2, manufactured by Takara Shuzo CO., LTD.). The reaction mixture was added to Escherichia coli competent for electroporation (Electro Max DH10B cell, manufactured by Gibco BRL), transferred to a cuvette for electroporation (Gene pulsar cuvette 0.1 cm, manufactured by Bio-Rad), and electroporation was carried out using electroporation equipment (Gene pulsar II, manufactured by Bio-Rad) under conditions that the voltage was 1.8kV, electric capacity was 25 µF per second, and the resistance was 200 ohms. Thereafter, one ml of SOC medium (manufactured by Gibco BRL) was added and incubated at 37 °C for 1 hour, and was spread on LB solid medium (containing 1.2 % bactoagar) containing ampicillin at a final concentration of 50 µg/ml. X-gal (manufactured by Gibco BRL) and IPTG (manufactured by Gibco BRL) at a final concentrations of 50 µg/ml, and cultured at 37 °C overnight.

### f) Preparation of DNA

White colonies obtained as a result of the above process e) were selected, and inoculated, one colony per well, in a microwell plate for cultivation (2.2 ml Deep well plate, manufactured by Japan Genetics) containing TBG medium (containing 12 g of tryptone, 24 g of yeast extract, 2.3 g of potassium phosphate, 12.5 g of potassium phosphate, 4 ml of glycerol and 20 ml of 1 M glucose per liter) containing ampicillin at a final concentration of 50 µg/ml. After sealing the upper surface of the plate with a gas permeability adhesion sheet (manufactured by Japan Genetics), the cells were cultured at 37 °C for 16.5 hours, with gentle agitation using a plate mixer. Then, 600 µl of each culture medium was taken, and transferred to a new microwell plate for cultivation, and centrifugation was carried out at 6000 rpm and 20 °C for 5 minutes using the centrifuge apparatus for microplates (4K15C, manufactured by Sigma). After removing the supernatant, DNA was extracted from the precipitated cells using a DNA extraction kit (QIAprep 96 Turbo Biorobot kit, manufactured by Qiagen), and a DNA extractor (Biorobot 9600, manufactured by Qiagen).

### g) Analysis of nucleotide sequences

Oligonucleotides consisting of the following nucleotide sequence were synthesized as primers using the DNA synthesizer:
5'-tgtaaaacga cggccagt-3'(-21M13forward, SEQ ID No. 57 of the Sequence Listing): and
5'-caggaaacag ctatgacc-3' (M13reverse, SEQ ID No. 58 of the Sequence Listing).

To 10 µl of each of the DNAs obtained in f) above, were added 3.2 pmol of a primer and 8 µl of the sequencing reagent (Bigdye terminator cycle sequence FS ready reaction kit, manufactured by Applied Biosystems), and thereto sterilized water was added to give a total volume of 20 µl. The cycle sequence reaction was carried out using Thermal cycler (Geneamp PCR system 9700, manufactured by Applied Biosystems) (25 cycles, one cycle consists of 96 °C for 10 seconds, 50 °C for 5 seconds, and 60 °C for 4 min), the obtained sample was subjected to nucleotide sequence analysis using a DNA sequencer (AB13700, manufactured by Applied Biosystems). The sequence data obtained was edited and sequenced using DNA sequence software (Sequencher, manufactured by GeneCodes). As a result of this analysis, DNA containing the nucleotide sequence shown in SEQ ID No. 27 of the Sequence Listing was chosen as a target promoter DNA. The transcription initiation site (5' end boundary part of exon 1) of the gene encoding the mouse "angiopoietin-related protein 3 " was determined with reference to "AI195524" which is the EST (Expressed Sequence Tag) with the longest 5' end side registered in the GenBank database. Namely, the first nucleotide after the vector part was determined to be the transcription initiation site of the nucleotide sequences of AI195524, and the position was defined as "1". As described above, DNA which consists of a nucleotide sequence corresponding to the domain from 418 bp at the 5' end side to 132 bp at the 3' end side from the transcription initiation site of mouse "angiopoietin-related protein 3 " gene was identified.

### Example 21. Construction of a luciferase expression plasmid vector

### a) Construction of the plasmid pGL8-3

Based on the result of Example 20, oligonucleotides which consist of the following nucleotide sequences were synthesized as PCR primers for amplifying specifically the 5' end side upstream region of a gene encoding mouse "angiopoietin-related protein 3 ":
5'-aaggtaccgc tgtttccaga taaacaaa-3' (Primer 1, SEQ ID No. 59 of the Sequence Listing): and
5'tcaaatgatg aaaggtctgg atccactctg gatgc-3' (Primer 2, SEQ ID No. 60 of the Sequence Listing).
Sterilized water was added to the above-mentioned primers (to a final concentration of 0.2 µM each), 5 µl of 10 x LA buffer (Mg+) provided with the LAPCR kit (manufactured by Takara Shuzo CO., LTD.), 8 µl of dNTP provided with the kit, 1 µl of the BAC clone 355L-1 DNA purified by the procedure described in a) of Example 20, so that the total volume was 49.5 µl, and then 0.5 µl of LATaq polymerase provided with the kit was added to give a total volume of 50 µl. PCR was carried out using a Thermal cycler (Geneamp PCR system 9600, manufactured by Applied Biosystems) (temperature conditions: heating at 94 °C for 2 minutes, repeating the temperature cycle of 98 °C for 20 seconds, 68 °C for 3 minutes, 30 times; cooling to 4 °C and keeping at that temperature).

After collecting the DNA in the reaction mixture after PCR by ethanol precipitation, it was suspended in 80 µl of sterilized water. The DNA was digested sequentially with the restriction enzymes BamHl and KpnI. Further, the luciferase expression vector pGL3-Basic (manufactured by Promega) was digested with KpnI and BglII and dephosphorylated using alkaline phosphatase (manufactured by Takara Shuzo CO., LTD.), and thereafter ligated to the above-mentioned DNA fragment using a DNA ligation kit (version 2 manufactured by Takara Shuzo CO., LTD.). Escherichia coli DH10B competent cells (manufactured by Gibco BRL) were transformed with this DNA, and colonies with ampicillin resistance were obtained.

Some colonies were cultured in a small volume, and then plasmid was extracted from the culture medium, and the nucleotide sequence of the insert was analyzed using a DNA sequencer (Model 3700, manufactured by Applied Biosystems); the plasmid into which was inserted DNA which consists of a nucleotide sequence shown in nucleotide numbers 1-526 of SEQ ID No. 27 of the Sequence Listing was named pGL8-3. The plasmid pGL8-3 was internationally deposited on June 7, 2001 at the International Patent Organism Depositary, National Institute of Advanced Industrial Science and Technology at Central 6, 1-1-1 Higashi, Tsukuba, Ibaraki, Japan, and was accorded the accession number FERM BP-7627. Transformed Escherichia coli carrying the plasmid were cultured at 37 °C overnight in 100 ml of liquid LB media containing 50 µg/ml of ampicillin, and the DNA was collected and purified from the media using a plasmid extraction purifying kit (Wizard Purefection kit, manufactured by Promega).

### b) Construction of the plasmid pGL13-1

Oligonucleotides having the following nucleotide sequence were synthesized.
5'-aaggtaccaa ttgcatccag agtggatcca a-3' (SEQ ID No. 61 of the Sequence Listing) and
5'-ttggatccac tctggatgca attggtacct t-3' (SEQ ID No. 62 of the Sequence Listing)

5 µg each of the above-mentioned oligonucleotides were mixed, annealed, and then digested sequentally with the restriction enzymes BamHI and KpnI. The luciferase expression vector pGL3-Basic (manufactured by Promega) was digested with KpnI and BglII and dephosphorylated using alkaline phosphatase (manufactured by Takara Shuzo CO., LTD.), and thereafter ligated to the above-mentioned DNA fragment using a DNA ligation kit (Version 2, manufactured by Takara Shuzo CO., LTD.). Escherichia coli DH10B competent cells (manufactured by Gibco BRL) were transformed with this DNA, and colonies with ampicillin resistance were obtained.

Some colonies were cultured in a small volume, and then plasmid was extracted from the culture medium, and the nucleotide sequence of the insert was analyzed using a DNA sequencer (Model 3700, manufactured by Applied Biosystems), and the plasmid pGL13-1 into which the DNA corresponding to the +93 to +108 domain of the mouse "angiopoietin-related protein 3 " gene (nucleotide numbers 511-526 of SEQ ID No. 27 of the Sequence Listing) was inserted was obtained. Transformed Escherichia coli carrying this plasmid were cultured in 100 ml liquid LB media containing 50 µg/ml ampicillin, at 37 °C overnight, and the DNA was collected and purified from the medium using a plasmid extraction purifying kit (manufactured by Promega).

Competent Escherichia coli DH10B cells (manufactured by Gibco BRL) were transformed also with a luciferase expression vector pGL3-Basic (manufactured by Promega), colonies with ampicillin resistance were cultured, and the DNA was collected and purified using a plasmid extraction purifying kit (manufactured by Promega). This plasmid DNA was used as a negative control.

### Example 22. Promoter activity of the angiopoietin-related protein 3 gene

A reporter assay was carried out in order to evaluate the promoter activity of DNA isolated in Example 21. Namely, the cell strain HepG2 originating from human liver was transfected with the firefly luciferase expression plasmid (pGL8-3 or pGL13-1) constructed in Example 21, changes in firefly luciferase activity were investigated. The Renilla luciferase expression plasmid pRL-CMV (manufactured by Promega) was used as an internal standard for rectifying the experimental difference between each plasmid due to cell transfection efficiency.

First, HepG2 cells (commercially available from Dainihon Pharaceuticals) were cultured in MEM medium (manufactured by Gibco BRL) containing 10 % fetal bovine serum (manufactured by Moagate), penicillin/streptomycin, 2 mM of L-glutamine and 1 mM of pyruvic acid in the incubator with 5 % carbon dioxide at 37 °C. After the cells became semiconfluent in a culture flask (50 ml scale, manufactured by Sumitomo bakelite), PBS (-) (manufactured by Nissui Pharmaceuticals) was added to the flask, and the cells were collected by scraping them with a cell scraper (manufactured by Sumitomo bakelite). The cells were seeded on a 6 well culture plate (manufactured by Coning) at 5 x 10⁵ cells/well, and cultured overnight. One µg of pGL3-Basic, pGL8-3 or pGL13-1 DNA and 0.04 ng of pRL-CMV DNA were mixed, and the total volume was adjusted to 5 µl with sterilized water to prepare the DNA solution. 6 µl of the transfection reagent (TransIT-LT1, manufactured by Takara Shuzo CO., LTD.) was added to 100 µl of the MEM medium (with components the same as above but which does not contain fetal bovine serum), and kept at room temperature for 5 minutes.) The DNA sample previously prepared was added to this solution, and kept at room temperature for 5 minutes. The cells were washed twice with 2 ml of the MEM medium which does not contain blood serum (other components are the same as above), and was transferred to 2 ml of the same medium. The DNA solution was transferred to three wells at 30 µl/well, and transferred to 2ml/well of the MEM medium which contains 10% fetal bovine serum (other components are the same as the above) after 4 hour cultivation, and cultured at 37 °C overnight. After the cultivation, the medium was removed, and the cells dissolved in 200 µl/well of a lysis agent (Passive Lysis Buffer (manufactured by Promega)), to prepare the cell extract. Firefly luciferase activity and the Renilla luciferase activity were measured (using Luminas CT-9000D, manufactured by Diatron) using 20 µl of them with a dual luciferase reporter assay system (manufactured by Promega) according to the protocol provided. The experimental results were shown as the firefly luciferase activity rectified using the Renilla luciferase activity as an internal standard.

As a result, it was observed that the luciferase activity in HepG2 cells in which pGL8-3 was introduced was about 19 times that of the cells into which pGL3-Basic was introduced. Therefore, it was revealed that promoter activity exists in DNA which consists of a nucleotide sequence shown in the SEQ ID No. 27 of the Sequence Listing incorporated in pGL8-3. In the cells in which pGL13-1 was introduced, luciferase activity was lowered to 1/10 of the activity found in the cells in which pGL8-3 was introduced. From the above results, it was revealed that promoter activity exists in DNA shown in nucleotide numbers 1-510 of SEQ ID No. 27 of the Sequence Listing, i.e., the domain equivalent to the upstream - 418 to +92 on the 5' end side of the mouse "angiopoietin-related protein 3 " gene.

In the procedure of this Example, the substance inhibiting the promoter activity of the DNA of this invention can be tested by measuring a luciferase activity when culturing HepG2 cells transformed with pGL8-3 in the presence of the test substance. The substance inhibiting the promoter activity may be used as a therapeutic or a preventive agent for one or more diseases selected from hyperlipidemia, arteriosclerosis, and hyperglycemia.

### Example 23. Analysis of the "angiopoietin-related protein 4" promoter region

### a) Isolation of the mouse "angiopoietin-related protein 4" promoter region:

Oligonucleotides which consist of the following nucleotide sequence were synthesized as PCR primers for amplifying specifically the 5' end side upstream region of the gene encoding the mouse "angiopoietin-related protein 4" using the DNA synthesizer (Amersham Bioscience):
5'-cggggtaccgagtgggtgctgggaagcaa-3'(primer 1, SEQ ID No. 63 of the Sequence Listing), and
5'-cccaagcttgccttgggtgcagcaacgct-3' (a primer 2, SEQ ID No. 64 of the Sequence Listing)

PCR was carried out using 100 ng of Genomic DNA of the mouse 129Sv (Sankyo Safety Laboratory) as a template, in the presence of the above-mentioned primers (300 nM each) and 1 µl of a KOD DNA polymerase (manufactured by Toyobo), 1 x PCR reaction buffer solution (1/10 amount of 10 x buffer solution #1 provided with the DNA polymerase), 1 mM of magnesium chloride and 0.2 mM of each dNTP, the total amount was made up to 50 µl by adding sterilized water. PCR was carried out using a Thermal cycler (Geneamp PCR system 9700, manufactured by Applied Biosystems) and heating the above-mentioned sample for 2 minutes at 94 °C, and repeating temperature cycles of 94 °C for 15 seconds, 60 °C for 30 seconds and 68 °C for 3 minutes for 35 cycles, and finally heating at 68 °C for 3 minutes. The resultant PCR product was purified with QIA quick PCR Purification Kit (manufactured by Qiagen), and digested with KpnI and Hind III, and then the target band was separated by 1.0 % 1 x TAE agarose electrophoresis. Then, the DNA was collected by SUPREC-01 (manufactured by Takara Shuzo CO., LTD.) and ethanol precipitation.

After digesting similarly the luciferase expression vector pGL3-Basic (manufactured by Promega) with KpnI and HindIII and subjecting it to dephosphorylation using alkaline phosphatase (manufactured by Takara Shuzo CO., LTD.), it was connected with the above-mentioned DNA fragment using a DNA ligation kit (Version 2, manufactured by Takara Shuzo CO., LTD.). Competent cells of Escherichia coli DH10B (manufactured by Gibco BRL) were transformed with this DNA, and colonies with ampicillin resistance were obtained. A small amount of cultivation of some colonies was carried out, and the plasmid was extracted from the cultured cells, and the nucleotide sequence of the insert was analyzed using a DNA sequencer (models 3700, manufactured by Applied Biosystems Japan), and plasmid was obtained in which a fragment of about 2.9 kb containing the DNA consisting of the nucleotide sequence shown in nucleotide numbers 1-260 of SEQ ID No. 28 of the Sequence Listing was inserted (pGL2-4).

The transformed Escherichia coli carrying this plasmid were cultured at 37 °C overnight in 100 ml of liquid LB medium containing 50 µg/ml of ampicillin, and the DNA was collected and purified from the medium using a plasmid extraction purifying kit (EndoFree Plasmid Maxi Kit, manufactured by Qiagen).

### b) Construction of the luciferase expression plasmid vectors pGL11-4 and pGL12-4

As PCR primers for amplifying specifically the 5' end side upstream region of the gene encoding the mouse "angiopoietin-related protein 4", oligonucleotides which consist of the following nucleotide sequence were synthesized using a DNA synthesizer:
5'-cggggtaccacaaagcctgtggcattgca -3' (primer 3, SEQ ID No. 65 of the Sequence Listing): and
5'-cggggtaccctcccccagaactccagctg -3' (primer 4, SEQ ID No. 66 of the Sequence Listing)

PCR was conducted using the plasmid pGL2-4 as a template, in the presence of the above-mentioned primers (300 nM each) and 1 µl of a KOD DNA polymerase (manufactured by Toyobo), 1 x PCR reaction buffer solution (1/10 amount of 10 x buffer solution #1 provided with the DNA polymerase), 1 mM of magnesium chloride and 0.2 mM of each dNTP, the total amount was made up to 50 µl by adding sterilized water. PCR was carried out using a thermal cycler (Geneamp PCR system 9700, manufactured by Applied Biosystems) and heating the above-mentioned sample for 2 minutes at 94 °C, and repeating temperature cycles of 94 °C for 15 seconds and 60 °C for 15 seconds for 30 cycles, and finally heating at 68 °C for 3 minutes. The resultant PCR product was purified with QIA quick PCR Purification Kit (manufactured by Qiagen), and digested with KpnI ad Hind III, and then the target band was separated by 1.0 % 1 x TAE agarose electrophoresis. Then, the DNA was collected by SUPREC-01 (manufactured by Takara Shuzo CO., LTD.) and ethanol precipitation.

The transcription initiation site (5' end boundary part of exon 1) of the gene encoding the mouse "angiopoietin-related protein 4" was determined with reference to "AK014564" which is the EST (Expressed Sequence Tag) with the longest 5' end side registered in the GenBank database. Namely, the first nucleotide after the vector part was determined as the transcription initiation site within the nucleotide sequences, and the position was defined as "1". As described above, DNA which consists of a nucleotide sequence corresponding to the domain from 90 bp at the 5' end side to 170 bp at the 3'-end side from the transcription initiation site of a mouse "angiopoietin-related protein 4" gene was isolated. Competent cells of Escherichia coli DH10B (manufactured by Gibco BRL) were transformed similarly with the luciferase expression vector pGL3-Basic (manufactured by Promega), and colonies with ampicillin resistance were cultured. DNA was collected and purified from the medium using the plasmid extraction purifying kit (EndoFree Plasmid Maxi Kit, manufactured by Qiagen). The plasmid DNA was used as a negative control.

### Example 24. Promoter activity of the angiopoietin related protein 4 gene

A reporter assay was carried out in order to evaluate the promoter activity of the DNA isolated in Example 23. Namely, the cell strain COS1 originating from ape kidney was transfected with the firefly luciferase expression plasmid (pGL11-4 or pGL12-4) constructed in Example 23, and change of the firefly luciferase activity was investigated. The Renilla luciferase expression plasmid pRL-TK (manufactured by Promega) was used as an internal standard for rectifying the experimental difference between the transfection efficiency of each plasmid into the cells.

First, COS1 cells (commercially available from American Type Culture Collection: ATCC) were cultured in D-MEM medium (manufactured by Gibco BRL, Catalog Number 11965-092) containing 10 % fetal bovine serum (manufactured by Gibco BRL), penicillin/streptomycin, in the incubator with 5 % carbon dioxide at 37 °C. In a 24 well culture plate (manufactured by Corning), the cells were proliferated until they became semiconfluent. 0.2 µg of pGL3-Basic, pGL11-4 or pGL12-4 DNA and 6 ng of pRL-TK DNA were mixed, and the total volume was adjusted to 5 µl with sterilized water to prepare the DNA solution. One µl of the transfection reagent (FuGENE6, manufactured by Roche) was added to 20 µl of D-MEM medium which does not contain fetal bovine serum (other components were the same as above). The DNA sample previously prepared was added to this solution, and kept at room temperature for 15 minutes. The DNA solution was transferred to three wells, and cultured at 37 °C over two nights. After cultivation, the medium was removed, and the cells dissolved in 250 µl/well of lysis agent (Passive lysis Buffer (manufactured by Promega)), to prepare the cell extract. The firefly luciferase activity and the Renilla luciferase activity were measured (using Luminas CT-9000D, manufactured by Diatron) using 20 µl of them with a dual luciferase reporter assay system (manufactured by Promega) according to the protocol provided. The experimental results were shown as the firefly luciferase activity rectified using the Renilla luciferase activity as an internal standard.

As a result, it was observed that the luciferase activity in COS-1 cells in which pGL11-4 was introduced was about 30 times that of the cells in which pGL3-Basic was introduced. Therefore, it was revealed that activity as a promoter exists in DNA which consists of a nucleotide sequence shown in SEQ ID No. 28 of the Sequence Listing incorporated in pGL8-3. On the other hand, in cells into which pGL12-4 was introduced, luciferase activity in the cell was about 1.4 times that of the cells into which pGL3-Basic was introduced. From the above results, it was revealed that promoter activity exists in the DNA shown in nucleotide numbers 1-260 of SEQ ID No. 28 of the Sequence Listing, i.e., the domain equivalent to the upstream -90 to +170 on the 5' end side of the mouse "angiopoietin-related protein 4" gene. In the table, (Mean ± SD) means the average and standard deviation of luciferase activity.

**[Table 13]**

| luciferase activity (Mean ± SD) | |
|---|---|
| pGL3 - Basic | 1.0 ± 0.1 |
| pGL11 - 4 | 30.3 ± 5.3 |
| pGL12 - 4 | 1.7 ± 0.2 |

In the procedure of this Example, a substance inhibiting the promoter activity of the DNA of this invention can be tested by measuring a luciferase activity when culturing COS1 cells transformed with pGL11-4 in the presence of the test substance. A substance inhibiting the promoter activity may be used as a therapeutic or a preventive agent for one or more diseases selected from hyperlipidemia, arteriosclerosis, and hyperglycemia.

### [Industrial Availability]

As described above, according to the present invention, it was confirmed that the genes consisting of the nucleotide sequence shown in nucleotide numbers 47-1411 of SEQ ID No. 1 of the Sequence Listing or the nucleotide sequence shown in nucleotide numbers 78-1457 of SEQ ID No. 3 of the Sequence Listing are hereditary disorders involved in one or more diseases selected from hyperlipidemia, arteriosclerosis, and hyperglycemia, and therefore substances which control the level of expression of these genes may serve as a therapeutic or preventive agent for one or more diseases selected from hyperlipidemia, arteriosclerosis, and hyperglycemia. That is, the methods of the present invention, a polynucleotide used as a probe or a primer in the mode which detects a nucleic acid among the methods, and an antibody used in the mode which similarly detects the polypeptide are useful for searching for a therapeutic or preventive agent for one or more diseases selected from hyperlipidemia, arteriosclerosis, and hyperglycemia. Furthermore, it was shown that a polypeptide of the present invention has an activity of increasing the concentration of neutral fat in blood, and that the activity can be maintained even with polypeptide in the form of a fused protein, that a polypeptide of the present invention has an inhibitory effect on LPL activity, that a test for a therapeutic or preventive agent for one or more diseases selected from hyperlipidemia, arteriosclerosis, and hyperglycemia can be conducted in vivo or in vitro using a polypeptide of the present invention.

Furthermore, there has been provided a novel DNA having a promoter activity according to the present invention. The DNA of the present invention can be used for a method for testing a substance suppressing the expression level of angiopoietin-related protein 3 and angiopoietin-related protein 4, it is useful for development of a therapeutic or preventive agent for one or more diseases selected from hyperlipidemia, arteriosclerosis, and hyperglycemia having a new mechanism of action.

## Claims

1. A method for testing a therapeutic or preventive effect of a test substance for one or more of diseases selected from hyperlipidemia, arteriosclerosis, and hyperglycemia comprising the following steps:
1) culturing cells originating from a mammal in the presence or absence of the test substance;
2) detecting the amount of expression of mRNA which has a nucleotide sequence of any one of the following sequences a) to e) (however, t in the sequence is read as u), in the cultured cells obtained in 1) above:
a) the nucleotide sequence shown in nucleotide numbers 47-1411 of SEQ ID No. 1 of the Sequence Listing;
b) the nucleotide sequence shown in nucleotide numbers 78-1457 of SEQ ID No. 3 of the Sequence Listing;
c) the nucleotide sequence of DNA incorporated into a phagemid carried by transformed Escherichia coli E.coli pBK/m55-1 SANK72199 (FERM BP-6940);
d) the nucleotide sequence of DNA incorporated into a phagemid carried by transformed Escherichia coli E.coli pTrip/h55-1 SANK72299 (FERM BP-6941);
e) the nucleotide sequence which hybridizes to a polynucleotide consisting of an antisense sequence of a nucleotide sequence described in any of the above a) to d) under stringent conditions, and encodes a polypeptide having an activity of raising the concentration of neutral fat in blood; and
3) comparing the amount of the detected mRNA expression between cells cultured in the absence of the test substance and cells cultured in the presence of the test substance, as a result of the detection in the above step 2).

2. The method according to claim 1, wherein the cultured cell originating from a mammal originates from the liver.

3. The method according to claim 1 or 2, wherein the cultured cell originating from a mammal originates from a primate or a rodent animal.

4. The method according to any one of claims 1 to 3, wherein the cultured cell originating from a mammal originates from a human or a mouse.

5. The method according to any one of claims 1 to 4, wherein the method for detecting the amount of expression of mRNA is a Northern blot, a dot blot or a slot blot.

6. The method according to any one of claims 1 to 4, wherein the method for detecting the amount of expression of mRNA is RT-PCR.

7. The method according to any one of claims 1 to 4, wherein the method for detecting the amount of expression of mRNA is a ribonuclease protection assay.

8. The method according to any one of claims 1 to 4, wherein the method for detecting the amount of expression of mRNA is a run-on assay.

9. The method according to any one of claims 1 to 4, wherein the method for detecting the amount of expression of mRNA is conducted using a gene chip produced with a group of complementary DNAs originating from a tissue or a cell of animals or partial sequences of the DNA.

10. A method for testing a therapeutic or preventive effect of a test substance against one or more of diseases selected from hyperlipidemia, arteriosclerosis, and hyperglycemia comprising the following steps:
1) culturing cells in the presence or absence of the test substance;
2) detecting the amount of production of a polypeptide, having an amino acid sequence encoded by the nucleotide sequence of any one of the following a) to e) or a part thereof, in the supernatant of the cultured cells obtained in 1) above using an antibody specifically binding to the polypeptide; and
a) the nucleotide sequence shown in nucleotide numbers 47-1411 of SEQ ID No. 1 of the Sequence Listing;
b) the nucleotide sequence shown in nucleotide numbers 78-1457 of SEQ ID No. 3 of the Sequence Listing;
c) the nucleotide sequence of DNA incorporated into a phagemid carried by transformed Escherichia coli E.coli pBK/m55-1 SANK72199 (FERM BP-6940);
d) the nucleotide sequence of DNA incorporated into a phagemid carried by transformed Escherichia coli E.coli pTrip/h55-1 SANK72299 (FERM BP-6941);
e) the nucleotide sequence which hybridizes to a polynucleotide consisting of an antisense sequence of the nucleotide sequence described in any of the above a) to d) under stringent conditions, and encodes a polypeptide having the activity of raising neutral fat concentration in blood; and
3) comparing the amount of the detected polypeptide between the cells cultured in the absence of the test substance and the cells cultured in the presence of the test substance, as a result of the above mentioned step 2);

11. A method according to claim 10 wherein the antibody which specifically binds to a polypeptide consisting of an amino acid sequence encoded by any one of the above-mentioned nucleotide sequences a) to e) or a part thereof is an antibody which specifically binds to a polypeptide consisting of the amino acid sequence shown in amino acid numbers 17-455 of SEQ ID No. 2 of the Sequence Listing or a part thereof, a polypeptide which consists of the amino acid sequence shown in amino acid numbers 19-455 of the same sequence as the above or a part thereof, or a polypeptide which consists of amino acid sequence shown in amino acid numbers 17-460 of SEQ ID No. 4 of the Sequence Listing or a part thereof.

12. A method according to claim 10 or 11 wherein the antibody which specifically binds to a polypeptide consisting of the amino acid sequence encoded by any one of the above-mentioned nucleotide sequences a) to e) or a part thereof is an antibody which specifically recognizes a polypeptide which consists of the amino acid sequence shown in amino acid numbers 1-13 of SEQ ID No. 9 of the Sequence Listing or amino acid numbers 1-14 of SEQ ID No. 10.

13. A method according to any one of claims 10 to 12, wherein the method for detecting the amount of production of polypeptide having an amino acid sequence encoded by the nucleotide sequence of any one of the above-mentioned a) to e) or a part thereof using an antibody which specifically recognizes the polypeptide is Western blotting, a dot blotting or a slot blotting.

14. A method according to any one of claims 10 to 12, wherein the method for detecting the amount of production of polypeptide having an amino acid sequence encoded by the nucleotide sequence of any one of the above-mentioned a) to e) or a part thereof using an antibody which specifically binds to the polypeptide is a solid-phase enzyme immunoassay (ELISA method), or a radioisotope immunoassay (RIA method).

15. A method according to claim 14, wherein a monoclonal antibody produced by mouse hybridoma 45B1 is used as the antibody immobilized on a plate in the ELISA method. The hybridoma 45B1 was deposited on March 13, 2002 in International Patent Organism Depositary, National Institute of Advanced Industrial Science and Technology, and was accorded the accession number FERM BP-7963.

16. A kit for testing a therapeutic or preventive agent for one or more diseases selected from hyperlipidemia, arteriosclerosis, and hyperglycemia comprising the following antibodies i) and/or ii)
i) an antibody which specifically binds to a polypeptide consisting of an amino acid sequence encoded by a nucleotide sequence of any one of the following a) to e) or a part thereof
a) the nucleotide sequence shown in nucleotide numbers 47-1411 of SEQ ID No. 1 of the Sequence Listing;
b) the nucleotide sequence shown in nucleotide numbers 78-1457 of SEQ ID No. 3 of the Sequence Listing;
c) the nucleotide sequence of DNA incorporated into a phagemid carried by transformed Escherichia coli E.coli pBK/m55-1 SANK72199 (FERM BP-6940);
d) the nucleotide sequence of DNA incorporated into a phagemid carried by transformed Escherichia coli E.coli pTrip/h55-1 SANK72299 (FERM BP-6941);
e) the nucleotide sequence which hybridizes to a polynucleotide consisting of an antisense sequence of the nucleotide sequence described in any of the above a) to d) under stringent conditions, and encodes a polypeptide having an activity of raising a concentration of neutral fat in blood; and
ii) an antibody which specifically binds to a polypeptide which consists of the amino acid sequence shown in the amino acid numbers 1-13 of SEQ ID No. 9 of the Sequence Listing or the amino acid numbers 1-14 of SEQ ID No. 10.

17. A composition for gene therapy of one or more diseases selected from hyperlipidemia, arteriosclerosis, and hyperglycemia which contains DNA or RNA which has a nucleotide sequence including the antisense sequence of a nucleotide sequence consisting of 15 or 30 continuous nucleotides in the nucleotide sequence shown in nucleotide numbers 78-1457 of SEQ ID No. 3 of the Sequence Listing as an active substance.

18. A polypeptide having a continuous amino acid sequence contained in the amino acid sequence shown in SEQ ID No. 12 of the Sequence Listing wherein the amino terminal is the amino acid of the amino acid number 17, and the carboxyl terminal is any one of amino acid numbers 147 to 207.

19. A polypeptide according to claim 18 which consists of an amino acid sequence shown in amino acid numbers 17 to 207 of SEQ ID No. 12 of the Sequence Listing.

20. A polypeptide according to claim 18 which consists of an amino acid sequence shown in amino acid numbers 17 to 195 of SEQ ID No. 12 of the Sequence Listing.

21. A polypeptide according to claim 18 which consists of an amino acid sequence shown in amino acid numbers 17 to 147 of SEQ ID No. 12 of the Sequence Listing.

22. A polypeptide which has a continuous amino acid sequence contained in the amino acid sequence shown in SEQ ID No. 16 of the Sequence Listing wherein the amino terminal is amino acid number 26 and the carboxyl terminal is amino acid number 143.

23. A polypeptide which has a continuous amino acid sequence contained in the amino acid sequence shown in SEQ ID No. 16 of the Sequence Listing wherein the amino terminal is amino acid number 26 and the carboxyl terminal is any one of amino acid numbers 144 to 183.

24. A polypeptide which has a continuous amino acid sequence contained in the amino acid sequence shown in SEQ ID No. 18 of the Sequence Listing wherein the amino terminal is amino acid number 22 and the carboxyl terminal is any one of amino acid numbers 202 to 276.

25. A polypeptide which has a continuous amino acid sequence contained in the amino acid sequence shown in SEQ ID No. 20 of the Sequence Listing wherein the amino terminal is amino acid number 23 and the carboxyl terminal is any one of amino acid numbers 206 to 275.

26. A polypeptide which has a continuous amino acid sequence contained in the amino acid sequence shown in SEQ ID No. 22 of the Sequence Listing wherein the amino terminal is amino acid number 21 and the carboxyl terminal is any one of amino acid numbers 185 to 258.

27. A polypeptide which has a continuous amino acid sequence contained in the amino acid sequence shown in SEQ ID No. 24 of the Sequence Listing wherein the amino terminal is amino acid number 27 and the carboxyl terminal is any one of amino acid numbers 122 to 129.

28. A polypeptide which consists of an amino acid sequence contained in the amino acid sequence shown in SEQ ID No. 16 of the Sequence Listing wherein the amino terminal is amino acid number 26 and the carboxyl terminal is amino acid number 406.

29. A polypeptide which consists of an amino acid sequence contained in the amino acid sequence shown in SEQ ID No. 18 of the Sequence Listing wherein the amino terminal is amino acid number 22 and the carboxyl terminal is amino acid number 491.

30. A polypeptide which consists of an amino acid sequence contained in the amino acid sequence shown in SEQ ID No. 20 of the Sequence Listing wherein the amino terminal is amino acid number 23 and the carboxyl terminal is amino acid number 493.

31. A polypeptide which consists of an amino acid sequence contained in the amino acid sequence shown in SEQ ID No. 22 of the Sequence Listing wherein the amino terminal is amino acid number 21 and the carboxyl terminal is amino acid number 470.

32. A polypeptide which consists of an amino acid sequence contained in the amino acid sequence shown in SEQ ID No. 24 of the Sequence Listing wherein the amino terminal is amino acid number 27 and the carboxyl terminal is amino acid number 346.

33. A polypeptide consisting of an amino acid sequence shown in SEQ ID No. 26 of the Sequence Listing.

34. An analogue of a polypeptide which has an activity of raising the neutral fat concentration in the blood of a mammal having an amino acid sequence wherein one or several amino acid residues are deleted, inserted, added or replaced in one or several sites of the amino acid sequences of any one of the polypeptides of claims 18 to 33.

35. A DNA which codes for any one of the polypeptides of claims 18 to 33.

36. A DNA consisting of a continuous sequence included in the nucleotide sequence shown in SEQ ID No. 11 of the Sequence Listing wherein nucleotide number 18 is the 5'-end and any one of nucleotide numbers 458 to 638 is the 3'-end;

37. A DNA consisting of a continuous sequence included in the nucleotide sequence shown in SEQ ID No. 11 of the Sequence Listing wherein nucleotide number 18 is the 5'-end and any one of nucleotide numbers 458 to 602 is the 3'-end.

38. A DNA consisting of a continuous sequence included in the nucleotide sequence shown in SEQ ID No. 15 of the Sequence Listing wherein nucleotide number 173 is the 5'-end and nucleotide number 601 is the 3'-end.

39. A DNA consisting of a continuous sequence included in the nucleotide sequence shown in SEQ ID No. 15 of the Sequence Listing wherein nucleotide number 173 is the 5'-end and nucleotide number 1393 is the 3'-end.

40. A DNA consisting of a continuous sequence included in the nucleotide sequence shown in SEQ ID No. 17 of the Sequence Listing wherein nucleotide number 434 is the 5'-end and any one of nucleotide numbers 1039 to 1261 is the 3'-end.

41. A DNA consisting of a continuous sequence included in the nucleotide sequence shown in SEQ ID No. 17 of the Sequence Listing wherein nucleotide number 434 is the 5'-end and nucleotide number 1909 is the 3'-end.

42. A DNA consisting of a continuous sequence included in the nucleotide sequence shown in SEQ ID No. 19 of the Sequence Listing wherein nucleotide number 22 is the 5'-end and any one of nucleotide numbers 639 to 846 is the 3'-end.

43. A DNA consisting of a continuous sequence included in the nucleotide sequence shown in SEQ ID No. 19 of the Sequence Listing wherein nucleotide number 22 is the 5'-end and nucleotide number 1503 is the 3'-end.

44. A DNA consisting of a continuous sequence included in the nucleotide sequence shown in SEQ ID No. 21 of the Sequence Listing wherein nucleotide number 242 is the 5'-end and any one of nucleotide numbers 796 to 1015 is the 3'-end.

45. A DNA consisting of a continuous sequence included in the nucleotide sequence shown in SEQ ID No. 21 of the Sequence Listing wherein nucleotide number 242 is the 5'-end and nucleotide number 1654 is the 3'-end.

46. A DNA consisting of a continuous sequence included in the nucleotide sequence shown in SEQ ID No. 23 of the Sequence Listing wherein nucleotide number 86 is the 5'-end and any one of nucleotide numbers 373 to 394 is the 3'-end.

47. A DNA consisting of a continuous sequence included in the nucleotide sequence shown in SEQ ID No. 23 of the Sequence Listing wherein nucleotide number 86 is the 5'-end and nucleotide number 1048 is the 3'-end.

48. A DNA which consists of a nucleotide sequence shown in nucleotide numbers 18 to 62 of SEQ ID No. 25 of the Sequence Listing.

49. A recombinant vector containing a DNA of any one of claims 35 to 48.

50. The recombinant vector of claim 49 wherein it is an adenovirus vector.

51. A host cell transformed by a recombinant vector of claim 49 or 50.

52. A method for producing a polypeptide having an activity of raising neutral fat concentration in the blood of a mammal **characterized by** culturing the host cell according to claim 51, and subsequently collecting from the culture the polypeptides which have an activity of raising neutral-fat concentration in the blood of a mammal.

53. A method for testing a therapeutic or preventive agent for one or more diseases selected from hyperlipidemia, arteriosclerosis, and hyperglycemia comprising the following steps:
1) administering to a non-human mammal one or more polypeptide selected from the polypeptides according to any one of claims 18 to 34; and
2) measuring the concentration of neutral fat in the blood of the animal of 1) above.

54. A method for testing a therapeutic or preventive agent for one or more diseases selected from hyperlipidemia, arteriosclerosis, and hyperglycemia comprising the following steps:
1) infecting a non-human mammal with adenovirus carrying a recombinant adenovirus vector according to claim 50;
2) administrating a test substance to the animal obtained in 1); and
3) measuring the concentration of neutral fat in the blood of the animal obtained in 2).

55. A method for testing a therapeutic or preventive agent for one or more diseases selected from hyperlipidemia, arteriosclerosis, and hyperglycemia **characterized in that** a polypeptide consisting of an amino acid sequence shown in amino acid number 17 to 460 of SEQ ID No. 4 of the Sequence Listing, a polypeptide wherein 1 to 253 amino acid residues at the carboxyl terminal are absent from the polypeptide, or a polypeptide according to any one of claims 18 to 34 are allowed to exist together with the test substance in the system, and in which a lipoprotein lipase (hereinafter referred to as "LPL") and/or hepatic triacylglycerol lipase (hereinafter referred to as "HTGL") are allowed to react with the substrates thereof, and the test substance is investigated for an effect of suppressing inhibition of the LPL and/or HTGL activity of the polypeptide.

56. A method for testing a substance for regulating LPL activity comprising the following steps:
1) preparing a mixture wherein the substance described in the following i-a to iii and the substance to be tested are allowed to exist together:
i-a material containing LPL,
ii-a material containing the substrate of LPL,
iii a polypeptide consisting of an amino acid sequence shown in amino acid numbers 17 to 460 of SEQ ID No. 4 of the Sequence Listing, a polypeptide wherein any length of from 1 to 253 amino acid residues at the carboxyl terminal is absent from the polypeptide, or a polypeptide according to any one of claims 18 to 34
2) preparing the mixture consisting of only the material i-a to iii of 1) above, independently from the mixture 1)
3) measuring an amount of fatty acid isolated from the substrate in the mixtures of 1 and 2 above respectively, and comparing the measured values.

57. A method according to claim 56 wherein the material containing LPL is a culture supernatant of a fat cell culture originating from a mammal.

58. A method according to claim 56 or 57 wherein the material containing LPL is a culture supernatant of fat cell culture originating from a rodent or a primate.

59. A method according to any one of claims 56 to 58 wherein the material containing LPL is a glycerol trioleic acid.

60. A method for testing a substance for regulating LPL and/or HTGL activity comprising the following steps:
1) preparing a mixture wherein the substance described in the following i-b to iii and the substance to be tested are allowed to exist together:
i-b material containing LPL and/or HTGL,
ii-b material containing the substrate of LPL and/or HTGL, iii polypeptide consisting of an amino acid sequence shown in amino acid numbers 17 to 460 of SEQ ID No. 4 of the Sequence Listing, a polypeptide wherein any length of from 1 to 253 amino acid residues at the carboxyl terminal is absent from the polypeptide, or a polypeptide according to any one of claims 18 to 34,
2) preparing the mixture consisting of only the material i-b to iii of 1) above, independently from the mixture 1),
3) measuring an amount of fatty acid isolated from the substrate in the mixtures of the above 1 and 2 respectively, and comparing the measured values.

61. A method according to claim 60 wherein the material containing LPL and/or HTGL is a plasma originating from a mammal to which heparin has been administered intravenously.

62. A method according to claim 61 or 62 wherein the material containing the substrate of LPL and/or HTGL is a glycerol trioleic acid.

63. A method according to claim 59 wherein the material containing LPL and/or HTGL is a culture supernatant of a cultured fat cell originating from a mammal or plasma originating from a mammal to which heparin has been administered intravenously.

64. A method according to any one of claims 59 to 63 wherein the material containing LPL and/or HTGL is a culture supernatant of cultured fat cells originating from a rodent or primate.

65. A method according claim 59, 63 or 64 wherein the material containing the substrate of LPL and/or HTGL is a glycerol trioleic acid.

66. A DNA shown in any one of 1) to 3)
1) DNA which consists of a nucleotide sequence shown in SEQ ID No. 27 of the Sequence Listing wherein the 5'-end is nucleotide number 1 and the 3'-end is any one of the nucleotides shown as nucleotide numbers 510 to 526;
2) DNA which hybridizes to the DNA in 1) above under stringent conditions, and has a promoter activity in a cell originating from mammalian liver;
3) DNA which includes a nucleotide sequence having 95% or more of nucleotide sequence identity with DNA in 1) above, and which has a promoter activity in a cell originating from mammalian liver.

67. A DNA including a nucleotide sequence shown as nucleotide numbers 1-510 of SEQ ID No. 27 of the Sequence Listing.

68. A DNA which consists of a nucleotide sequence shown as nucleotide numbers 1-510 of SEQ ID No. 27 of the Sequence Listing.

69. A DNA which consists of a nucleotide sequence shown as nucleotide numbers 1-526 of SEQ ID No. 27 of the Sequence Listing.

70. A DNA shown in any one of the following 1) to 3)
1) DNA which consists of a nucleotide sequence shown in SEQ ID No. 28 of the Sequence Listing wherein the 5'-end is nucleotide number 1 and the 3'-end is any one of the nucleotides shown as nucleotide number 188 to 260;
2) DNA which hybridizes to the DNA of 1) above under stringent conditions, and which has a promoter activity in a cell originating from mammalian liver;
3) DNA which includes a nucleotide sequence having 95% or more nucleotide sequence identity with DNA of 1) above, and which has a promoter activity in a cell originating from mammalian liver.

71. A DNA including a nucleotide sequence shown as nucleotide numbers 1-188 of SEQ ID No. 28 of the Sequence Listing, and which has a promoter activity in a cell originating from a mammal.

72. A DNA which consists of a nucleotide sequence shown as nucleotide numbers 1-188 of SEQ ID No. 28 of the Sequence Listing.

73. An expression vector containing a DNA of any one of claims 66 to 72.

74. An expression vector wherein a DNA according to any one of claims 66 to 72 is connected to the upstream side of a foreign gene in the same transcription direction as that of the sequence of the foreign gene and which can express the foreign gene in a a mammal cell.

75. An expression vector according to claim 74 wherein the sequence of the foreign gene is a nucleotide sequence coding for a protein selected from the group consisting of luciferase, horseradish peroxidase, alkaline phosphatase, beta-galactosidase, and green fluorescence protein.

76. An expression vector according to claim 74 wherein the sequence of the foreign gene is a nucleotide sequence coding for luciferase.

77. A recombinant plasmid pGL 8-3 (FERM BP-7627).

78. An expression vector pGL 11-4.

79. A host cell transformed with an expression vector according to any one of claims 73 to 78.

80. A method for testing a therapeutic or preventive effect of a test substance for one or more diseases selected from hyperlipidemia, arteriosclerosis, and hyperglycemia comprising the following steps:
1) culturing a host cell transformed with an expression vector according to any one of claims 73 to 78 in the presence or absence of the test substance;
2) detecting or measuring the amount of production of a protein which is encoded by the foreign gene in the expression vector according to any one of claims 73 to 78;
3) comparing the result of the detection or measuring in the above step 2) between the cells cultured in the presence of the substance and the cells cultured in the absence of the substance.

81. A method for identifying cDNA coding for a protein having a therapeutic or preventive effect in one or more diseases selected from hyperlipidemia, arteriosclerosis, and hyperglycemia comprising the following steps:
i) individually culturing a cell selected from the group consisting of:
1) a host cell transformed with an expression vector according to any one of claims 73 to 78 (hereinafter referred to as "cell A");
2) a cell which is obtained by transforming the cell A with a recombinant plasmid in which the cDNA to be tested is incorporated into a mammalian expression vector and which expresses the cDNA to be tested transient or stably (hereinafter referred to "cell B");
3) a cell obtained by transforming the cell A with a second recombinant plasmid, which does not express the cDNA to be tested (hereinafter referred to as "cell C");
ii) measuring the amount of production of the protein which is encoded by the foreign gene in the expression vector according to any one of claims 73 to 78 in the cell A, the cell B and the cell C described in i), and comparing the results.

82. A method for detecting a protein which regulates "an angiopoietin-related protein 3 " gene promoter activity **characterized in that** a sample containing the protein is brought into contact with a DNA according to any one of claims 65 to 69 and then binding of the protein to the DNA is detected.

83. A method for testing a substance having an activity of regulating "an angiopoietin-related protein 3 " gene promoter activity which comprises the following steps:
i) preparing a sample wherein a nuclear extract of a mouse cell and a test substance are added to a DNA according to any one of claims 65 to 69 which is radio labeled, and a sample wherein only a nuclear extract of a mouse cell is added to the DNA.
ii) subjecting each of the sample mixtures described in i) above to electrophoresis, and comparing the migration rate of the band found in the sample to which only the nuclear extract of a mouse cell is added, with that of the band found in the sample to which the test substance is also added.

84. A nucleic acid which consists of a nucleotide sequence including DNA which consists of at least 10 continuous nucleotides in the nucleotide sequence shown by nucleotide numbers 1-526 of SEQ ID No. 27 of the Sequence Listing, the antisense sequence thereof, or a derivative thereof.

85. A method for detecting a protein having an activity of regulating "an angiopoietin-related protein 4" gene promoter activity **characterized in that** a sample containing the protein is brought into contact with a DNA according any one of claims 70 to 72 and then binding of the protein to the DNA is detected.

86. A method for testing a substance having an activity of regulating "an angiopoietin-related protein 4" gene promoter activity which comprises the following steps:
i) preparing a sample wherein a nuclear extract of a mouse cell and a test substance are added to a DNA according to any one of claims 70 to 72 which is radio labeled, and a sample wherein only a nuclear extract of a mouse cell is added to the DNA.
ii) subjecting each of the sample mixtures described in i) above to electrophoresis, and comparing the migration rate of the band found in the sample to which only the nuclear extract of a mouse cell is added, with that of the band found in the sample to which the test substance is also added.

87. A nucleic acid which consists of a nucleotide sequence including DNA which consists of at least 10 continuous nucleotides in the nucleotide sequence shown by the nucleotide numbers 1-260 of SEQ ID No. 28 of the Sequence Listing, the antisense sequence thereof, or a derivative thereof.
